# EUROPEAN PATENT APPLICATION

(11) **EP 1 785 418 A1**
(43) Date of publication of application: **16.05.2007**
(21) Application number: 05768611.5
(22) Date of filing: 08.08.2005
(51) Int. Cl.: C07D 401/14, A61K 31/444, A61K 31/496, A61K 31/497, A61K 31/501, A61K 31/506, A61K 31/5377, A61P 7/02, A61P 9/10, C07D 403/14, C07D 413/14

(54) **PYRAZOLE DERIVATIVE**

(30) Priority: 06.08.2004 JP 2004230012
(71) Applicant: DAIICHI PHARMACEUTICAL CO., LTD., Chuo-ku, Tokyo 103-8234 (JP)
(72) Inventor: KANAYA, Naoaki, Daiichi Pharmaceutical Co., Ltd., Tokyo 134-8630 (JP); ISHIYAMA, Takashi, Tokyo 134-8630 (JP); MUTO, Ryo, Daiichi Pharmaceutical Co., Ltd., Tokyo 134-8630 (JP); WATANABE, Toshiyuki, Tokyo 134-8630 (JP); OCHIAI, Yuichi, Daiichi Pharmaceutical Co., Ltd., Tokyo 134-8630 (JP)
(74) Representative: Hartz, Nikolai
(86) International application number: PCT/JP2005/014544
(87) International publication number: WO 2006/014005

(57) **Abstract**

A compound represented by Formula (I): 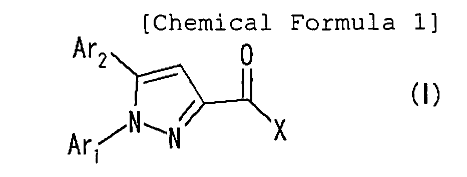 wherein Ar₁ represents Formula (II): 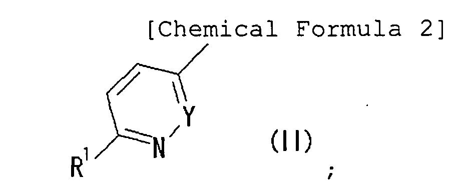 Ar₂ represents a 5- or 6-membered aromatic heterocyclic group which may be substituted; and
X represents Formula (III): 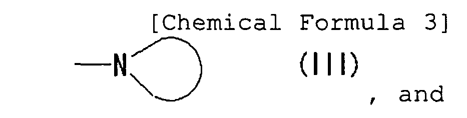 a salt thereof, or a solvate of the compound or the salt.

A potent platelet aggregation suppressant which does not inhibit COX-1 and COX-2 is provided.

## Description

### Technical Field

The present invention relates to a pyrazole derivative having an effect of suppressing platelet aggregation.

### Background Art

Platelets play an important role in preventing hemorrhage by aggregating and forming hemostatic plugs upon damages to blood vessels. On the other hand, platelets also aggregate at the sites where vascular endothelium in a blood vessel is damaged, or where a blood vessel has been narrowed, and the like, thereby inducing thrombus or embolus formation. These thrombi and emboli cause ischemic diseases such as myocardial infarction, angina pectoris, ischemic cerebrovascular disorder, peripheral vascular diseases and the like. Therefore, platelet aggregation suppressants are used for the prevention or treatment of ischemic diseases. Among such drugs, low-dose aspirin has been traditionally used as a platelet aggregation suppressant, and its effects have been demonstrated by APT (Antiplatelet Trialists' Collaboration), in which plural sets of clinical test results obtained by administering the low-dose aspirin to 100,000 patients were subjected to a meta-analysis (See Non-Patent Document 1).
However, aspirin is known to have side effects such as gastrointestinal hemorrhage and the like, namely, the so-called "aspirin-induced ulcer", and this side effect occurs at a rate of one in 100 patients, without relation to dose (See Non-Patent Document 2).

The platelet aggregation suppressing effect of aspirin is known to be based on the action of inhibiting cyclooxygenases. Cyclooxygenases include cyclooxygenase-1 (COX-1) and cyclooxygenase-2 (COX-2), and aspirin selectively and irreversibly inhibits COX-1 even at a low dose, resulting in suppression of platelet aggregation. But, the inhibition of COX-1 also causes the aspirin-induced ulcer (See Non-Patent Documents 3 and 4). Moreover, nonsteroidal antiinflammatory drugs are known to exhibit antiinflammatory action by selectively inhibiting COX-2.

As described above, although aspirin is useful as a platelet aggregation suppressant, it is associated with a side effect of gastrointestinal disorder attributable to the COX-1-inhibiting action, which is an action mechanism of the drug. Thus, there is a strong demand for a new platelet aggregation suppressant exhibiting no COX-1 inhibitory effect.

Meanwhile, as pyrazole derivatives having antithrombotic activity, compound (A) (See Patent Document 1 and Non-Patent Document 5) and compound (B) (See Patent Document 2) are known heretofore.

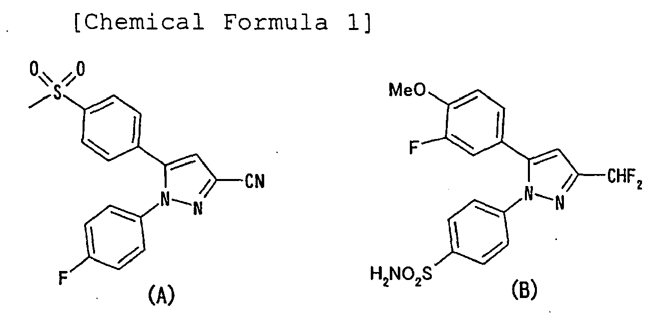

[Patent Document 1] Japanese Patent No. 2586713
[Patent Document 2] WO 97/29774
[Non-Patent Document 1] BMJ, vol.308, pp. 81-106 (1994)
[Non-Patent Document 2] BMJ, vol.321, pp. 1183-1187 (2000)
[Non-Patent Document 3]Neurology, vol.57, Suppl.2, pp. S5-S7 (2001)
[Non-Patent Document 4] Drugs Today, vol.35, pp. 251-265 (1999)
[Non-Patent Document 5]Chem. Pharm. Bull., vol.45, pp. 987-995 (1997)

### Disclosure of the Invention

### [Problems to be Solved by the Invention]

However, compound (A) has an IC₅₀ value of 5.3×10⁻⁶ M against collagen-induced platelet aggregation, and exhibits an even stronger inhibitory activity against COX-2 (IC₅₀, 2.4×10⁻⁷ M). Likewise, the platelet aggregation inhibitory action of compound (B) is not as strong as the inhibitory activity of the compound against COX-2. As described above, since inhibition of COX-2 may lead to an antiinflammatory action, a drug having a COX-2 inhibitory action is not necessarily favorable as a platelet aggregation suppressant. In this light, an object of the present invention is to provide a potent platelet aggregation suppressant which does not inhibit COX-1 and COX-2.

### [Means for solving the problems]

The inventors of the present invention have conducted a keen study in search of such a platelet aggregation suppressant, and found that a pyrazole derivative represented by the following formula (I) exhibits a potent platelet aggregation suppressing effect without inhibiting COX-1 or COX-2, thus accomplishing the invention.

Thus, the present invention provides a compound represented by Formula (I):

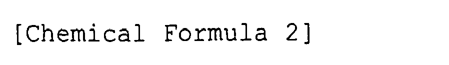

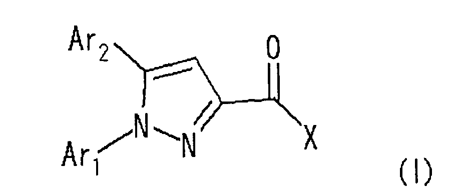

wherein Ar₁ represents a group represented by Formula (II) :

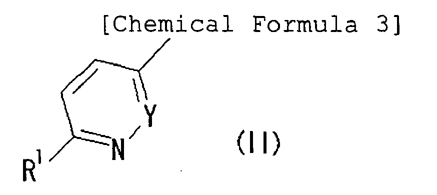

(wherein Y represents CH or a nitrogen atom; and R¹ represents a lower alkyl group or a lower alkoxy group);
Ar₂ represents a 5- or 6-membered aromatic heterocyclic group which may be substituted with 1 to 3 groups selected from a lower alkyl group which may be substituted, a lower alkynyl group, a carbamoyl group which may be substituted, a cyano group, an amino group which may be substituted, a lower alkoxy group which may be substituted, and a lower alkanoyl group; and
X represents a group represented by Formula (III):

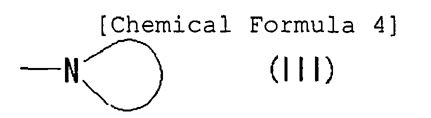

(wherein the cyclic structure represents a 4- to 7-membered alicyclic heterocyclic group which may contain a nitrogen atom or an oxygen atom as constituent atoms in addition to the nitrogen atom described in the formula, and which may be substituted with 1 to 2 groups or atoms selected from a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom), and
a salt thereof or a solvate of the compound or the salt.

The invention also provides a pharmaceutical composition containing the above-described compound, a salt thereof or a solvate of the compound or the salt; a medicine containing the above-described compound, a salt thereof or a solvate of the compound or the salt as an active ingredient; a platelet aggregation suppressant containing the above-described compound, a salt thereof or a solvate of the compound or the salt as an active ingredient; and a prophylactic and/or therapeutic agent for ischemic diseases, containing the above-described compound, a salt thereof or a solvate of the compound or the salt as an active ingredient.
The invention also provides a method for preventing and/or treating ischemic diseases by administering the above-described compound, a salt thereof or a solvate of the compound or the salt as an active ingredient.
The invention additionally provides a use of the above-described compound, a salt thereof or a solvate of the compound or the salt, for the production of medicine.

### [Effect of the Invention]

The compound (I) of the invention, a salt thereof or a solvate of the compound or the salt has an effect of inhibiting thrombus formation by strongly suppressing platelet aggregation without inhibiting COX-1 and COX-2. Thus, the compound (I), a salt thereof and a solvate of the compound or the salt are useful for the prevention and/or treatment of ischemic diseases caused by thrombi and emboli, such as myocardial infarction, angina pectoris (chronic stable angina, unstable angina, and the like), ischemic cerebrovascular disorder (transient ischemic attack (TIA), cerebral infarction, and the like), peripheral vascular disorder, occlusion after replacement with an artificial vessel, thrombotic occlusion after coronary artery intervention (coronary artery bypass grafting(CABG), percutaneous transluminal coronary angioplasty (PTCA), stent placement, and the like), diabetic retinopathy and nephropathy, occlusion upon replacement with an artificial heart valve, and the like. They are also useful for the prevention and/or treatment of thrombi and emboli associated with vascular surgery, blood extracorporeal circulation and the like. Furthermore, they are useful for an improvement in ischemic symptoms associated with chronic arterial occlusion, such as ulcer, pain, cold sensation and the like.

### Best Mode for Carrying Out the Invention

The Formula (I) will be described in the following.
First, Ar₁ will be described. Ar₁ represents a group represented by the Formula (II). In the formula, Y is CH or a nitrogen atom, while R¹ is a lower alkyl group or a lower alkoxy group.
A lower alkyl group means a straight-chained, branched or cyclic alkyl group having 1 to 6 carbon atoms. Specifically, a methyl group, an ethyl group, an n-propyl group, an isopropyl group, an n-butyl group, an isobutyl group, a tert-butyl group, an n-pentyl group, an isopentyl group, an n-hexyl group, a cyclopropyl group, a cyclobutyl group, a cyclopentyl group, a cyclohexyl group, a cyclopropylmethyl group, a cyclopentylmethyl group and the like may be mentioned. Among these, a methyl group, an ethyl group or an n-propyl group is preferred, with a methyl group being particularly preferred.

A lower alkoxy group means an alkoxy group containing the lower alkyl group in its structure. Specifically, a methoxy group, an ethoxy group, an n-propoxy group, an isopropoxy group, an n-butoxy group, an isobutoxy group, a tert-butoxy group, an n-pentoxy group, a cyclopentyloxy group and the like may be mentioned. Among these, a methoxy group or an ethoxy group is preferred, with a methoxy group being particularly preferred.

Therefore, Ar₁ may be exemplified by a 6-methyl-3-pyridyl group, a 6-ethyl-3-pyridyl group, a 6-methoxy-3-pyridyl group, a 6-ethoxy-3-pyridyl group, a 6-methyl-3-pyridazinyl group, a 6-ethyl-3-pyridazinyl group, a 6-methoxy-3-pyridazinyl group, a 6-ethoxy-3-pyridazinyl group or the like. Among these, a 6-methyl-3-pyridyl group, a 6-methoxy-3-pyridyl group, a 6-methyl-3-pyridazinyl group or a 6-methoxy-3-pyridazinyl group is preferred.

In particular, a 6-methyl-3-pyridyl group and a 6-methyl-3-pyridazinyl group are preferred in view of achieving reduction in the COX-1 inhibitory action and enhancement of oral absorbability.

Next, Ar₂ will be described. Ar₂ represents a 5- or 6-membered aromatic heterocyclic group which may be substituted with 1 to 3 groups selected from a lower alkyl group which may be substituted, a lower alkynyl group, a carbamoyl group which may be substituted, a cyano group, an amino group which may be substituted, a lower alkoxy group which may be substituted, and a lower alkanoyl group.

Here, the 5-membered aromatic heterocyclic group may be exemplified by a pyrrolyl group, a pyrazolyl group, an imidazolyl group, a triazolyl group, a furyl group, a thienyl group, an oxazolyl group, an isoxazolyl group, an oxadiazolyl group, a thiazolyl group, an isothiazolyl group, a thiadiazolyl group, or the like. Among these, a 5-membered nitrogen-containing heterocyclic group is preferred, and inter alia, a pyrrolyl group, an imidazolyl group, a pyrazolyl group, a thiazolyl group, an oxazolyl group or a triazolyl group is preferred. In addition, a 1H-pyrrol-1-yl group, a 1H-pyrrol-2-yl group, a 1H-pyrrol-3-yl group, a 1H-imidazol-2-yl group, a 1H-imidazol-4-yl group, a 1H-pyrazol-3-yl group, a 1H-pyrazol-4-yl group, a thiazol-2-yl group, a thiazol-4-yl group, a thiazol-5-yl group, an oxazol-2-yl group, an oxazol-4-yl group, or a 1H-1, 2, 4-triazolyl group is particularly preferred.

A 6-membered aromatic heterocyclic group may be exemplified by a 6-membered nitrogen-containing aromatic heterocyclic group, and examples thereof may include a pyridyl group, a pyridazinyl group, a pyrimidinyl group, a pyrazinyl group, a triazinyl group, and the like. Among these, a pyridyl group, a pyrimidinyl group and a pyrazinyl group are preferred. Moreover, among these, a 2-pyridyl group, a 3-pyridyl group, a 4-pyrimidinyl group or a 2-pyrazinyl group is preferred.

The 5- or 6-membered aromatic heterocyclic group may be substituted with (1) a lower alkyl group which may be substituted, (2) a lower alkynyl group, (3) a carbamoyl group which may be substituted, (4) a cyano group, (5) an amino group which may be substituted, (6) a lower alkoxy group which may be substituted, or (7) a lower alkanoyl group. The number of substituents is one, or two to three which may be identical or different, with one being preferred. In regard to the 6-membered aromatic heterocyclic group, the position of substitution is preferably the p-position with respect to the bonding to the pyrazole ring.

The substituents (1) to (7) for the 5- or 6-membered aromatic heterocyclic group will be described.
(1) Lower alkyl group which may be substituted: The lower alkyl group in a lower alkyl group which may be substituted means the same ones as the lower alkyl groups described above for R¹. Specifically, a methyl group, an ethyl group, an n-propyl group, an isopropyl group, an n-butyl group, an isobutyl group, a tert-butyl group, an n-pentyl group, an isopentyl group, an n-hexyl group, a cyclopropyl group, a cyclobutyl group, a cyclopentyl group, a cyclohexyl group, a cyclopropylmethyl group, a cyclopentylmethyl group and the like may be mentioned. Among these, a methyl group, an ethyl group or an n-propyl group is preferred, with a methyl group being particularly preferred.

These lower alkyl groups may be substituted with one, or two or three of substituents or atoms which may be identical or different, selected from the substituent group consisting of the following (a) to (e). These substituents or atoms may substitute for the same carbon atom in a lower alkyl group if it may be substituted, or may substitute for different carbon atoms.

(a) A carbamoyl group which may be substituted with one or two of identical or different lower alkyl groups: the carbamoyl group means an unsubstituted carbamoyl group, or a carbamoyl group substituted with one or two of the aforementioned lower alkyl groups. Specifically, a methylcarbamoyl group, an ethylcarbamoyl group, a dimethylcarbamoyl group, an N-methyl-N-ethylcarbamoyl group, and the like may be mentioned. Among these, an unsubstituted carbamoyl group, a methylcarbamoyl group or a dimethylcarbamoyl group is preferred.

(b) An amino group which may be substituted with one or two of identical or different substituents selected from a lower alkyl group, a lower alkanoyl group and a lower alkylsulfonyl group: the amino group means an unsubstituted amino group, or an amino group substituted with one or two of identical or different substituents selected from a lower alkyl group, a lower alkanoyl group and a lower alkylsulfonyl group.
The lower alkyl group means the aforementioned lower alkyl groups.
The lower alkanoyl group means a straight-chained or branched alkanoyl group having 1 to 6 carbon atoms. Specifically, a formyl group, an acetyl group, an n-propionyl group, an n-butyryl group, an isobutyryl group and the like may be mentioned.
The lower alkylsulfonyl group means a sulfonyl group substituted with the aforementioned lower alkyl group. Specifically, a methylsulfonyl group, an ethylsulfonyl group, an n-propylsulfonyl group, an isopropylsulfonyl group, an n-butylsulfonyl group, an isobutylsulfonyl group, a tert-butylsulfonyl group, an n-pentylsulfonyl group, an isopentylsulfonyl group, a cyclopropylsulfonyl group, a cyclohexylsulfonyl group and the like may be mentioned.
Therefore, the amino group which is substituted with one, or two of identical or different substituents selected from the group consisting of a lower alkyl group, a lower alkanoyl group and a lower alkylsulfonyl group, may be exemplified by a methylamino group, an ethylamino group, an n-propylamino group, an isopropylamino group, a cyclopropylamino group, an n-butylamino group, an isobutylamino group, a cyclopentylmethylamino group, a dimethylamino group, a diethylamino group, a di-n-propylamino group, a di-n-butylamino group, an N-methyl-N-ethylamino group, an N-ethyl-N-n-propylamino group, an N-methyl-N-cyclopentylmethylamino group, a formylamino group, an acetylamino group, an n-propionylamino group, an N-methyl-N-acetylamino group, an N-ethyl-N-acetylamino group, a methylsulfonylamino group, an ethylsulfonylamino group, an isopropylsulfonylamino group, an n-butylsulfonylamino group, a cyclopropylsulfonylamino group, a cyclobutanesulfonylamino group, an N-methyl-N-methylsulfonylamino group, an N-ethyl-N-methylsulfonylamino group and the like may be mentioned.

(c) A hydroxyl group

(d) A lower alkoxy group: the lower alkoxy group means the same one as the lower alkoxy group described above for R¹, that is, an alkoxy group including the aforementioned lower alkyl group in its structure. Specifically, a methoxy group, an ethoxy group, an n-propoxy group, an isopropoxy group, an n-butoxy group, an isobutoxy group, an n-pentoxy group, a cyclopentyloxy group and the like may be mentioned. Among these, a methoxy group or an ethoxy group is preferred, with a methoxy group being particularly preferred.

(e) A halogen atom: the halogen atom may include fluorine, chlorine, bromine and iodine. Among these, fluorine or chlorine is preferred, with fluorine being particularly preferred.

(2) Lower alkynyl group: the lower alkynyl group means a straight-chained, branched or cyclic alkynyl group having 2 to 6 carbon atoms. Specifically, an ethynyl group, a 1-propynyl group, a 2-propynyl group, a 1-butynyl group, a 2-butynyl group, a 1-pentynyl group, a 2-pentynyl group and the like may be mentioned. Among these, an ethynyl group, a 1-propynyl group or a 2-propynyl group is preferred, with an ethynyl group being particularly preferred.

(3) Carbamoyl group which may be substituted: the carbamoyl group which may be substituted means an unsubstituted carbamoyl group, or a carbamoyl group substituted one, or two of the aforementioned lower alkyl groups, which may be identical or different. Specifically, a methylcarbamoyl group, an ethylcarbamoyl group, an n-propylcarbamoyl group, a dimethylcarbamoyl group, a diethylcarbamoyl group, an N-methyl-N-ethylcarbamoyl group and the like may be mentioned.

(4) Cyano group

(5) Amino group which may be substituted: The amino group which may be substituted means an unsubstituted amino group, or an amino group substituted with one, or two of the aforementioned lower alkyl groups, which may be identical or different. Specifically, a methylamino group, an ethylamino group, an n-propylamino group, a dimethylamino group, a diethylamino group, an N-methyl-N-ethylamino group and the like may be mentioned. Among these, an unsubstituted amino group, a methylamino group or a dimethylamino group is preferred.

(6) Lower alkoxy group which may be substituted: the lower alkoxy group in a lower alkoxy group which may be substituted represents the same ones as the lower alkoxy groups described above for R¹, and means an alkoxy group including the aforementioned lower alkyl group in its structure. Specifically, a methoxy group, an ethoxy group, an n-propoxy group, an isopropoxy group, an n-butoxy group, an isobutoxy group, a tert-butoxy group, an n-pentoxy group, a cyclopentyloxy group and the like may be mentioned. Among these, a methoxy group or an ethoxy group is preferred, with a methoxy group being particularly preferred. Here, the lower alkyl group may be further substituted with one to three substituents or atoms selected from the substituent groups (a) to (e), as in the case of the lower alkyl group described in (1). The lower alkoxy group which may be substituted is preferably an unsubstituted lower alkoxy group, or a lower alkoxy group substituted with a carbamoyl group, more preferably a methoxy group, an ethoxy group or a carbamoylmethoxy group, and most preferably a methoxy group or a carbamoylmethoxy group.

(7) Lower alkanoyl group: the lower alkanoyl group represents the same ones as the lower alkanoyl groups mentioned in (b) above, and means an alkanoyl group including the aforementioned lower alkyl group in its structure. Among these, an acetyl group or an n-propionyl group is preferred, with an acetyl group being particularly preferred.

Among these substituents, a lower alkyl group which may be substituted, an unsubstituted carbamoyl group, a cyano group, an unsubstituted amino group, an unsubstituted lower alkoxy group, or a lower alkanoyl group is preferred.

Therefore, specific examples of the 5- or 6-membered aromatic heterocyclic group which may be substituted, Ar₂, include a pyrrolyl group, a methylpyrrolyl group, an ethylpyrrolyl group, a carbamoylpyrrolyl group, a dimethylcarbamoylpyrrolyl group, a cyanopyrrolyl group, a methoxypyrrolyl group, an aminopyrrolyl group, a methylaminopyrrolyl group, a dimethylaminopyrrolyl group, a hydroxymethylpyrrolyl group, an aminomethylpyrrolyl group, a methylaminomethylpyrrolyl group, a dimethylaminomethylpyrrolyl group, an acetylpyrrolyl group, a pyrazolyl group, a methylpyrazolyl group, a carbamoylpyrazolyl group, a dimethylcarbamoylpyrazolyl group, a cyanopyrazolyl group, a methoxypyrazolyl group, an aminopyrazolyl group, a methylaminopyrazolyl group, a dimethylaminopyrazolyl group, a hydroxymethylpyrazolyl group, an aminomethylpyrazolyl group, a methylaminomethylpyrazolyl group, a dimethylaminomethylpyrazolyl group, an acetylpyrazolyl group, an imidazolyl group, a methylimidazolyl group, a carbamoylimidazolyl group, a dimethylcarbamoylimidazolyl group, a cyanoimidazolyl group, a methoxyimidazolyl group, an aminoimidazolyl group, a methylaminoimidazolyl group, a dimethylaminoimidazolyl group, a hydroxymethylimidazolyl group, an aminomethylimidazolyl group, a methylaminomethylimidazolyl group, a dimethylaminomethylimidazolyl group, an acetylimidazolyl group, a thiazolyl group, a methylthiazolyl group, an aminothiazolyl group, a triazolyl group, a methyltriazolyl group, a carbamoyltriazolyl group, a dimethylcarbamoyltriazolyl group, a cyanotriazolyl group, a methoxytriazolyl group, an aminotriazolyl group, a methylaminotriazolyl group, a dimethylaminotriazolyl group, a hydroxymethyltriazolyl group, an aminomethyltriazolyl group, a methylaminomethyltriazolyl group, a dimethylaminomethyltriazolyl group, an acetyltriazolyl group, a pyridyl group, a methylpyridyl group, a fluoromethylpyridyl group, a carbamoylpyridyl group, a dimethylcarbamoylpyridyl group, a cyanopyridyl group, a methoxypyridyl group, an aminopyridyl group, a methylaminopyridyl group, a dimethylaminopyridyl group, a hydroxymethylpyridyl group, an aminomethylpyridyl group, a methylaminomethylpyridyl group, a dimethylaminomethylpyridyl group, an acetylpyridyl group, a pyridazinyl group, a methylpyridazinyl group, a carbamoylpyridazinyl group, a dimethylcarbamoylpyridazinyl group, a cyanopyridazinyl group, a methoxypyridazinyl group, an aminopyridazinyl group, a methylaminopyridazinyl group, a dimethylaminopyridazinyl group, a hydroxymethylpyridazinyl group, an aminomethylpyridazinyl group, a methylaminomethylpyridazinyl group, a dimethylaminomethylpyridazinyl group, an acetylpyrimidinyl group, a pyrimidinyl group, a methylpyrimidinyl group, a carbamoylpyrimidinyl group, a dimethylcarbamoylpyrimidinyl group, a cyanopyrimidinyl group, a methoxypyrimidinyl group, an aminopyrimidinyl group, a methylaminopyrimidinyl group, a dimethylaminopyrimidinyl group, a hydroxymethylpyrimidinyl group, an aminomethylpyrimidinyl group, a methylaminomethylpyrimidinyl group, a dimethylaminomethylpyrimidinyl group, an acetylpyrimidinyl group, a pyrazinyl group, a methylpyrazinyl group, a carbamoylpyrazinyl group, a dimethylcarbamoylpyrazinyl group, a cyanopyrazinyl group, a methoxypyrazinyl group, an aminopyrazinyl group, a methylaminopyrazinyl group, a dimethylaminopyrazinyl group, a hydroxymethylpyrazinyl group, an aminomethylpyrazinyl group, a methylaminomethylpyrazinyl group, a dimethylaminomethylpyrazinyl group, an acetylpyrazinyl group, an oxazolyl group, a methyloxazolyl group, a carbamoyloxazolyl group, a dimethylcarbamoyloxazolyl group, a cyanooxazolyl group, a methoxyoxazolyl group, an aminooxazolyl group, a methylaminooxazolyl group, a dimethylaminooxazolyl group, a hydroxymethyloxazolyl group, an aminomethyloxazolyl group, a methylaminomethyloxazolyl group, a dimethylaminomethyloxazolyl group, an acetyloxazolyl group, and the like.

Among these, a pyrrolyl group, a methylpyrrolyl group, a hydroxymethylpyrrolyl group, an ethylpyrrolyl group, a carbamoylpyrrolyl group, an acetylpyrrolyl group, an imidazolyl group, a methylimidazolyl group, a pyrazolyl group, a methylpyrazolyl group, a thiazolyl group, a methylthiazolyl group, an aminothiazolyl group, a methyltriazolyl group, a methoxyoxazolyl group, a methyloxazolyl group, a pyridyl group, a methylpyridyl group, a carbamoylpyridyl group, a cyanopyridyl group, an aminopyridyl group, a methoxypyridyl group, a hydroxymethylpyridyl group, an aminomethylpyridyl group, a fluoromethylpyridyl group, a pyrimidinyl group, a methylpyrimidinyl group, a pyrazinyl group, a methylpyrazinyl group, a carbamoylpyrazinyl group or an aminopyrazinyl group is preferred.

More specifically, a 1H-pyrrol-1-yl group, a 3-methyl-1H-pyrrol-1-yl group, a 3-hydroxymethyl-1H-pyrrol-1-yl group, a 3-aminomethyl-1H-pyrrol-1-yl group, a 3-methylaminomethyl-1H-pyrrol-1-yl group, a 3-dimethylaminomethyl-1H-pyrrol-1-yl group, a 3-carbamoyl-1H-pyrrol-1-yl group, a 1H-pyrrol-2-yl group, a 1-methyl-1H-pyrrol-2-yl group, a 1H-pyrrol-3-yl group, a 1-methyl-1H-pyrrol-3-yl group, a 1-ethyl-1H-pyrrol-3-yl group, a 1-aminoethyl-1H-pyrrol-3-yl group, a 1-acetyl-1H-pyrrol-3-yl group, a 1H-imidazol-2-yl group, a 1-methyl-1H-imidazol-2-yl group, a 1H-imidazol-4-yl group, a 1-methyl-1H-imidazol-4-yl group, a 1H-pyrazol-3-yl group, a 1H-pyrazol-4-yl group, a 1-methyl-1H-pyrazol-3-yl group, a 1-methyl-1H-pyrazol-4-yl group, a thiazol-2-yl group, a thiazol-4-yl group, a thiazol-5-yl group, a 2-methyloxazol-4-yl group, a 5-methoxyoxazol-2-yl group, a 1H-1, 2, 4-triazol-3-yl group, a 1-methyl-1H-1, 2, 4-triazol-3-yl group, a 2-pyridyl group, a 3-pyridyl group, a 4-pyridyl group, a 3-methoxy-2-pyridyl group, a 3-methyl-2-pyridyl group, a 4-methyl-2-pyridyl group, a 4-ethyl-2-pyridyl group, a 4-cyano-2-pyridyl group, a 4-carbamoyl-2-pyridyl group, a 4-methoxy-2-pyridyl group, a 4-ethoxy-2-pyridyl group, a 4-amino-2-pyridyl group, a 4-methylamino-2-pyridyl group, a 4-dimethylamino-2-pyridyl group, a 4-aminomethyl-2-pyridyl group, a 4-methylaminomethylpyridyl group, a 4-dimethylaminomethyl-2-pyridyl group, a 5-methyl-2-pyridyl group, a 5-ethynyl-2-pyridyl group, a 5-methoxy-2-pyridyl group, a 5-carbamoylmethoxy-2-pyridyl group, a 5-cyano-2-pyridyl group, a 5-amino-2-pyridyl group, a 5-methylamino-2-pyridyl group, a 5-dimethylamino-2-pyridyl group, a 5-carbamoyl-2-pyridyl group, a 5-methylcarbamoyl-2-pyridyl group, a 5-dimethylcarbamoyl-2-pyridyl group, a 5-hydroxymethyl-2-pyridyl group, a 5-aminomethyl-2-pyridyl group, a 5-methylaminomethyl-2-pyridyl group, a 5-dimethylaminomethyl-2-pyridyl group, a 5-fluoromethyl-2-pyridyl group, a 6-methoxy-2-pyridyl group, a 6-methyl-2-pyridyl group, a 6-methyl-3-pyridyl group, a 6-methoxy-3-pyridyl group, a 6-amino-3-pyridyl group, a 3-pyridazinyl group, a 6-methoxy-3-pyridazinyl group, a 6-methyl-3-pyridazinyl group, a 2-pyrimidinyl group, a 5-methoxy-2-pyrimidinyl group, a 5-methyl-2-pyrimidinyl group, a 4-pyrimidinyl group, a 2-pyrazinyl group, a 5-methoxy-2-pyrazinyl group, a 5-methyl-2-pyrazinyl group or a 5-amino-2-pyrazinyl group is preferred.

Among these, particularly a 1H-pyrrol-1-yl group, a 3-methyl-1H-pyrrol-1-yl group, a 3-hydroxymethyl-1H-pyrrol-1-yl group, a 3-aminomethyl-1H-pyrrol-1-yl group, a 3-methylaminomethyl-1H-pyrrol-1-yl group, a 3-carbamoyl-1H-pyrrol-1-yl group, a 3-dimethylaminomethyl-1H-pyrrol-1-yl group, a 1H-pyrrol-2-yl group, a 1-methyl-1H-pyrrol-2-yl group, a 1H-pyrrol-3-yl group, a 1-methyl-1H-pyrrol-3-yl group, a 1-ethyl-1H-pyrrol-3-yl group, a 1-aminoethyl-1H-pyrrol-3-yl group, a 1-acetyl-1H-pyrrol-3-yl group, a 1H-imidazol-2-yl group, a 1-methyl-1H-imidazol-2-yl group, a 1H-imidazol-4-yl group, a 1-methyl-1H-imidazol-4-yl group, a 1H-pyrazol-3-yl group, a 1-methyl-1H-pyrazol-3-yl group, a 1H-pyrazol-4-yl group, a 1-methyl-1H-pyrazol-4-yl group, a thiazol-2-yl group, a thiazol-4-yl group, a thiazol-5-yl group, a 1-methyl-1, 2, 4-triazol-3-yl group, a 2-pyridyl group, a 3-pyridyl group, a 4-pyridyl group, a 3-methoxy-2-pyridyl group, a 3-methyl-2-pyridyl group, a 4-methyl-2-pyridyl group, a 4-amino-2-pyridyl group, a 5-methyl-2-pyridyl group, a 5-ethynyl-2-pyridyl group, a 5-methoxy-2-pyridyl group, a 5-carbamoylmethoxy-2-pyridyl group, a 5-cyano-2-pyridyl group, a 5-carbamoyl-2-pyridyl group, a 5-cyano-2-pyridyl group, a 5-amino-2-pyridyl group, a 5-hydroxymethyl-2-pyridyl group, a 5-aminomethyl-2-pyridyl group, a 5-fluoromethyl-2-pyridyl group, a 6-methyl-2-pyridyl group, a 6-methoxy-2-pyridyl group, a 6-methoxy-3-pyridyl group, a 6-methyl-3-pyridyl group, a 6-amino-3-pyridyl group, a 6-methoxy-3-pyridazinyl group, a 6-methyl-3-pyridazinyl group, a 2-pyrimidinyl group, a 5-methoxy-2-pyrimidinyl group, a 5-methyl-2-pyrimidinyl group, a 4-pyrimidinyl group, a 2-pyrazinyl group, a 5-methoxy-2-pyrazinyl group, a 5-methyl-2-pyrazinyl group, or a 5-amino-2-pyrazinyl group is preferred.

Furthermore, among these, a 1H-pyrrol-1-yl group, a 3-hydroxymethyl-1H-pyrrol-1-yl group, a 1H-pyrrol-2-yl group, a 1H-pyrrol-3-yl group, a 1-methyl-1H-pyrrol-3-yl group, a 1-ethyl-1H-pyrrol-3-yl group, a 1-aminoethyl-1H-pyrrol-3-yl group, a 1-acetyl-1H-pyrrol-3-yl group, a 1H-imidazol-2-yl group, a 1-methyl-1H-imidazol-4-yl group, a 1H-pyrazol-3-yl group, a 1-methyl-1H-pyrazol-3-yl group, a 1-methyl-1, 2, 4-triazol-3-yl group, a thiazol-2-yl group, a thiazol-5-yl group, a 2-pyridyl group, a 4-methyl-2-pyridyl group, a 5-methyl-2-pyridyl group, a 5-aminomethyl-2-pyridyl group, a 5-hydroxymethyl-2-pyridyl group, a 5-fluoromethyl-2-pyridyl group, a 5-cyano-2-pyridyl group, a 5-carbamoyl-2-pyridyl group, a 5-methoxy-2-pyridyl group, a 4-pyrimidinyl group, a 2-pyrazinyl group, a 5-methyl-2-pyrazinyl group or a 5-amino-2-pyrazinyl group is most preferred.

Next, X will be described. X represents a group represented by Formula (III), and the Formula (III) means a 4- to 7-membered alicyclic heterocyclic group which may contain a nitrogen atom or an oxygen atom, in addition to the nitrogen atom described in the Formula (III).

The 4- to 7-membered alicyclic heterocyclic group may be exemplified by an azetidinyl group, a pyrrolidinyl group, a pyrazolidinyl group, a piperidinyl group, a piperazinyl group, a hexahydropyridazinyl group, a hexahydropyrimidinyl group, an imidazolidinyl group, a homopiperazinyl group, a morpholinyl group, an oxazepanyl group or the like. Among these, particularly an azetidinyl group, a pyrrolidinyl group, a pyrazolidinyl group, a piperidinyl group, a piperazinyl group, a hexahydropyridazinyl group, a morpholinyl group and an oxazepanyl group are preferred.

These alicyclic heterocyclic groups may be further substituted with one, or two to four groups or atoms, which may be identical or different, selected from the following substituents (i) to (ix).

(i) A lower alkyl group which may be substituted: the lower alkyl group which may be substituted means the same groups as the lower alkyl groups which may be substituted, which have been described as the substituents for Ar₂. That is, the lower alkyl group indicates a lower alkyl group which may be substituted with one to three groups or atoms selected from (a) to (e) mentioned above. Furthermore, the lower alkyl group may be substituted with an oxo group alone, or may be substituted with an oxo group in combination with the group or atom selected from (a) to (e). The lower alkyl group may be exemplified by the aforementioned lower alkyl groups, and among these, a methyl group or a cyclopropyl group is particularly preferred. Furthermore, the group or atom substituting for the lower alkyl group is preferably a halogen atom, a hydroxyl group, a lower alkoxy group or an amino group. Thus, the lower alkyl group substituting for the cyclic structure of Formula (III) is preferably a halogeno-lower alkyl group, a hydroxy-lower alkyl group, a lower alkoxy-lower alkyl group, or an amino-lower alkyl group. The halogeno-lower alkyl group means the aforementioned lower alkyl group substituted with the aforementioned halogen atom. Specifically, a fluoromethyl group, a difluoromethyl group, a trifluoromethyl group, a chloromethyl group, a dichloromethyl group, a trichloromethyl group and the like may be mentioned, and among these, a methyl group, a fluoromethyl group, a difluoromethyl group or a trifluoromethyl group is preferred, with a fluoromethyl group being particularly preferred. The hydroxyl-lower alkyl group means the aforementioned lower alkyl group substituted with a hydroxyl group, and specifically, a hydroxymethyl group, a 1-hydroxyethyl group, a 2-hydroxyethyl group, a 1-hydroxypropyl group, a 2-hydroxypropyl group, a 3-hydroxypropyl group and the like may be mentioned. The lower alkoxy-lower alkyl group means the aforementioned lower alkyl group substituted with the aforementioned lower alkoxy group, and may be exemplified by a methoxymethyl group, an ethoxymethyl group, a methoxyethyl group, an ethoxyethyl group or the like. Among these, a methoxymethyl group is preferred. The amino lower alkyl group means the aforementioned lower alkyl group substituted with an amino group, and specifically, an aminomethyl group, a 2-aminoethyl group, a 1-aminocyclopropyl group and the like may be mentioned, with a 1-aminocyclopropyl group inter alia being preferred.

(ii) A carbamoyl group which may be substituted: the carbamoyl group which may be substituted may be exemplified by the same ones as those in (3) among the substituents for Ar₂. Among these, an unsubstituted carbamoyl group, a methylcarbamoyl group or a dimethylcarbamoyl group is preferred, with an unsubstituted carbamoyl group being particularly preferred.

(iii) An amino group which may be substituted: the amino group which may be substituted may be exemplified by the same ones as the amino groups listed in (5) among the substituents for Ar₂. Among these, an unsubstituted amino group, a methylamino group, a dimethylamino group, an ethylamino group or a diethylamino group is preferred, with an unsubstituted amino group or a dimethylamino group being particularly preferred.

(iv) A hydroxyl group

(v) A lower alkoxy group: the lower alkoxy group may be exemplified by the same ones as R¹ mentioned above, and a methoxy group or an ethoxy group is preferred, with a methoxy group being particularly preferred.

(vi) An oxo group

(vii) A lower alkanoyl group: the lower alkanoyl group means, as described above, a straight-chained or branched alkanoyl group having 1 to 6 carbon atoms. Specifically, a formyl group, an acetyl group, an n-propionyl group, an n-butyryl group, an isobutyryl group, a pivaloyl group and the like may be mentioned, and among these, a formyl group is particularly preferred.

(viii) A lower alkylsulfonyl group: the lower alkylsulfonyl group may be exemplified by the same ones as those mentioned above. That is, the lower alkylsulfonyl group means a sulfonyl group substituted with the aforementioned lower alkyl group, and specifically, a methylsulfonyl group, an ethylsulfonyl group, an n-propylsulfonyl group, an isopropylsulfonyl group, an n-butylsulfonyl group, an isobutylsulfonyl group, a tert-butylsulfonyl group, an n-pentylsulfonyl group, an isopentylsulfonyl group, a cyclopropylsulfonyl group, a cyclohexylsulfonyl group and the like may be mentioned. Among these, a methylsulfonyl group, an ethylsulfonyl group or an n-propylsulfonyl group is preferred.

(ix) A halogen atom: the same ones as those mentioned above may be mentioned. Among these, fluorine or chlorine is preferred, and particularly, fluorine is preferred.

The group or atom selected from these (i) to (ix), may be used in the substitution alone, or two to four of them which are identical or different may be used in the substitution, as long as substitutions can be made. In the case of a plurality of groups being used, they may substitute for the same element or different elements of the alicyclic heterocyclic group.

For the Formula (III), specific representative examples thereof include an azetidin-1-yl group, a 3-oxoazetidin-1-yl group, a 2-oxoazetidin-1-yl group, a 3-aminoazetidin-1-yl group, a 3-methylaminoazetidin-1-yl group, a 3-dimethylaminoazetidin-1-yl group, a 2-methylazetidin-1-yl group, a 3-methylazetidin-1-yl group, a 2, 2-dimethylazetidin-1-yl group, a 3, 3-dimethylazetidin-1-yl group, a 2, 2-dimethyl-3-dimethylaminoazetidin-1-yl group, a 2-hydroxymethylazetidin-1-yl group, a 3-hydroxymethylazetidin-1-yl group, a 2-fluoromethylazetidin-1-yl group, a 3-fluoromethylazetidin-1-yl group, a 3-methoxyazetidin-1-yl group, a 2-carbamoylazetidin-1-yl group, a 2-methylcarbamoylazetidin-1-yl group, a 2-dimethylcarbamoylazetidin-1-yl group, a 3-carbamoylazetidin-1-yl group, a 3-methylcarbamoylazetidin-1-yl group, a 3-dimethylcarbamoylazetidin-1-yl group, a 3, 3-difluoroazetidin-1-yl group;

a pyrrolidino group, a 2-methylpyrrolidino group, a 2-ethylpyrrolidino group, a 2-aminomethylpyrrolidino group, a 2-methylaminomethylpyrrolidino group, a 2-dimethylaminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2, 2-dimethylpyrrolidino group, a 2, 3-dimethylpyrrolidino group, a 2, 4-dimethylpyrrolidino group, a 2, 5-dimethylpyrrolidino group, a 2-carbamoylpyrrolidino group, a 2-methylcarbamoylpyrrolidino group, a 2-dimethylcarbamoylpyrrolidino group, a 2-methoxypyrrolidino group, a 2-oxopyrrolidino group, a 2, 5-dioxopyrrolidino group, a 2-methoxymethyl-5-methylpyrrolidino group, a 2, 2-dimethyl-3-dimethylaminopyrrolidino group, a 3-methylpyrrolidino group, a 3-methoxymethylpyrrolidino group, a 3-fluoromethylpyrrolidino group, a 3-trifluoromethylpyrrolidino group, a 3-aminopyrrolidino group, a 3-methylaminopyrrolidino group, a 3-dimethylaminopyrrolidino group, a 3-oxopyrrolidino group, a 3, 3-dimethylpyrrolidino group, a 3-hydroxymethylpyrrolidino group, a 3-carbamoylpyrrolidino group, a 3-methylcarbamoylpyrrolidino group, a 3-dimethylcarbamoylpyrrolidino group, a 3-methoxypyrrolidino group, a 3-fluoropyrrolidino group, a 3, 3-difluoropyrrolidino group;

an imidazolidin-1-yl group, a 3-methylimidazolidin-1-yl group, a 2-oxoimidazolidin-1-yl group, a 4-oxoimidazolidin-1-yl group, a 3-methyl-2-oxoimidazolidin-1-yl group, a 3-methyl-4-oxoimidazolidin-1-yl group, a 2, 2-dimethylimidazolin-1-yl group;

a pyrazolidin-1-yl group, a 2-methylpyrazolidin-1-yl group, a 3-oxopyrazolidin-1-yl group, a 3, 5-dioxopyrazolidin-1-yl group, a 2-formyl-pyrazolidin-1-yl group, a 2-methylsulfonylpyrazolidin-1-yl group;

a piperidino group, a 2-oxopiperidino group, a 3-oxopiperidino group, a 4-oxopiperidino group, a 2-hydroxypiperidino group, a 3-hydroxypiperidino group, a 4-hydroxypiperidino group, a 2-methoxypiperidino group, a 3-methoxypiperidino group, a 4-methoxypiperidino group, a 3-aminopiperidino group, a 4-aminopiperidino group, a 3-methylaminopiperidino group, a 4-methylaminopiperidino group, a 3-dimethylaminopiperidino group, a 4-dimethylaminopiperidino group, a 2-methylpiperidino group, a 3-methylpiperidino group, a 4-methylpiperidino group, a 2, 2-dimethylpiperidino group, a 3, 3-dimethylpiperidino group, a 4, 4-dimethylpiperidino group, a 3-fluoropiperidino group, a 4-fluoropiperidino group, a 4-chloropiperidino group, a 3, 3-difluoropiperidino group, a 4, 4-difluoropiperidino group, a 3, 3-dichloropiperidino group, a 4, 4-dichloropiperidino group, a 4-fluoromethylpiperidino group, a 2-hydroxymethylpiperidino group, a 3-hydroxymethylpiperidino group, a 4-hydroxymethylpiperidino group, a 2-carbamoylpiperidino group, a 3-carbamoylpiperidino group, a 4-carbamoylpiperidino group, a 2-methylcarbamoylpiperidino group, a 3-methylcarbamoylpiperidino group, a 4-methylcarbamoylpiperidino group, a 2-dimethylcarbamoylpiperidino group, a 3-dimethylcarbamoylpiperidino group, a 4-dimethylcarbamoylpiperidino group, a 2-methoxymethylpiperidino group, a 3-methoxymethylpiperidino group, a 4-methoxymethylpiperidino group, a 2-aminomethylpiperidino group, a 3-aminomethylpiperidino group, a 4-aminomethylpiperidino group, a 2-methylaminomethylpiperidino group, a 3-methylaminomethylpiperidino group, a 4-methylaminomethylpiperidino group, a 2-dimethylaminomethylpiperidino group, a 3-dimethylaminomethylpiperidino group, a 4-dimethylaminomethylpiperidino group, a 2-aminoethylpiperidino group, a 3-aminoethylpiperidino group, a 4-aminoethylpiperidino group, a 2-methylaminoethylpiperidino group, a 3-methylaminoethylpiperidino group, a 4-methylaminoethylpiperidino group, a 2-dimethylaminoethylpiperidino group, a 3-dimethylaminoethylpiperidino group, a 4-dimethylaminoethylpiperidino group, a 2-aminocyclopropylpiperidino group;

a piperazino group, a 2-oxopiperazino group, a 3-oxopiperazino group, a 2-oxo-4-methylpiperazino group, a 3-oxo-4-methylpiperazino group, a 3-oxo-4-ethylpiperazino group, a 4-formylpiperazino group, a 2, 3-dioxopiperazino group, a 3, 5-dioxopiperazino group, a 2, 6-dioxopiperazino group, a 2, 3-dioxo-4-methylpiperazino group, a 3, 5-dioxo-4-methylpiperazino group, a 2, 6-dioxo-4-methylpiperazino group, a 2-methylpiperazino group, a 3-methylpiperazino group, a 4-methylpiperazino group, a 2-ethylpiperazino group, a 3-ethylpiperazino group, a 4-ethylpiperazino group, a 2-isopropylpiperazino group, a 3-isopropylpiperazino group, a 4-isopropylpiperazino group, a 2-cyclopropylpiperazino group, a 3-cyclopropylpiperazino group, a 4-cyclopropylpiperazino group, a 4-cyclobutylpiperazino group, a 2, 2-dimethylpiperazino group, a 3, 3-dimethylpiperazino group, a 2, 3-dimethylpiperazino group, a 2, 4-dimethylpiperazino group, a 3, 4-dimethylpiperazino group, a 3, 5-dimethylpiperazino group, a 2, 6-dimethylpiperazino group, a 2-ethyl-4-methylpiperazino group, a 3-ethyl-4-methylpiperazino group, a 2-isopropyl-4-methylpiperazino group, a 3-isopropyl-4-methylpiperazino group, a 2-cyclopropyl-4-methylpiperazino group, a 3-cyclopropyl-4-methylpiperazino group, a 1, 2, 6-trimethylpiperazino group, a 3, 4, 5-trimethylpiperazino group, a 2, 2, 4-trimethylpiperazino group, a 3, 3, 4-trimethylpiperazino group, a 3, 3, 4-trimethyl-5-oxopiperazino group, a 2, 2, 4-trimethyl-3-oxopiperazino group, a 4-acetylpiperazino group, a 2-hydroxymethylpiperazino group, a 3-hydroxymethylpiperazino group, a 4-methoxypiperazino group, a 2-methoxymethylpiperazino group, a 3-methoxymethylpiperazino group, a 2-hydroxyethylpiperazino group, a 3-hydroxyethylpiperazino group, a 4-hydroxyethylpiperazino group, a 2-hydroxymethyl-4-methylpiperazino group, a 3-hydroxymethyl-4-methylpiperazino group, a 2-methoxymethyl-4-methylpiperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoylpiperazino group, a 3-carbamoylpiperazino group, a 4-carbamoylpiperazino group, a 2-methylcarbamoylpiperazino group, a 3-methylcarbamoylpiperazino group, a 4-methylcarbamoylpiperazino group, a 2-dimethylcarbamoylpiperazino group, a 3-dimethylcarbamoylpiperazino group, a 4-dimethylcarbamoylpiperazino group, a 2-carbamoylmethylpiperazino group, a 3-carbamoylmethylpiperazino group, a 4-carbamoylmethylpiperazino group, a 2-methylcarbamoylmethylpiperazino group, a 3-methylcarbamoylmethylpiperazino group, a 4-methylcarbamoylpiperazino group, a 2-dimethylcarbamoylmethylpiperazino group, a 3-dimethylcarbamoylmethylpiperazino group, a 2-carbamoyl-4-methylpiperazino group, a 3-carbamoyl-4-methylpiperazino group, a 4-carbamoylpiperazino group, a 2-methylcarbamoyl-4-methylpiperazino group, a 3-methylcarbamoyl-4-methylpiperazino group, a 4-methylcarbamoylpiperazino group, a 2-dimethylcarbamoyl-4-methylpiperazino group, a 3-dimethylcarbamoyl-4-methylpiperazino group, a 4-dimethylcarbamoylpiperazino group, a 2-carbamoylmethyl-4-methylpiperazino group, a 3-carbamoylmethyl-4-methylpiperazino group, a 4-carbamoylmethylpiperazino group, a 2-methylcarbamoylmethyl-4-methylpiperazino group, a 3-methylcarbamoylmethyl-4-methylpiperazino group, a 4-methylcarbamoylpiperazino group, a 2-dimethylcarbamoylmethyl-4-methylpiperazino group, a 3-dimethylcarbamoylmethyl-4-methylpiperazino group, a 2-aminomethylpiperazino group, a 3-aminomethylpiperazino group, a 2-methylaminomethylpiperazino group, a 3-methylaminomethylpiperazino group, a 2-dimethylaminomethylpiperazino group, a 3-dimethylaminomethylpiperazino group, a 2-aminoethylpiperazino group, a 3-aminoethylpiperazino group, 4-aminoethylpiperazino group, a 2-methylaminoethylpiperazino group, a 3-methylaminoethylpiperazino group, a 4-methylaminoethylpiperazino group, a 2-dimethylaminoethylpiperazino group, a 3-dimethylaminoethylpiperazino group, a 4-dimethylaminoethylpiperazino group, a 2-aminomethyl-4-methylpiperazino group, a 3-aminomethyl-4-methylpiperazino group, a 2-methylaminomethyl-4-methylpiperazino group, a 3-methylaminomethyl-4-methylpiperazino group, a 2-dimethylaminomethyl-4-methylpiperazino group, a 3-dimethylaminomethyl-4-methylpiperazino group, a 2-aminoethyl-4-methylpiperazino group, a 3-aminoethyl-4-methylpiperazino group, a 2-methylaminoethyl-4-methylpiperazino group, a 3-methylaminoethyl-4-methylpiperazino group, a 2-dimethylaminoethyl-4-methylpiperazino group, a 3-dimethylaminoethyl-4-methylpiperazino group, a 4-methylsulfonylpiperazino group;

a morpholino group, a 2-methylmorpholino group, a 3-methylmorpholino group, a 2-ethylmorpholino group, a 3-ethylmorpholino group, a 2, 2-dimethylmorpholino group, a 3, 3-dimethylmorpholino group, a 3-methylmorpholin-4-yl group, a 2-hydroxymethylmorpholino group, a 3-hydroxymethylmorpholino group, a 2-methoxymethylmorpholino group, a 3-methoxymethylmorpholino group, a 2-hydroxyethylmorpholino group, a 3-hydroxyethylmorpholino group, a 2-methoxyethylmorpholino group, a 3-methoxyethylmorpholino group, a 2-carbamoylmorpholino group, a 3-carbamoylmorpholino group, a 2-methylcarbamoylmorpholino group, a 3-methylcarbamoylmorpholino group, a 2-dimethylcarbamoylmorpholino group, a 3-dimethylcarbamoylmorpholino group, a 2-carbamoylmethylmorpholino group, a 3-carbamoylmethylmorpholino group, a 2-methylcarbamoylmethylmorpholino group, a 3-methylcarbamoylmethylmorpholino group, a 2-dimethylcarbamoylmethylmorpholino group, a 3-dimethylcarbamoylmethylmorpholino group, a 2-carbamoylethylmorpholino group, a 3-carbamoylethylmorpholino group, a 2-methylcarbamoylethylmorpholino group, a 3-methylcarbamoylethylmorpholino group, a 2-dimethylcarbamoylethylmorpholino group, a 3-dimethylcarbamoylethylmorpholino group, a 2-aminomethylmorpholino group, a 3-aminomethylmorpholino group, a 2-methylaminomethylmorpholino group, a 3-methylaminomethylmorpholino group, a 2-dimethylaminomethylmorpholino group, a 3-dimethylaminomethylmorpholino group, a 2-aminoethylmorpholino group, a 3-aminoethylmorpholino group, a 2-methylaminoethylmorpholino group, a 3-methylaminoethylmorpholino group, a 2-dimethylaminoethylmorpholino group, a 3-dimethylaminoethylmorpholino group;

a hexahydropyridazin-1-yl group, a 2-formylhexahydropyridazin-1-yl group, a 2-acetylhexahydropyridazin-1-yl group, a 3-oxohexahydropyridazin-1-yl group, a 6-oxohexahydropyridazin-1-yl group, a 4-aminohexahydropyridazin-1-yl group, a 4-methylaminohexahydropyridazin-1-yl group, a 4-dimethylaminohexahydropyridazin-1-yl group, a 2-methylhexahydropyridazin-1-yl group, a 3-methylhexahydropyridazin-1-yl group, a 4-methylhexahydropyridazin-1-yl group, a 2, 3-dimethylhexahydropyridazin-1-yl group, a 3, 3-dimethylhexahydropyridazin-1-yl group, a 4, 4-dimethylhexahydropyridazin-1-yl group, a 3-hydroxymethylhexahydropyridazin-1-yl group, a 4-hydroxymethylhexahydropyridazin-1-yl group, a 5-hydroxymethylhexahydropyridazin-1-yl group, a 6-hydroxymethylhexahydropyridazin-1-yl group, a 2-carbamoylhexahydropyridazin-1-yl group, a 3-carbamoylhexahydropyridazin-1-yl group, a 4-carbamoylhexahydropyridazin-1-yl group, a 5-carbamoylhexahydropyridazin-1-yl group, a 6-carbamoylhexahydropyridazin-1-yl group, a 2-methylcarbamoylhexahydropyridazin-1-yl group, a 3-methylcarbamoylhexahydropyridazin-1-yl group, a 4-methylcarbamoylhexahydropyridazin-1-yl group, a 5-methylcarbamoylhexahydropyridazin-1-yl group, a 6-methylcarbamoylhexahydropyridazin-1-yl group, a 2-dimethylcarbamoylhexahydropyridazin-1-yl group, a 3-dimethylcarbamoylhexahydropyridazin-1-yl group, a 4-dimethylcarbamoylhexahydropyridazin-1-yl group, a 5-dimethylcarbamoylhexahydropyridazin-1-yl group, a 6-dimethylcarbamoylhexahydropyridazin-1-yl group, a 3-methoxymethylhexahydropyridazin-1-yl group, a 4-methoxymethylhexahydropyridazin-1-yl group, a 5-methoxymethylhexahydropyridazin-1-yl group, a 6-methoxymethylhexahydropyridazin-1-yl group, a 2-aminoethylhexahydropyridazin-1-yl group, a 3-aminoethylhexahydropyridazin-1-yl group, a 4-aminoethylhexahydropyridazin-1-yl group, a 5-aminoethylhexahydropyridazin-1-yl group, a 6-aminoethylhexahydropyridazin-1-yl group, a 2-methylaminoethylhexahydropyridazin-1-yl group, a 3-methylaminoethylhexahydropyridazin-1-yl group, a 4-methylaminoethylhexahydropyridazin-1-yl group, a 5-methylaminoethylhexahydropyridazin-1-yl group, a 6-methylaminoethylhexahydropyridazin-1-yl group, a 3-aminomethylhexahydropyridazin-1-yl group, a 4-aminomethylhexahydropyridazin-1-yl group, a 5-aminomethylhexahydropyridazin-1-yl group, a 6-aminomethylhexahydropyridazin-1-yl group, a 3-methylaminomethylhexahydropyridazin-1-yl group, a 4-methylaminomethylhexahydropyridazin-1-yl group, a 5-methylaminomethylhexahydropyridazin-1-yl group, a 6-methylaminomethylhexahydropyridazin-1-yl group, a 3-dimethylaminomethylhexahydropyridazin-1-yl group, a 4-dimethylaminomethylhexahydropyridazin-1-yl group, a 5-dimethylaminomethylhexahydropyridazin-1-yl group, a 6-dimethylaminomethylhexahydropyridazin-1-yl group, a 2-dimethylaminoethylhexahydropyridazin-1-yl group, a 3-dimethylaminoethylhexahydropyridazin-1-yl group, a 4-dimethylaminoethylhexahydropyridazin-1-yl group, a 5-dimethylaminoethylhexahydropyridazin-1-yl group, a 6-dimethylaminoethylhexahydropyridazin-1-yl group;

a hexahydropyrimidin-1-yl group, a 2-oxohexahydropyrimidin-1-yl group, a 4-oxohexahydropyrimidin-1-yl group, a 5-oxohexahydropyrimidin-1-yl group, a 6-oxohexahydropyrimidin-1-yl group, a 2-methylhexahydropyrimidin-1-yl group, a 3-methylhexahydropyrimidin-1-yl group, a 4-methylhexahydropyrimidin-1-yi group, a 4-methylhexahydropyrimidin-1-yl group, a 2, 2-dimethylhexahydropyrimidin-1-yl group, a 4, 4-dimethylhexahydropyrimidin-1-yl group, a 5, 5-dimethylhexahydropyrimidin-1-yl group, a 6, 6-dimethylhexahydropyrimidin-1-yl group, a 2-hydroxymethylhexahydropyrimidin-1-yl group, a 4-hydroxymethylhexahydropyrimidin-1-yl group, a 5-hydroxymethylhexahydropyrimidin-1-yl group, a 6-hydroxymethylhexahydropyrimidin-1-yl group, a 2-carbamoylhexahydropyrimidin 1-yl group, a 3-carbamoylhexahydropyrimidin 1-yl group, a 4-carbamoylhexahydropyrimidin 1-yl group, a 5-carbamoylhexahydropyrimidin 1-yl group, a 6-carbamoylhexahydropyrimidin 1-yl group, a 2-methylcarbamoylhexahydropyrimidin 1-yl group, a 3-methylcarbamoylhexahydropyrimidin 1-yl group, a 4-methylcarbamoylhexahydropyrimidin 1-yl group, a 5-methylcarbamoylhexahydropyrimidin 1-yl group, a 6-methylcarbamoylhexahydropyrimidin 1-yl group, a 2-dimethylcarbamoylhexahydropyrimidin 1-yl group, a 3-dimethylcarbamoylhexahydropyrimidin 1-yl group, a 4-dimethylcarbamoylhexahydropyrimidin 1-yl group, a 5-dimethylcarbamoylhexahydropyrimidin 1-yl group, a 6-dimethylcarbamoylhexahydropyrimidin 1-yl group, a 3-methoxymethylhexahydropyrimidin-1-yl group, a 4-methoxymethylhexahydropyrimidin-1-yl group, a 5-methoxymethylhexahydropyrimidin-1-yl group, a 6-methoxymethylhexahydropyrimidin-1-yl group, a 2-aminoethylhexahydropyrimidin-1-yl group, a 3-aminoethylhexahydropyrimidin-1-yl group, a 4-aminoethylhexahydropyrimidin-1-yl group, a 5-aminoethylhexahydropyrimidin-1-yl group, a 6-aminoethylhexahydropyrimidin-1-yl group, a 2-methylaminoethylhexahydropyrimidin-1-yl group, a 3-methylaminoethylhexahydropyrimidin-1-yl group, a 4-methylaminoethylhexahydropyrimidin-1-yl group, a 5-methylaminoethylhexahydropyrimidin-1-yl group, a 6-methylaminoethylhexahydropyrimidin-1-yl group, a 2-dimethylaminoethylhexahydropyrimidin-1-yl group, a 3-dimethylaminoethylhexahydropyrimidin-1-yl group, a 4-dimethylaminoethylhexahydropyrimidin-1-yl group, a 5-dimethylaminoethylhexahydropyrimidin-1-yl group, a 6-dimethylaminoethylhexahydropyrimidin-1-yl group;

a homopiperazino group, a 2-oxohomopiperazino group, a 3-oxohomopiperazino group, a 5-oxohomopiperazino group, a 6-oxohomopiperazino group, a 7-oxohomopiperazino group, a 2-oxo-4-methylhomopiperazino group, a 3-oxo-4-methylhomopiperazino group, a 5-oxo-4-methylhomopiperazino group, a 6-oxo-4-methylhomopiperazino group, a 7-oxo-4-methylhomopiperazino group, a 2, 3-dioxohomopiperazino group, a 2, 7-dioxohomopiperazino group, a 3, 5-dioxohomopiperazino group, a 3, 7-dioxohomopiperazino group, a 2, 3-dioxo-4-methylhomopiperazino group, a 2, 7-dioxo-4-methylhomopiperazino group, a 3, 5-dioxo-4-methylhomopiperazino group, a 3, 7-dioxo-4-methylhomopiperazino group, a 2-methylhomopiperazino group, a 3-methylhomopiperazino group, a 4-methylhomopiperazino group, a 5-methylhomopiperazino group, a 6-methylhomopiperazino group, a 7-methylhomopiperazino group, a 2-ethylhomopiperazino group, a 3-ethylhomopiperazino group, a 4-ethylhomopiperazino group, a 5-ethylhomopiperazino group, a 6-ethylhomopiperazino group, a 7-ethylhomopiperazino group, a 4-cyclopropylhomopiperazino group, a 2, 2-dimethylhomopiperazino group, a 3, 3-dimethylhomopiperazino group, a 5, 5-dimethylhomopiperazino group, a 6, 6-dimethylhomopiperazino group, a 7, 7-dimethylhomopiperazino group, a 2, 3-dimethylhomopiperazino group, a 2, 4-dimethylhomopiperazino group, a 3, 4-dimethylhomopiperazino group, a 3, 5-dimethylhomopiperazino group, a 3, 4, 5-trimethylhomopiperazino group, a 2-hydroxymethylhomopiperazino group, a 3-hydroxymethylhomopiperazino group, a 5-hydroxymethylhomopiperazino group, a 6-hydroxymethylhomopiperazino group, a 7-hydroxymethylhomopiperazino group, a 2-hydroxymethyl-4-methylhomopiperazino group, a 3-hydroxymethyl-4-methylhomopiperazino group, a 5-hydroxymethyl-4-methylhomopiperazino group, a 6-hydroxymethyl-4-methylhomopiperazino group, a 7-hydroxymethyl-4-methylhomopiperazino group, a 2-methoxymethylhomopiperazino group, a 3-methoxymethylhomopiperazino group, a 5-methoxymethylhomopiperazino group, a 6-methoxymethylhomopiperazino group, a 7-methoxymethylhomopiperazino group, a 2-methoxymethyl-4-methylhomopiperazino group, a 3-methoxymethyl-4-methylhomopiperazino group, a 5-methoxymethyl-4-methylhomopiperazino group, a 6-methoxymethyl-4-methylhomopiperazino group, a 7-methoxymethyl-4-methylhomopiperazino group, a 2-hydroxyethylhomopiperazino group, a 3-hydroxyethylhomopiperazino group, a 4-hydroxyethylhomopiperazino group, a 5-hydroxyethylhomopiperazino group, a 6-hydroxyethylhomopiperazino group, a 7-hydroxyethylhomopiperazino group, a 2-hydroxyethyl-4-methylhomopiperazino group, a 3-hydroxyethyl-4-methylhomopiperazino group, a 5-hydroxyethyl-4-methylhomopiperazino group, a 6-hydroxyethyl-4-methylhomopiperazino group, a 7-hydroxyethyl-4-methylhomopiperazino group, a 2-methoxyethylhomopiperazino group, a 3-methoxyethylhomopiperazino group, a 4-methoxyethylhomopiperazino group, a 5-methoxyethylhomopiperazino group, a 6-methoxyethylhomopiperazino group, a 7-methoxyethylhomopiperazino group, a 2-methoxyethyl-4-methylhomopiperazino group, a 3-methoxyethyl-4-methylhomopiperazino group, a 5-methoxyethyl-4-methylhomopiperazino group, a 6-methoxyethyl-4-methylhomopiperazino group, a 7-methoxyethyl-4-methylhomopiperazino group, a 2-carbamoylhomopiperazino group, a 3-carbamoylhomopiperazino group, a 4-carbamoylhomopiperazino group, a 5-carbamoylhomopiperazino group, a 6-carbamoylhomopiperazino group, a 7-carbamoylhomopiperazino group, a 2-carbamoyl-4-methylhomopiperazino group, a 3-carbamoyl-4-methylhomopiperazino group, a 4-carbamoylhomopiperazino group, a 5-carbamoyl-4-methylhomopiperazino group, a 6-carbamoyl-4-methylhomopiperazino group, a 7-carbamoyl-4-methylhomopiperazino group, a 2-methylcarbamoylhomopiperazino group, a 3-methylcarbamoylhomopiperazino group, a 4-methylcarbamoylhomopiperazino group, a 5-methylcarbamoylhomopiperazino group, a 6-methylcarbamoylhomopiperazino group, a 7-methylcarbamoylhomopiperazino group, a 2-methylcarbamoyl-4-methylhomopiperazino group, a 3-methylcarbamoyl-4-methylhomopiperazino group, a 5-methylcarbamoyl-4-methylhomopiperazino group, a 6-methylcarbamoyl-4-methylhomopiperazino group, a 7-methylcarbamoyl-4-methylhomopiperazino group, a 2-dimethylcarbamoylhomopiperazino group, a 3-dimethylcarbamoylhomopiperazino group, a 4-dimethylcarbamoylhomopiperazino group, a 5-dimethylcarbamoylhomopiperazino group, a 6-dimethylcarbamoylhomopiperazino group, a 7-dimethylcarbamoylhomopiperazino group, a 2-dimethylcarbamoyl-4-methylhomopiperazino group, a 3-dimethylcarbamoyl-4-methylhomopiperazino group, a 5-dimethylcarbamoyl-4-methylhomopiperazino group, a 6-dimethylcarbamoyl-4-methylhomopiperazino group, a 7-dimethylcarbamoyl-4-methylhomopiperazino group, a 2-carbamoylmethylhomopiperazino group, a 3-carbamoylmethylhomopiperazino group, a 4-carbamoylmethylhomopiperazino group, a 5-carbamoylmethylhomopiperazino group, a 6-carbamoylmethylhomopiperazino group, a 7-carbamoylmethylhomopiperazino group, a 2-carbamoylmethyl-4-methylhomopiperazino group, a 3-carbamoylmethyl-4-methylhomopiperazino group, a 5-carbamoylmethyl-4-methylhomopiperazino group, a 6-carbamoylmethyl-4-methylhomopiperazino group, a 7-carbamoylmethyl-4-methylhomopiperazino group, a 2-methylcarbamoylmethylhomopiperazino group, a 3-methylcarbamoylmethylhomopiperazino group, a 4-methylcarbamoylhomopiperazino group, a 5-methylcarbamoylhomopiperazino group, a 6-methylcarbamoylhomopiperazino group, a 7-methylcarbamoylhomopiperazino group, a 2-methylcarbamoylmethyl-4-methylhomopiperazino group, a 3-methylcarbamoylmethyl-4-methylhomopiperazino group, a 5-methylcarbamoyl-4-methylhomopiperazino group, a 6-methylcarbamoyl-4-methylhomopiperazino group, a 7-methylcarbamoyl-4-methylhomopiperazino group, a 2-dimethylcarbamoylmethylhomopiperazino group, a 3-dimethylcarbamoylmethylhomopiperazino group, a 4-dimethylcarbamoylmethylhomopiperazino group, a 5-dimethylcarbamoylmethylhomopiperazino group, a 6-dimethylcarbamoylmethylhomopiperazino group, a 7-dimethylcarbamoylmethylhomopiperazino group, a 2-dimethylcarbamoylmethyl-4-methylhomopiperazino group, a 3-dimethylcarbamoylmethyl-4-methylhomopiperazino group, a 5-dimethylcarbamoylmethyl-4-methylhomopiperazino group, a 6-dimethylcarbamoylmethyl-4-methylhomopiperazino group, a 7-dimethylcarbamoylmethyl-4-methylhomopiperazino group, a 2-aminomethylhomopiperazino group, a 3-aminomethylhomopiperazino group, a 5-aminomethylhomopiperazino group, a 6-aminomethylhomopiperazino group, a 7-aminomethylhomopiperazino group, a 2-aminomethyl-4-methylhomopiperazino group, a 3-aminomethyl-4-methylhomopiperazino group, a 5-aminomethyl-4-methylhomopiperazino group, a 6-aminomethyl-4-methylhomopiperazino group, a 7-aminomethyl-4-methylhomopiperazino group, a 2-methylaminomethylhomopiperazino group, a 3-methylaminomethylhomopiperazino group, a 4-methylaminomethylhomopiperazino group, a 5-methylaminomethylhomopiperazino group, a 6-methylaminomethylhomopiperazino group, a 7-methylaminomethylhomopiperazino group, a 2-methylaminomethyl-4-methylhomopiperazino group, a 3-methylaminomethyl-4-methylhomopiperazino group, a 5-methylaminomethyl-4-methylhomopiperazino group, a 6-methylaminomethyl-4-methylhomopiperazino group, a 7-methylaminomethyl-4-methylhomopiperazino group, a 2-dimethylaminomethylhomopiperazino group, a 3-dimethylaminomethylhomopiperazino group, a 4-dimethylaminomethylhomopiperazino group, a 5-dimethylaminomethylhomopiperazino group, a 6-dimethylaminomethylhomopiperazino group, a 7-dimethylaminomethylhomopiperazino group, a 2-dimethylaminomethyl-4-methylhomopiperazino group, a 3-dimethylaminomethyl-4-methylhomopiperazino group, a 5-dimethylaminomethyl-4-methylhomopiperazino group, a 6-dimethylaminomethyl-4-methylhomopiperazino group, a 7-dimethylaminomethyl-4-methylhomopiperazino group, a 2-aminoethylhomopiperazino group, a 3-aminoethylhomopiperazino group, a 4-aminoethylhomopiperazino group, a 5-aminoethylhomopiperazino group, a 6-aminoethylhomopiperazino group, a 7-aminoethylhomopiperazino group, a 2-aminoethyl-4-methylhomopiperazino group, a 3-aminoethyl-4-methylhomopiperazino group, a 5-aminoethyl-4-methylhomopiperazino group, a 6-aminoethyl-4-methylhomopiperazino group, a 7-aminoethyl-4-methylhomopiperazino group, a 2-methylaminoethylhomopiperazino group, a 3-methylaminoethylhomopiperazino group, a 4-methylaminoethylhomopiperazino group, a 5-methylaminoethylhomopiperazino group, a 6-methylaminoethylhomopiperazino group, a 7-methylaminoethylhomopiperazino group, a 2-methylaminoethyl-4-methylhomopiperazino group, a 3-methylaminoethyl-4-methylhomopiperazino group, a 5-methylaminoethyl-4-methylhomopiperazino group, a 6-methylaminoethyl-4-methylhomopiperazino group, a 7-methylaminoethyl-4-methylhomopiperazino group, a 2-dimethylaminoethylhomopiperazino group, a 3-dimethylaminoethylhomopiperazino group, a 4-dimethylaminoethylhomopiperazino group, a 5-dimethylaminoethylhomopiperazino group, a 6-dimethylaminoethylhomopiperazino group, a 7-dimethylaminoethylhomopiperazino group, a 2-dimethylaminoethyl-4-methylhomopiperazino group, a 3-dimethylaminoethyl-4-methylhomopiperazino group, a 5-dimethylaminoethyl-4-methylhomopiperazino group, a 6-dimethylaminoethyl-4-methylhomopiperazino group, a 7-dimethylaminoethyl-4-methylhomopiperazino group, a 4-methanesulfonylhomopiperazino group, a 4-methanesulfonylaminohomopiperazino group, a 4-(azetidin-1-yl)homopiperazino group, a 4-pyrrolidinohomopiperazino group, a 4-piperidinohomopiperazino group;

a 1, 4-oxazepan-4-yl group, and the like.

Among these, the following are preferred.
An azetidin-1-yl group, a 3-dimethylaminoazetidin-1-yl group, a 2-methylazetidin-1-yl group, a 3-methylazetidin-1-yl group, a 2, 2-dimethylazetidin-1-yl group, a 3, 3-dimethylazetidin-1-yl group, a 2, 2-dimethyl-3-dimethylaminoazetidin-1-yl group, a 2-hydroxymethylazetidin-1-yl group, a 3-hydroxymethylazetidin-1-yl group, a 2-carbamoylazetidin-1-yl group, a 2-methylcarbamoylazetidin-1-yl group, a 2-dimethylcarbamoylazetidin-1-yl group, a 3, 3-difluoroazetidin-1-yl group;

a pyrrolidino group, a 2-methylpyrrolidino group, a 2-aminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2, 2-dimethylpyrrolidino group, a 2, 5-dimethylpyrrolidino group, a 2-carbamoylpyrrolidino group, a 2-methoxypyrrolidino group, a 2-oxopyrrolidino group, a 2-methoxymethyl-5-methylpyrrolidino group, a 3-methylpyrrolidino group, a 3-methoxymethylpyrrolidino group, a 3-fluoromethylpyrrolidino group, a 3-trifluoromethylpyrrolidino group, a 3-aminopyrrolidino group, a 3, 3-dimethylpyrrolidino group, a 3-hydroxymethylpyrrolidino group, a 3-carbamoylpyrrolidino group, a 3-methoxypyrrolidino group, a 3-fluoropyrrolidino group, a 3, 3-difluoropyrrolidino group;

an imidazolidin-1-yl group, a 3-methylimidazolidin-1-yl group, a 2-oxoimidazolidin-1-yl group, a 4-oxoimidazolidin-1-yl group, a 3-methyl-2-oxoimidazolidin-1-yl group, a 3-methyl-4-oxoimidazolidin-1-yl group, a 2, 2-dimethylimidazolin-1-yl group;

a pyrazolidin-1-yl group, a 2-methylpyrazolidin-1-yl group, a 3-oxopyrazolidin-1-yl group, a 2-formyl-pyrazolidin-1-yl group, a 2-methylsulfonylpyrazolidin-1-yl group;

a piperidino group, a 2-oxopiperidino group, a 3-oxopiperidino group, a 4-oxopiperidino group, a 2-hydroxymethylpiperidino group, a 3-hydroxymethylpiperidino group, a 4-hydroxymethylpiperidino group, a 2-methoxypiperidino group, a 3-methoxypiperidino group, a 4-methoxypiperidino group, a 2-methylpiperidino group, a 3-methylpiperidino group, a 4-methylpiperidino group, a 2, 2-dimethylpiperidino group, a 3, 3-dimethylpiperidino group, a 4, 4-dimethylpiperidino group, a 3-fluoropiperidino group, a 4-fluoropiperidino group, a 4-chloropiperidino group, a 3, 3-difluoropiperidino group, a 4, 4-difluoropiperidino group, a 2-fluoromethylpiperidino group, a 3-fluoromethylpiperidino group, a 4-fluoromethylpiperidino group, a 3, 3-dichloropiperidino group, a 4, 4-dichloropiperidino group, a 2-hydroxymethylpiperidino group, a 2-carbamoylpiperidino group, a 2-methylcarbamoylpiperidino group, a 2-dimethylcarbamoylpiperidino group, a 2-methoxymethylpiperidino group, a 4-methyl-4-methoxypiperidino group, a 2-aminomethylpiperidino group, a 2-methylaminomethylpiperidino group, a 2-dimethylaminomethylpiperidino group, a 2-aminoethylpiperidino group, a 2-methylaminoethylpiperidino group, a 2-dimethylaminoethylpiperidino group, a 2-aminocyclopropylpiperidino group;

a piperazino group, a 2-oxo-4-methylpiperazino group, a 3-oxo-4-methylpiperazino group, a 3-oxo-4-ethylpiperazino group, a 4-formylpiperazino group, a 2, 3-dioxo-4-methylpiperazino group, a 3, 5-dioxo-4-methylpiperazino group, a 2, 6-dioxo-4-methylpiperazino group, a 4-methylpiperazino group, a 4-ethylpiperazino group, a 4-isopropylpiperazino group, a 4-cyclopropylpiperazino group, a 2, 4-dimethylpiperazino group, a 3, 4-dimethylpiperazino group, a 2-ethyl-4-methylpiperazino group, a 3-ethyl-4-methylpiperazino group, a 2-isopropyl-4-methylpiperazino group, a 3-isopropyl-4-methylpiperazino group, a 2-cyclopropyl-4-methylpiperazino group, a 3-cyclopropyl-4-methylpiperazino group, a 3, 4, 5-trimethylpiperazino group, a 2, 2, 4-trimethylpiperazino group, a 3, 3, 4-trimethylpiperazino group, a 3, 3, 4-trimethyl-5-oxopiperazino group, a 2, 2, 4-trimethyl-3-oxopiperazino group, a 2-hydroxymethyl-4-methylpiperazino group, a 3-hydroxymethyl-4-methylpiperazino group, a 2-methoxymethyl-4-methylpiperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoyl-4-methylpiperazino group, a 3-carbamoyl-4-methylpiperazino group, a 4-carbamoylpiperazino group, a 2-methylcarbamoyl-4-methylpiperazino group, a 3-methylcarbamoyl-4-methylpiperazino group, a 4-methylcarbamoylpiperazino group, a 2-dimethylcarbamoyl-4-methylpiperazino group, a 3-dimethylcarbamoyl-4-methylpiperazino group, a 4-dimethylcarbamoylpiperazino group, a 2-carbamoylmethyl-4-methylpiperazino group, a 3-carbamoylmethyl-4-methylpiperazino group, a 4-carbamoylmethylpiperazino group, a 2-methylcarbamoylmethyl-4-methylpiperazino group, a 3-methylcarbamoylmethyl-4-methylpiperazino group, a 4-methylcarbamoylpiperazino group, a 2-dimethylcarbamoylmethyl-4-methylpiperazino group, a 3-dimethylcarbamoylmethyl-4-methylpiperazino group, a 2-aminomethyl-4-methylpiperazino group, a 2-methylaminomethyl-4-methylpiperazino group, a 2-dimethylaminomethyl-4-methylpiperazino group, a 2-aminoethyl-4-methylpiperazino group, a 2-methylaminoethyl-4-methylpiperazino group, a 2-dimethylaminoethyl-4-methylpiperazino group;

a morpholino group, a 2-methylmorpholino group, a 3-methylmorpholino group, a 2-ethylmorpholino group, a 3-ethylmorpholino group, a 2, 2-dimethylmorpholino group, a 3, 3-dimethylmorpholino group, a 3-methylmorpholin-4-yl group, a 3-hydroxymethylmorpholino group, a 3-methoxymethylmorpholino group, a 3-hydroxyethylmorpholino group, a 3-methoxyethylmorpholino group, a 3-carbamoylmorpholino group, a 3-methylcarbamoylmorpholino group, a 3-dimethylcarbamoylmorpholino group, a 3-carbamoylmethylmorpholino group, a 3-methylcarbamoylmethylmorpholino group, a 3-dimethylcarbamoylmethylmorpholino group, a 3-carbamoylethylmorpholino group, a 3-methylcarbamoylethylmorpholino group, a 3-dimethylcarbamoylethylmorpholino group, a 3-aminomethylmorpholino group, a 3-methylaminomethylmorpholino group, a 3-dimethylaminomethylmorpholino group, a 3-aminoethylmorpholino group, a 3-methylaminoethylmorpholino group, a 3-dimethylaminoethylmorpholino group;

a 2-acetylhexahydropyridazin-1-yl group, a 2-formylhexahydropyridazin-1-yl group, a 2-methylhexahydropyridazin-1-yl group, a 3-oxohexahydropyridazin-1-yl group, a 6-oxohexahydropyridazin-1-yl group, a 2, 3-dimethylhexahydropyridazin-1-yl group, a 3-hydroxymethylhexahydropyridazin-1-yl group, a 5-hydroxymethylhexahydropyridazin-1-yl group, a 6-hydroxymethylhexahydropyridazin-1-yl group, a 2-carbamoylhexahydropyridazin-1-yl group, a 2-methylcarbamoylhexahydropyridazin-1-yl group, a 2-dimethylcarbamoylhexahydropyridazin-1-yl group;

a 2-oxohexahydropyrimidin-1-yl group, a 4-oxohexahydropyrimidin-1-yl group, a 6-oxohexahydropyrimidin-1-yl group, a 2-methylhexahydropyrimidin-1-yl group, a 3-methylhexahydropyrimidin-1-yl group, a 3-carbamoylhexahydropyrimidin-1-yl group, a 3-methylcarbamoylhexahydropyrimidin-1-yl group, a 3-dimethylcarbamoylhexahydropyrimidin-1-yl group, a 6-hydroxymethylpyrimidin-1-yl group;

a 2-oxo-4-methylhomopiperazino group, a 3-oxo-4-methylhomopiperazino group, a 5-oxo-4-methylhomopiperazino group, a 6-oxo-4-methylhomopiperazino group, a 7-oxo-4-methylhomopiperazino group, a 2, 3-dioxohomopiperazino group, a 2, 7-dioxohomopiperazino group, a 3, 5-dioxohomopiperazino group, a 3, 7-dioxohomopiperazino group, a 2, 3-dioxo-4-methylhomopiperazino group, a 2, 7-dioxo-4-methylhomopiperazino group, a 3, 5-dioxo-4-methylhomopiperazino group, a 3, 7-dioxo-4-methylhomopiperazino group, a 4-methylhomopiperazino group, a 4-ethylhomopiperazino group, a 4-cyclopropylhomopiperazino group, a 2, 4-dimethylhomopiperazino group, a 3, 4-dimethylhomopiperazino group, a 3, 4, 5-trimethylhomopiperazino group, a 2-hydroxymethyl-4-methylhomopiperazino group, a 7-hydroxymethyl-4-methylhomopiperazino group, a 2-methoxymethyl-4-methylhomopiperazino group, a 3-methoxymethyl-4-methylhomopiperazino group, a 5-methoxymethyl-4-methylhomopiperazino group, a 6-methoxymethyl-4-methylhomopiperazino group, a 7-methoxymethyl-4-methylhomopiperazino group, a 2-hydroxyethyl 4-methylhomopiperazino group, a 7-hydroxyethyl-4-methylhomopiperazino group, a 2-methoxyethyl-4-methylhomopiperazino group, a 3-methoxyethyl-4-methylhomopiperazino group, a 5-methoxyethyl-4-methylhomopiperazino group, a 6-methoxyethyl-4-methylhomopiperazino group, a 7-methoxyethyl-4-methylhomopiperazino group, a 2-carbamoyl-4-methylhomopiperazino group, a 7-carbamoyl-4-methylhomopiperazino group, a 2-methylcarbamoyl-4-methylhomopiperazino group, a 7-methylcarbamoyl-4-methylhomopiperazino group, a 2-dimethylcarbamoylhomopiperazino group, a 7-dimethylcarbamoylhomopiperazino group; a 1, 4-oxazepan-4-yl group; and a 3-methyl-4-oxoimidazolidin-1-yl group.

Among these, the following are more preferred.
An azetidin-1-yl group, a 3-dimethylaminoazetidin-1-yl group, a 2, 2-dimethyl-3-dimethylaminoazetidin-1-yl group, a 2-hydroxymethylazetidin-1-yl group, a 2-carbamoylazetidin-1-yl group, a 2-methylcarbamoylazetidin-1-yl group, a 2-dimethylcarbamoylazetidin-1-yl group; a pyrrolidino group, a 2-methylpyrrolidino group, a 2-aminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2, 2-dimethylpyrrolidino group, a 2, 5-dimethylpyrrolidino group, a 2-carbamoylpyrrolidino group, a 2-methoxypyrrolidino group, a 2-oxopyrrolidino group, a 2-methoxymethyl-5-methylpyrrolidino group, a 3-methylpyrrolidino group, a 3-methoxymethylpyrrolidino group, a 3-fluoromethylpyrrolidino group, a 3-trifluoromethylpyrrolidino group, a 3-aminopyrrolidino group, a 3-hydroxymethylpyrrolidino group, a 3-carbamoylpyrrolidino group, a 3-methoxypyrrolidino group, a 3-fluoropyrrolidino group, a 3, 3-difluoropyrrolidino group;

an imidazolidin-1-yl group, a 3-methylimidazolidin-1-yl group, a 2-oxoimidazolidin-1-yl group, a 4-oxoimidazolidin-1-yl group, a 3-methyl-2-oxoimidazolidin-1-yl group, a 3-methyl-4-oxoimidazolidin-1-yl group, a 2, 2-dimethylimidazolin-1-yl group; a pyrazolidin-1-yl group, a 2-methylpyrazolidin-1-yl group, a 2-formyl-pyrazolidin-1-yl group, a 2-methylsulfonylpyrazolidin-1-yl group; a piperidino group, a 2-oxopiperidino group, a 2-methoxypiperidino group, a 3-methoxypiperidino group, a 4-methoxypiperidino group, a 2-hydroxymethylpiperidino group, a 2-carbamoylpiperidino group, a 2-methylcarbamoylpiperidino group, a 2-dimethylcarbamoylpiperidino group, a 2-methoxymethylpiperidino group, a 2-aminomethylpiperidino group, a 2-methylaminomethylpiperidino group, a 2-dimethylaminomethylpiperidino group, a 2-aminoethylpiperidino group, a 2-methylaminoethylpiperidino group, a 2-dimethylaminoethylpiperidino group, a 3-fluoropiperidino group, a 4-fluoropiperidino group, 4-methylpiperidino group, a 4-methoxypiperidino group, a 3, 3-difluoropiperidino group, a 4, 4-difluoropiperidino group, a 3-fluoromethylpiperidino group, a 4-fluoromethylpiperidino group, a 4-methyl-4-methoxypiperidino group, a 2-aminocyclopropylpiperidino group; a piperazino group, a 2-oxo-4-methylpiperazino group, a 3-oxo-4-methylpiperazino group, a 3-oxo-4-ethylpiperazino group, a 4-formylpiperazino group, a 2, 3-dioxo-4-methylpiperazino group, a 3, 5-dioxo-4-methylpiperazino group, a 2, 6-dioxo-4-methylpiperazino group, a 4-methylpiperazino group, a 4-ethylpiperazino group, a 4-isopropylpiperazino group, a 4-cyclopropylpiperazino group, a 2, 4-dimethylpiperazino group, a 3, 4-dimethylpiperazino group, a 2-ethyl-4-methylpiperazino group, a 3-ethyl-4-methylpiperazino group, a 3, 4, 5-trimethylpiperazino group, a 2, 2, 4-trimethylpiperazino group, a 3, 3, 4-trimethylpiperazino group, a 3, 3, 4-trimethyl-5-oxopiperazino group, a 2, 2, 4-trimethyl-3-oxopiperazino group, a 2-hydroxymethyl-4-methylpiperazino group, a 3-hydroxymethyl-4-methylpiperazino group, a 2-methoxymethyl-4-methyl-piperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoyl-4-methylpiperazino group, a 2-methylcarbamoyl-4-methylpiperazino group, a 2-dimethylcarbamoyl-4-methylpiperazino group, a 2-carbamoylmethyl-4-methylpiperazino group, a 2-methylcarbamoylmethyl-4-methylpiperazino group, a 2-dimethylcarbamoylmethyl-4-methylpiperazino group, a 2-aminomethyl-4-methylpiperazino group, a 2-methylaminomethyl-4-methylpiperazino group, a 2-dimethylaminomethyl-4-methylpiperazino group, a 2-aminoethyl-4-methylpiperazino group, a 2-methylaminoethyl-4-methylpiperazino group, a 2-dimethylaminoethyl-4-methylpiperazino group;

a morpholino group, a 2, 2-dimethylmorpholino group, a 3, 3-dimethylmorpholino group, a 3-methylmorpholin-4-yl group, a 3-hydroxymethylmorpholino group, a 3-methoxymethylmorpholino group, a 3-hydroxyethylmorpholino group, a 3-methoxyethylmorpholino group, a 3-carbamoylmorpholino group, a 3-methylcarbamoylmorpholino group, a 3-dimethylcarbamoylmorpholino group, a 3-aminomethylmorpholino group, a 3-methylaminomethylmorpholino group, a 3-dimethylaminomethylmorpholino group, a 3-aminoethylmorpholino group, a 3-methylaminoethylmorpholino group, a 3-dimethylaminoethylmorpholino group; a 2-acetylhexahydropyridazin-1-yl group, a 2-formylhexahydropyridazin-1-yl group, a 3-oxohexahydropyridazin-1-yl group, a 2-methylhexahydropyridazin-1-yl group, a 2-carbamoylhexahydropyridazin-1-yl group; a 2-oxohexahydropyrimidin-1-yl group, a 4-oxohexahydropyrimidin-1-yl group, a 3-methylhexahydropyrimidin-1-yl group, a 6-hydroxymethylhexahydropyrimidin-1-yl group; a 2-oxo-4-methylhomopiperazino group, a 3-oxo-4-methylhomopiperazino group, a 5-oxo-4-methylhomopiperazino group, a 7-oxo-4-methylhomopiperazino group, a 2, 3-dioxo-4-methylhomopiperazino group, a 2, 7-dioxo-4-methylhomopiperazino group, a 3, 5-dioxo-4-methylhomopiperazino group, a 3, 7-dioxo-4-methylhomopiperazino group, a 4-methylhomopiperazino group, a 4-ethylhomopiperazino group, a 4-cyclopropylhomopiperazino group; a 1, 4-oxazepan-4-yl group; and a 3-methyl-4-oxoimidazolidin-1-yl group.

Among these, the following are particularly preferred.
A 3-dimethylaminoazetidin-1-yl group, a 2, 2-dimethyl-3-dimethylaminoazetidin-1-yl group, a 2-hydroxymethylazetidin-1-yl group, a 2-carbamoylazetidin-1-yl group; a pyrrolidino group, a 2-methylpyrrolidino group, a 2-fluoromethylpyrrolidino group, a 2-trifluoromethylpyrrolidino group, a 2-hydroxymethylpyrrolidino group, a 2, 5-dimethylpyrrolidino group, a 2-carbamoylpyrrolidino group, a 3-fluoropyrrolidino group, a 3, 3-difluoropyrrolidino group; a pyrazolidino group, a 2-methyl-pyrazolidin-1-yl group, a 2-formyl-pyrazolidin-1-yl group, a 2-methylsulfonylpyrazolidin-1-yl group; a piperidino group, a 2-hydroxymethylpiperidino group, a 2-carbamoylpiperidino group, a 2-methylcarbamoylpiperidino group, a 2-dimethylcarbamoylpiperidino group, a 3-methoxypiperidino group, a 3-fluoropiperidino group, a 4-fluoropiperidino group, a 4-methylpiperidino group, a 4-methoxypiperidino group, a 3, 3-difluoropiperidino group, a 4, 4-difluoropiperidino group, a 4-fluoromethylpiperidino group, a 4-methyl-4-methoxypiperidino group, a 2-aminocyclopropylpiperidino group; a 3-oxo-4-methylpiperazino group, a 3-oxo-4-ethylpiperazino group, a 4-methylpiperazino group, a 4-ethylpiperazino group, a 4-isopropylpiperazino group, a 4-cyclopropylpiperazino group, a 2, 4-dimethylpiperazino group, a 3, 4-dimethylpiperazino group, a 3, 4, 5-trimethylpiperazino group, a 2, 2, 4-trimethylpiperazino group, a 3, 3, 4-trimethylpiperazino group; a morpholino group, a 3-methylmorpholin-4-yl group, a 3-carbamoylmorpholino group; a thiomorpholino group, a 1, 1-dioxothiomorpholino group; a 2-methylhexahydropyridazin-1-yl group, a 3-methylhexahydropyridazin-1-yl group; a 3-oxo-4-methylhomopiperazino group, a 5-oxo-4-methylhomopiperazino group, a 4-methylhomopiperazino group, a 4-ethylhomopiperazino group, a 4-cyclopropylhomopiperazino group; a 1, 4-oxazepan-4-yl group; a 3-methyl-4-oxoimidazolidin-1-yl group; a 2-acetylhexahydropyridazin-1-yl group, and a 2-carbamoylhexahydropyridazin-1-yl group.

Among these, the following are most preferred.
A 2-methylpyrrolidino group, a 2, 5-dimethylpyrrolidino group, a 2-fluoromethylpyrrolidino group, a 2-trifluoromethylpyrrolidino group, a 2-hydroxymethylpyrrolidino group, a 2-methoxymethyl-5-methylpyrrolidino group, a 2-carbamoylpyrrolidino group, a 3-fluoropyrrolidino group, a 3, 3-difluoropyrrolidino group; a 2-methyl-pyrazolidin-1-yl group, a 2-formyl-pyrazolidino group, a 2-methylsulfonylpyrazolidin-1-yl group; a piperidino group, a 3-methoxypiperidin-1-yl group, a 3-fluoropiperidino group, a 4-methylpiperidino group, a 4-methoxypiperidino group, a 4-fluoropiperidino group, a 4, 4-difluoropiperidino group, a 2-aminocyclopropylpiperidino group; a 3-oxo-4-methylpiperazino group, a 3-oxo-4-ethylpiperazino group, a 4-methylpiperazino group; a hexahydropyridazin-1-yl group; a morpholino group, a 3-methylmorpholin-4-yl group; and a 1, 4-oxazepan-4-yl group.

For the salt of compound (I) of the present invention, it cannot be said that all of the compounds of the invention form salts, but those containing a carboxyl group, an amino group or the like, or those in which Ar₁ or Ar₂ is a pyridine ring, form salts. Moreover, in some cases, these salts may form solvates. The salt as used herein may be exemplified by a salt of an inorganic acid such as hydrochloric acid, hydrobromic acid, sulfuric acid, nitric acid or the like; a salt of an organic acid such as methanesulfonic acid, p-toluenesulfonic acid, fumaric acid, trifluoroacetic acid or the like; and a salt with the ion of an alkali metal or an alkaline earth metal such as sodium, potassium, calcium or the like.

For a solvate of compound (I) of the invention or a salt thereof, the solvate includes those formed by absorbing the moisture from the air as well as those to which the solvent used in crystallization etc. has been added. Examples of the solvent include, for example, water, lower alcohols such as methanol, ethanol and the like, organic solvents such as acetone, acetonitrile and the like.

Among the compound (I) of the invention, more preferred one which potently suppressing platelet aggregation without inhibiting COX-1 and COX-2, include 4-[1-(6-methoxy-3-pyridyl)-5-(1-methyl-1H-pyrazol-4-yl)-1H-pyrazole-3-carbonyl]morpholine, 1-[1-(6-methoxy-3-pyridyl)-5-(1-methyl-1H-imidazol-4-yl)-1H-pyrazole-3-carbonyl]-4-methoxypiperidine, 1-[1-(6-methyl-3-pyridyl)-5-(1-methyl-1H-pyrrol-3-yl)-1H-pyrazole-3-carbonyl]-4-methyl-3-oxopiperazine, 1-[1-(6-methoxy-3-pyridazinyl)-5-(1-methyl-1H-pyrrol-3-yl)-1H-pyrazole-3-carbonyl]-4-methyl-3-oxopiperazine, 1-[1-(6-methoxy-3-pyridyl)-5-(thiazol-4-yl)-1H-pyrazole-3-carbonyl]-4-methyl-3-oxopiperazine, 1-[1-(6-methoxy-3-pyridyl)-5-(thiazol-4-yl)-1H-pyrazole-3-carbonyl]-4-methylpiperazine, 1-[1-(6-methoxy-3-pyridyl)-5-(thiazol-4-yl)-1H-pyrazole-3-carbonyl]-4-methoxypiperidine, 1-[1-(6-methoxy-3-pyridyl)-5-(thiazol-4-yl)-1H-pyrazole-3-carbonyl]-2-carbamoylpiperidine, 1-[1-(6-methoxy-3-pyridazinyl)-5-(thiazol-4-yl)-1H-pyrazole-3-carbonyl]-4-methylpiperazine, 1-[1-(6-methoxy-3-pyridazinyl)-5-(thiazol-4-yl)-1H-pyrazole-3-carbonyl]-4-methoxypiperidine, 1-[1-(6-methoxy-3-pyridazinyl)-5-(thiazol-4-yl)-1H-pyrazole-3-carbonyl]-4, 4-difluoropiperidine, 1-[1-(6-methoxy-3-pyridyl)-5-(1-methyl-1H-pyrazol-4-yl)-1H-pyrazole-3-carbonyl]hexahydropyridazine, 1-[1-(6-methoxy-3-pyridazinyl)-5-(thiazol-4-yl)-1H-pyrazole-3-carbonyl]hexahydropyridazine, 4-[1-(6-methoxy-3-pyridyl)-5-(1-methyl-1H-pyrazol-4-yl)-1H-pyrazole-3-carbonyl]-1, 4-oxazepane, 1-[1-(6-methoxy-3-pyridyl)-5-(thiazol-4-yl)-1H-pyrazole-3-carbonyl]hexahydropyridazine, 4-[1-(6-methoxy-3-pyridyl)-5-(thiazol-4-yl)-1H-pyrazole-3-carbonyl]-1, 4-oxazepane, 1-[1-(6-methoxy-3-pyridazinyl)-5-(1-methyl-1H-pyrazol-4-yl)-1H-pyrazole-3-carbonyl]-4, 4-difluoropiperidine, salts thereof and solvates of the compounds and the salts. Among these, preferred is 4-[1-(6-methoxy-3-pyridyl)-5-(1-methyl-1H-pyrazol-4-yl)-1H-pyrazole-3-carbonyl]morpholine or 1-[1-(6-methoxy-3-pyridyl)-5-(1-methyl-1H-imidazol-4-yl)-1H-pyrazole-3-carbonyl]-4-methoxypiperidine, a salt thereof or a solvate of the compound or the salt.
Further, among these, 4-[1-(6-methoxy-3-pyridyl)-5-(1-methyl-1H-pyrazol-4-yl)-1H-pyrazole-3-carbonyl]morpholine, a salt or solvate thereof is preferred because they exhibit a potent effect of suppressing platelet aggregation without inhibiting COX-1 and COX-2, and also are excellent as a pharmaceutical product in view of primary efficacy, safety, oral absorbability and solubility.

The compound (I) of the invention can be prepared by a method as follows. Hereinafter, representative methods for preparation of the compound (I) of the invention will be described.

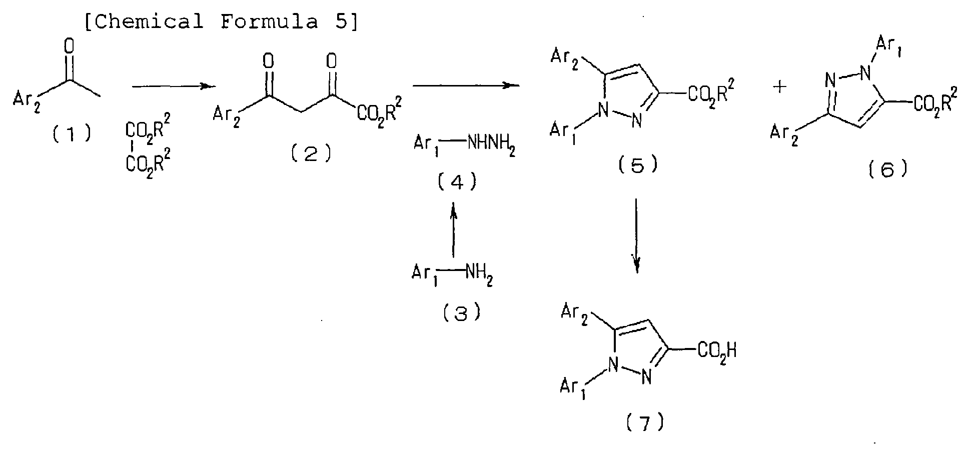

wherein Ar₁ and Ar₂ represent the same ones as described above; and R² represents a lower alkyl group such as a methyl group, an ethyl group or the like.

An aromatic ketone (1) and an oxalic acid dialkyl ester can be treated in a solution of alcohol (methanol or ethanol) in the presence of sodium alkoxide (sodium methoxide or sodium ethoxide), to obtain compound (2). The reaction temperature is preferably -10 to 100°C.

Compound (2) can also be prepared by dissolving or suspending compound (1) and an oxalic acid dialkyl ester in an appropriate solvent such as N, N-dimethylformamide or the like, and reacting the solution or suspension with sodium hydride at -20 to 20°C under an argon stream.

Furthermore, compound (2) can also be prepared by dissolving compound (1) in an inert solvent such as tetrahydrofuran or the like, and treating the resultant with a base such as lithium bis(trimethylsilyl)amide or the like under cooling, and reacting the resultant with an oxalic acid dialkyl ester. The reaction temperature is preferably -78 to 20°C, and particularly preferably -78°C.

In addition, for compound (1), commercially available products may be used, or those prepared by a method described in Reference Examples or a method equivalent thereto may be used.

In a case where compound (1) has a functional group such as a hydroxyl group, an amino group or the like, it is desirable to protect such functional groups in advance using an appropriate protective group. As the protective group for a hydroxyl group, a tert-butyl group, a benzyl group and the like may be mentioned, while as the protective group for an amino group, a trifluoroacetyl group, a tert-butoxycarbonyl group, a benzyloxycarbonyl group, a benzenesulfonyl group, a p-toluenesulfonyl group and the like may be mentioned. These protective groups can be detached under the conditions suitable for the respective protective groups.

Next, compound (5) can be prepared by dissolving the compound (2) in an alcohol (methanol or ethanol), adding thereto a hydrazine derivative (4) or a salt thereof at room temperature, then adding an appropriate amount of acetic acid, and heating the mixture to reflux. Here, although a regioisomer (6) is produced as a side product, the desired compound (5) can be easily separated and purified by silica gel column chromatography or the like.

The hydrazine derivative (4) or a salt thereof can be prepared by dissolving an aromatic amine (3) in concentrated hydrochloric acid, adding sodium nitrite under ice-cooling to derive a diazo product, and then treating the resultant with tin(II) chloride. The reaction temperature is preferably -10 to 20°C.

For the hydrazine derivative (4), commercially available ones, or those prepared by a method of reacting halogenated Ar₁ with hydrazine as described in the Reference Examples or a method equivalent thereto, may be used. Also for the aromatic amine (3), commercially available compounds may be used, or the compound may be prepared by a method described in the Reference Examples or a method equivalent thereto.

With regard to the reaction for forming the aforementioned pyrazole ring, the heating and refluxing may be performed after adding an appropriate amount of triethylamine, concentrated hydrochloric acid, p-toluenesulfonic acid or the like instead of acetic acid, and in some cases, the compound (5) can be obtained even without adding acetic acid, triethylamine, concentrated hydrochloric acid, p-toluenesulfonic acid or the like.

Furthermore, various pyrazole derivatives (5) can be prepared on the basis of common knowledge in organic chemistry. For example, 5-(1H-pyrrol-1-yl)-1H-pyrazol derivertive (5a), in which Ar₂ of the pyrazole derivative (5) is a pyrrolyl group, can be prepared as shown in the following.

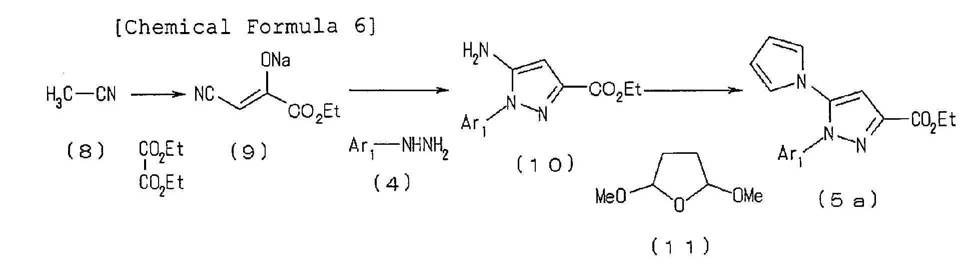

wherein Ar₁ represents the same ones as described above.

5-Aminopyrazole product (10) can be prepared by adding a 1 M hydrochloric acid-ethanol solution to a solution of compound (9) and the hydrazine derivative (4) in ethanol, and heating to reflux the mixture. The compound (9) can be prepared by suspending sodium ethoxide and oxalic acid diethyl ester in an appropriate solvent such as tert-butyl methyl ether or the like, adding acetonitrile (8), and heating the mixture to reflux. The compound (5a) can be prepared by dissolving the compound (10) in a solvent such as acetic acid or the like, adding 2, 5-dimethoxytetrahydrofuran, and heating the mixture to reflux.

The aforementioned compound (5) can be derived to carboxylic acid (7) on the basis of common knowledge in organic chemistry, by hydrolyzing the ester using a base, a Lewis acid or the like. As the base, hydroxides of alkali metals (for example, lithium, sodium, potassium, and the like) may be mentioned. Further, as the Lewis acid, for example, boron tribromide may be mentioned. The reaction temperature is preferably -20 to 100°C, and particularly preferably -5 to 50°C.

Furthermore, the compound (5) can be converted to various derivatives by being subjected to further modifications on the basis of common knowledge in organic chemistry. For example, from compound (5b), respective derivatives of alcohol, triflate and nitrile (5c to 5e) can be prepared.

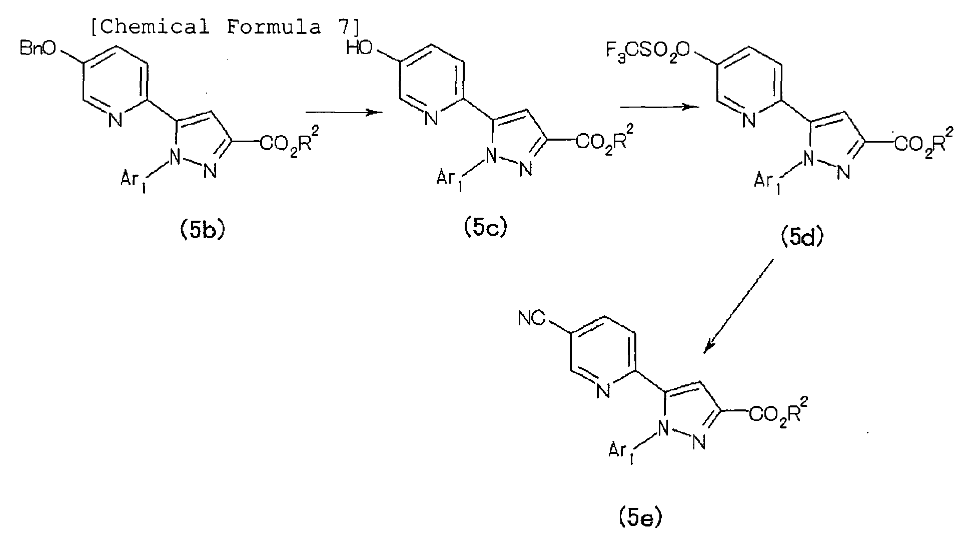

wherein Ar₁ represents the same ones as described above; Bn represents a benzyl group; and R² represents a lower alkyl group such as a methyl group, an ethyl group or the like.

Specifically, a hydroxy product (5c) can be prepared by dissolving a benzyloxy product (5b) in ethanol or the like, and catalytically reducing the benzyloxy product using 10% palladium-carbon as the catalyst. When the hydroxy product (5c) is dissolved in methylene chloride or the like and reacted with anhydrous trifluoromethanesulfonic acid at -50 to 50°C in the presence of a base such as pyridine or the like, a triflate (5d) can be prepared. Moreover, when the triflate (5d) is dissolved in 1, 2-dichloroethane or the like, and reacted with tri-n-butyltin cyanide and tetrakis(triphenylphosphine)palladium(0), a cyano derivative (5e) can be prepared. The reaction temperature is preferably 10 to 100°C. The aforementioned reaction conditions, reagents and the like may be appropriately selected on the basis of common knowledge in organic chemistry.

Furthermore, as shown in the following, a carboxylic acid derivative (5g) or an amine derivative (5h) can be prepared from compound (5f).

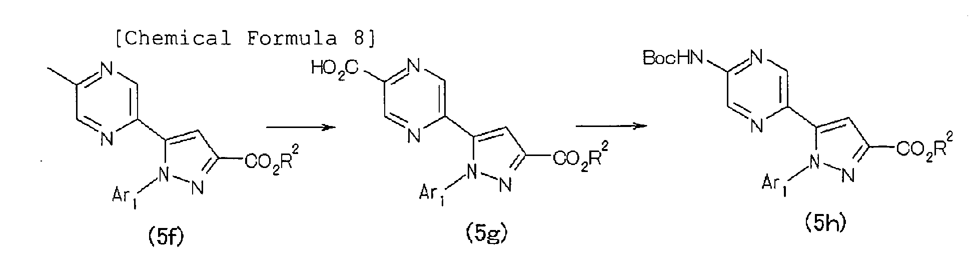

wherein Ar₁ and R² represent the same ones as described above; and Boc represents a tert-butoxycarbonyl group.

Specifically, the carboxylic acid derivative (5g) can be prepared by dissolving a methylpyrazine derivative (5f) in pyridine or the like, adding selenium dioxide at room temperature, and then heating the mixture to reflux. The amine derivative (5h) can be prepared by dissolving the carboxylic acid derivative (5g) in 1, 4-dioxane or the like, adding tert-butanol, triethylamine and diphenylphosphorylazide at room temperature, and then heating the mixture to reflux. The aforementioned reaction conditions, reagents and the like may be appropriately selecting on the basis of common knowledge in organic chemistry.

A pyrazole derivative (5j) in which Ar₂ is a 3-hydroxymethylpyrrolyl group, can be prepared as follows.

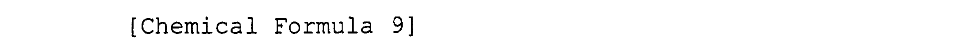

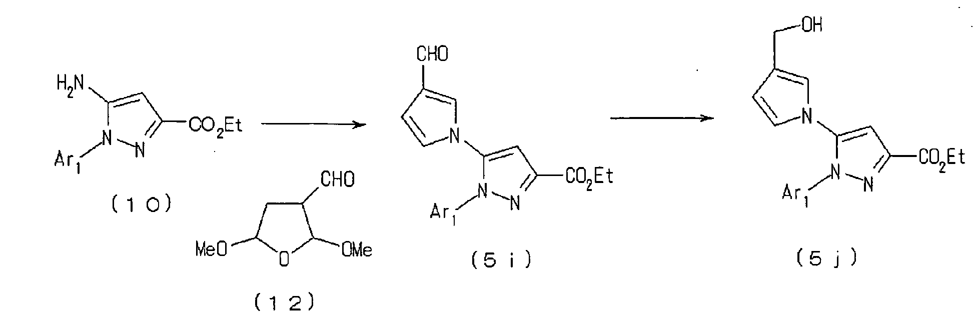

wherein Ar₁ represents the same ones as described above.

The hydroxymethyl derivative (5j) can be prepared by dissolving the 5-aminopyrazole product (10) in a solvent such as acetic acid or the like, adding 2, 5-dimethoxytetrahydro-3-furan carboaldehyde (12), and heating the mixture to reflux, thus to prepare a 3-formylpyrrolyl derivative (5i), and reducing the product with sodium borohydride. The hydroxymethyl group of the hydroxymethyl derivative (5j) thus obtained can be easily converted to a cyanomethyl group, an aminomethyl group or the like.

The aforementioned pyrazole derivative (5) can be derived to a carboxylic acid derivative (7) by hydrolyzing the ester as described above.

By condensation of the carboxylic acid derivative (7) and an amine (13), the compound (I) of the invention can be obtained.

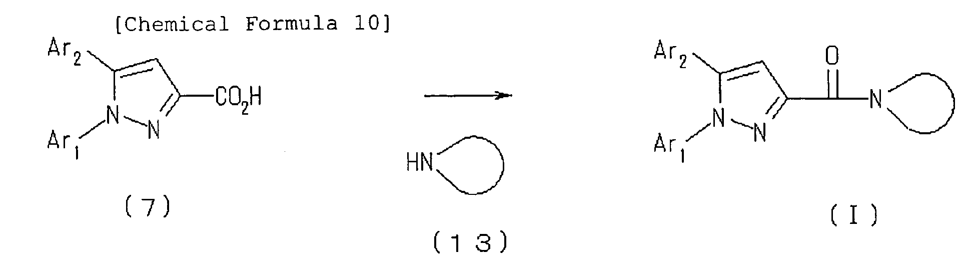

wherein Ar₁ and Ar₂ represent the same ones as described above.

For the condensation reaction described above, a method generally used as a method for peptide synthesis may be applied. As the method for peptide synthesis, for example, an azide method, an acid chloride method, an acid anhydride method, a DCC (dicyclohexylcarbodiimide) method, an active ester method, a carbonyldiimidazole method, a DCC/HOBT (1-hydroxybenzotriazole) method, a method of using a water-soluble carbodiimide, a method of using diethylcyanophosphate, and the like may be mentioned, and these methods are described in M. Bodanszky, Y.S. Klausner and M.A. Ondetti, "Peptide Synthesis" (A Wiley-Interscience publication, New York, 1976); G.R. Pettit, "Synthetic Peptides" (Elsevier Scientific Publication Company, New York, 1976); The Chemical Society of Japan, "Lectures on Experimental Chemistry, 4th ed., Vol. 22, Organic Synthesis IV" (Maruzen Corp., 1992); and the like. The solvent used in this condensation reaction may be exemplified by N, N-dimethylformamide, pyridine, chloroform, methylene chloride, tetrahydrofuran, dioxane, acetonitrile or the like, or a mixed solvent thereof. The reaction temperature is preferably -20 to 50°C, and more preferably -10 to 30°C. For the amine (13), commercially available compounds may be used, or those prepared by a method described in literature, a method described in the Reference Examples, or a method equivalent thereto, may be used.

Furthermore, for the condensation reaction described above, in the case where the amine product (13) has a functional group such as a hydroxyl group, an amino group, a carboxyl group or the like, it is desirable to protect such functional group in advance using an appropriate protective group. It is preferable to provide the amine product to the condensation reaction after deriving, in the case of a hydroxyl group, to a tert-butyl group, a benzyl group or the like; in the case of an amino group, to a trifluoroacetyl group, a tert-butoxycarbonyl group, a benzyloxycarbonyl group or the like; and in the case of a carboxyl group, to a methyl ester, a tert-butyl ester or the like.

The compound (I) of the invention prepared by the above-described methods can be converted to a derivative of the compound (I) by further modifications based on common knowledge in organic chemistry. For example, from compound (Ia), respective derivatives of alcohol, triflate, nitrile and amide (Ib to Ie) can be obtained.

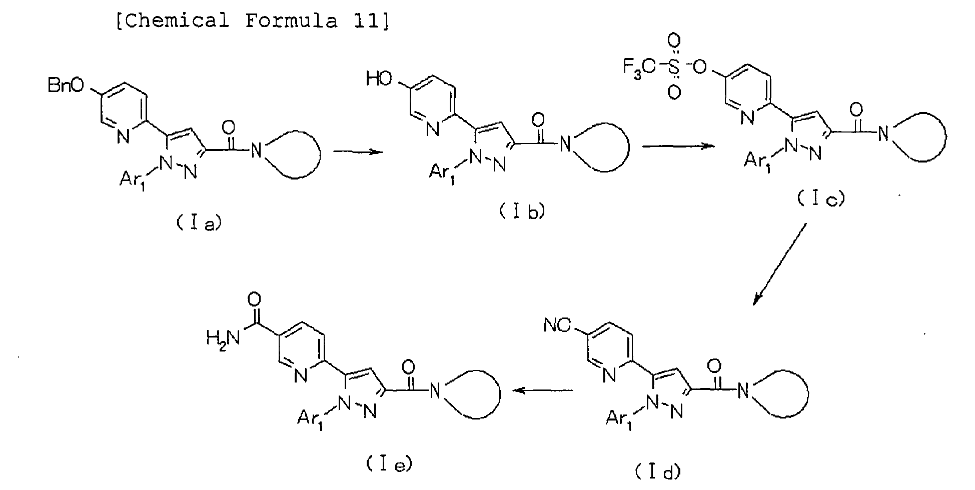

wherein Ar₁ and a moiety structure as follows:

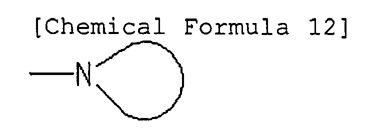

represent the same ones as described above; and Bn represents a benzyl group.

Specifically, when the compound (Ia) is dissolved in ethanol or the like and catalytically reduced using 10% palladium-carbon as the catalyst, an alcohol (Ib) can be prepared. When the alcohol (Ib) is dissolved in methylene chloride or the like and reacted with anhydrous trifluoromethanesulfonic acid at -50 to 50°C in the presence of a base such as pyridine or the like, a triflate (Ic) can be prepared. When the triflate (Ic) is dissolved in 1, 2-dichloroethane or the like, and tri-n-butyltin cyanide and tetrakis(triphenylphosphine)palladium(0) are added with stirring, a nitrile (Id) can be prepared. The reaction temperature is preferably 10 to 100°C. When the nitrile (Id) is dissolved in methanol, tetrahydrofuran or the like, and hydrolyzed using sodium hydroxide, an amide (Ie) can be obtained. The reaction temperature is preferably 0 to 100°C. The amide (Ie) can also be prepared by deriving the nitrile (Id) to a carboxylic acid, and then condensing the carboxylic acid using an appropriate condensing agent such as aqueous ammonia, ammonium chloride or the like.
In addition, the aforementioned compounds (Ia to Ie) of the invention can be converted to other derivatives of the invention by further modifications based on common knowledge in organic chemistry.

The compound (I) of the present invention, a salt thereof, or a solvate of the compound or the salt has a strong anti-platelet aggregation effect, and potently inhibited thrombus formation even in a high shear stress-induced thrombosis model. Therefore, the compound (I) of the invention, a salt thereof, or a solvate of the compound or the salt is useful in mammals including humans as a prophylactic and/or therapeutic agent for ischemic diseases caused by thrombi or emboli, such as myocardial infarction, angina pectoris (chronic stable angina, unstable angina, and the like), ischemic cerebrovascular disorder (transient ischemic attack (TIA), cerebral infarction, and the like), peripheral vascular disorder, occlusion after replacement with an artificial vessel, thrombotic occlusion after coronary artery intervention (coronary artery bypass grafting (CABG), percutaneous transluminal coronary angioplasty (PTCA), stent placement, and the like), diabetic retinopathy and nephropathy, occlusion upon replacement with an artificial heart valve, and the like. Also, the aforementioned compound is useful as a prophylactic and/or therapeutic agent for thrombosis and embolism associated with vascular surgery, extracorporeal blood circulation, and the like. The aforementioned compound is also useful for the improvement of ischemic symptoms associated with chronic arterial occlusion, such as ulcer, pain, cold sensation and the like.
When the compound (I) of the invention, a salt thereof, or a solvate of the compound or the salt is used as a medicine, the dose may vary depending on the age, sex, symptoms and the like of the patient, but a daily dose for an adult is preferably 0.1 mg to 1 g, and particularly preferably 0.5 mg to 500 mg. In this case, the daily dose can be administered in several portions in a divided manner, or if necessary, the aforementioned compound can also be administered at a dose exceeding the daily dose described above.

The medicine containing the compound (I) of the invention, a salt thereof, or a solvate of the compound or the salt as an active ingredient can be administered via any method of administration and in any dosage form according to the need. The preparation of the medicine may be formulated according to various conventionally used methods for formulating preparations,optionally together with a pharmacologically acceptable carrier, and selecting any dosage form which conform to the method of administration. The method of administration and the dosage form are not particularly limited.

For oral preparations, for example, solid preparations such as tablets, powders, granules, pills, capsules and the like, as well as liquid preparations such as liquids, syrups, elixirs, suspensions, emulsions and the like, may be mentioned.

For injective preparations, the compound (I), a salt thereof, or a solvate of the compound or the salt may be dissolved and filled in a container, or alternatively, the solution thus formed may be solidified by lyophilization or the like, as a preparation prepared at the time of use.

In the case of producing such preparations, pharmaceutically acceptable additives, for example, binder, disintegrant, dissolution accelerator, lubricant, filler, excipient and the like, can be selected as necessary and used.

### EXAMPLES

Next, the present invention will be described in detail with reference to Reference Examples, Examples and Test Examples.
[Reference Example 1] 5-Hydrazino-2-methoxypyridine hydrochloride

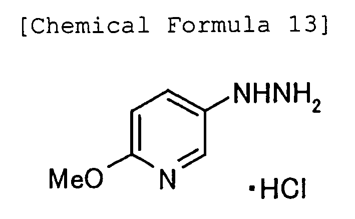

A solution of sodium nitrite (3.795 g) in water (20 mL) was added dropwise to a solution of 5-amino-2-methoxypyridine (6.21 g) in concentrated hydrochloric acid (50 mL) for 60 minutes under ice cooling, and the resultant mixture was stirred at the same temperature for 30 minutes. A solution of tin(II) chloride dihydrate (39.5 g) in concentrated hydrochloric acid (30 mL) was added dropwise to the reaction solution at an internal temperature of about 10°C over 30 minutes, and then the resultant mixture was stirred for 2 hours at room temperature. A solution of sodium hydroxide (75 g) in water (300 mL) and diethyl ether were added to the reaction solution under ice cooling, to partition the reaction solution. The aqueous layer was extracted twice with diethyl ether. Further, the aqueous layer was saturated with sodium chloride and then extracted with diethyl ether. The organic layers were combined, and dried over anhydrous sodium sulfate. After separation by filtration, a 1 M hydrochloric acid-ethanol solution (50 mL) was added to the filtrate, and the mixture was stirred. The solid that had precipitated was collected by filtration, washed with diethyl ether, and dried, thus to obtain the title compound (5.02 g, 57%).
¹H-NMR(400MHz, DMSO-d₆)δ: 3.81(3H, s), 6.82(1H, d, J=8.8Hz), 7.57(1H, dd, J=8.8, 2.9Hz), 7.97(1H, d, J=2.9Hz), 8.55-9.20(1H, br), 10.13-10.50(3H, br).
MS(ESI)m/z: 140(M+H)⁺.

[Reference Example 2] 5- Hydrazino-2-methoxypyridine

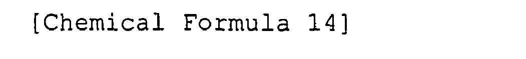

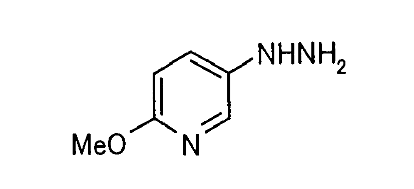

A solution of sodium nitrite (3.795 g) in water (20 mL) was added dropwise to a solution of 5-amino-2-methoxypyridine (6.207 g) in concentrated hydrochloric acid (50 mL) under ice cooling over 80 minutes, and the resultant mixture was stirred at the same temperature for 30 minutes. A solution of tin(II) chloride dihydrate (39.5 g) in concentrated hydrochloric acid (30 mL) was added dropwise to the reaction solution at an internal temperature of about 10°C over 60 minutes, and then the resultant mixture was stirred for 12.5 hours at room temperature. A solution of sodium hydroxide (54 g) in water (200 mL) and chloroform were added to the reaction solution under ice cooling, the insoluble matter was filtered off, and the reaction solution was partitioned. Further, the aqueous layer was extracted twice with chloroform. The organic layers were combined, and dried over anhydrous sodium sulfate. After separation by filtration, the solvent was evaporated under reduced pressure, thus to obtain the title compound (4.23 g, 60%).
¹H-NMR(400MHz, CDCl₃)δ: 3.50-3.68 (2H, br), 3.88(3H, s), 4.86-5.03(1H, br), 6.66(1H, d, J=8.8Hz), 7.20(1H, dd, J=8.8, 2.9Hz), 7.77(1H, d, J=2.9Hz).
MS(ESI)m/z: 140(M+H)⁺.

[Reference Example 3] 1-(6-Methoxy-3-pyridyl)-5-(2-pyridyl)-1H-pyrazole-3-carboxylic acid

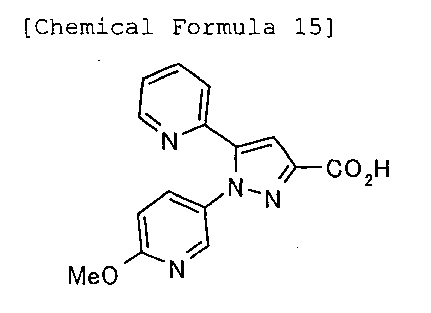

### 1) 4-(2-Pyridyl)-2, 4-dioxobutanoic acid ethyl ester

Under an argon atmosphere, 2-acetylpyridine (1.39 mL) was added dropwise to a suspension of 60% sodium hydride (0.991 g) in N, N-dimethylformamide (30 mL) at 0°C, and the resultant mixture was stirred for 5 minutes, and then stirred at room temperature for 30 minutes. Diethyl oxalate (3.36 mL) at 0°C was added dropwise to the reaction solution, and the resultant mixture was stirred for 10 minutes and then stirred at room temperature for 18 hours. Water and diethyl ether were added to the reaction solution, and the mixture was partitioned. The aqueous layer was neutralized with an aqueous 1 N hydrochloric acid solution (24.8 mL), ethyl acetate was added thereto, and the resultant mixture was partitioned. The organic layer was washed twice with water, and then dried over anhydrous sodium sulfate. After separation by filtration, a residue obtained by evaporating the solvent under reduced pressure was purified by silica gel column chromatography (methanol-chloroform), thus to obtain 4-(2-pyridyl)-2, 4-dioxobutanoic acid ethyl ester (1.12 g, 41%) as a solid.
¹H-NMR(400MHz, CDCl₃)δ: 1.40-1.43(3H, m), 4.38-4.43(2H, m), 7.51-7.54(1H, m), 7.62(1H, s), 7.89-7.93(1H, m), 8.18(1H, d, J=8.0Hz), 8.73(1H, d, J=4.4Hz).
MS(EI)m/z: 221(M⁺).

### 2) 5-Hydroxy-1-(6-methoxy-3-pyridyl)-5-(2-pyridyl)-4, 5-dihydro-1H-pyrazole-3-carboxylic acid ethyl ester

4-(2-Pyridyl)-2, 4-dioxo butanoic acid ethyl ester (1.10 g) of the above and a solution of 5-hydrazino-2-methoxypyridine (0.692 g) of Reference Example 2 in ethanol (22 mL) were heated to reflux for 14 hours. After air cooling, a residue obtained by evaporating the solvent under reduced pressure was purified by silica gel column chromatography (hexane-ethyl acetate), then further purified by silica gel column chromatography (toluene-acetone) to obtain 5-hydroxy-1-(6-methoxy-3-pyridyl)-5-(2-pyridyl)-4, 5-dihydro-1H-pyrazole-3-carboxylic acid ethyl ester (0.575 g, 34%) as a solid.
¹H-NMR(400MHz, CDCl₃)δ: 1.37-1.40(3H, m), 3.47-3.64(2H, m), 3.81(3H, s), 4.35-4.40(2H, m), 6.57-6.59(1H, m), 6.85(1H, m), 7.34-7.38(1H, m), 7.45-7.48(1H, m), 7.52-7.59(2H, m), 7.79-7.83(1H, m), 8.55-8.57(1H, m).

### 3) 1-(6-Methoxy-3-pyridyl)-5-(2-pyridyl)-1H-pyrazole-3-carboxylic acid ethyl ester

Acetic acid (0.456 mL) was added to a solution of 5-hydroxy-1-(6-methoxy-3-pyridyl)-5-(2-pyridyl)-4, 5-dihydro-1H-pyrazole-3-carboxylic acid ethyl ester (0.546 g) in ethanol (11 mL), and the resultant mixture was heated to reflux for 4 hours. After air cooling, a saturated aqueous solution of sodium hydrogen carbonate, water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (hexane-ethyl acetate), to obtain 1-(6-methoxy-3-pyridyl)-5-(2-pyridyl)-1H-pyrazole-3-carboxylic acid ethyl ester (0.516 g, quantitative) as a solid.
¹H-NMR(400MHz, CDCl₃)δ: 1.43(3H, t, J=7.2Hz), 3.95(3H, s), 4.46(2H, q, J=7.2Hz), 6.76-6.78(1H, m), 7.22-7.28(2H, m), 7.35-7.37(1H, m), 7.66-7.71(2H, m), 8.11(1H, m), 8.52-8.54(1H, m).
MS(FAB)m/z: 325(M+H)⁺.

### 4) Title compound

A 1 N aqueous sodium hydroxide solution (3.38 mL) was added to a solution of 1-(6-methoxy-3-pyridyl)-5-(2-pyridyl)-1H-pyrazole-3-carboxylic acid ethyl ester (0.438 g) of the above in methanol (8.8 mL) at room temperature, and the resultant mixture was stirred for 4 hours. A 1 N aqueous hydrochloric acid solution (3.38 mL) was added to a residue obtained by evaporating the reaction solvent under reduced pressure, and the mixture was neutralized. Water and ethyl acetate were added to the resultant, and the mixture was partitioned. The organic layer was dried over anhydrous sodium sulfate. After separation by filtration, the solvent was evaporated under reduced pressure, to obtain the title compound (0.344 g, 86%) as a solid.
¹H-NMR(400MHz, DMSO-d₆)δ: 3.89(3H, s), 6.89(1H, d, J=8.8Hz), 7.33-7.37(2H, m), 7.67-7.73(2H, m), 7.85-7.89(1H, m), 8.14(1H, d, J=2.4Hz), 8.44-8.46(1H, m), 13.06(1H, br).
MS (FAB)m/z: 297(M+H)⁺.

[Reference Example 4] 1-(6-Methoxy-3-pyridazinyl)-5-(2-pyridyl)-1H-pyrazole-3-carboxylic acid

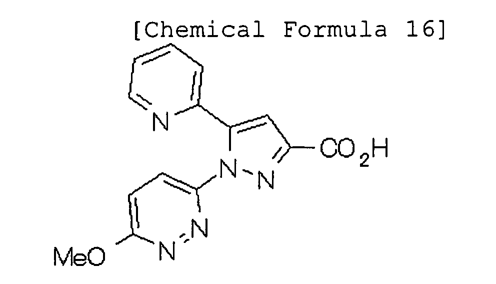

### Method (A)

### 1) 1-(6-Chloro-3-pyridazinyl)-5-(2-pyridyl)-1H-pyrazol 3-carboxylic acid ethyl ester

3-Chloro-6-hydrazinopyridazine (1.59 g) and a solution of 4-(2-pyridyl)-2, 4-dioxobutanoic acid ethyl ester (2.45 g) of Reference Example 3-(1) in ethanol (60 mL) were heated to reflux for 6 hours, and then concentrated hydrochloric acid (1 mL) was added to the reaction solution, which was further heated to reflux for 1 hour. After air cooling, ethyl acetate and a saturated aqueous solution of sodium hydrogen carbonate were added to a residue obtained by evaporating the reaction solvent under reduced pressure, and the resultant mixture was partitioned. The organic layer was washed with saturated saline, and dried over anhydrous magnesium sulfate. After separation by filtration, a residue obtained by evaporating the solvent under reduced pressure was purified by silica gel chromatography (ethyl acetate-hexane), to obtain 1-(6-chloro-3-pyridazinyl)-5-(2-pyridyl)-1H-pyrazole-3-carboxylic acid ethyl ester (1.50 g, 41%) as a solid.
¹H-NMR(400MHz, CDCl₃)δ: 1.44(3H, t, J=7.0Hz), 4.46(2H, q, J=7.0Hz), 7.23(1H, s), 7.24-7.27(1H, m), 7.62-7.65(1H, m), 7.69(1H, d, J=9.0Hz), 7.76-7.81(1H, m), 8.10(1H, d, J=9.0Hz), 8.40(1H, d, J=4.6Hz).
LC-MSm/z: 330(M+H)⁺.

### 2) 1-(6-Methoxy-3-pyridazinyl)-5-(2-pyridyl)-1H-pyrazole-3-carboxylic acid methyl ester

A solution (3 mL) of 28% sodium methoxide in methanol was added to a solution of 1-(6-chloro-3-pyridazinyl)-5-(2-pyridyl)-1H-pyrazole-3-carboxylic acid ethyl ester (1.50 g) of the above in methanol (45 mL), and the resultant mixture was heated to reflux for 2 hours. After air cooling, ethyl acetate and a saturated aqueous solution of sodium hydrogen carbonate were added to a residue obtained by evaporating the reaction solvent under reduced pressure, and the mixture was partitioned. The organic layer was dried over anhydrous magnesium sulfate. After separation by filtration, a residue obtained by evaporating the solvent under reduced pressure was purified by silica gel chromatography (ethyl acetate-hexane), to obtain 1-(6-methoxy-3-pyridazinyl)-5-(2-pyridyl)-1H-pyrazole-3-carboxylic acid methyl ester (480 mg, 34%) as a solid.
¹H-NMR(400MHz, CDCl₃)δ: 3.99(3H, s), 4.10(3H, s), 7.15(1H, d, J=9.3Hz), 7.21-7.23(1H, m), 7.24(1H, s), 7.58-7.61(1H, m), 7.73-7.78(1H, m), 7.93(1H, d, J=9.3Hz), 8.40-8.41(1H, m).
LC-MSm/z: 312(M+H)⁺.

### 3) Title compound

An aqueous solution of 1 N sodium hydroxide (3 mL) was added to a solution of 1-(6-methoxy-3-pyridazinyl)-5-(2-pyridyl)-1H-pyrazole-3-carboxylic acid methyl ester (475 mg) of the above in ethanol (10 mL) and tetrahydrofuran (10 mL), and the resultant mixture was stirred for 20 hours at room temperature. An aqueous solution (3 mL) of 1 N hydrochloric acid was added to the reaction solution under ice cooling, and the mixture was neutralized. A mixed solvent of chloroform-methanol (10:1) was added to the reaction solution, and the mixture was partitioned. The organic layer was dried over anhydrous magnesium sulfate. After separation by filtration, the solvent was evaporated under reduced pressure, to obtain the title compound (300 mg, 66%) as a solid.
¹H-NMR(400MHz, DMSO-d₆)δ: 4.04(3H, s), 7.32-7.35(1H, m), 7.41(1H, s), 7.49(1H, d, J=9.3Hz), 7.80-7.82(1H, m), 7.87-7.91(1H, m), 7.99(1H, d, J=9.3Hz), 8.35-8.36(1H, m).
LC-MSm/z: 298(M+H)⁺.

### Method (B)

### 1) 4-(2-Pyridyl)-2, 4-dioxobutanoic acid methyl ester

Under an argon atmosphere, a solution of 2-acetylpyridine (2.56 g) in methanol (26 mL) was added to a solution of dimethyl oxalate (5.00 g) and sodium methoxide (2.29 g) in methanol (26 mL) at room temperature, and the resultant mixture was stirred for 15 minutes, and then stirred at 60°C for 45 minutes. After air cooling, water was added to the reaction solution, followed by washing with diethyl ether. A saturated aqueous ammonium chloride solution and chloroform were added to the aqueous layer, and the mixture was partitioned. The organic layer was dried over anhydrous sodium sulfate. After separation by filtration, the solvent was evaporated under reduced pressure, to obtain 4-(2-pyridyl)-2, 4-dioxobutanoic acid methyl ester (3.44 g, 79%) as a solid.
¹H-NMR(400MHz, CDCl₃)δ: 3. 94 (3H, s), 7.54-7.50(1H, m), 7.64(1H, s), 7.93-7.89(1H, m), 8.19-8.16(1H, m), 8.74-8.72(1H, m).
EI-MSm/z: 207(M⁺).

### 2) 1-(6-Chloro-3-pyridazinyl)-5-(2-pyridyl)-1H-pyrazole-3-carboxylic acid methyl ester

A solution of 4-(2-pyridyl)-2, 4-dioxobutanoic acid methyl ester (4.143 g) of the above and 3-chloro-6-hydrazinopyridazine (2.891 g) in methanol (100 mL) was heated to reflux for 109 hours. Concentrated hydrochloric acid (2 mL) was added to the reaction solution and further heated to reflux for 6 hours. After air cooling, a saturated aqueous sodium hydrogen carbonate solution and ethyl acetate were added to the reaction solution, and the mixture was partitioned. The organic layer was washed with water and saturated saline, and then dried over anhydrous sodium sulfate. After separation by filtration, the solvent was evaporated under reduced pressure, to obtain 1-(6-chloro-3-pyridazinyl)-5-(2-pyridyl)-1H-pyrazole-3-carboxylic acid methyl ester (3.169 g, 50%) as a solid.
¹H-NMR(400MHz, CDCl₃)δ: 4.00(3H, s), 7.24-7.28(1H, m), 7.24 (1H, s), 7.64(1H, dt, J=7.8, 1.2Hz), 7.70(1H, d, J=9.0Hz), 7.79(1H, td, J=7.8, 1.7Hz), 8.09(1H, d, J=9.0Hz), 8.38-8.41(1H, m).
ESI-MSm/z: 316(M+H)⁺.

### 3) 1-(6-Methoxy-3-pyridazinyl)-5-(2-pyridyl)-1H-pyrazole-3-carboxylic acid methyl ester

Sodium methoxide (1.530 g) was added to a solution of 1-(6-chloro-3-pyridazinyl)-5-(2-pyridyl)-1H-pyrazole-3-carboxylic acid methyl ester (2.981 g) of the above in methanol (190 mL) at room temperature, and the resultant mixture was stirred for 19 hours. An aqueous 1 N hydrochloric acid solution (19 mL) was added to the reaction solution, and methanol was evaporated under reduced pressure. Water was added to the residue thus obtained, and insoluble solids were filtered and dried, to obtain 1-(6-methoxy-3-pyridazinyl)-5-(2-pyridyl)-1H-pyrazole-3-carboxylic acid methyl ester (2.571 g, 87%) as a solid.

### 4) Title compound

An aqueous 1 N sodium hydroxide solution (15 mL) was added to a mixed solution of 1-(6-methoxy-3-pyridazinyl)-5-(2-pyridyl)-1H-pyrazole-3-carboxylic acid methyl ester (2.20 g) of the above in methanol (30 mL) and tetrahydrofuran (30 mL) at room temperature, and the resultant mixture was stirred for 2.5 hours. An aqueous 1 N hydrochloric acid solution (15 mL) and a mixed solvent of chloroform-methanol (10:1) were added to the reaction solution under ice cooling, and the mixture was partitioned. The organic layer was dried over anhydrous sodium sulfate. After separation by filtration, isopropyl ether was added to a residue obtained by evaporating the solvent under reduced pressure, and thus precipitatant was filtered, to obtain the title compound (1.42 g, 47.6%).

[Reference Example 5] 1-(6-Methoxy-3-pyridyl)-5-(1H-pyrrol-2-yl)pyrazole-3-carboxylic acid

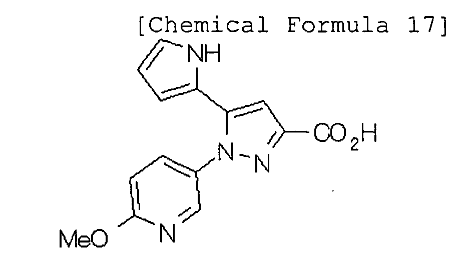

Diethyl oxalate (3.10 mL) and 1-[1-(phenylsulfonyl)-1H-pyrrol-2-yl]-1-ethanone (2.49 g) were added to a solution of sodium ethoxide (1.63 g) in ethanol (20 mL) under ice cooling, and the resultant mixture was stirred for 5 hours at room temperature. To this reaction solution, 5-hydrazino-2-methoxypyridine hydrochloride (2.52 g) of Reference Example 1 and ethanol (20 mL) were added, and the mixture was heated to reflux for 14.5 hours. After air cooling, ethyl acetate and a saturated aqueous solution of sodium hydrogen carbonate were added to a residue obtained by evaporating the reaction solvent under reduced pressure, and the mixture was partitioned. The aqueous layer was further extracted with ethyl acetate. The organic layers were combined and dried over anhydrous sodium sulfate. After separation by filtration, a residue obtained by evaporating the solvent under reduced pressure was purified by silica gel column chromatography (ethyl acetate-hexane), to obtain 1-(6-methoxy-3-pyridyl)-5-[1-(phenylsulfonyl)-1H-pyrrol-2-yl]pyrazole-3-carboxylic acid ethyl ester (3.28 g, 72%) as an oily product. To a solution of this ethyl ester product (3.28 g) in ethanol (22 mL), an aqueous 1 N sodium hydroxide solution (22 mL) was added, and the mixture was stirred for 2 days at room temperature. An aqueous 1 N hydrochloric acid solution was added to the reaction solution, and the precipitated solid was filtered, to obtain the title compound (1.40 g, 68%) as a solid.
¹H-NMR(400MHz, DMSO-d₆)δ: 3.94(3H, s), 5.49-5.51(1H, m), 5.98-6.00(1H, m), 6.87-6.89(1H, m), 6.98(1H, dd, J=8.8, 0.5Hz), 7.08(1H, s), 7.80(1H, dd, J=8.8, 2.7Hz), 8.25(1H, dd, J=2.7, 0.5Hz), 11.39(1H, br s).
ESI-MSm/z: 285(M+H)⁺.

[Reference Example 6] 1-(6-Methoxy-3-pyridyl)-5-(2-pyrazinyl)-1H-pyrazole-3-carboxylic acid

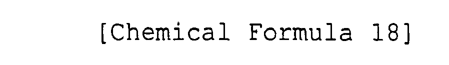

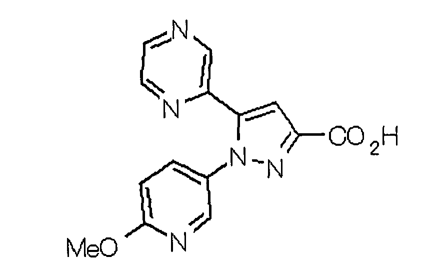

Lithium bis(trimethylsilyl)amide (a 1.0 M solution in tetrahydrofuran, 11.0 mL) was added to a solution of 1-(2-pyrazinyl)-1-ethanone (1.22 g) in tetrahydrofuran (10 mL) under cooling to -78°C, and the resultant mixture was stirred for 55 minutes. Diethyl oxalate (2.05 mL) was added thereto, and the mixture was slowly returned to room temperature, and stirred for 6.5 hours. An aqueous 1 N hydrochloric acid solution (11 mL), water and diethyl ether were added to the reaction solution, and the mixture was partitioned. Then, sodium chloride was added to the aqueous layer to be saturated, and then ethyl acetate was added for extraction. The organic layers were combined, and the solvent was evaporated under reduced pressure, to obtain a crude product of 4-(2-pyrazinyl)-2, 4-dioxobutanoic acid ethyl ester (1.83 g, 82%) as a solid. To a suspension of this crude product (1.58 g) in ethanol (20 mL), a solution formed from a suspension of 5-hydrazino-2-methoxypyridine hydrochloride (1.50 g) of Reference Example 1 in ethanol (80 mL) conditioned with triethylamine (1.9 mL), was added, and the mixture was heated to reflux for 19 hours. Acetic acid (5 mL) was further added to the reaction solution, and the mixture was heated to reflux for 1.5 days. After air cooling, ethyl acetate and a saturated aqueous solution of sodium hydrogen carbonate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(6-methoxy-3-pyridyl)-5-(2-pyrazinyl)-1H-pyrazole-3-carboxylic acid ethyl ester (1.05 g, 45%) as a solid. To a solution of this obtained 1H-pyrazole-3-carboxylic acid ethyl ester product (1.05 g) in ethanol (30 mL), an aqueous 1 N sodium hydroxide solution (10.0 mL) was added, and the mixture was stirred for 16 hours at room temperature. An aqueous 1 N hydrochloric acid solution (15 mL), water and ethyl acetate were added to the reaction solution, and the mixture was partitioned. The organic layer was dried over anhydrous magnesium sulfate. After separation by filtration, the solvent was evaporated under reduced pressure, to obtain a crude product of the title compound (0.883 g, 92%) as a solid. This product was supplied to the subsequent reaction without being purified.

[Reference Example 7] 5-Hydrazino-2-methylpyridine

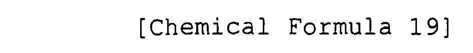

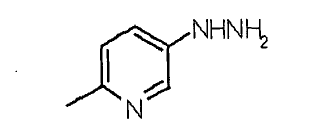

### 1) (6-Methylpyridin-3-yl)carbamic acid tert-butyl ester

Triethylamine (23.0 mL), diphenylphosphorylazide (35.6 mL) and tert-butanol (30.0 mL) were added to a suspension of 6-methylnicotinic acid (21.58 g) in 1, 4-dioxane (300 mL) at room temperature, and the resultant mixture was heated to reflux for 18 hours. After air cooling, the reaction solvent was evaporated under reduced pressure, chloroform and water were added to the residue thus obtained, and the mixture was partitioned. The organic layer was washed with saturated saline, and dried over anhydrous sodium sulfate. After separation by filtration, the solvent was evaporated under reduced pressure, and the residue thus obtained was purified by silica gel column chromatography (chloroform-ethyl acetate), to obtain (6-methylpyridin-3-yl)carbamic acid tert-butyl ester (28.7 g, 88%) as a solid.
¹H-NMR(400MHz, CDCl₃)δ: 1.54(9H, s), 2.49(3H, s), 6.52(1H, br), 7.09(1H, d, J=8.6Hz), 7.86(1H, br), 8.29(1H, d, J=2.7Hz).
EI-MSm/z: 208(M)⁺.

### 2) Title compound

Concentrated hydrochloric acid (100 mL) was slowly added to (6-methylpyridin-3-yl)carbamic acid tert-butyl ester (28.7 g) at 0°C, and the resultant mixture was stirred for 30 minutes. While maintaining the internal temperature of the reaction solution at 0 to 5°C, a solution of sodium nitrite (10.51 g) in water (38 mL) was added dropwise over 30 minutes, and the mixture was stirred for 15 minutes. This reaction solution was added dropwise to a solution of tin(II) chloride dihydrate (108.7 g) in concentrated hydrochloric acid (54 mL) over 50 minutes, while maintaining the internal temperature of the reaction solution at 0 to 5°C, and then the mixture was stirred for 1 hour. While maintaining the internal temperature of the reaction solution at 0 to 10°C, an aqueous solution (700 mL) of 6 N sodium hydroxide and a mixed solvent of chloroform-methanol (10:1) were added to the reaction solution, and the mixture was partitioned. The insoluble suspended matter in the organic layer was separated by filtration using Celite, and then the solvent of the filtrate was washed with water. The aqueous layer was extracted with a mixed solvent of chloroform-methanol (10:1), and the organic layers were combined and washed with saturated saline. Then, the organic layer was dried over anhydrous sodium sulfate. After separation by filtration, the solvent was evaporated under reduced pressure, to obtain the title compound (7.66 g, 45%) as a solid.
¹H-NMR(400MHz, DMSO-d₆)δ: 2.29(3H, s), 3.97(2H, br), 6.66(1H, br), 6.94(1H, d, J=8.3Hz), 7.05(1H, dd, J=3.0, 8.3Hz), 7.98(1H, d, J=3.0Hz).
ESI-MSm/z: 124(M+H)⁺.

[Reference Example 8] 5-(5-Methyl-2-pyrazinyl)-1-(6-methyl-3-pyridyl)-1H-pyrazole-3-carboxylic acid

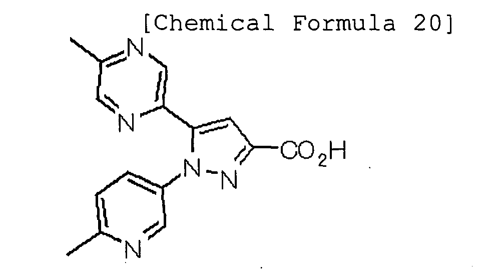

### 1) 5-Methylpyrazine-2-carboxylic acid N-methoxy-N-methylamide

Triethylamine (28.9 mL) was added to a solution of 5-methylpyrazine-2-carboxylic acid (13.0 g), 3-(3-dimethylaminopropyl)-1-ethylcarbodiimide hydrochloride (19.8 g), 1-hydroxybenzotriazole (14.0 g), and N, O-dimethylhydroxyamine hydrochloride (10.1 g) in N, N-dimethylformamide (130 mL) at room temperature, and the resultant mixture was stirred for 63 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (hexane-ethyl acetate), to obtain 5-methylpyrazine-2-carboxylic acid N-methoxy-N-methylamide (12.3 g, 72%) as an oily product.
¹H-NMR(400MHz, CDCl₃)δ: 2.63(3H, s), 3.41(3H, s), 3.74(3H, s), 8.46(1H, s), 8.82(1H, s).
FAB-MSm/z: 182(M+H)⁺.

### 2) 1-(5-Methylpyrazin-2-yl)-1-ethanone

Under an argon atmosphere, methyllithium (a 1.02 M solution in diethyl ether, 72.6 mL) was added dropwise to a solution of 5-methylpyrazine-2-carboxylic acid N-methoxy-N-methylamide (12.2 g) in tetrahydrofuran (183 mL) over 20 minutes under cooling to -78°C, and the resultant mixture was stirred for 130 minutes. Water and ethyl acetate were added to the reaction solution at 0°C, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(5-methylpyrazin-2-yl)-1-ethanone (7.90 g, 86%) as a solid.
¹H-NMR(400MHz, CDCl₃)δ: 2.66(3H, s), 2.70(3H, s), 8.50(1H, m), 9.11(1H, d, J=1.5Hz).
ESI-MSm/z: 137(M+H)⁺.

### 3) 4-(5-Methylpyrazin-2-yl)-2, 4-dioxobutanoic acid ethyl ester

Under an argon atmosphere, lithium bis(trimethylsilyl)amide (a 1.0 M solution in tetrahydrofuran, 63.7 mL) was added dropwise to a solution of 1-(5-methylpyrazin-2-yl)-1-ethanone (7.89 g) in tetrahydrofuran (118 mL) over 20 minutes under cooling to - 78°C, and the resultant mixture was stirred for 30 minutes. Diethyl oxalate (11.8 mL) was added dropwise to the reaction solution, and stirred for 10 minutes at -78°C, for 30 minutes at 0°C, and again for 1.5 hours at room temperature. Water and diethyl ether were added to the reaction solution, and the mixture was partitioned. A saturated aqueous solution of ammonium chloride was added to the aqueous layer, and the aqueous layer was extracted with chloroform. The aqueous layer was further extracted with chloroform, and the organic layers were combined and dried over anhydrous sodium sulfate. After separation by filtration, the solvent was evaporated under reduced pressure, to obtain 4-(5-methylpyrazin-2-yl)-2, 4-dioxobutanoic acid ethyl ester (4.92 g, 36%) as a solid.
¹H-NMR(400MHz, CDCl₃)δ: 1.39-1.43(3H, m), 2.69(3H, s), 4.38-4.43(2H, m), 7.60(1H, s), 8.55(1H, m), 9.21(1H, d, J=1.2Hz).
FAB-MSm/z: 237(M+H)⁺.

### 4) 5-(5-Methyl-2-pyrazinyl)-1-(6-methyl-3-pyridyl)-1H-pyrazole-3-carboxylic acid ethyl ester

A solution of 4-(5-methylpyrazin-2-yl)-2, 4-dioxobutanoic acid ethyl ester (4.51 g) and 5-hydrazino-2-methylpyridine (2.35 g) of Reference Example 7 in ethanol (90 mL) was heated to reflux for 80 minutes, and then acetic acid (5.46 mL) was added to the reaction solution, which was further heated to reflux for 15 hours. Furthermore, concentrated hydrochloric acid (3 mL) was added to the reaction solution, and the mixture was heated to reflux for 1 hour. After air cooling, saturated sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (acetone-toluene), to obtain 5-(5-methyl-2-pyrazinyl)-1-(6-methyl-3-pyridyl)-1H-pyrazole-3-carboxylic acid ethyl ester (1.72 g, 28%) as a solid.
¹H-NMR(400MHz, CDCl₃)δ: 1.44(3H, t, J=7.1Hz), 2.58(3H, s), 2.62(3H, s), 4.48(2H, q, J=7.1Hz), 7.25(1H, d, J=8.3Hz), 7.34(1H, s), 7.73(1H, dd, J=8.3, 2.7Hz), 8.34(1H, m), 8.40-8.41(1H, m), 8.59(1H, d, J=1.5Hz).
FAB-MSm/z: 324(M+H)⁺.

### 5) Title compound

A solution of lithium hydroxide monohydrate (0.244 g) in water (17 mL) was added to a suspension of 5-(5-methyl-2-pyrazinyl)-1-(6-methyl-3-pyridyl)-1H-pyrazole-3-carboxylic acid ethyl ester (1.71 g) in tetrahydrofuran (34 mL) at room temperature, and the resultant mixture was stirred for 100 minutes. An aqueous 1 N hydrochloric acid solution (5.82 mL) was added to the reaction solution, the reaction solution was neutralized, and then water (250 mL) was added. The precipitated solid was filtered, and thus the title compound (1.15 g, 74%) was obtained as a solid.
¹H-NMR(400MHz, DMSO-d₆)δ: 2.50-2.53(6H, m), 7.35(1H, d, J=8.3Hz), 7.48(1H, m), 7.72(1H, dd, J=8.3, 2.4Hz), 8.39(1H, m), 8.42(1H, d, J=2.4Hz), 8.87(1H, s), 13.14(1H, br s).
FAB-MSm/z: 296(M+H)⁺.

[Reference Example 9] 5-(5-Methoxy-2-pyridyl)-1-(6-methyl-3-pyridyl)-1H-pyrazole-3-carboxylic acid

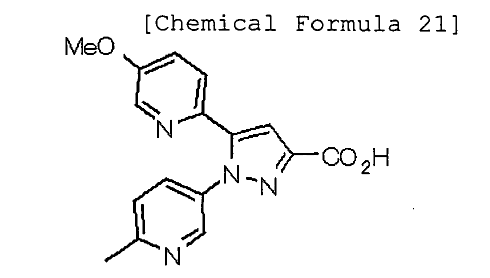

### 1) 5-Methoxypyridine-2-carboxylic acid N-methoxy-N-methylamide

Under an argon atmosphere, a solution of 5-hydroxy-2-methylpyridine (30.0 g) in dimethylsulfoxide (200 mL) cooled to 0°C was added dropwise to a suspension of sodium hydride (55% in oil, 12.6 g) in dimethylsulfoxide (100 mL) over 20 minutes, and then the resultant mixture was stirred for 35 minutes. At the same temperature, methyl iodide (18.0 mL) was added dropwise to the reaction solution over 15 minutes, and the mixture was stirred for 2 hours at room temperature. Water and diethyl ether were added to the reaction solution, and the mixture was partitioned. The organic layer was dried over anhydrous sodium sulfate. After separation by filtration, the solvent was evaporated under reduced pressure, and 5-methoxy-2-methylpyridine (18.7 g) was obtained. To a solution of this crude product (18.7 g) in pyridine (187 mL), selenium dioxide (33.7 g) was added, and the mixture was heated to reflux for 62 hours. After air cooling, water and chloroform were added to the reaction solution, and the mixture was partitioned. The organic layer was dried over anhydrous sodium sulfate. After separation by filtration, the solvent was evaporated under reduced pressure, and 5-methoxypyridine-2-carboxylic acid (19.1 g) was obtained. To a suspension of the crude product thus obtained (19.1 g), N, O-dimethylhydroxyamine hydrochloride (16.3 g), 3-(3-dimethylaminopropyl)-1-ethylcarbodiimide hydrochloride (32.1 g) and 1-hydroxybenzotriazole (22.6 g) in dichloromethane (250 mL), triethylamine (46.6 mL) was added at 0°C, and the mixture was stirred for 30 minutes, and then stirred for 14 hours at room temperature. Water and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-chloroform), to obtain 5-methoxypyridine-2-carboxylic acid N-methoxy-N-methylamide (15.3 g, 51%) as an oily product.
¹H-NMR(400MHz, CDCl₃)δ: 3.44 (3H, s), 3.79-3.84 (3H, m), 3.91(3H, s), 7.24-7.28(1H, m), 7.75(1H, d, J=8.5Hz), 8.30(1H, d, J=2.9Hz).
FAB-MSm/z: 197(M+H)⁺.

### 2) 1-(5-Methoxypyridin-2-yl)-1-ethanone

1-(5-Methoxypyridin-2-yl)-1-ethanone (5.41 g, 46%) was obtained as an oily product by the same method as that of Reference Example 8-(2), using 5-methoxypyridine-2-carboxylic acid N-methoxy-N-methyl amide (15.3 g) and methyllithium (a 0.98 M solution in diethyl ether, 87.5 mL) under an argon atmosphere.
¹H-NMR(400MHz, CDCl₃)δ: 2.69(3H, s), 3.94(3H, s), 7.27(1H, dd, J=8.5, 2.9Hz), 8.06(1H, d, J=8.5Hz), 8.34(1H, d, J=2.9Hz).
FAB-MSm/z: 152(M+H)⁺.

### 3) 4-(5-Methoxy-2-pyridyl)-2, 4-dioxobutanoic acid ethyl ester

Under an argon atmosphere, diethyl oxalate (9.70 mL) was added to a solution of sodium ethoxide (4.86 g) in ethanol (54 mL) at room temperature, and then a solution of 1-(5-methoxypyridin-2-yl)-1-ethanone (5.40 g) in ethanol (54 mL) was added dropwise to the reaction solution. After stirring the mixture at room temperature for 140 minutes, water and diethyl ether were added to the reaction solution, and the mixture was partitioned. A saturated aqueous solution of ammonium chloride was added to the aqueous solution, and the aqueous layer was extracted with chloroform. The organic layer was dried over anhydrous sodium sulfate. After separation by filtration, the solvent was evaporated under reduced pressure, and 4-(5-methoxy-2-pyridyl)-2, 4-dioxobutanoic acid ethyl ester (5.10 g, 57%) was obtained as a solid.
¹H-NMR(400MHz, CDCl₃)δ: 1.39-1.43(3H, m), 3.96(3H, s), 4.37-4.42(2H, m), 7.31(1H, dd, J=8.8, 2.9Hz), 7.60(1H, s), 8.16(1H, d, J=8.8Hz), 8.38(1H, d, J=2.9Hz).
EI-MSm/z: 251(M⁺).

### 4) 5-(5-Methoxy-2-pyridyl)-1-(6-methyl-3-pyridyl)-1H-pyrazole-3-carboxylic acid ethyl ester

A solution of 4-(5-methoxy-2-pyridyl)-2, 4-dioxobutanoic acid ethyl ester (4.79 g) and 5-hydrazino-2-methylpyridine (2.35 g) of Reference Example 7 in ethanol (96 mL) was heated to reflux for 1 hour, and then acetic acid (5.47 mL) was added to the reaction solution, which was further heated to reflux for 63 hours. After air cooling, a saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (acetone-toluene), to obtain 5-(5-methoxy-2-pyridyl)-1-(6-methyl-3-pyridyl)-1H-pyrazole-3-carboxylic acid ethyl ester (1.49 g, 23%) as a solid.
¹H-NMR(400MHz, CDCl₃)δ: 1.41-1.44(3H, m), 2.59(3H, s), 3.86(3H, s), 4.43-4.48(2H, m), 7.16-7.34(4H, m), 7.71-7.73(1H, m), 8.19(1H, d, J=2.8Hz), 8.38(1H, d, J=2.4Hz).
FAB-MSm/z: 339(M+H)⁺.

### 5) Title compound

The title compound (0.970 g, 71%) was obtained as a solid by the same method as that in Reference Example 4, Method (B)-(4), using 5-(5-methoxy-2-pyridyl)-1-(6-methyl-3-pyridyl)-1H-pyrazole-3-carboxylic acid ethyl ester (1.48 g).
¹H-NMR(400MHz, DMSO-d₆)δ: 2.53(3H, s), 3.84(3H, m), 7.23(1H, m), 7.34(1H, d, J=8.5Hz), 7.45-7.48(1H, m), 7.63-7.67(2H, m), 8.16(1H, d, J=3.2Hz), 8.37(1H, d, J=2.7Hz), 13.03(1H, br s).
FAB-MSm/z: 311(M+H)⁺.

[Reference Example 10] 1-(6-Methoxy-3-pyridazinyl)-5-(1H-pyrrol-2-yl)-1H-pyrazole-3-carboxylic acid

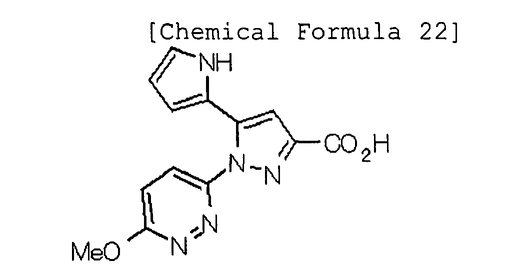

Under cooling to -78°C, lithium bis(trimethylsilyl)amide (a 1.0 M solution in tetrahydrofuran, 11.0 mL) was added to a solution of 1-[1-(phenylsulfonyl)-1H-pyrrol-2-yl]-1-ethanone (2.49 g) in tetrahydrofuran (10 mL), and the resultant mixture was stirred for 35 minutes. Further, dimethyl oxalate (1.77 g) was added to the reaction solution, and the mixture was stirred for 10 minutes. The mixture was slowly returned to room temperature and stirred for 3.5 hours. Diethyl ether and water were added to the reaction solution, and the mixture was partitioned. Diethyl ether, an aqueous 1 N hydrochloric acid solution (11 mL) and ethyl acetate were added to the aqueous layer, and the mixture was partitioned. The organic layer was dried over anhydrous sodium sulfate. After separation by filtration, the solvent was evaporated under reduced pressure, to obtain a crude product of 4-[1-(phenylsulfonyl)-1H-pyrrol-2-yl]-2, 4-dioxobutanoic acid methyl ester (2.88 g, 85%) as a solid. To a suspension of this butanoic acid methyl ester product (2.70 g) in methanol (40 mL), 3-chloro-6-hydrazinopyridazine (1.16 g) was added, and the mixture was heated to reflux for 16.5 hours. Furthermore, concentrated hydrochloric acid (0.200 mL) and methanol (40 mL) were added to the reaction solution, and the mixture was heated to reflux for 2 hours. Then, concentrated hydrochloric acid (0.200 mL) was added, and the mixture was heated to reflux for 2 hours. Concentrated hydrochloric acid (0.200 mL) was further added, and the mixture was heated to reflux for 40 minutes. After air cooling, ethyl acetate and a saturated aqueous solution of sodium hydrogen carbonate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(6-chloro-3-pyridazinyl)-5-[1-(phenylsulfonyl)-1H-pyrrol-2-yl]-1H-pyrazole-3-carboxylic acid methyl ester (2.57 g, 71%) as an amorphous form. This pyrazole product (2.34 g) was dissolved in methanol (50 mL) and tetrahydrofuran (50 mL), sodium methoxide (0.854 g) was added thereto at room temperature, and the resultant mixture was stirred for 7.5 hours. Further, an aqueous 1 N sodium hydroxide solution (11.0 mL) was added to the reaction solution, and the mixture was stirred for 23 hours. Diethyl ether and water were added to the reaction solution, and the mixture was partitioned. The aqueous layer was acidified with an aqueous 1 N hydrochloric acid solution and extracted with diethyl ether, and the aqueous layer was extracted with ethyl acetate. The aqueous layer was further saturated with sodium chloride, and then extracted with ethyl acetate. The organic layers were combined and dried over anhydrous sodium sulfate. After separation by filtration, methanol and diethyl ether were added to a residue obtained by evaporating the solvent under reduced pressure, and the precipitated solid was filtered off. The solvent of the filtrate was evaporated under reduced pressure, and the title compound (0.984 g, 65%) was obtained as a solid.
¹H-NMR(400MHz, DMSO-d₆)δ: 4.12(3H, s), 5.67-5.69(1H, m), 6.00-6.02(1H, m), 6.89-6.91(1H, m), 7.11(1H, s), 7.53(1H, d, J=9.3Hz), 7.92(1H, d, J=9.3Hz), 11.40(1H, br s).
ESI-MSm/z: 286(M+H)⁺.

[Reference Example 11] 1-(6-Methoxy-3-pyridyl)-5-(1H-pyrrol-1-yl)-1H-pyrazole-3-carboxylic acid

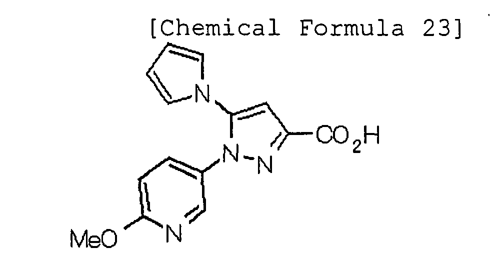

### 1) 1-(6-Methoxy-3-pyridyl)-5-(1H-pyrrol-1-yl)-1H-pyrazole-3-carboxylic acid ethyl ester

Diethyl oxalate (6.79 mL) was slowly added to a suspension of sodium ethoxide (3.40 g) in tert-butyl methyl ether (30 mL) at room temperature, and the resultant mixture was stirred at 60°C for 10 minutes. Acetonitrile (2.61 mL) was slowly added to the reaction solution, and the mixture was heated to reflux for 4 hours. After air cooling, the precipitated solid was filtered, and 1-cyano-3-ethoxy-3-oxo-1-propen-2-ol sodium salt (6.17 g, 75%) was obtained as a solid. To a solution of 5-hydrazino-2-methoxypyridine (5.00 g) of Reference Example 2 in ethanol (100 mL), a 1 M hydrochloric acid-ethanol solution (36.0 mL) and the 1-cyano-3-ethoxy-3-oxo-1-propen-2-ol sodium salt (5.86 g) were added, and the mixture was heated to reflux for 16 hours. After air cooling, the reaction solvent was evaporated under reduced pressure, ethyl acetate and a saturated aqueous solution of sodium hydrogen carbonate were added to the residue thus obtained, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-amino-1-(6-methoxy-3-pyridyl)-1H-yrazole-3-carboxylic acid ethyl ester (5. 09 g, 54%) as a solid. To a solution of this 5-aminopyrazole product (2.62 g) in acetic acid (50 mL), 2, 5-dimethoxytetrahydrofuran (1.94 mL) was added, and the mixture was heated to reflux for 3 hours. After air cooling, the reaction solvent was evaporated under reduced pressure, ethyl acetate and a saturated aqueous solution of sodium hydrogen carbonate were added to the residue thus obtained, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(6-methoxy-3-pyridyl)-5-(1H-pyrrol-1-yl)-1H-pyrazole-3-carboxylic acid ethyl ester (2.92 g, 93%) as a solid.
¹H-NMR(400MHz, CDCl₃)δ: 1.43(3H, t, J=7.1Hz), 3.94(3H, s), 4.46(2H, q, J=7.2Hz), 6.28-6.30(2H, m), 6.64-6.65(2H, m), 6.72(1H, d, J=9.3Hz), 6.92(1H, s), 7.42(1H, dd, J=8.8, 2.7Hz), 8.00(1H, d, J=2.4Hz).
ESI-MSm/z: 313(M+H)⁺.

### 2) Title compound

An aqueous 1 N sodium hydroxide solution (15.0 mL) was added to a mixed solution of 1-(6-methoxy-3-pyridyl)-5-(1H-pyrrol-1-yl)-1H-pyrazole-3-carboxylic acid ethyl ester (2.91 g) in ethanol (30 mL) and tetrahydrofuran (15 mL), and the resultant mixture was stirred for 3 hours at room temperature. The reaction solution was acidified with an aqueous 1 N hydrochloric acid, ethyl acetate was added thereto, and the mixture was partitioned. The organic layer was dried over anhydrous sodium sulfate. After separation by filtration, the solvent was evaporated under reduced pressure, and the title compound (2.96 g, quantitative) was obtained as a solid.
¹H-NMR(400MHz, DMSO-d₆)δ: 3.87(3H, s), 6.21-6.22 (2H, m), 6.88-6.90(3H, m), 7.02(1H, s), 7.61(1H, dd, J=8.9, 2.8Hz), 8.00(1H, d, J=2.7Hz).
ESI-MSm/z: 285(M+H)⁺.

[Reference Example 12] 1-(6-Methoxy-3-pyridazinyl)-5-(2-pyrazinyl)-1H-pyrazole-3-carboxylic acid

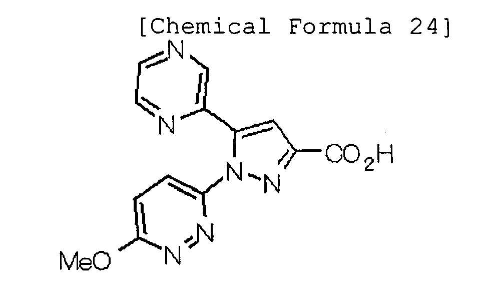

### 1) 1-(6-Chloro-3-pyridazinyl)-5-(2-pyrazinyl)-1H-pyrazole-3-carboxylic acid methyl ester

Under cooling to -78°C, lithium bis(trimethylsilyl) amide(a 1.0 M solution in tetrahydrofuran, 55.0 mL) was added to a solution of 1-(2-pyrazinyl)-1-ethanone (6.10 g) in tetrahydrofuran (50 mL), and the resultant mixture was stirred for 45 minutes. Dimethyl oxalate (8.85 g) was added to the reaction solution, and the mixture was stirred for 10 minutes. Then, while slowly returning the temperature of the mixture to room temperature, the mixture was stirred for 2.5 hours. Diethyl ether and water were added to the reaction solution, and the mixture was partitioned. An aqueous 1 N hydrochloric acid solution (55 mL) was added to the aqueous layer, and the aqueous layer was further saturated with sodium chloride, and then extracted with diethyl ether. The organic layers were combined and dried over anhydrous sodium sulfate. After separation by filtration, the solvent was evaporated under reduced pressure, and 4-(2-pyrazinyl)-2, 4-dioxobutanoic acid methyl ester (10.0 g, 96%) was obtained as a solid. To a suspension of a crude product of this butanoic acid methyl ester product (6.27 g) in methanol (150 mL), 3-chloro-6-hydrazinopyridazine (4.35 g) was added, and the mixture heated to reflux for 18.5 hours. Concentrated hydrochloric acid (0. 750 mL) was further added to the reaction solution, and the mixture was heated to reflux for 2 hours. After air cooling, ethyl acetate and a saturated aqueous solution of sodium hydrogen carbonate were added to the reaction solution, and the mixture was partitioned. The organic layer was dried over anhydrous sodium sulfate. After separation by filtration, the solvent was evaporated under reduced pressure, and methanol was added to the residue thus obtained. The precipitated solid was filtered, and 1-(6-chloro-3-pyridazinyl)-5-(2-pyrazinyl)-1H-pyrazole-3-carboxylic acid methyl ester (5.74 g, 60%) was obtained as a solid.
¹H-NMR(400MHz, CDCl₃)δ: 4. 02 (3H, s), 7.33(1H, s), 7.72(1H, d, J=9.3Hz), 8.16(1H, d, J=9.0Hz), 8.42(1H, dd, J=2.4, 1.5Hz), 8.57(1H, d, J=2.7Hz), 8.90(1H, d, J=1.5Hz).
ESI-MSm/z: 317[(M+H)⁺, ³⁵Cl], 319[(M+H)⁺, ³⁷Cl].

### 2) Title compound

Sodium methoxide (1.60 g) was added to a suspension of 1-(6-chloro-3-pyridazinyl)-5-(2-pyrazinyl)-1H-pyrazole-3-carboxylic acid methyl ester (6.25 g) in methanol (50 mL) and tetrahydrofuran (100 mL) at room temperature, and the resultant mixture was stirred for 8 hours. Further, an aqueous 1 N sodium hydroxide solution (40.0 mL) was added to the reaction solution, and the mixture was stirred for 19 hours. Diethyl ether and water were added to the reaction solution, and the mixture was partitioned. The aqueous layer was acidified with hydrochloric acid, and extracted with diethyl ether. The aqueous layer was further extracted with ethyl acetate, and the organic layers were combined and dried over anhydrous sodium sulfate. After separation by filtration, the solvent was evaporated under reduced pressure, and methanol and diethyl ether were added to the residue thus obtained. The precipitated solid was filtered, and the title compound (4.37 g, 74%) was obtained as a solid.
¹H-NMR(400MHz, DMSO-d₆)δ: 4.04(3H, s), 7.51(1H, d, J=9.3Hz), 7.55(1H, s), 8.06(1H, d, J=9.3Hz), 8.49(1H, dd, J=2.4, 1.5Hz), 8.62(1H, d, J=2.4Hz), 9.07(1H, d, J=1.5Hz).
ESI-MSm/z: 299(M+H)⁺.

[Reference Example 13] 1-(6-Methoxy-3-pyridyl)-5-(1H-pyrazol-3-yl)-1H-pyrazole-3-carboxylic acid

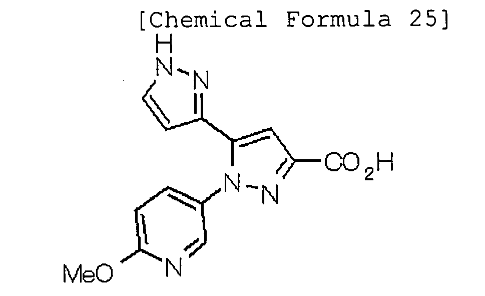

### 1) 1-{1-[(4-Methylphenyl)sulfonyl]-1H-pyrazol-3-yl}-1-ethanone

4-Methylbenzenesulfonyl chloride (5.72 g) was added to a solution of 1-(1H-pyrazol-5-yl)-1-ethanone hydrochloride (2.93 g) in pyridine (60 mL), and the resultant mixture was heated to reflux for 3.5 hours. After air cooling, the reaction solvent was evaporated under reduced pressure, ethyl acetate and water were added to the residue thus obtained, and the mixture was partitioned. The organic layer was washed with an aqueous 1 N hydrochloric acid solution, and then dried over anhydrous sodium sulfate. After separation by filtration, a residue obtained by evaporating the solvent under reduced pressure was solidified from methanol, diethyl ether and hexane, and thus 1-{1-[(4-methylphenyl)sulfonyl]-1H-pyrazol-3-yl}-1-ethanone (1.89 g, 35%) was obtained. Further, a residue obtained by evaporating the filtrate solvent under reduced pressure was solidified from methanol, diethyl ether and hexane, and thus 1-{1-[(4-methylphenyl)sulfonyl]-1H-pyrazol-3-yl}-1-ethanone (2.09 g, 39%) was obtained.
¹H-NMR(400MHz, CDCl₃)δ: 2.45(3H, s), 2.57(3H, s), 6.83(1H, d, J=2.7Hz), 7.36-7.38(2H, m), 7.92-7.96(2H, m), 8.10(1H, d, J=2.9Hz).
ESI-MSm/z: 265(M+H)⁺.

### 2) 1-(6-Methoxy-3-pyridyl)-5-{1-[(4-methylphenyl)sulfonyl]-1H-pyrazol-3-yl}-1H-pyrazole-3-carboxylic acid ethyl ester

Under cooling to -78°C, lithium bis(trimethylsilyl)amide (a 1.0 M solution in tetrahydrofuran, 16.5 mL) was added to a suspension of 1-{1-[(4-methylphenyl)sulfonyl]-1H-pyrazol-3-yl}-1-ethanone (3.97 g) in tetrahydrofuran (15 mL), and the resultant mixture was stirred for 35 minutes. Diethyl oxalate (3.05 mL) was added to the reaction solution, and the mixture was stirred for 15 minutes. Then, while slowly returning the mixture to room temperature, the mixture was stirred for 3.5 hours. Diethyl ether and water were added to the reaction solution, and the mixture was partitioned. An aqueous 1 N hydrochloric acid solution was added to the aqueous layer to acidify the aqueous layer, which was then extracted with diethyl ether. The aqueous layer was further extracted with ethyl acetate, and the organic layers were combined and dried over anhydrous sodium sulfate. After separation by filtration, the solvent was evaporated under reduced pressure, and dichloromethane was added to the residue thus obtained. The precipitated solid was filtered, and then the filtrate solvent was evaporated under reduced pressure, to obtain 4-{1-[(4-methylphenyl)sulfonyl]-1H-pyrazol-3-yl}-2, 4-dioxobutanoic acid ethyl ester (5.08 g, 92%) as an oily product. A solution of this butanoic acid ethyl ester product (5.08 g) and 5-hydrazino-2-methoxypyridine (1.93 g) of Reference Example 2 in ethanol (70 mL) was heated to reflux for 14.5 hours. After air cooling, ethyl acetate and a saturated aqueous solution of sodium hydrogen carbonate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(6-methoxy-3-pyridyl)-5-{1-[(4-methylphenyl)sulfonyl]-1H-pyrazol-3-yl}-1H-pyrazole-3-carboxylic acid ethyl ester (2.58 g, 39%) as an amorphous form.
¹H-NMR(400MHz, CDCl₃)δ: 1.41(3H, t, J=7.1Hz), 2.45(3H, s), 4.01(3H, s), 4.44(2H, q, J=7.1Hz), 6.24(1H, d, J=2.7Hz), 6.73(1H, d, J=8.8Hz), 7.23(1H, s), 7.31(2H, d, J=8.5Hz), 7.58(1H, dd, J=8.8, 2.7Hz), 7.76(2H, d, J=8.3Hz), 8.04(1H,
d, J=2.7Hz), 8.15(1H, d, J=2.7Hz).
ESI-MSm/z: 468(M+H)⁺.

### 3) Title compound

The title compound (1.33 g, 84%) was obtained as a solid by the same method as that in Reference Example 11-(2), using 1-(6-methoxy-3-pyridyl)-5-{1-[(4-methylphenyl)sulfonyl]-1H-pyrazol-3-yl}-1H-pyrazole-3-carboxylic acid ethyl ester (2.58 g).
¹H-NMR(400MHz, DMSO-d₆)δ: 3.92(3H, s), 6.29(1H, br s), 6.94(1H, d, J=8.8Hz), 7.14(1H, s), 7.75(1H, d, J=2.2Hz), 7.80(1H, dd, J=8.8, 2.7Hz), 8.24-8.25(1H, m), 13.09(1H, br s).
ESI-MSm/z: 286(M+H)⁺.

[Reference Example 14] 1-(6-Methyl-3-pyridyl)-5-(1-methyl-1H-pyrrol-3-yl)-1H-pyrazole-3-carboxylic acid

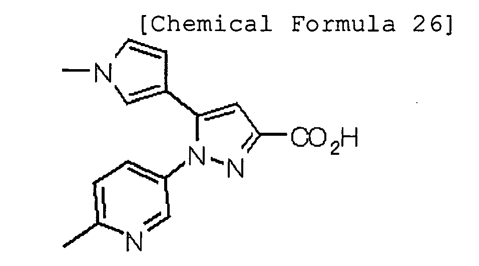

### 1) 4-(1-Methyl-1H-pyrrol-3-yl)-2, 4-dioxobutanoic acid methyl ester

4-(1-methyl-1H-pyrrol-3-yl)-2, 4-dioxobutanoic acid methyl ester (6.52 g, 76%) was obtained as a solid by the same method as that in Reference Example 8-(3), using 3-acetyl-1-methyl-1H-pyrrole (5.03 g), lithium bis(trimethylsilyl)amide (a 1.0 M solution in tetrahydrofuran, 45 mL) and dimethyl oxalate (9.53 g).
¹H-NMR(400MHz, CDCl₃)δ: 3.72(3H, s), 3.91(3H, s), 6.60-6.66(2H, m), 6.70(1H, s), 7.37(1H, s like).
FAB-MSm/z: 210(M+H)⁺.

### 2) 1-(6-Methyl-3-pyridyl)-5-(1-methyl-1H-pyrrol-3-yl)-1H-pyrazole-3-carboxylic acid methyl ester

A solution of 4-(1-methyl-1H-pyrrol-3-yl)-2, 4-dioxobutanoic acid methyl ester (3.00 g) and 5-hydrazino-2-methyl pyridine (2.00 g) of Reference Example 7 in methanol (80 mL) was heated to reflux for 20 minutes. After air cooling, acetic acid (3.3 mL) was added to the reaction solution, and the mixture was heated to reflux for 14 hours. After air cooling, the reaction solvent was evaporated under reduced pressure, chloroform and a saturated aqueous sodium bicarbonate solution were added to the residue thus obtained, and the mixture was partitioned. The organic layer was washed with saturated saline, and then was dried over anhydrous sodium sulfate. After separation by filtration, a residue obtained by evaporating the solvent under reduced pressure was purified by silica gel column chromatography (acetone-chloroform), to obtain 1-(6-methyl-3-pyridyl)-5-(1-methyl-1H-pyrrol-3-yl)-1H-pyrazole-3-carboxylic acid methyl ester (2.96 g, 70%) as a solid.
¹H-NMR(400MHz, CDCl₃)δ: 2.62(3H, s), 3.58(3H, s), 3.94 (3H, s), 5.85-5.92(1H, m), 6.41-6.46(1H, m), 6.48-6.53(1H, m), 6.91(1H, s like), 7.23(1H, d, J=8.1Hz), 7.66-7.74(1H, m), 8.53-8.60(1H, m).
FAB-MSm/z: 297(M+H)⁺.

### 3) Title compound

To a suspension of 1-(6-methyl-3-pyridyl)-5-(1-methyl-1H-pyrrol-3-yl)-1H-pyrazole-3-carboxylic acid methyl ester (2.96 g) in methanol (25 mL) and water (15 mL), lithium hydroxide monohydrate (0.475 g) was added at room temperature, and the resultant mixture was stirred for 1.5 hours. A residue obtained by evaporating the reaction solvent under reduced pressure was neutralized with an aqueous 1 N hydrochloric acid solution, and the precipitated solid was filtered to obtain the title compound (1.32 g, 47%).
¹H-NMR(400MHz, DMSO-d₆)δ: 2.56(3H, s), 3.55(3H, s), 5.72-5.76(1H, m), 6.65-6.76(2H, m), 6.87-6.90(1H, m), 7.37-7.44(1H, m), 7.76-7.81(1H, m), 8.44-8.50(1H, m).
ESI-MSm/z: 283(M+H)⁺.

[Reference Example 15] 1-(6-Methyl-3-pyridazinyl)-5-(5-methyl-2-pyridyl)-1H-pyrazole-3-carboxylic acid

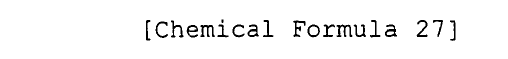

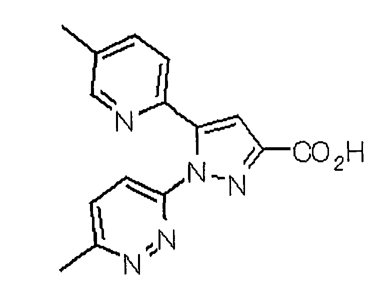

### 1) 3-Hydrazino-6-methylpyridazine

Hydrazine monohydrate (45 mL) was added to a suspension of 3-chloro-6-methylpyridine (3.00 g) in ethanol (45 mL), and the resultant mixture was heated to reflux for 2.5 hours. After air cooling, a residue obtained by evaporating the reaction solvent was purified by silica gel chromatography (a lower layer mixed solvent of chloroform-methanol-water (7:3:1)), to obtain 3-hydrazino-6-methylpyridazine (2.35 g, 81%) as a solid.
¹H-NMR(400MHz, DMSO-d₆)δ: 2.39(3H, s), 4.20(2H, br), 6.94(1H, d, J=9.3Hz), 7.18(1H, d, J=9.3Hz), 7.64(1H, br).
ESI-MSm/z: 125(M+H)⁺.

### 2) 1-(5-Methyl-2-pyridyl)-1-ethanone

n-Butyllithium (a 1.58 M solution in hexane, 24 mL) was added dropwise to a solution of 2-bromo-5-picoline (5.0 g) in diethyl ether (100 mL) over 5 minutes under cooling to -78°C, and then the resultant mixture was stirred for 5 minutes. N, N-dimethylacetamide (3.5 mL) was added dropwise to the reaction solution, and then the mixture was stirred for 2 hours. Water and ethyl acetate were added to the reaction solution, and the mixture was partitioned. The organic layer was dried over anhydrous magnesium sulfate. After separation by filtration, a residue obtained by evaporating the solvent under reduced pressure was purified by silica gel column chromatography (hexane-ethyl acetate), to obtain 1-(5-methyl-2-pyridyl)-1-ethanone (3.43 g, 87%) as an oily product.
¹H-NMR(400MHz, CDCl₃)δ: 2.42(3H, s), 2.71(3H, s), 7.62(1H, dd, J=1.59, 7.93Hz), 7.94(1H, d, J=7.93Hz), 8.54(1H, s).

### 3) 4-(5-Methyl-2-pyridyl)-2, 4-dioxobutanoic acid ethyl ester

Diethyl oxalate (7 mL) was added dropwise to a solution of sodium ethoxide (3.5 g) in ethanol (60 mL) at room temperature, then a solution of 1-(5-methyl-2-pyridyl)-1-ethanone (3.43 g) in ethanol (40 mL) was added to the reaction solution, and the mixture was stirred for 2 hours. Water and diethyl ether were added to the reaction solution, and the mixture was partitioned. An aqueous 1 N hydrochloric acid solution was added to the aqueous layer to acidify the layer, and chloroform was further added thereto. The mixture was partitioned, and the organic layer was dried over anhydrous magnesium sulfate. After separation by filtration, the solvent was evaporated under reduced pressure, and 4-(5-methyl-2-pyridyl)-2, 4-dioxobutanoic acid ethyl ester (6.8 g) was obtained as a solid.
¹H-NMR(400MHz, CDCl₃)δ: 1.40(3H, t, J=7.08Hz), 2.47(3H, s), 4.39(2H, q, J=7.08Hz), 7.49(1H, br), 7.74(1H, dd, J=1.47, 8.06Hz), 8.08(1H, d, J=8.06Hz), 8.58(1H, d, J=0.73Hz).
EI-MSm/z: 236(M+H)⁺.

### 4) 1-(6-Methyl-3-pyridazinyl)-5-(5-methyl-2-pyridyl)-1H-pyrazole-3-carboxylic acid ethyl ester

To a solution of 4-(5-methyl-2-pyridyl)-2, 4-dioxobutanoic acid ethyl ester (3.54 g) and 3-hydrazino-6-methylpyridazine (1.87 g) of (1) above in ethanol (71 mL), acetic acid (4.31 mL) was added at room temperature, and the resultant mixture was heated to reflux for 15 hours. Further, concentrated hydrochloric acid (4.7 mL) was added to the reaction solution, and the mixture was heated to reflux for 3 hours. After air cooling, saturated sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-ethyl acetate), to obtain 1-(6-methyl-3-pyridazinyl)-5-(5-methyl-2-pyridyl)-1H-pyrazole-3-carboxylic acid ethyl ester (1.13 g, 23%) as a solid.
¹H-NMR(400MHz, CDCl₃)δ: 1.41-1.44(3H, m), 2.32(3H, s), 2.72(3H, s), 4.43-4.48(2H, m), 7.18(1H, s), 7.46-7.56(3H,
m), 7.98(1H, d, J=8.8Hz), 8.21(1H, m).
EI-MSm/z: 323(M⁺).

### 5) Title compound

The title compound (0.759 g, 74%) was obtained as a solid by the same method as that in Reference Example 4, Method (B)-(4), using 1-(6-methyl-3-pyridazinyl)-5-(5-methyl-2-pyridyl)-1H-pyrazole-3-carboxylic acid ethyl ester (1.12 g).
¹H-NMR(400MHz, DMSO-d₆)δ: 2.27 (3H, s), 2. 67 (3H, s), 7.32(1H, s), 7.64-7.69(2H, m), 7.81(1H, d, J=8.8Hz), 7.94(1H, d, J=8.8Hz), 8.15-8.16(1H, m), 13.16(1H, s).
EI-MSm/z: 295(M⁺).

[Reference Example 16] 1-(6-Methoxy-3-pyridyl)-5-(4-pyrimidinyl)-1H-pyrazole-3-carboxylic acid

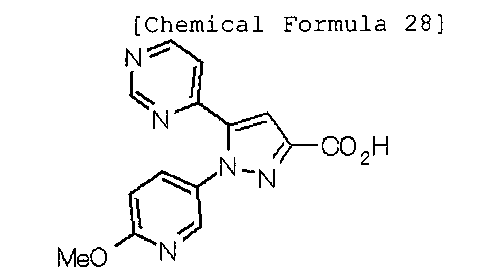

### 1) 4-Acetyl-2-methylthiopyrimidine

A mixture of 3, 3-dimethylbutan-2-one (25.15 g) and N, N-dimethylformamide dimethylacetal (126 mL) was stirred at an external temperature of 100°C for 48 hours. After air cooling, the low boiling point components generated during the reaction were evaporated under reduced pressure, and methanol (400 mL), thiourea (28.92 g) and sodium methoxide (15.39 g) were added to the residue thus obtained. The mixture was heated to reflux for 118 hours. After air cooling, sodium methoxide (10.26 g) was added to the reaction solution, methyl iodide (17.8 mL) was added dropwise to the mixture over 5 minutes under ice cooling, and the mixture was stirred for 5 hours. Water and ethyl acetate were added to a residue obtained by evaporating the reaction solvent under reduced pressure, and the mixture was partitioned. The organic layer was washed with water and saturated saline, and then dried over anhydrous sodium sulfate. After separation by filtration, the solvent was evaporated under reduced pressure, and an aqueous 3 N hydrochloric acid solution (400 mL) was added to the residue thus obtained and stirred for 15 hours at room temperature. Ethyl acetate was added to the reaction solution and the mixture was partitioned, and the organic layer was dried over anhydrous sodium sulfate. After separation by filtration, a residue obtained by evaporating the solvent under reduced pressure was purified by silica gel column chromatography (ethyl acetate-hexane), to obtain 4-acetyl-2-methylthiopyrimidine (26.34 g, 82%) as a solid.
¹H-NMR(400MHz, CDCl₃)δ: 2.63(3H, s), 2.70(3H, s), 7.51(1H, d, J=4.9Hz), 8.74(1H, d, J=4.9Hz).
ESI-MSm/z: 169(M+H)⁺.

### 2) 4-(2-Methylthio-4-pyrimidinyl)-2, 4-dioxobutanoic acid methyl ester

Under an argon atmosphere, 4-(2-methylthio-4-pyrimidinyl)-2, 4-dioxobutanoic acid methyl ester (294 mg, 98%) was obtained as a solid by the same method as that in Reference Example 8-(3), using 4-acetyl-2-methylthiopyrimidine (197 mg), lithium bis(trimethylsilyl)amide (a 1.0 M solution in tetrahydrofuran, 1.40 mL) and dimethyl oxalate (276 mg).
ESI-MSm/z: 255(M+H)⁺.

### 3) 1-(6-Methoxy-3-pyridyl)-5-(2-methylthio-4-pyrimidinyl)-1H-pyrazole-3-carboxylic acid methyl ester

1-(6-Methoxy-3-pyridyl)-5-(2-methylthio-4-pyrimidinyl)-1H-pyrazole-3-carboxylic acid methyl ester (204 mg, 49%) was obtained as a solid by the same method as that in Reference Example 14-(2), using 4-(2-methylthio-4-pyrimidinyl)-2, 4-dioxobutanoic acid methyl ester (294 mg) and 5-hydrazino-2-methoxypyridine (161 mg) of Reference Example 2.
¹H-NMR(400MHz, CDCl₃)δ: 2.14(3H, s), 3.98(3H, s), 3.99(3H, s), 6.83(1H, d, J=8.8Hz), 7.03(1H, d, J=5.1Hz), 7.45(1H, s), 7.63(1H, dd, J=8.8, 2.7Hz), 8.16(1H, d, J=2.7Hz), 8.52(1H, d, J=5.1Hz).
ESI-MSm/z: 358(M+H)⁺.

### 4) 1-(6-Methoxy-3-pyridyl)-5-(4-pyrimidinyl)-1H-pyrazole-3-carboxylic acid methyl ester

Raney nickel (an excessive amount, an activated catalyst washed with water and methanol was used) was added to a solution of 1-(6-methoxy-3-pyridyl)-5-(2-methylthio-4-pyrimidinyl)-1H-pyrazole-3-carboxylic acid methyl ester (198 mg) in methanol (25 mL), and the resultant mixture was stirred in a sealed tube at an external temperature of 120°C for 16 hours. After air cooling, chloroform was added to the reaction solution, and the insoluble matter was filtered. A residue obtained by evaporating the solvent of the filtrate under reduced pressure was purified by silica gel column chromatography (methanol-chloroform), to obtain 1-(6-methoxy-3-pyridyl)-5-(4-pyrimidinyl)-1H-pyrazole-3-carboxylic acid methyl ester (123 mg, 71%) as a solid.
¹H-NMR(400MHz, CDCl₃)δ: 3.98(3H, s), 3.99(3H, s), 6.82(1H, d, J=8.8Hz), 7.34(1H, d, J=5.1Hz), 7.48(1H, s), 7.67(1H, dd, J=8.8, 2.7Hz), 8.14(1H, d, J=2.7Hz), 8.75(1H, d, J=5.1Hz), 9.11(1H, s).
ESI-MSm/z: 312(M+H)⁺.

### 5) Title compound

The title compound (100 mg, 86%) was obtained as a solid by the same method as that in Reference Example 4, Method (B)-(4), using 1-(6-methoxy-3-pyridyl)-5-(4-pyrimidinyl)-1H-pyrazole-3-carboxylic acid methyl ester (122 mg).
¹H-NMR(400MHz, CDCl₃)δ: 4.00(3H, s), 6.84(1H, d, J=8.8Hz), 7.37(1H, dd, J=5.4, 1.2Hz), 7.51(1H, s), 7.67(1H, dd, J=8.8, 2.7Hz), 8.16(1H, d, J=2.7Hz), 8.78(1H, d, J=5.4Hz), 9.12(1H, d, J=1.2Hz).
ESI-MSm/z: 298(M+H)⁺.

[Reference Example 17] 4-Methoxypiperidine trifluoroacetate

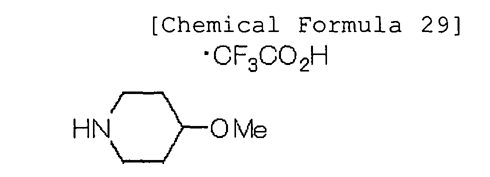

### 1) 4-Methoxypiperidine-1-carboxylic acid tert-butyl ester

Under an argon atmosphere, a solution of 4-hydroxypiperidine-1-carboxylic acid tert-butyl ester (2.00 g) in N, N-dimethylformamide (20 mL) was added dropwise to a suspension of sodium hydride (60%, 0.477 g) in N, N-dimethylformamide (20 mL) at room temperature, and the resultant mixture was stirred for 15 minutes. Methyl iodide (0.742 mL) was added dropwise to the reaction solution, and the mixture was stirred for 2 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 4-methoxypiperidine-1-carboxylic acid tert-butyl ester (1.43 g, 67%) as an oily product.
¹H-NMR(400MHz, CDCl₃)δ: 1.39-1.54(2H, m), 1.46(9H, s), 1.81-1.84(2H, m), 3.05-3.12(2H, m), 3.31-3.39(1H, m), 3.35(3H, s), 3.74-3.77(2H, m).

### 2) Title compound

To a solution of 4-methoxypiperidine-1-carboxylic acid tert-butyl ester (1.42 g) of the above in dichloromethane (28 mL), trifluoroacetic acid (14 mL) was added at room temperature, and the resultant mixture was stirred for 2.5 hours. The solvent of the reaction solution was evaporated under reduced pressure, and the title compound (2.65 g, quantitative) was obtained as an oily product.
¹H-NMR(400MHz, CDCl₃)δ: 1.98-2.02(4H, m), 3.19-3.23(2H, m), 3.30-3.42(2H, m), 3.37(3H, s), 3.54-3.60(1H, m).

[Reference Example 18] 4, 4-Difluoropiperidine hydrochloride

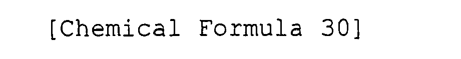

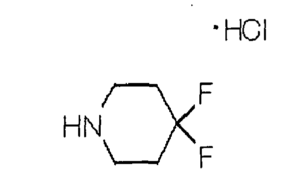

### 1) 1-Benzyl-4, 4-difluoropiperidine

Under an argon atmosphere, diethylaminosulfur trifluoride (8.38 mL) was added dropwise to a solution of 1-benzyl-4-piperidone (5.00 g) in benzene (200 mL) at 0°C, and the resultant mixture was stirred for 30 minutes, and then heated to reflux for 18 hours. Under cooling to 0°C, a saturated aqueous solution of sodium hydrogen carbonate and ethyl acetate were added, and the mixture was partitioned. The organic layer was dried over anhydrous sodium sulfate. After separation by filtration, a residue obtained by evaporating the solvent under reduced pressure was purified by silica gel column chromatography (hexane-ethyl acetate), to obtain 1-benzyl-4, 4-difluoropiperidine (4.67 g, 84%) as an oily product.
¹H-NMR(400MHz, CDCl₃)δ: 1.93-2.04(4H, m), 2.53-2.55(4H, m), 3.54(2H, s), 7.24-7.34(5H, m).
EI-MSm/z: 211(M⁺).

### 2) Title compound

Under an argon atmosphere, 1-chloroethyl chloroformate (2.62 mL) was added dropwise to a solution of 1-benzyl-4, 4-difluoropiperidine (4.66 g) of the above in dichloromethane (93 mL) at 0°C, and then the resultant mixture was heated to reflux for 2 hours. After air cooling, the reaction solvent was evaporated under reduced pressure, and a solution of the residue thus obtained in methanol (93 mL) was heated to reflux for 4 hours. After air cooling, the reaction solvent was evaporated under reduced pressure, diethyl ether was added to the residue thus obtained, and the precipitated solid was filtered, to obtain the title compound (3.03 g, 87%).
¹H-NMR(400MHz, D₂O)δ: 2.31-2.41(4H, M), 3. 43-3.46 (4H, m). FAB-MSm/z: 122(M+H)⁺.

[Reference Example 19] 4-Fluoropiperidine hydrochloride

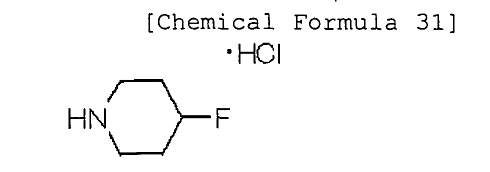

### 1) 4-Fluoropiperidine-1-carboxylic acid tert-butyl ester

Under an argon atmosphere, [bis(2-methoxyethyl)amino]sulfur trifluoride (7.33 mL) was added dropwise to a solution of 4-hydroxypiperidine-1-carboxylic acid tert-butyl ester (4.00 g) in dichloromethane (80 mL) Under cooling to -78°C, and the resultant mixture was stirred for 30 minutes, then stirred for 30 minutes at 0°C, and further stirred for 2 hours at room temperature. A saturated aqueous solution of sodium hydrogen carbonate and chloroform were to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-ethyl acetate), to obtain 4-fluoropiperidine-1-carboxylic acid tert-butyl ester (1.77 g, 44%) as an oily product.
¹H-NMR(400MHz, CDCl₃)δ: 1.45(9H, s), 1.76-1.86(4H, m), 3.41-3.54(4H, m), 4.70-4.87(1H, m).
EI-MSm/z: 203(M⁺).

### 2) Title compound

A 4 N hydrochloric acid-dioxane solution (12 mL) was added to a solution of 4-fluoropiperidine-1-carboxylic acid tert-butyl ester (1.74 g) of the above in dichloromethane (35 mL) at room temperature, and the resultant mixture was stirred for 40 minutes. Diethyl ether was added to a residue obtained by evaporating the solvent of the reaction solution under reduced pressure, and the precipitated solids was filtered to obtain the title compound (0.870 g, 73%).
¹H-NMR(400MHz, DMSO-d₆)δ: 1.92-2.13(4H, m), 3.01-3.12(4H, m), 4.83-4.97(1H, m).
FAB-MSm/z: 104(M+H)⁺.

[Reference Example 20] Hexahydropyridazine hydrochloride

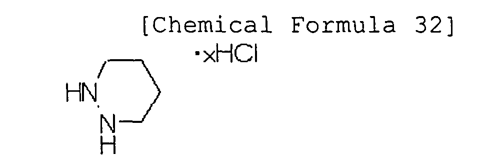

### 1) 3, 6-Dihydropyridazine-1, 2-dicarboxylic acid=dibenzyl ester

1, 3-Butadiene (14.2 g) was bubbled into a solution of 1, 2-azodicarboxylic acid=dibenzyl ester (10.28 g) in benzene (50 mL) under cooling to -10°C, and then the resultant mixture was stirred for 16 hours at room temperature. A residue obtained by evaporating the reaction solvent under reduced pressure was purified by silica gel column chromatography (ethyl acetate-hexane), to obtain 3, 6-dihydropyridazine-1, 2-dicarboxylic acid=dibenzyl ester (2.57 g, 21%) as an oily product.
¹H-NMR(400MHz, CDCl₃)δ: 3.70-3.85(2H, br), 4.35-4.52(2H, br), 5.05-5.25(4H, br), 5.78(2H, br), 7.03-7.40(10H, m).
FAB-MSm/z: 353(M+H)⁺.

### 2) Hexahydropyridazine

To a solution of 3, 6-dihydropyridazine-1, 2-dicarboxylic acid=dibenzyl ester (2.57 g) of the above in methanol (25 mL), 10% palladium-carbon (0.754 g) was added, and the resultant mixture was stirred for 19 hours in the presence of hydrogen. The catalyst was filtered from the reaction solution, and then the solvent of the filtrate was evaporated under reduced pressure, to obtain hexahydropyridazine (0.629 g, quantitative) as an oily product.
¹H-NMR(400MHz, DMSO-d₆)δ: 1.67-1.75(2H, m), 1.96-2.05(2H, m), 2.60-3.10(4H, m).
ESI-MSm/z: 87(M+H)⁺.

### 3) Hexahydropyridazine-1-carboxylic acid tert-butyl ester

### Method (A)

Di-tert-butoxydicarbonate (2.10 g) was added to a solution of hexahydropyridazine (0.72 g) in methanol (20 mL) at room temperature, and the resultant mixture was stirred for 15 hours. A residue obtained by evaporating the reaction solvent under reduced pressure was purified by silica gel column chromatography (acetone-chloroform), to obtain hexahydropyridazine-1-carboxylic acid tert-butyl ester (0.671 g, 43%) as an oily product.
¹H-NMR(400MHz, CDCl₃)δ: 1.48(9H, s), 1.50-1.73(4H, m), 2.87(2H, t like, J=4.5Hz), 3.51(2H, t like, J=4.5Hz), 4.65(1H, br).

### Method (B)

To a solution of 2-(tert-butoxycarbonyl)hexahydropyridazine-1-carboxylic acid benzyl ester (Bioorg. Med. Chem., 2002, 10, 953; 26.94 g) in methanol (250 mL), 10% palladium-carbon (50% wet, 5.21 g) was added, and the resultant mixture was stirred for 4 hours under a hydrogen atmosphere. After separation by filtration, a residue obtained by evaporating the solvent of the filtrate under reduced pressure was purified by silica gel column chromatography (chloroform-methanol), to obtain hexahydropyridazine-1-carboxylic acid tert-butyl ester (18.24 g, 85%) as an oily product.
¹H-NMR(400MHz, CDCl3)δ: 1.48(9H, s), 1.50-1.73(4H, m), 2.87(2H, t like, J=4.5Hz), 3.51(2H, t like, J=4.5Hz).

### 4) Title compound

A 4 N hydrochloric acid-dioxane solution (4 mL) was added to a solution of hexahydropyridazine-1-carboxylic acid tert-butyl ester (0.671 g) of the above in dichloromethane (8 mL) at room temperature, and the resultant mixture was stirred for 1 hour. Diethyl ether and pentane were added to the reaction solution, and the supernatant was removed by decantation. The remaining fraction was dried under reduced pressure, thus to obtain the title compound (0.292 g, 66%) as a solid.
¹H-NMR(400MHz, DMSO-d₆)δ: 1.66(2H, br), 2.50(2H, br), 2.98(2H, br), 4.35(4H, br).
ESI-MSm/z: 87(M+H)⁺.

[Reference Example 21] 1-Methylpiperazin-2-one hydrochloride

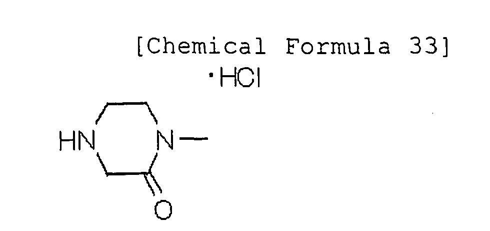

### 1) 3-Oxopiperazine-1-carboxylic acid tert-butyl ester

Triethylamine (3.83 mL) and di-tert-butoxydicarbonate (6.32 mL) were added to a mixed solution of piperazin-2-one (2.5 g) in tetrahydrofuran (50 mL) and methanol (50 mL) at room temperature, and the resultant mixture was stirred for 4 hours. The reaction solvent was evaporated under reduced pressure, water and ethyl acetate were added to the residue thus obtained, and the mixture was partitioned. The organic layer was washed with water and saturated saline in this order, and then the washing water layer was combined for further extraction with ethyl acetate. The organic layers were combined and dried over anhydrous magnesium sulfate. After separation by filtration, a residue obtained by evaporating the solvent under reduced pressure was solidified using ethyl acetate-hexane, thus to obtain 3-oxopiperazine-1-carboxylic acid tert-butyl ester (3.6 g, 72%).
¹H-NMR(400MHz, CDCl₃)δ: 1.48(9H, s), 3.37-3.40(2H, m), 3.62-3.65(2H, m), 4.01(2H, s), 6.32(1H, br s).

### 2) 4-Methyl-3-oxopiperazine-1-carboxylic acid tert-butyl ester

Sodium hydride (60%, 960 mg) was added to a solution of 3-oxopiperazine-1-carboxylic acid tert-butyl ester (3.0 g) of the above in N, N-dimethylformamide (50 mL) at 0°C, and then methyl iodide (2.33 mL) was added to the reaction solution, which was then stirred for 15 hours at room temperature. Water and ethyl acetate were added to the reaction solution, and the mixture was partitioned. The organic layer was washed with water and saturated saline in this order, and then the washing water layers were combined for further extraction with ethyl acetate. The organic layers were combined and dried over anhydrous magnesium sulfate. After separation by filtration, the solvent was evaporated under reduced pressure, and thus 4-methyl-3-oxopiperazine-1-carboxylic acid tert-butyl ester (2.32 g, 72%) was obtained as an oily product.
¹H-NMR(400MHz, CDCl₃)δ: 1.47(9H, s), 3.01(3H, s), 3.34(2H, t, J=5.6Hz), 3.65(2H, t, J=5.6Hz), 4.07(2H, s).

### 3) Title compound

A 4 N hydrochloric acid-dioxane solution (20 mL) was added to 4-methyl-3-oxopiperazine-1-carboxylic acid tert-butyl ester (2.06 g) obtained above, and the resultant mixture was stirred for 1 hour at room temperature. The reaction solvent was evaporated under reduced pressure, and toluene was added to the residue thus obtained. Then, the solvent was azeotropically evaporated under reduced pressure, and the residue thus obtained was dried, to obtain the title compound (1.44 g, 99%) as an oily product.
¹H-NMR(400MHz, DMSO-d₆)δ: 2.86(3H, s), 3.34(2H, br m), 3.50(2H, m), 3.64(2H, s).
MS(ESI)m/z: 115(M+H)⁺.

### [Reference Example 22] 1-Methylpiperazin-2-one trifluoroacetate

Trifluoroacetic acid (3 mL) was added to a solution of 4-methyl-3-oxopiperazine-1-carboxylic acid tert-butyl ester (0. 308 g) of Reference Example 21-(2) in dichloromethane (6 mL) at room temperature, and the resultant mixture was stirred for 1.5 hours. A residue obtained by evaporating the reaction solvent under reduced pressure was dried to obtain the title compound (0.485 g, quantitative).
¹H-NMR(400MHz, CDCl₃-CD₃OD(15: 1))δ: 2.98(3H, s), 3.39(2H, t-like, J=6.1Hz), 3.54(2H, t-like, J=6.1Hz), 3.72(2H, s). MS(EI)m/z: 114(M⁺).

[Reference Example 23] 4-Methoxypiperidine hydrochloride

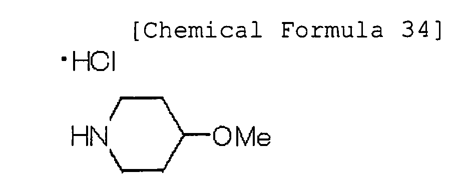

A 4 N hydrochloric acid-dioxane solution (10 mL) was added to a solution of 4-methoxypiperidine-1-carboxylic acid tert-butyl ester (5.34 g) of Reference Example 17-(1) in 1, 4-dioxane (10 mL) at room temperature, and the resultant mixture was stirred for 30 minutes. Further, a 4 N hydrochloric acid-dioxane solution (20 mL) was added to the mixture, which was then stirred for 30 minutes. The reaction solvent was evaporated under reduced pressure, and the solid thus obtained was filtered with ethyl acetate, to obtain the title compound (3.55 g).
¹H-NMR(400MHz, DMSO-d₆)δ: 1.68(2H, m), 1.93(2H, m), 2.91(2H, m), 3.08(2H, m), 3.23(3H, s), 3.42(1H, q, J=3.90Hz).

[Reference Example 24] (3S)-3-Fluoropyrrolidine hydrochloride

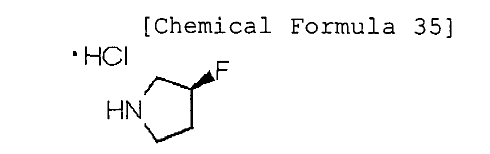

### 1) (3S)-3-Fluoropyrrolidine-1-carboxylic acid tert-butyl ester

Diethylaminosulfur trifluoride (2.22 mL) was added to a solution of (3R)-3-hydroxypyrrolidine-1-carboxylic acid tert-butyl ester (2.62 g) in dichloromethane (50 mL) at - 78°C, and the resultant mixture was stirred for 75 minutes at room temperature. The reaction solution was poured into ice water and partitioned. The organic layer was dried over anhydrous magnesium sulfate. After separation by filtration, a residue obtained by evaporating the solvent under reduced pressure was purified by silica gel column chromatography (hexane-ethyl acetate), to obtain (3S)-3-fluoropyrrolidine-1-carboxylic acid tert-butyl ester (676 mg, 26%) as an oily product.
¹H-NMR(400MHz, CDCl₃)δ: 1.45(9H, s), 2.17-2.26(1H, m), 3.52-3.68(5H, m), 5.20(1H, dt, J=52.7, 3.4Hz).

### 2) Title compound

A 4 N-hydrochloric acid-dioxane solution (5 mL) was added to a solution of (3S)-3-fluoropyrrolidine-1-carboxylic acid tert-butyl ester (600 mg) of the above in dichloromethane (10 mL) at room temperature, and the resultant mixture was stirred for 75 minutes. Diethyl ether was added to the reaction solution, and the precipitated solid was filtered, to obtain the title compound (341 mg, 86%).
¹H-NMR(400MHz, DMSO-d₆)δ: 2.00-2.26(2H, m), 3.15-3.56(4H, m), 5.43(1H, dt, J=52.9, 3.8Hz), 9.83(2H, br s).

[Reference Example 25] 4-Fluoromethylpiperidine hydrochloride

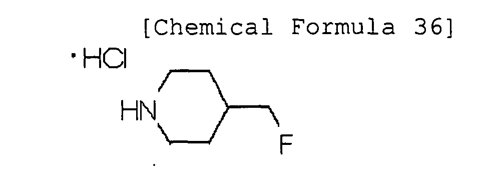

### 1) 4-Fluoromethylpiperidine-1-carboxylic acid tert-butyl ester

To a solution of 4-hydroxymethylpiperidine-1-carboxylic acid tert-butyl ester (1.17 g) in dichloromethane (8 mL), [bis(2-methoxyethyl)amino]sulfur trifluoride (1.2 mL) and [bis(2-methoxyethyl)amino]sulfur trifluoride (a 50% solution in tetrahydrofuran, 3 mL) were added dropwise under ice cooling, and the resultant mixture was stirred for 17 hours at room temperature. Water, a saturated aqueous solution of sodium hydrogen carbonate, and ethyl acetate were added to the reaction solution, and the mixture partitioned. The organic layer was dried over anhydrous magnesium sulfate. After separation by filtration, a residue obtained by evaporating the solvent under reduced pressure was purified by silica gel column chromatography (hexane-ethyl acetate), to obtain 4-fluoromethylpiperidine-1-carboxylic acid tert-butyl ester (597 mg, 51%) as a solid.
¹H-NMR(400MHz, CDCl₃)δ: 1.22(2H, m), 1.46(9H, s), 1.70(2H, d, J=12.94Hz), 1.83(1H, m), 2.71(2H, br), 4.13(2H, br), 4.21(1H, d, J=6.10Hz),
4.32(1H, d, J=6.10Hz).

### 2) Title compound

The title compound (526 mg, quantitative) was obtained as a solid by the same method as that in Reference Example 24, using 4-fluoromethylpiperidine-1-carboxylic acid tert-butyl ester (635 mg).
¹H-NMR(400MHz, CDCl₃)δ: 1.76(3H, m), 1.96(2H, d, J=13.4Hz), 2.90(2H, br), 3.54(2H, d, J=12.1Hz), 4.26(1H, d, J=6.10Hz), 4.37(1H, d, J=6.10Hz).

[Reference Example 26] (3R)-3-Fluoropiperidine hydrochloride

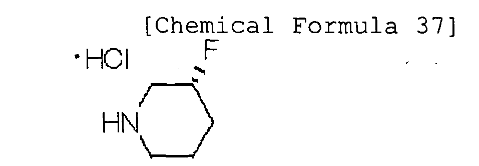

### [Method A]

(3R)-3-Fluoropiperidine-1-carboxylic acid tert-butyl ester (346 mg) was obtained as an oily product by the same method as that in Reference Example 24-(1), using (2S)-2-hydroxymethylpyrrolidine-1-carboxylic acid tert-butyl ester (3.0 g) and diethylaminosulfur trifluoride (2.95 mL). This ester product was dissolved in dichloromethane (20 mL), 4N hydrochloric acid-dioxane (7 mL) was added thereto at room temperature, and the mixture was stirred for 30 minutes. Diethyl ether was added to the reaction solution, and the precipitated solid was filtered, to obtain the title compound (162 mg, 8% from two processes) as a solid.
¹H-NMR(400MHz, DMSO-d₆)δ: 1.65-1.93(4H, m), 3.03-3.20(4H, m), 4.97(1H, dd, J=45.7, 2.4Hz), 9.34(2H, br s).

### [Method B]

Diethylaminosulfur trifluoride (20.6 mL) was added to a solution of (2S)-1-benzyl-2-hydroxymethylpyrrolidine (19.89 g) in dichloromethane (300 mL) at 0°C, and the resultant mixture was stirred for 90 minutes at room temperature. The reaction solution was poured into a saturated aqueous solution of sodium hydrogen carbonate and extracted with dichloromethane. The organic layer was dried over anhydrous magnesium sulfate. After separation by filtration, a residue obtained by evaporating the solvent under reduced pressure was purified by silica gel column chromatography (ethyl acetate-hexane), to obtain a mixture of (2S)-1-benzyl-2-fluoromethylpyrrolidine and (3R)-1-benzyl-3-fluoropiperidine (14.56 g, 73%) as an oily product. A solution of this mixture (7.25 g) and chloroformic acid 1-chloroethyl ester (4.50 mL) in dichloromethane (100 mL) was heated to reflux for 1.5 hours. After air cooling, a residue obtained by evaporating the reaction solvent under reduced pressure was dissolved in methanol (50 mL), and then the solution was heated to reflux for 75 minutes. After air cooling, a residue obtained by evaporating the reaction solvent under reduced pressure was crystallized from methanol-diethyl ether, to obtain the title compound (1.58 g, 30%).
¹H-NMR(400MHz, DMSO-d₆)δ: 1.72-1.81(4H, m), 2.91-3.33(4H, m), 4.98(1H, d, J=46.1Hz), 9.33(2H, s).

[Reference Example 27] (2S)-2-Fluoromethylpyrrolidine hydrochloride

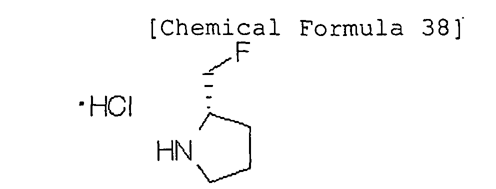

### 1) (2S)-1-Benzoyl-2-hydroxymethylpyrrolidine

To a mixed solution of (2S)-2-hydroxymethylpyrrolidine (3.00 mL) and benzoyl chloride (6.92 mL) in dichloromethane (100 mL) and water (100 mL), sodium hydrogen carbonate (7. 51 g) was added at room temperature, and the resultant mixture was stirred for 1.5 hours. Dichloromethane was added to the reaction solution, and the mixture was partitioned. The organic layer was dried over anhydrous sodium sulfate. After separation by filtration, a residue obtained by evaporating the solvent under reduced pressure was dissolved in tetrahydrofuran (120 mL) and water (60 mL), then lithium hydroxide dihydrate (6. 25 g) was added to the reaction solution at room temperature, and the mixture was stirred overnight. The reaction solution was partitioned, and the organic layer was dried over anhydrous sodium sulfate. After separation by filtration, the solvent was evaporated under reduced pressure, and thus (2S)-1-benzoyl-2-hydroxymethylpyrrolidine (5.79 g, 98%) was obtained as an oily product.
¹H-NMR(400MHz, CDCl₃)δ: 1.60-2.27(4H, m), 3.43-3.52(2H, m), 3.71-3.82(2H, m), 4.40(1H, d, J=7.3Hz), 4.92(1H, s), 7.40-7.52(5H, m).

### 2) (2S)-1-Benzoyl-2-fluoromethylpyrrolidine

Diethylaminosulfur trifluoride (1.93 mL) was added to a solution of (2S)-1-benzoyl-2-hydroxymethylpyrrolidine (1.50 g) in dichloromethane (50 mL) under ice cooling, and the resultant mixture was stirred overnight. A saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (hexane-ethyl acetate), to obtain (2S)-1-benzoyl-2-fluoromethylpyrrolidine (772 mg, 51%) as an oily product. ¹H-NMR(400MHz, CDCl₃)δ: 1.74-2.18(4H, m), 3.49(2H, br s), 4.44-4.90(3H, m), 7.38-7.54(5H, m).

### 3) (2S)-1-Benzyl-2-fluoromethylpyrrolidine

Under a nitrogen atmosphere, lithium aluminum hydride (128 mg) was added to a solution of (2S)-1-benzoyl-2-fluoromethylpyrrolidine (350 mg) in tetrahydrofuran (20 mL) at room temperature, and the resultant mixture was heated to reflux for 1 hour. After air cooling, ice was added to the reaction solution, which was then treated with an excess of lithium aluminum hydride. Subsequently, diethyl ether and water were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain (2S)-1-benzyl-2-fluoromethylpyrrolidine (142 mg, 44%) as an oily product.
¹H-NMR(400MHz, CDCl₃)δ: 1.62-1.76(3H, m), 1.88-1.97(1H, m), 2.25-2.31(1H, m), 2.84-2.97(2H, m), 3.48(1H, d, J=12.9Hz), 4.04(1H, d, J=13.2Hz), 4.21-4.42(2H, m), 7.22-7.34(5H, m).

### 4) Title compound

1-Chloroethyl chloroformate (289 µL) was added to a solution of (2S)-1-benzyl-2-fluoromethylpyrrolidine (466 mg) in dichloromethane (20 mL) at room temperature, and the resultant mixture was heated to reflux for 1 hour. After air cooling, a residue obtained by evaporating the reaction solvent under reduced pressure was dissolved in methanol (20 mL), and the solution was heated to reflux for 1 hour. After air cooling, the reaction solvent was evaporated under reduced pressure, and a solid thus obtained was filtered with diethyl ether, to obtain the title compound (292 mg, 87%).
¹H-NMR(400MHz, DMSO-d₆)δ: 1.56-2.06(4H, m), 3.15(2H, t, J=7.2Hz), 3.75-3.85(1H, m), 4.55-4.76(2H, m), 9.66(2H, s).

[Reference Example 28] 3-Methoxypiperidine hydrochloride

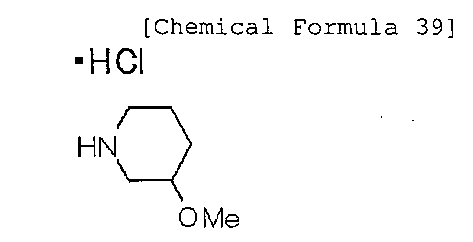

### 1) 3-Hydroxypiperidine-1-carboxylic acid tert-butyl ester

A solution of triethylamine (15.2 mL) and di-tert-butoxydicarbonate (11.9 g) in methanol (50 mL) was added to a solution of 3-hydroxypiperidine (5.00 g) in methanol (50 mL) at room temperature, and the resultant mixture was stirred for 15 hours. A residue obtained by evaporating the solvent of the reaction solution under reduced pressure was purified by silica gel column chromatography (ethyl acetate-chloroform), to obtain 3-hydroxypiperidine-1-carboxylic acid tert-butyl ester (9.86 g, 99%) as a solid.
¹H-NMR(400MHz, CDCl₃)δ: 1.36-1.55(2H, m), 1.45(9H, s), 1.71-1.78(1H, m), 1.88(1H, m), 3.02-3.13(2H, m), 3.52(1H, m), 3.72-3.76(2H, m).
EI-MSm/z: 201(M⁺).

### 2) 3-Methoxypiperidine-1-carboxylic acid tert-butyl ester

3-Methoxypiperidine-1-carboxylic acid tert-butyl ester (9.24 g, 88%) was obtained as an oily product by the same method as that in Reference Example 17-(1), using 3-hydroxypiperidine-1-carboxylic acid tert-butyl ester (9.86 g) and methyl iodide (3. 66 mL).
¹H-NMR(400MHz, CDCl₃)δ: 1.30-1.54(2H, m), 1.46(9H, s), 1.72-1.73(1H, m), 1.92(1H, m), 3.04-3.21(3H, m), 3.37(3H, s), 3.55-3.74(2H, m).
EI-MSm/z: 215(M⁺).

### 3) Title compound

The title compound (6.36 g, 98%) was obtained as a solid by the same method as that in Reference Example 19-(2), using 3-methoxypiperidine-1-carboxylic acid tert-butyl ester (9.24 g).
¹H-NMR(400MHz, DMSO-d₆)δ: 1.58-1.61(2H, m), 1.76-1.81(2H, m), 2.88-2.94(3H, m), 3.09-3.13(1H, m), 3.28(3H, s), 3.55-3.51(1H, m).
EI-MSm/z: 115(M⁺).

[Reference Example 29] (2S, 5R)-2-Methoxymethyl-5-methylpyrrolidine hydrochloride

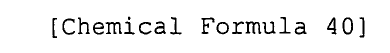

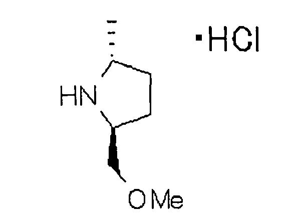

### 1) (2S, 5S)-2-(4-Methylphenyl)sulfonyloxymethyl-5-methoxypyrrolidine-1-carboxylic acid tert-butyl ester

Under ice cooling, sodium hydride (60% in oil, 568 mg) was added to a solution of (2S, 5S)-2-benzyloxymethyl-5-hydroxymethylpyrrolidine-1-carboxylic acid tert-butyl ester (3.04 g, S. Takano, et al., Tetrahedron Lett., 1989, 30, 3805-3806) and methyl iodide (1 mL) in N, N-dimethylformamide (30 mL), and the resultant mixture was stirred for 30 minutes at room temperature. Water and diethyl ether were added to the reaction solution, and the mixture was partitioned. The organic layer was washed with water and saturated saline, and dried over anhydrous sodium sulfate. After separation by filtration, a residue obtained by evaporating the solvent under reduced pressure was purified by silica gel column chromatography (ethyl acetate-hexane), to obtain (2S, 5S)-2-benzyloxymethyl-5-methoxymethylpyrrolidine-1-carboxylic acid tert-butyl ester (2.68 g, 85%) as an oily product. To a solution of this 5-methoxymethylpyrrolidine product (2.68 g) in methanol (50 mL), 10% palladium-carbon (50% wet, 1.50 g) was added, and the mixture was stirred overnight at room temperature under a hydrogen atmosphere. The reaction solution was filtered, and then the solvent of the filtrate was evaporated under reduced pressure, to obtain (2S, 5S)-2-hydroxymethyl-5-methoxymethylpyrrolidine-1-carboxylic acid tert-butyl ester (1.92 g, 98%) as an oily product. To a solution of this 2-hydroxymethylpyrrolidine product (1.44 g) and p-toluenesulfonyl chloride (1.34 g) in dichloromethane (50 mL), pyridine (5 mL) was added at room temperature, and the mixture was stirred overnight. 1 N Hydrochloric acid and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (methanol-chloroform), to obtain (2S, 5S)-2-(4-methylphenyl)sulfonyloxymethyl-5-methoxypyrrolidine-1-carboxylic acid tert-butyl ester (1.64 g, 70%) as an oily product.
¹H-NMR(400MHz, CDCl₃)δ: 1.35 and 1.43(9H, s), 1.80-2.10(4H, m), 2.44 and 2.45(3H, s), 3.23-3.52(2H, m), 3.31(3H, s), 3.83-4.23(4H, m), 7.34(2H, t, J=9.0Hz), 7.78(2H, t, J=4.0Hz).

### 2) Title compound

Under a nitrogen atmosphere, sodium borohydride (55% in oil, 313 mg) was added to a solution of (2S, 5S)-2-(4-methylphenyl)sulfonyloxymethyl-5-methoxypyrrolidine-1-carboxylic acid tert-butyl ester (1.601 g) in dimethylsulfoxide (30 mL) at room temperature, and the resultant mixture was stirred for 1 hours at 85°C. After air cooling, water and diethyl ether were added to the reaction solution, and the mixture was partitioned. The organic layer was washed with water and saturated saline, and then the organic layer was dried over anhydrous sodium sulfate. After separation by filtration, a residue obtained by evaporating the solvent under reduced pressure was dissolved in dichloromethane (30 mL), 4 N hydrochloric acid-dioxane (10 mL) was added thereto at room temperature, and the mixture was stirred for 1.5 hours. The reaction solvent was evaporated under reduced pressure, and thus the title compound (216 mg, 33%) was obtained as a solid.
ESI-MSm/z: 130(M+H)⁺.

[Reference Example 30] 1-(6-Methoxy-3-pyridyl)-5-(1H-pyrrol-3-yl)-1H-pyrazole-3-carboxylic acid

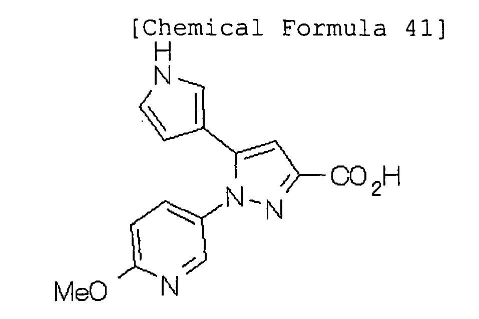

4-[1-(Phenylsulfonyl)-1H-pyrrol-3-yl]-2, 4-dioxobutanoic acid ethyl ester (12. 4 g) was obtained as a solid by the same method as that in Reference Example 8-(3), using 1-[1-(phenylsulfonyl)-1H-pyrrol-3-yl]-1-ethanone (10.7 g) and diethyl oxalate (8.60 mL), and lithium bis(trimethylsilyl)amide (a 1.0 M solution in tetrahydrofuran, 46. 3 mL). To a solution of this butanoic acid ethyl ester product (4.00 g) in ethanol (50 mL), 3-hydrazino-2-methoxypyridine (1.90 g) of Reference Example 2 and acetic acid (3.91 mL) were added at room temperature, and the mixture was heated to reflux overnight. Further, concentrated hydrochloric acid (1.00 mL) was added to the reaction solution, and the mixture was heated to reflux for 6 days. After air cooling, the reaction solvent was evaporated under reduced pressure, a saturated aqueous solution of sodium hydrogen carbonate, water and ethyl acetate were added to a residue thus obtained, and the mixture was partitioned. The organic layer was dried over anhydrous sodium sulfate. After separation by filtration, the solvent was evaporated under reduced pressure, ethanol (50 mL) and sodium hydroxide (816 mg) were added at room temperature to a residue thus obtained, and the mixture was stirred for 3 hours. Water (20 mL) was added to the reaction solution, and the mixture was stirred overnight. Ethyl acetate was added to a residue obtained by evaporating the reaction solvent under reduced pressure, and the mixture was partitioned. The organic layer was dried over anhydrous sodium sulfate. After separation by filtration, a residue obtained by evaporating the solvent under reduce pressure was purified by silica gel column chromatography (dichloromethane-ethyl acetate), to obtain 1-(6-methoxy-3-pyridinyl)-5-(1H-pyrrol-3-yl)-1H-pyrazol 3-carboxylic acid ethyl ester (210 mg, 5%).
¹H-NMR(400MHz, CDCl₃)δ: 1. 41-1.44 (3H, t, J=7.1Hz), 3. 98 (3H, s), 4.45(2H, q, J=7.1Hz), 6.04-6.09(1H, m), 6.57-6.60(1H, m), 6.73-6.76(1H, m), 6.80(1H, d, J=8.8Hz), 6.95(1H, d, J=0.7Hz), 7.66(1H, ddd, J=8.8, 2.7, 0.7Hz), 8.25(1H, d, J=2.7Hz), 8.34(1H, br s).
FAB-MSm/z: 313(M+H)⁺.

Furthermore, a precipitated solid from the aqueous layer during the partition operation was filtered to obtain the title compound (1.60, 41%).
¹H-NMR(400MHz, DMSO-d₆)δ: 3.89(3H, d, J=1.5Hz), 5.82(1H, d, J=1.5Hz), 6.53(1H, d, J=1.5Hz), 6.61(1H, s), 6.66-6.73(1H, m), 6.88(1H, dd, J=8.8, 1.0Hz), 7.70(1H, ddd, J=8.7, 1.5, 1.5Hz), 8.16-8.20(1H, m), 11.14(1H, s).
FAB-MSm/z: 285(M+H)⁺.

[Reference Example 31] 1-(6-Methoxy-3-pyridyl)-5-(1-methyl-1H-pyrazol-3-yl)-1H-pyrazole-3-carboxylic acid

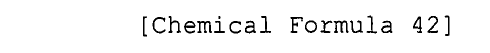

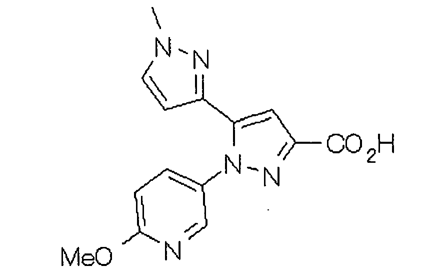

### 1) 1-Methyl-1H-pyrazole-3-carboxylic acid N-methoxy-N-methylamide

1-Hydroxybenzotriazole (2.37 g), 1-(3-dimethylaminopropyl)-3-ethylcarbodiimide hydrochloride (6:79 g), triethylamine (13.4 mL), and N, O-dimethylhydroxylamine hydrochloride (2.93 g) were added to a solution of 1-methyl-1H-pyrazole-3-carboxylic acid (2.025 g) in dichloromethane (80 mL) at room temperature, and the resultant mixture was stirred for 22 hours. Water and chloroform were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (acetone-chloroform), to obtain 1-methyl-1H-pyrazole-3-carboxylic acid N-methoxy-N-methylamide (2.78 g, quantitative) as an oily product.
¹H-NMR(400MHz, CDCl₃)δ: 3.43(3H, br s), 3.75(3H, s), 3.97(3H, s), 6.76(1H, d, J=2.4Hz), 7.36(1H, d, J=2.4Hz).
FAB-MSm/z: 170(M+H)⁺.

### 2) 3-Acetyl-1-methyl-1H-pyrazole

3-Acetyl-1-methyl-1H-pyrazole (2.03 g, quantitative) was obtained as an oily product by the same method as that in Reference Example 8-(2), using 1-methyl-1H-pyrazole-3-carboxylic acid N-methoxy-N-methylamide (2.78 g) and methyllithium (a 0.98 M solution in diethyl ether, 23 mL).
¹H-NMR(400MHz, CDCl₃)δ: 2.57(3H, s), 3.97(3H, s), 6.77(1H, d, J=2.4Hz), 7.37(1H, d, J=2.4Hz).
EI-MSm/z: 124(M⁺).

### 3) 4-(1-Methyl-1H-pyrazol-3-yl)-2, 4-dioxobutanoic acid methyl ester

Under cooling to -78°C, lithium bis(trimethylsilyl)amide (a 1.0 M solution in tetrahydrofuran, 20 mL) was added dropwise to a solution of 3-acetyl-1-methyl-1H-pyrazole (2.03 g) in tetrahydrofuran (50 mL), and the resultant mixture was stirred for 1 hour. A solution of dimethyl oxalate (3.90 g) in tetrahydrofuran (15 mL) was added dropwise to the reaction solution, and then the mixture was stirred for 2 hours at 0°C. Water and diethyl ether were added to the reaction solution, and the mixture was partitioned. The aqueous layer was acidified (pH 3) using an aqueous 1 N hydrochloric acid solution, and then extracted with chloroform. The organic layer was washed with saturated saline, and then dried over anhydrous sodium sulfate. After separation by filtration, the solvent was evaporated under reduced pressure, and 4-(1-methyl-1H-pyrazol-3-yl)-2, 4-dioxobutanoic acid methyl ester (0.975 g, 28%) was obtained as a solid.
¹H-NMR(400MHz, CDCl₃)δ: 3.85(3H, s), 3.92(3H, s), 6.88(1H, br), 7.24(1H, br), 7.43(1H, br).EI-MSm/z: 210(M⁺).

### 4) 1-(6-Methoxy-3-pyridyl)-5-(1-methyl-1H-pyrazol-3-yl)-1H-pyrazole-3-carboxylic acid methyl ester

A solution of 4-(1-methyl-1H-pyrazol-3-yl)-2, 4-dioxobutanoic acid methyl ester (0.975 g) and 5-hydrazino-2-methoxypyridine (0.885 g) of Reference Example 2 in methanol (30 mL) was heated to reflux for 40 minutes. After air cooling, acetic acid (1.06 mL) was added to the reaction solution, and the mixture was heated to reflux for 15 hours. After air cooling, the reaction solvent was evaporated under reduced pressure, chloroform and a saturated aqueous solution of sodium hydrogen carbonate were added to a residue thus obtained, and the mixture was partitioned. Furthermore, the aqueous layer was extracted with chloroform-methanol (10: 1), and the organic layers were combined, washed with saturated saline, and then dried over anhydrous sodium sulfate. After separation by filtration, a residue obtained by evaporating the solvent under reduced pressure was purified by silica gel column chromatography (acetone-chloroform), to obtain 1-(6-methoxy-3-pyridyl)-5-(1-methyl-1H-pyrazol-3-yl)-1H-pyrazole-3-carboxylic acid methyl ester (0.665 g, 46%) as a solid.
¹H-NMR(400MHz, CDCl₃)δ: 3.88(3H, s), 3.96(3H, s), 3.97(3H, s), 5.93(1H, d like, J=2.2Hz), 6.78(1H, d, J=8.5Hz), 7.20(1H, s like), 7.28-7.30(1H, m), 7.65-7.72(1H, m), 8.21(1H, d, J=2.5Hz).
FAB-MSm/z: 314(M+H)⁺.

### 5) Title compound

To a mixed solution of 1-(6-methoxy-3-pyridyl)-5-(1-methyl-1H-pyrazol-3-yl)-1H-pyrazole-3-carboxylic acid methyl ester (0.665 g) in tetrahydrofuran (8 mL), methanol (4 mL) and water (4 mL), lithium hydroxide monohydrate (0.0995 g) was added at room temperature, and the resultant mixture was stirred for 2 hours. The reaction solvent was evaporated under reduced pressure, and a residue thus obtained was acidified (pH 4) by adding an aqueous 1 N hydrochloric acid solution. The precipitated solid was filtered, and thus the title compound (0.348 g, 55%) was obtained.
¹H-NMR(400MHz, DMSO-d₆)δ: 3.78 (3H, s), 3. 91 (3H, s), 6.07-6.13(1H, m), 6.94(1H, d, J=8.8Hz), 7.07(1H, s like), 7.69(1H, s like), 7.76-7.82(1H, m), 8.21-8.25(1H, m).
FAB-MSm/z: 300(M+H)⁺.

[Reference Example 32] 1-(6-Methoxy-3-pyridyl)-5-(1-methyl-1H-pyrrol-3-yl)-1H-pyrazole-3-carboxylic acid

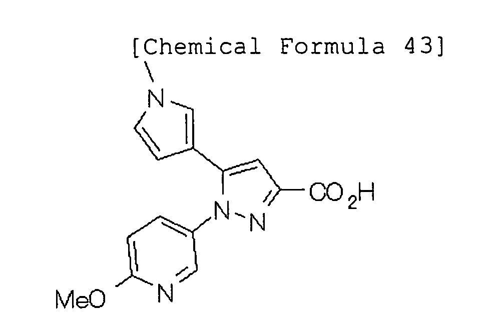

### 1) 1-(6-Methoxy-3-pyridyl)-5-(1-methyl-1H-pyrrol-3-yl)-1H-pyrazole-3-carboxylic acid ethyl ester

Lithium bis(trimethylsilyl)amide (a 1.0 M solution in tetrahydrofuran, 11.1 mL) was added dropwise to a solution of 3-acetyl 1-methylpyrrole (1.2 mL) in tetrahydrofuran (10 mL) under cooling to -78°C, and the resultant mixture was stirred for 30 minutes. Diethyl oxalate (2.06 mL) was added dropwise to the reaction solution, and then the mixture was stirred for 1 hour at room temperature. 3-Methoxy-6-hydrazinopyridine (2.50 g) of Reference Example 2, acetic acid (0.6 mL) and ethanol (50 mL) were added to the reaction solution, and the mixture was heated to reflux for 18 hours. After air cooling, a saturated aqueous solution of sodium hydrogen carbonate, water and ethyl acetate were added to the reaction solution, and the mixture was partitioned. The organic layer was dried over anhydrous magnesium sulfate. After separation by filtration, a residue obtained by evaporating the solvent under reduced pressure was purified by silica gel column chromatography (hexane-ethyl acetate), to obtain 1-(6-methoxy-3-pyridyl)-5-(1-methyl-1H-pyrrol-3-yl)-1H-pyrazole-3-carboxylic acid ethyl ester (2.45 g, 73%) as an oily product.
¹H-NMR(400MHz, DMSO-d₆)δ: 1.41(3H, t, J=7.20Hz), 3.59(3H, s), 3.98(3H, s), 4.43(2H, q, J=7.20Hz), 5.91(1H, dd, J=2.81, 1.83Hz), 6.41(1H, t, J=1.95Hz), 6.51(1H, t, J=2.32Hz), 6.79(1H, d, J=8.79Hz), 6.90(1H, s), 7.65(1H, dd, J=8.79, 2.69Hz), 8.25(1H, d, J=2.32Hz).

### 2) Title compound

An aqueous 1 N sodium hydroxide solution (9 mL) was added to a suspension of 1-(6-methoxy-3-pyridyl)-5-(1-methyl-1H-pyrrol-3-yl)-1H-pyrazole-3-carboxylic acid ethyl ester (2.45 g) in tetrahydrofuran (30 mL) at room temperature, and the resultant mixture was stirred for 16 hours. Furthermore, an aqueous 1 N sodium hydroxide solution (4 mL) was added to the reaction solution, and the mixture was stirred for 3.5 hours. Water and diethyl ether were added to the reaction solution, and the mixture was partitioned. The aqueous layer was acidified using hydrochloric acid, and extracted with chloroform. The organic layer was dried over anhydrous magnesium sulfate. After separation by filtration, the solvent was evaporated under reduced pressure, and the title compound (1.53 g, 68%) was obtained as a solid.
¹H-NMR(400MHz, DMSO-d₆)δ: 3.54(3H, s), 3.91(3H, s), 5.78(1H, dd, J=2.69, 1.83Hz), 6.68(1H, dt, J=8.67, 2.56Hz), 6.84(1H, s), 6.95(1H, d, J=8.79Hz), 7.77(1H, dd, J=8.89, 2.69Hz), 8.23(1H, d, J=2.69Hz), 12.81(1H, br).
EI-MSm/z: 299(M⁺).

[Reference Example 33] 1-(6-Methoxy-3-pyridazinyl)-5-(1H-pyrrol-3-yl)-1H-pyrazole-3-carboxylic acid

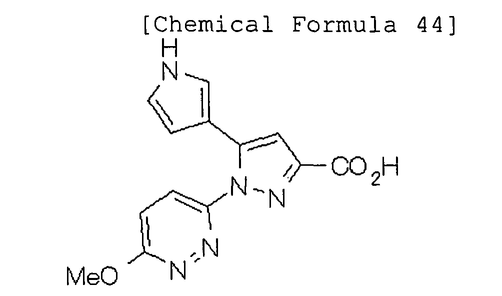

4-[1-(Phenylsulfonyl)-1H-pyrrol-3-yl]-2, 4-dioxobutanoic acid methyl ester (1.76 g) was obtained as a solid by the same method as that in Reference Example 31-(3), using 3-acetyl-1-phenylsulfonylpyrrole (2.23 g), dimethyl oxalate (1.60 g), and lithium bis(trimethylsilyl)amide (a 1.0 M solution in tetrahydrofuran, 9.85 mL).
A solution of this butanoic acid methyl ester product (1.76 g) and 3-chloro-6-hydrazinopyridazine (759 mg) in methanol (30 mL) was heated to reflux overnight. Concentrated hydrochloric acid (1.00 mL) was added to the reaction solution, and the mixture was further heated to reflux for 3 hours. After air cooling, the reaction solvent was evaporated under reduced pressure, a saturated aqueous solution of sodium hydrogen carbonate, water and ethyl acetate were added to a residue thus obtained, and the mixture was partitioned. The organic layers were combined, and dried over anhydrous sodium sulfate. After separation by filtration, a residue obtained by evaporating the solvent under reduced pressure was purified by silica gel column chromatography (diethyl ether-dichloromethane), to obtain a mixture of 1-(6-chloro-3-pyridazinyl)-5-(1H-pyrrol-3-yl)-1H-pyrazole-3-carboxylic acid methyl ester and 1-(6-methoxy-3-pyridazinyl)-5-(1H-pyrrol-3-yl)-1H-pyrazole-3-carboxylic acid (6-chloropyridazine product: 6-methoxypyridazine product = 8: 1, 1.85 g) as a solid.
To a mixed solution of this mixture (1.85 g) in methanol (30 mL) and tetrahydrofuran (50 mL), sodium methoxide (676 mg) was added at room temperature, and the mixture was stirred for 4 hours. Further, an aqueous 1 N sodium hydroxide solution (9.0 mL) was added to the reaction solution at room temperature, and the mixture was stirred overnight. An aqueous 1 N hydrochloric acid solution (22 mL) was added to the reaction solution, and methanol and tetrahydrofuran of the reaction solvent were evaporated under reduced pressure. The precipitated solid was filtered to obtain the title compound (1.20 g, 47%).
¹H-NMR(400MHz, DMSO-d₆)δ: 4.44(3H, s), 6.20-6.24(1H, m), 7.02-7.06(1H, m), 7.09-7.12(1H, m), 7.26(1H, d, J=0.7Hz), 7.82(1H, dd, J=9.2, 0.8Hz), 8.14(1H, dd, J=9.2, 0.7Hz), 11.36(1H, br s), 13.29(1H, br s).
FAB-MSm/z: 286(M+H)⁺.

[Reference Example 34] 1-(6-Methoxy-3-pyridyl)-5-(1-methyl-1H-imidazol-4-yl)-1H-pyrazole-3-carboxylic acid

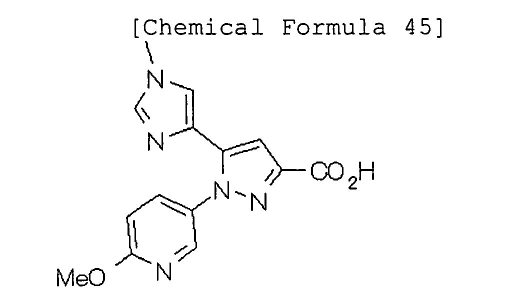

### 1) 1-Methyl-1H-imidazole-4-carboxylic acid N-methoxy-N-methylamide

1-Methyl-1H-imidazole-4-carboxylic acid N-methoxy-N-methylamide (1.08 g, 64%) was obtained as a solid by the same method as that in Reference Example 31-(1), using 1-methyl-1H-imidazole-4-carboxylic acid (1.26 g) and N, O-dimethylhydroxylamine hydrochloride (1.17 g).
¹H-NMR(400MHz, CDCl₃)δ: 3.45(3H, s), 3.73(3H, s), 3.78(3H, s), 7.45(1H, s), 7.54(1H, s).

### 2) 4-Acetyl-1-methyl-1H-imidazole

4-Acetyl-1-methyl-1H-imidazole (309 mg, 39%) was obtained as a solid by the same method as that in Reference Example 8-(2), using 1-methyl-1H-imidazole-4-carboxylic acid N-methoxy-N-methylamide (1.08 g) and methyllithium (a 0.98 M solution in diethyl ether, 6.84 mL).
¹H-NMR(400MHz, CDCl₃)δ: 2.56(3H, s), 3.75(3H, s), 7.44(1H, s), 7.56(1H, s).

### 3) 1-(6-Methoxy-3-pyridyl)-5-(1-methyl-1H-imidazol-4-yl)-1H-pyrazole-3-carboxylic acid ethyl ester

Under a nitrogen atmosphere, lithium bis(trimethylsilyl)amide (a 1.0 M solution in tetrahydrofuran, 2.71 mL) was added dropwise to a solution of 4-acetyl-1-methyl-1H-imidazole (306 mg) in tetrahydrofuran (25 mL) under cooling to -78°C, and then the resultant mixture was stirred for 30 minutes. Diethyl oxalate (502 µL) was added to the reaction solution, and the mixture was stirred for 105 minutes at room temperature. Ethanol (50 mL), 5-hydrazino-2-methoxypyridine (343 mg) of Reference Example 2, and 1 M hydrochloric acid-ethanol (2.71 mL) were added to the reaction solution, and the mixture was heated to reflux overnight. After air cooling, a saturated aqueous solution of sodium hydrogen carbonate and ethyl acetate were added to the reaction solution, and the mixture was partitioned. The organic layer was dried over anhydrous sodium sulfate. After separation by filtration, a residue obtained by evaporating the solvent under reduced pressure was purified by silica gel thin layer chromatography (ethyl acetate), to obtain 1-(6-methoxy-3-pyridyl)-5-(1-methyl-1H-imidazol-4-yl)-1H-pyrazole-3-carboxylic acid ethyl ester (302 mg, 37%) as an oily product.
¹H-NMR(400MHz, CDCl₃)δ: 1.41(3H, t, J=7.1Hz), 3.64(3H, s), 3.98(3H, s), 4.43(2H, q, J=7.1Hz), 6.55(1H, s), 6.81(1H, d, J=8.8Hz), 7.17(1H, s), 7.41(1H, s), 7.71(1H, dd, J=8.8, 2.7Hz), 8.24(1H, d, J=2.4Hz).
ESI-MSm/z: 328(M+H)⁺.

### 4) Title compound

Lithium hydroxide monohydrate (110 mg) was added to a solution of 1-(6-methoxy-3-pyridyl)-5-(1-methyl-1H-imidazol-4-yl)-1H-pyrazole-3-carboxylic acid ethyl ester (286 mg) in tetrahydrofuran (15 mL) and water (5 mL) at room temperature, and the resultant mixture was stirred overnight. An aqueous 1 N hydrochloric acid solution (2.63 mL) and a mixed solvent of chloroform-methanol (10: 1) was added to the reaction solution, and the mixture was partitioned. The organic layer was dried over anhydrous sodium sulfate. After separation by filtration, the solvent was evaporated under reduced pressure, and the title compound (184 mg, 70%) was obtained as a solid.
ESI-MSm/z: 300(M+H)⁺.

[Reference Example 35] 3-Hydrazino-6-methylpyridazine

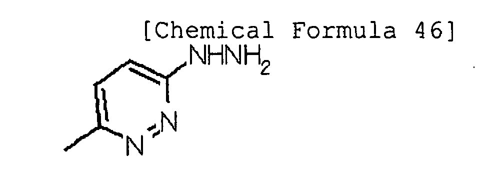

Hydrazine monohydrate (45 mL) was added to a suspension of 3-chloro-6-methylpyridazine (3.00 g) in ethanol (45 mL), and the resultant mixture was heated to reflux for 2.5 hours. After air cooling, a residue obtained by evaporating the solvent of the reaction solution under reduced pressure was purified by silica gel chromatography (chloroform-methanol-water (a lower layer solvent of 7: 3: 1 (v/v)), to obtain the title compound (2.35 g, 81%) as a solid.
¹H-NMR(400MHz, DMSO-d₆)δ: 2.39(3H, s), 4.20(2H, br), 6.94(1H, d, J=9.3Hz), 7.18(1H, d, J=9.3Hz), 7.64(1H, br).
ESI-MSm/z: 125(M+H)⁺.

[Reference Example 36] 1-(6-Methyl-3-pyridazinyl)-5-(1-methyl-1H-pyrrol-3-yl)-1H-pyrazole-3-carboxylic acid

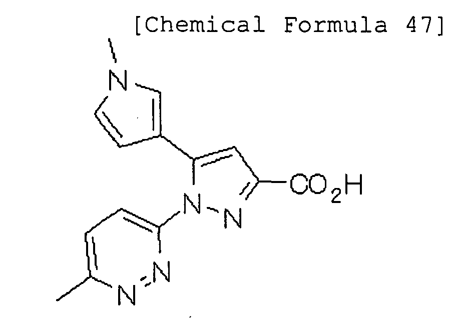

### 1) 1-(6-Methyl-3-pyridazinyl)-5-(1-methyl-1H-pyrrol-3-yl)-1H-pyrazole-3-carboxylic acid methyl ester

1-(6-Methyl-3-pyridazinyl)-5-(1-methyl-1H-pyrrol-3-yl)-1H-pyrazole-3-carboxylic acid methyl ester (4.14 g, 70%) was obtained as a solid by the same method as that in Reference Example 14-(2), using 4-(1-methyl-1H-pyrrol-3-yl)-2, 4-dioxobutanoic acid methyl ester (3.49 g) of Reference Example 14-(1) and 6-methyl-3-hydrazinopyridazine (2.30 g) of Reference Example 35.
¹H-NMR(400MHz, CDCl₃)δ: 2.79(3H, s), 3.62(3H, s), 3.96(3H, s), 6.08-6.13(1H, m), 6.53(1H, t, J=2.7Hz), 6.92-6.97(1H, m), 6.95(1H, s), 7.48(1H, d, J=8.8Hz), 7.76(1H, d, J=8.8Hz).
FAB-MSm/z: 298(M+H)⁺.

### 2) Title compound

The title compound (3.07 g, 78%) was obtained as a solid by the same method as that in Reference Example 14-(3), using 1-(6-methyl-3-pyridazinyl)-5-(1-methyl-1H-pyrrol-3-yl)-1H-pyrazole-3-carboxylic acid methyl ester (4.14 g) and lithium hydroxide monohydrate (0.656 g).
¹H-NMR(400MHz, DMSO-d₆)δ: 2.73(3H, s), 3.55(3H, s), 5.79-5.84(1H, m), 6.66(1H, t like, J=2.0Hz), 6.81(1H, t like, J=2.0Hz), 6.91(1H, s), 7.85(2H, s).
ESI-MSm/z: 284(M+H)⁺.

[Reference Example 37] 5-(5-Cyano-2-pyridyl)-1-(6-methyl-3-pyridyl)-1H-pyrazole-3-carboxylic acid

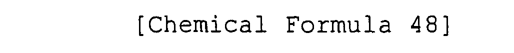

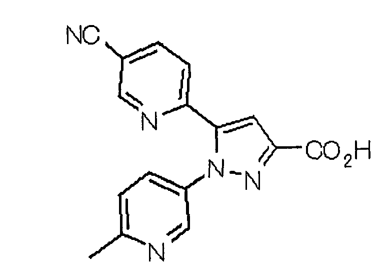

### 1) 5-Benzyloxy-2-methylpyridine

Benzyl bromide (10.9 mL) was added to a solution of 5-hydroxy-2-methylpyridine (10.0 g) and potassium carbonate (38.0 g) in acetonitrile (200 mL) at room temperature, and the resultant mixture was stirred for 12 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-benzyloxy-2-methylpyridine (4.14 g, 23%) as an oily product.
¹H-NMR(400MHz, CDCl₃)δ: 2.48(3H, s), 5.08(2H, s), 7.05(1H, d, J=8.5Hz), 7.16(1H, dd, J=8.5, 2.9Hz), 7.31-7.43(5H, m), 8.26(1H, d, J=2.9Hz).
EI-MSm/z: 199(M⁺).

### 2) 1-(5-Benzyloxy-2-pyridyl)-1-ethanone

Selenium dioxide (9.20 g) was added to a solution of 5-benzyloxy-2-methylpyridine (4.13 g) in pyridine (83 mL) at room temperature, and the resultant mixture was heated to reflux for 61 hours. After air cooling, water and chloroform were added to the reaction solution, and the mixture was partitioned. The organic layer was dried over anhydrous magnesium sulfate. After separation by filtration, the solvent was evaporated under reduced pressure, triethylamine (6.35 mL) was added to a solution of a residue thus obtained, N, O-dimethylhydroxylamine hydrochloride (2.22 g), 1-(3-dimethylaminopropyl)-3-ethylcarbodiimide hydrochloride (4.37 g) and 1-hydroxybenzotriazole (3.08 g) in N, N-dimethylformamide (95 mL), and the mixture was stirred for 61 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-benzyloxypyridine-2-carboxylic acid N-methoxy-N-methylamide (3.75 g, 66%) as an oily product. (FAB-MSm/z: 273(M+H)+.)
Under an argon atmosphere, methyllithium (a 1.10 M solution in diethyl ether, 13.7 mL) was added dropwise to a solution of 5-benzyloxypyridine-2-carboxylic acid N-methoxy-N-methylamide (3.74 g) in tetrahydrofuran (75 mL) at 0°C, and the resultant mixture was stirred for 40 minutes. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(5-benzyloxy-2-pyridyl)-1-ethanone (1.47 g, 47%) as an oily product.
¹H-NMR(400MHz, CDCl₃)δ: 2.67(3H, s), 5.18(2H, s), 7.30-7.45(6H, m), 8.03(1H, d, J=8.8Hz), 8.39(1H, d, J=2.7Hz).
EI-MSm/z: 227(M⁺).

### 3) 4-(5-Benzyloxy-2-pyridyl)-2, 4-dioxobutanoic acid ethyl ester

Under an argon atmosphere, a solution of diethyl oxalate (1.75 mL) and 1-(5-benzyloxy-2-pyridyl)-1-ethanone (1.46 g) of the above in ethanol (15 mL) was added to a solution of sodium ethoxide (0.874 g) in ethanol (15 mL), and the resultant mixture was stirred for 7 hours at room temperature, and then stirred for 1 hour at 60°C. After air cooling, sodium ethoxide (0.874 g) and diethyl oxalate (1.75 mL) were further added to the reaction solution, and the mixture was stirred for 1 hour at 60°C. After air cooling, water was added to the reaction solution, which was then washed with diethyl ether. A saturated aqueous solution of ammonium chloride and chloroform were added to the aqueous layer, and the mixture was partitioned. The organic layer was dried over anhydrous sodium sulfate. After separation by filtration, the solvent was evaporated under reduced pressure, and 4-(5-benzyloxy-2-pyridyl)-2, 4-dioxobutanoic acid ethyl ester (1.38 g, 66%) was obtained as a solid.
¹H-NMR(400MHz, CDCl₃)δ: 1.38-1.42(3H, m), 4.35-4.42(2H, m), 5.20(2H, s), 7.35-7.44(6H, m), 7.59(1H, s), 8.14(1H, d, J=8.8Hz), 8.44(1H, d, J=2.7Hz).
EI-MSm/z: 327(M⁺).

### 4) 5-(5-Benzyloxy-2-pyridyl)-1-(6-methyl-3-pyridyl)-1H-pyrazole-3-carboxylic acid ethyl ester

5-(5-Benzyloxy-2-pyridyl)-1-(6-methyl-3-pyridyl)-1H-pyrazole-3-carboxylic acid ethyl ester (1.74 g, 52%) was obtained as a solid by the same method as that in Reference Example 9-(4), using 4-(5-benzyloxy-2-pyridyl)-2, 4-dioxobutanoic acid ethyl ester (2.62 g) and 5-hydrazino-2-methylpyridine (0.986 g) of Reference Example 7.
¹H-NMR(400MHz, CDCl₃)δ: 1.42(3H, t, J=7.1Hz), 2.60(3H, s), 4.46(2H, q, J=7.1Hz), 5.10(2H, s), 7.19-7.42(9H, m), 7.72(1H, dd, J=8.3, 2.4Hz), 8.26(1H, d, J=2.9Hz), 8.38(1H, d, J=2.4Hz).FAB-MSm/z: 415(M+H)⁺.

### 5) 5-(5-Hydroxy-2-pyridyl)-1-(6-methyl-3-pyridyl)-1H-pyrazole-3-carboxylic acid ethyl ester

To a mixed solution of 5-(5-benzyloxy-2-pyridyl)-1-(6-methyl-3-pyridyl)-1H-pyrazole-3-carboxylic acid ethyl ester (1.73 g) in ethanol (34 mL) and ethyl acetate (34 mL), 10% palladium-carbon (1.73 g) was added, and the resultant mixture was stirred for 3.5 hours at room temperature in the presence of hydrogen. The reaction solution was separated by filtration, and the solvent of the filtrate thus obtained was evaporated under reduced pressure, to obtain 5-(5-hydroxy-2-pyridyl)-1-(6-methyl-3-pyridyl)-1H-pyrazole-3-carboxylic acid ethyl ester (1.28 g, 95%) as a solid.
¹H-NMR(400MHz, DMSO-d₆)δ: 1.33(3H, t, J=7.1Hz), 3.33(3H, s), 4.34(2H, q, J=7.1Hz), 7.18-7.25(2H, m), 7.34(1H, d, J=8.5Hz), 7.51(1H, d, J=8.5Hz), 7.64-7.67(1H, m), 7.97(1H, d, J=2.9Hz), 8.35(1H, d, J=2.4Hz), 10.31(1H, s).
FAB-MSm/z: 325(M+H)⁺.

### 6) Title compound

Under an argon atmosphere, trifluoromethanesulfonic anhydride (0.791 mL) was added dropwise to a mixed solution of 5-(5-hydroxy-2-pyridyl)-1-(6-methyl-3-pyridyl)-1H-pyrazole-3-carboxylic acid ethyl ester (1.27 g) in dichloromethane (25 mL) and pyridine (8.3 mL), and the resultant mixture was stirred for 1 hour. Furthermore, trifluoromethanesulfonic anhydride (0.791 mL) was added dropwise to the reaction solution, and the mixture was stirred or 1 hour. Water and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-chloroform), to obtain 1-(6-methyl-3-pyridyl)-5-(5-trifluoromethanesulfonyloxy-2-pyridyl)-1H-pyrazole-3-carboxylic acid ethyl ester (1.77 g, 99%) as a solid.
¹H-NMR(400MHz, CDCl₃)δ: 1.42-1.45(3H, m), 2.63(3H, s), 4.44-4.50(2H, m), 7.24(1H, d, J=8.3Hz), 7.34(1H, s), 7.55-7.57(1H, m), 7.65-7.70(2H, m), 8.43-8.45(2H, m).
FAB-MSm/z: 457(M+H)⁺.

Under an argon atmosphere, a suspension of tri-n-butyltin cyanide (4.88 g) and tetrakis(triphenylphosphine)palladium(0) (6.68 g) in dichloroethane (48 mL) was heated to reflux for 2 hours. A solution of 1-(6-methyl-3-pyridyl)-5-(5-trifluoromethanesulfonyloxy-2-pyridyl)-1H-pyrazole-3-carboxylic acid ethyl ester (1.76 g) in dichloroethane (39 mL) was added dropwise to the reaction solution, and the mixture was stirred for 23 hours at 80°C. After air cooling, a saturated aqueous solution of sodium hydrogen carbonate was added to the reaction solution, and the mixture was filtered using Celite. Water and chloroform were added to a filtrate thus obtained, and the mixture was partitioned. The organic layer was dried over anhydrous sodium sulfate. After separation by filtration, a residue obtained by evaporating the solvent under reduced pressure was purified by silica gel column chromatography (ethyl acetate-chloroform), to obtain 5-(5-cyano-2-pyridyl)-1-(6-methyl-3-pyridyl)-1H-pyrazole-3-carboxylic acid ethyl ester (2.07 g). To a suspension of the obtained ethyl ester product in tetrahydrofuran (41 mL), a solution of lithium hydroxide monohydrate (0.162 g) in water (21 mL) was added dropwise at room temperature, and then the mixture was stirred for 1.5 hours. Water and chloroform were added to the reaction solution, and the mixture was partitioned. An aqueous 1 N hydrochloric acid solution (3. 86 mL) was added to the aqueous layer, which was then neutralized. A precipitated solid was filtered, and the title compound (0.750 g, 64%) was obtained as a solid.
¹H-NMR(400MHz, DMSO-d₆)δ: 2.54(3H, s), 7.36(1H, d, J=8.3Hz), 7.57(1H, s), 7.71-7.73(1H, m), 7.99(1H, d, J=8.3Hz), 8.40-8.43(2H, m), 8.84(1H, d, J=1.2Hz), 13.20(1H, br s).
FAB-MSm/z: 306(M+H)⁺.

[Reference Example 38] 5-(3-Formyl-1H-pyrrol-1-yl)-1-(6-methoxy-3-pyridyl)-1H-pyrazole-3-carboxylic acid ethyl ester

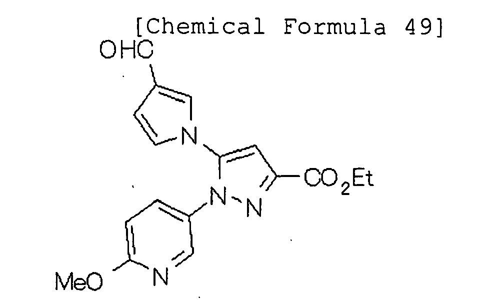

2, 5-Dimethoxytetrahydro-3-furan carbaldehyde (1.62 g) was added to a solution of 5-amino-1-(6-methoxy-3-pyridyl)-1H-pyrazole-3-carboxylic acid ethyl ester (1.77 g) obtained in Reference Example 11-(1) in acetic acid (35 mL), and the resultant mixture was heated to reflux for 14.5 hours. After air cooling, the reaction solvent was evaporated under reduced pressure, ethyl acetate and a saturated aqueous solution of sodium hydrogen carbonate were added to a residue thus obtained, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain the title compound (1.38 g, 60%) as a solid.
¹H-NMR(400MHz, CDCl₃)δ: 1.43(3H, t, J=7.2Hz), 3.94(3H, s), 4.47(2H, q, J=7.2Hz), 6.70-6.71(1H, m), 6.75-6.77(2H, m), 7.02(1H, s), 7.28(1H, dd, J=2.2, 1.5Hz), 7.48(1H, dd, J=8.8, 2.8Hz), 8.02(1H, dd, J=2.8, 0.6Hz), 9.79(1H, s).
ESI-MSm/z: 341(M+H)⁺.

[Reference Example 39] 1-(6-Methoxy-3-pyridazinyl)-5-(5-methyl-2-pyridyl)-1H-pyrazole-3-carboxylic acid

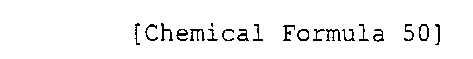

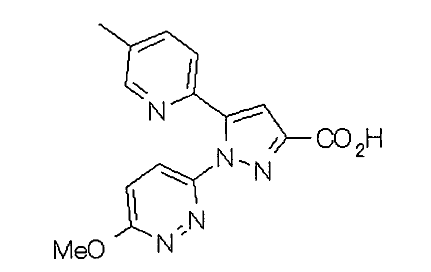

### 1) 4-(5-Methyl-2-pyridyl)-2, 4-dioxobutanoic acid methyl ester

Dimethyl oxalate (10.4 g) was added to a solution of sodium methoxide (4.74 g) in methanol (200 mL), and the resultant mixture was stirred for 5 minutes. 1-(5-Methyl-2-pyridyl)-1-ethanone (5.93 g) of Reference Example 15-(2) was added to the reaction solution at room temperature, and the mixture was stirred for 5 hours. Water and diethyl ether were added to the reaction solution, and the mixture was partitioned. The aqueous layer was acidified with an aqueous 1 N hydrochloric acid solution, and then extracted with chloroform. The organic layer was dried over anhydrous magnesium sulfate. After separation by filtration, the solvent was evaporated under reduced pressure, and 4-(5-methyl-2-pyridyl)-2, 4-dioxobutanoic acid methyl ester (7.31 g, 75%) was obtained as a solid.
¹H-NMR(400MHz, CDCl₃)δ: 2.46(3H, s), 3.92(3H, s), 7.58(1H, br), 7.70(1H, dd, J=8.06, 1.83Hz), 8.08(1H, d, J=8.06Hz),
8.54(1H, d, J=1.22Hz).

### 2) 1-(6-Methoxy-3-pyridazinyl)-5-(5-methyl-2-pyridyl)-1H-pyrazole-3-carboxylic acid ethyl ester

3-Chloro-6-hydrazinopyridazine (2.6 g) was added to a solution of 4-(5-methyl-2-pyridyl)-2, 4-dioxobutanoic acid methyl ester (3.34 g) in ethanol (200 mL), and the resultant mixture was heated to reflux for 1.5 hours. Concentrated hydrochloric acid (3 mL) was added to the reaction solution, and the mixture was further heated to reflux for 4 hours. After air cooling, a saturated aqueous solution of sodium hydrogen carbonate and ethyl acetate were added to the reaction solution, and the mixture was partitioned. The organic layer was dried over anhydrous magnesium sulfate. After separation by filtration, a residue obtained by evaporating the solvent under reduced pressure was purified by silica gel column chromatography (hexane-ethyl acetate), to obtain 1-(6-methoxy-3-pyridazinyl)-5-(5-methyl-2-pyridyl)-1H-pyrazole-3-carboxylic acid ethyl ester (4.08 g, 82%) as a solid.
¹H-NMR(400MHz, CDCl₃)δ: 1.43(3H, t, J=7.08Hz), 2.41(3H, s), 4.48(2H, q, J=7.08Hz), 7.07(1H, m), 7.20(1H, s), 7.47(1H, s), 7.69(1H, d, J=9.03Hz), 8.08(1H, d, J=9.03Hz), 8.23(1H, d, J=4.88Hz).
EI-MSm/z: 344(M+H)⁺.

### 3) Title compound

1-(6-Methoxy-3-pyridazinyl)-5-(5-methyl-2-pyridyl)-1H-pyrazole-3-carboxylic acid ethyl ester (4.08 g) was added to a solution of sodium methoxide (1.3 g) in methanol (100 mL) at room temperature, and the resultant mixture was stirred for 21 hours. Diethyl ether and water were added to the reaction solution, and the mixture was partitioned. Then, an aqueous 1 N hydrochloric acid solution was added to the aqueous layer, which was then extracted with chloroform. The organic layer was dried over anhydrous magnesium sulfate. After separation by filtration, the solvent was evaporated under reduced pressure, and the title compound (3.0 g 79%) was obtained as a solid.
¹H-NMR(400MHz, CDCl₃)δ: 2.35(3H, s), 4.10(3H, s), 7.15(1H, d, J=9.28Hz), 7.23(1H, s), 7.51(1H, d, J=7.93Hz), 7.60(1H, d, J=7.93Hz), 7.96(1H, d, J=9.28Hz), 8.32(1H, s).

[Reference Example 40] 1-(6-Methyl-3-pyridyl)-5-(4-methyl-2-pyridyl)-1H-pyrazole-3-carboxylic acid

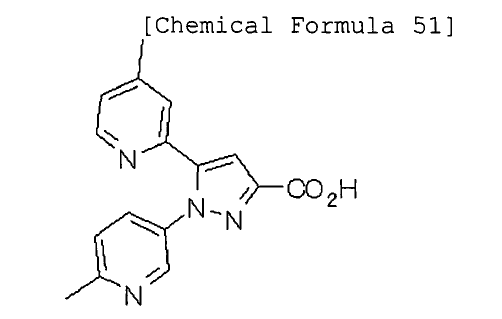

### 1) 1-(4-Methyl-2-pyridyl)-1-ethanone

Under cooling to -78°C, n-butyllithium (a 1.58 M solution in hexane, 22 mL) was added dropwise to a solution of 2-bromo-4-picoline (4.0 g) in diethyl ether (60 mL) over 5 minutes, and then the resultant mixture was stirred for 5 minutes. N, N-Dimethyl acetamide (3.3 mL) was added dropwise to the reaction solution, and the mixture was gradually warmed to room temperature, and stirred for 14.5 hours. Water was added to the reaction solution, which was then extracted with ethyl acetate. The organic layer was dried over anhydrous magnesium sulfate. After separation by filtration, a residue obtained by evaporating the solvent under reduced pressure was purified by silica gel column chromatography (ethyl acetate-hexane), to obtain 1-(4-methyl-2-pyridyl)-1-ethanone (1.86 g, 59%) as an oily product.
¹H-NMR(400MHz, CDCl₃)δ: 2.42(3H, s), 2.72(3H, s), 7.29(1H, dd, J=4.94, 0.67Hz), 7.86(1H, d, J=0.67Hz), 8.54(1H, d, J=4.94Hz).

### 2) 4-(4-Methyl-2-pyridyl)-2, 4-dioxobutanoic acid ethyl ester

4-(4-Methyl-2-pyridyl)-2, 4-dioxobutanoic acid ethyl ester (3.82 g) was obtained as a solid by the same method as that in Reference Example 9-(3), using 1-(4-methyl-2-pyridyl)-1-ethanone (1.86 g), sodium ethoxide (1.90 g) and diethyl oxalate (3.74 mL).
¹H-NMR(400MHz, CDCl₃)δ: 1.38(3H, t, J=7.08Hz), 2.51(3H, s), 4.36(2H, q, J=7.08Hz), 7.43(2H, br), 8.03(1H, s), 8.65(1H, d, J=5.01Hz).
EI-MSm/z: 236(M+H)⁺.

### 3) 1-(6-Methyl-3-pyridyl)-5-(4-methyl-2-pyridyl)-1H-pyrazole-3-carboxylic acid ethyl ester

1-(6-Methyl-3-pyridyl)-5-(4-methyl-2-pyridyl)-1H-pyrazole-3-carboxylic acid ethyl ester (6.97 g, 45%) was obtained as an oily product by the same method as that in Reference Example 39-(2), using 4-(4-methyl-2-pyridyl)-2, 4-dioxobutanoic acid ethyl ester (11.37 g) and 5-hydrazino-2-methylpyridine (6.0 g) of Reference Example 7.
¹H-NMR(400MHz, CDCl₃)δ: 1.43(3H, t, J=7.08Hz), 2.35(3H, s), 2.59(3H, s), 4.46(2H, q, J=7.08Hz), 7.05(1H, d, J=5.01Hz), 7.20(1H, d, J=8.30Hz), 7.24(1H, s), 7.26(1H, s), 7.72(1H, dd, J=8.30, 2.44Hz), 8.33(1H, d, J=5.01Hz), 8.38(1H, d, H=2.44Hz).
EI-MSm/z: 323(M+H)⁺.

### 4) Title compound

The title compound (5.26 g, 83%) was obtained as an amorphous material by the same method as that in Reference Example 3-(4), using 1-(6-methyl-3-pyridyl)-5-(4-methyl-2-pyridyl)-1H-pyrazole-3-carboxylic acid ethyl ester (6.97 g) and an aqueous 1 N sodium hydroxide solution (24 mL).
¹H-NMR(400MHz, CDCl₃)δ: 2.37(3H, s), 2.61(3H, s), 7.08(1H, d, J=5.01Hz), 7.20(1H, d, J=8.30Hz), 7.27(1H, s), 7.30(1H, s), 7.73(1H, dd, J=8.30, 2.44Hz), 8.36(1H, d, J=5.01Hz), 8.45(1H, d, J=2.44Hz).
EI-MSm/z: 295(M+H)⁺.

[Reference Example 41] 1-(6-Methoxy-3-pyridyl)-5-(5-methyl-2-pyrazinyl)-1H-pyrazole-3-carboxylic acid

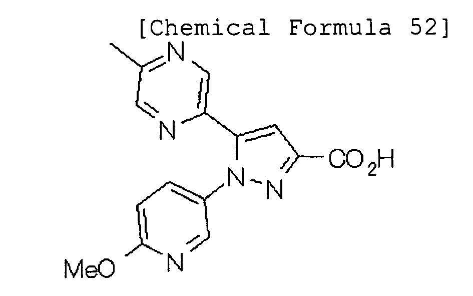

### 1) 1-(6-Methoxy-3-pyridyl)-5-(5-methyl-2-pyrazinyl)-1H-pyrazole-3-carboxylic acid ethyl ester

1-(6-Methoxy-3-pyridyl)-5-(5-methyl-2-pyrazinyl)-1H-pyrazole-3-carboxylic acid ethyl ester (4.80 g, 46%) was obtained as a solid by the same method as that in Reference Example 9-(4), using 4-(5-methyl-2-pyrazinyl)-2, 4-dioxobutanoic acid ethyl ester (7.32 g) of Reference Example 8-(3) and 5-hydrazino-2-methoxypyridine (4.31 g) of Reference Example 2.
¹H-NMR(400MHz, CDCl₃)δ: 1.41-1.45(3H, m), 2.57(3H, s), 3.96(3H, s), 4.44-4.49(2H, m), 6.79(1H, dd, J=8.8, 0.7Hz), 7.33(1H, s), 7.68(1H, dd, J=8.8, 2.7Hz), 8.10-8.11(1H, m), 8.36(1H, m), 8.54(1H, d, J=1.5Hz).
FAB-MSm/z: 340(M+H)⁺.

### 2) Title compound

The title compound (1.43 g, 87%) was obtained as a solid by the same method as that in Reference Example 4, Method B-(4), using 1-(6-methoxy-3-pyridyl)-5-(5-methyl-2-pyrazinyl)-1H-pyrazole-3-carboxylic acid ethyl ester (1.79 g).
¹H-NMR(400MHz, DMSO-d₆)δ: 2.50(3H, s), 3.90(3H, s), 6.90(1H, d, J=8.8Hz), 7.47(1H, s), 7.76(1H, dd, J=8.8, 2.7Hz), 8.19(1H, d, J=2.7Hz), 8.41(1H, m), 8.85(1H, d, J=1.5Hz), 13.12(1H, br s).
FAB-MSm/z: 312(M+H)⁺.

[Reference Example 42] 5-(5-tert-Butoxycarbonylamino-2-pyrazinyl)-1-(6-methoxy-3-pyridyl)-1H-pyrazole-3-carboxylic acid

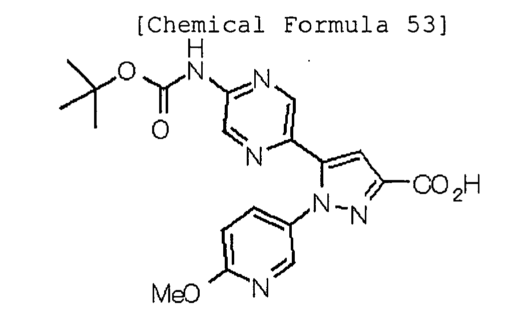

### 1) 5-(5-Carboxy-2-pyrazinyl)-1-(6-methoxy-3-pyridyl)-1H-pyrazole-3-carboxylic acid ethyl ester

Under an argon atmosphere, selenium dioxide (3.92 g) was added to a solution of 1-(6-methoxy-3-pyridyl)-5-(5-methyl-2-pyrazinyl)-1H-pyrazole-3-carboxylic acid ethyl ester (3.00 g) of Reference Example 41-(1) in pyridine (60 mL), and the resultant mixture was heated to reflux for 23 hours. After air cooling, water and chloroform were added to the reaction solution, and the mixture was partitioned. The organic layer was dried over anhydrous sodium sulfate. After separation by filtration, the solvent was evaporated under reduced pressure, and 5-(5-carboxy 2-pyrazinyl)-1-(6-methoxy-3-pyridyl)-1H-pyrazole-3-carboxylic acid ethyl ester (2.83 g, 87%) was obtained as a solid.
¹H-NMR(400MHz, DMSO-d₆)δ: 1.35(3H, t, J=7.1Hz), 3.92(3H, s), 4.37(2H, q, J=7.1Hz), 6.92(1H, d, J=8.8Hz), 7.36-7.40(1H, m), 7.76-7.84(1H, m), 8.25(1H, m), 8.96(1H, d, J=1.5Hz), 9.18(1H, d, J=1.5Hz).
EI-MSm/z: 369(M⁺).

### 2) 5-(5-tert-Butoxycarbonylamino-2-pyrazinyl)-1-(6-methoxy-3-pyridyl)-1H-pyrazole-3-carboxylic acid ethyl ester

Under an argon atmosphere, triethylamine (1.17 mL), diphenylphosphorylazide (1.80 mL) and tert-butanol (1.60 mL) were added to a suspension of 5-(5-carboxy 2-pyrazinyl)-1-(6-methoxy-3-pyridyl)-1H-pyrazole-3-carboxylic acid ethyl ester (2.81 g) in 1, 4-dioxane (56 mL) at room temperature, and the resultant mixture was stirred for 25 minutes at 100°C. After air cooling, a residue obtained by evaporating the reaction solvent under reduced pressure was purified by silica gel column chromatography (ethyl acetate-hexane), to obtain 5-(5-tert-butoxycarbonylamino-2-pyrazinyl)-1-(6-methoxy-3-pyridyl)-1H-pyrazole-3-carboxylic acid ethyl ester (1.29 g, 39%) as a solid.
¹H-NMR(400MHz, CDCl₃)δ: 1.42-1.45(3H, m), 1.54(9H, s), 3.97(3H, s), 4.44-4.50(2H, m), 6.79(1H, d, J=8.8Hz), 7.28(1H, s), 7.36(1H, br s), 7.68(1H, dd, J=8.8, 2.9Hz), 8.11-8.12(1H, m), 8.29(1H, d, J=1.7Hz), 9.18(1H, d, J=1.5Hz).
EI-MSm/z: 440(M⁺).

### 3) Title compound

The title compound (1.19 g, 99%) was obtained as a solid by the same method as that in Reference Example 4, Method B-(4), using 5-(5-tert-butoxycarbonylamino-2-pyrazinyl)-1-(6-methoxy-3-pyridyl)-1H-pyrazole-3-carboxylic acid ethyl ester (1.28 g).
¹H-NMR(400MHz, DMSO-d₆)δ: 1.48(9H, s), 3.91(3H, m), 6.90(1H, d, J=8.8Hz), 7.41(1H, m), 7.75-7.7.8(1H, m), 8.19(1H, d, J=2.7Hz), 8.62-8.63(1H, m), 8.86-8.87(1H, m), 10.39(1H, s), 13.10(1H, br s).
EI-MSm/z: 412(M⁺).

[Reference Example 43] 5-(1H-Imidazol-2-yl)-1-(6-methoxy-3-pyridyl)-1H-pyrazole-3-carboxylic acid ethyl ester

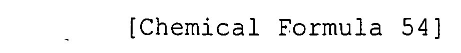

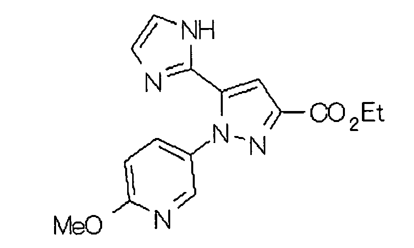

### 1) 1-(1H-Imidazol-2-yl)-1-ethanone

n-Butyllithium (a 1.58 M solution in hexane, 25.3 mL) was added dropwise to a solution of 1-diethoxymethyl-1H-imidazole (6.80 g) in tetrahydrofuran (100 mL) for 5 minutes under cooling to -55°C, while maintaining the internal temperature at -35°C or lower, and the resultant mixture was stirred for 15 minutes at an internal temperature of -40°C. N, N-Dimethylacetamide (5.57 mL) was added dropwise to the reaction solution for 5 minutes, and the mixture was stirred for 25.5 hours at room temperature. Diethyl ether and an aqueous 0.1 N hydrochloric acid solution were added to the reaction solution, and the mixture was partitioned. The aqueous layer was neutralized with sodium hydrogen carbonate, and then extracted with chloroform. The organic layer was dried over anhydrous sodium sulfate. After separation by filtration, the solvent was evaporated under reduced pressure, and 1-(1H-imidazol-2-yl)-1-ethanone (3.06 g, 69%) was obtained as a solid.
¹H-NMR(400MHz, CD₃OD)δ: 2.56(3H, s), 7.27(2H, br s).
ESI-MSm/z: 111(M+H)⁺.

### 2) 1-{1-[(4-methylphenyl)sulfonyl]-1H-imidazol-2-yl}-1-ethanone

Triethylamine (2.09 mL) and 4-methylbenzenesulfonyl chloride (2.29 g) were added to a suspension of 1-(1H-imidazol-2-yl)-1-ethanone (1.10 g) in dichloromethane (10 mL), and the resultant mixture was stirred for 24.5 hours. Ethyl acetate and water were added to the reaction solution, and the mixture was partitioned. The organic layer was dried over anhydrous sodium sulfate. After separation by filtration, a residue obtained by evaporating the solvent under reduced pressure was purified by silica gel column chromatography (hexane-dichloromethane), to obtain 1-{1-[(4-methylphenyl)sulfonyl]-1H-imidazol-2-yl}-1-ethanone (2.05 g, 77%) as a solid.
¹H-NMR(400MHz, CDCl₃)δ: 2.44(3H, s), 2.57(3H, s), 7.17(1H, d, J=1.5Hz), 7.36(2H, d, J=8.3Hz), 7.87(1H, d, J=1.5Hz), 7.99(2H, d, J=8.3Hz).
ESI-MSm/z: 265(M+H)⁺.

### 3) Title compound

Under cooling to -78°C, lithium bis(trimethylsilyl)amide (a 1.0 M solution in tetrahydrofuran, 7.95 mL) was added to a solution of 1-{1-[(4-methylphenyl)sulfonyl]-1H-imidazol-2-yl}-1-ethanone (1.91 g) in tetrahydrofuran (7.5 mL), and the resultant mixture was stirred for 30 minutes. Diethyl oxalate (1.47 mL) was added to the reaction solution, and the mixture was stirred for 10 minutes, then gradually warmed to room temperature, and then stirred for 3 hours. Diethyl ether and water were added to the reaction solution, and the mixture was partitioned. Diethyl ether and an aqueous 1 N hydrochloric acid solution (7.95 mL) were added to the aqueous layer, and the mixture was partitioned. The aqueous layer was further extracted with ethyl acetate, and the organic layers were combined and dried over anhydrous sodium sulfate. After separation by filtration, the solvent was evaporated under reduced pressure, and 4-{1-[(4-methylphenyl)sulfonyl]-1H-imidazol-2-yl}-2, 4-dioxobutanoic acid ethyl ester was obtained. A solution of this ethyl ester product and 5-hydrazino-2-methoxypyridine (1.00 g) of Reference Example 2 in ethanol (75 mL) was heated to reflux for 15.5 hours. After air cooling, to a residue obtained by evaporating the reaction solvent under reduced pressure, ethyl acetate and a saturated aqueous solution of sodium hydrogen carbonate were added, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain the title compound (0.210 g, 9.2%) as a solid.
¹H-NMR(400MHz, CDCl₃)δ: 1.42(3H, t, J=7.1Hz), 3.96(3H, s), 4.44(2H, q, J=7.1Hz), 6.78(1H, d, J=8.8Hz), 7.10(2H, br s), 7.23(1H, s), 7.68(1H, dd, J=8.8, 2.7Hz), 8.22(1H, d, J=2.7Hz), 9.60(1H, br s).
ESI-MSm/z: 314(M+H)⁺.

[Reference Example 44] 3, 3-Difluoropyrrolidine hydrochloride

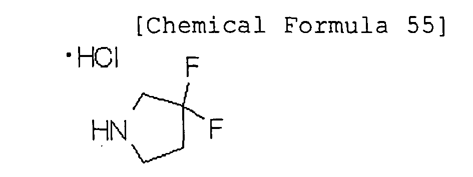

### 1) N-tert-Butoxycarbonyl-3-pyrrolidinone

Pyridine-sulfur trioxide (4.12 g) was added to a solution of (3R)-N-tert-butoxycarbonyl-3-hydroxypyrrolidine (2.47 g) and triethylamine (9.19 mL) in dimethylsulfoxide (30 mL) at room temperature, and the resultant mixture was stirred overnight. The reaction solution was poured onto water and extracted with diethyl ether. The organic layer was washed with water and saturated saline, and then dried over anhydrous sodium sulfate. After separation by filtration, a residue obtained by evaporating the solvent under reduced pressure was purified by silica gel column chromatography (ethyl acetate-hexane), to obtain N-tert-butoxycarbonyl-3-pyrrolidinone (855 mg, 34%) as an oily product.
¹H-NMR (400MHz, CDCl₃)δ: 1.47(9H, d, J=9.3Hz), 2.59(2H, t, J=7.8Hz), 3.75-3.80(4H, t, J=7.9Hz).

### 2) 3, 3-Difluoropyrrolidine-1-carboxylic acid tert-butyl ester

Under a nitrogen atmosphere, (diethylamino)sulfur trifluoride (1.45 mL) was added to a solution of N-tert-butoxycarbonyl-3-pyrrolidinone (846 mg) in benzene (30 mL) under ice cooling, and the resultant mixture was stirred for 3 days at room temperature. Diethyl ether was added to the reaction solution, and the mixture was partitioned. The organic layer was dried over anhydrous sodium sulfate. After separation by filtration, a residue obtained by evaporating the solvent under reduced pressure was purified by silica gel thin layer chromatography (ethyl acetate-hexane), to obtain 3, 3-difluoropyrrolidine-1-carboxylic acid tert-butyl ester (669 mg, 71%) as an oily product.
¹H-NMR(400MHz, CDCl₃)δ: 1.45(9H, s), 2.26-2.36(2H, m), 3.57-3.65(4H, m).

### 3) Title compound

The title compound (371 mg, 81%) was obtained as a solid by the same method as that in Reference Example 24-(2), using 3, 3-difluoropyrrolidine-1-carboxylic acid tert-butyl ester (659 mg).
¹H-NMR(400MHz, DMSO-d₆)δ: 2.42-2.53(2H, m), 3.39-3.43(2H, m), 3.62(2H, t, J=12.6Hz), 10.17(2H, s).
ESI-MSm/z: 108(M+H)⁺.

[Reference Example 45] 5-(2-Methyloxazol-4-yl)-1-(6-methyl-3-pyridyl)-1H-pyrazole-3-carboxylic acid

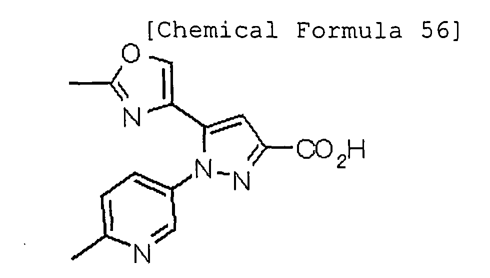

### 1) 2-Methyloxazole-4-carboxylic acid

To a solution of 2-methyloxazole-4-carboxylic acid methyl ester (2.63 g, D.R. Williams, et al. Tetrahedron Lett., 1997, 38(3), 331) in a mixed solvent of tetrahydrofuran (20 mL), methanol (10 mL) and water (10 mL), lithium hydroxide monohydrate (0.867 g) was added at room temperature, and the resultant mixture was stirred for 2 hours. The reaction solvent was evaporated under reduced pressure, and an aqueous 1 N hydrochloric acid solution was added (pH 4) to a residue thus obtained. The precipitated solid was filtered, and 2-methyloxazol-4-carboxylic acid (0.987 g, 42%) was obtained as a solid.
¹H-NMR(400MHz, CDCl₃)δ: 2.55(3H, s), 8.21(1H, s).
ESI-MSm/z: 128(M+H)⁺.

### 2) 4-Acetyl-2-methyloxazole

2-Methyloxazole-4-carboxylic acid N-methoxy-N-methylamide (4.32 g, quantitative) was obtained as an oily product by the same method as that in Reference Example 31-(1), using 2-methyloxazole-4-carboxylic acid (3.23 g) and N, O-dimethylhydroxylamine hydrochloride (5.00 g).
¹H-NMR(400MHz, CDCl₃)δ: 2.50(3H, s), 3.37(3H, s), 3.74(3H, s), 8.06 (1H, s).
EI-MSm/z: 170(M⁺).
Using this 2-methyloxazole-4-carboxylic acid N-methoxy-N-methylamide (4.23 g) and methyllithium (a 0.98 M solution in diethyl ether, 40 mL), 4-acetyl-2-methyloxazole (1.14 g, 37%) was obtained as an oily product by the same method as that in Reference Example 8-(2).
¹H-NMR(400MHz, CDCl₃)δ: 2.50(3H, s), 2.51(3H, s), 8.10(1H, s).
ESI-MSm/z: 126(M+H)⁺.

### 3) 4-(2-Methyloxazol-4-yl)-2, 4-dioxobutanoic acid methyl ester

4-(2-Methyloxazol-4-yl)-2, 4-dioxobutanoic acid methyl ester (0.641 g) was obtained as a solid by the same method as that in Reference Example 31-(3), using 4-acetyl-2-methyloxazole (1.14 g) and lithium bis(trimethylsilyl)amide (a 1.0 M solution in tetrahydrofuran, 10.0 mL) and dimethyl oxalate (2.15 g).
¹H-NMR(400MHz, CDCl₃)δ: 2.50(3H, s), 3.85(3H, s), 7.05(1H, s), 8.20(1H, s).
ESI-MSm/z: 212(M+H)⁺.

### 4) 5-(2-Methyloxazol-4-yl)-1-(6-methyl-3-pyridyl)-1H-pyrazole-3-carboxylic acid methyl ester

5-(2-Methyloxazol-4-yl)-1-(6-methyl-3-pyridyl)-1H-pyrazole-3-carboxylic acid methyl ester (76.9 mg) was obtained as an oily product by the same method as that in Reference Example 31-(4), using 4-(2-methyloxazol-4-yl)-2, 4-dioxobutanoic acid methyl ester (0.641 g) and 5-hydrazino-2-methylpyridine (0.459 g) of Reference Example 7.
¹H-NMR(400MHz, CDCl₃)δ: 2.44(3H, s), 2.65(3H, s), 3.96(3H, s), 7.18(1H, s), 7.27-7.36(2H, m), 7.72-7.83(1H, m), 8.56(1H, d, J=6.4Hz).
FAB-MSm/z: 299(M+H)⁺.

### 5) Title compound

The title compound (95.9 mg) was obtained as an oily product by the same method as that in Reference Example 31-(5), using 5-(2-methyloxazol-4-yl)-1-(6-methyl-3-pyridyl)-1H-pyrazole-3-carboxylic acid methyl ester (76.9 mg) and lithium hydroxide monohydrate (15.8 mg).
FAB-MSm/z: 285(M+H)⁺.

[Reference Example 46] 1-Methylpiperazin-2-one

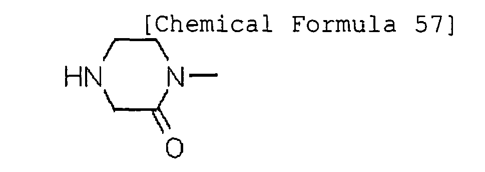

An aqueous 1 N sodium hydroxide solution was added to a suspension of 1-methylpiperazin-2-one hydrochloride (19.6 g) of Reference Example 21-(3) in dichloromethane, and the resultant mixture was partitioned. Further, sodium chloride was added to the aqueous layer to saturate the layer, and then the aqueous layer was extracted with dichloromethane. The organic layers were combined and dried over anhydrous sodium sulfate. After separation by filtration, the solvent was evaporated under reduced pressure, and the title compound (5.90 g) was obtained as an oily product.
ESI-MSm/z: 115(M+H)⁺.

[Reference Example 47] 1-(6-Methoxy-3-pyridazinyl)-5-(5-methyl-2-pyrazinyl)-1H-pyrazole-3-carboxylic acid

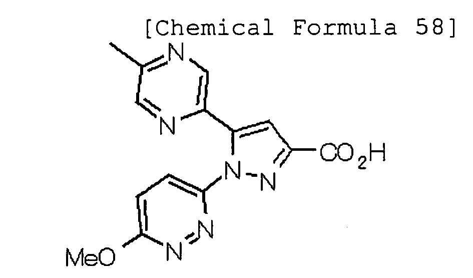

### 1) 4-(5-Methyl-2-pyrazinyl)-2, 4-dioxobutanoic acid methyl ester

4-(5-Methyl-2-pyrazinyl)-2, 4-dioxobutanoic acid methyl ester (7.84 g, 66%) was obtained as a solid by the same method as that in Reference Example 8-(3), using 1-(5-methyl-2-pyrazinyl)-1-ethanone (7.24 g) of Reference Example 8-(2), dimethyl oxalate (12.6 g) and lithium bis(trimethylsilyl)amide (a 1.0 M solution in tetrahydrofuran, 58.5 mL).
¹H-NMR(400MHz, DMSO-d₆)δ: 2. 63 (3H, s), 3.87 (3H, s), 7.41(1H, br s), 8.73(1H, s), 9.13(1H, s).
EI-MSm/z: 222(M⁺).

### 2) 1-(6-Chloro-3-pyridazinyl)-5-(5-methyl-2-pyrazinyl)-1H-pyrazole-3-carboxylic acid methyl ester

1-(6-Chloro-3-pyridazinyl)-5-(5-methyl-2-pyrazinyl)-1H-pyrazole-3-carboxylic acid methyl ester (6.60 g, 56%) was obtained as a solid by the same method as that in Reference Example 4, Method B-(2), using 4-(5-methyl-2-pyrazinyl)-2, 4-dioxobutanoic acid methyl ester (7.83 g) and 3-chloro-6-hydrazinopyridazine (5.09 g).
¹H-NMR(400MHz, DMSO-d₆)δ: 2.53(3H, s), 3.92(3H, s), 7.63(1H, s), 8.20-8.29(2H, m), 8.37(1H, m), 8.99(1H, d, J=1.5Hz).
EI-MSm/z: 330(M⁺).

### 3) Title compound

Under an argon atmosphere, sodium methoxide (3.22 g) was added to a suspension of 1-(6-chloro-3-pyridazinyl)-5-(5-methyl-2-pyrazinyl)-1H-pyrazole-3-carboxylic acid methyl ester (6.58 g) in methanol (132 mL) at room temperature, and the resultant mixture was heated to reflux for 2 hours. After air cooling, water (132 mL) was added thereto, and the mixture was stirred for 1 hour at room temperature. An aqueous 1 N hydrochloric acid solution (50 mL) was added to the reaction solution, and the mixture was stirred for 10 minutes at room temperature. The precipitated solid was filtered, and the title compound (5.50 g, 89%) was obtained as a solid.
¹H-NMR(400MHz, DMSO-d₆)δ: 2.51(3H, s), 4.05(3H, s), 7.48-7.50(2H, m), 8.04(1H, d, J=9.3Hz), 8.37(1H, m), 8.91(1H, d, J=1.5Hz), 13.28(1H, br s).
EI-MSm/z: 312(M⁺).

[Reference Example 48] 1-(6-Methyl-3-pyridyl)-5-(5-methyl-2-pyridyl)-1H-pyrazole-3-carboxylic acid

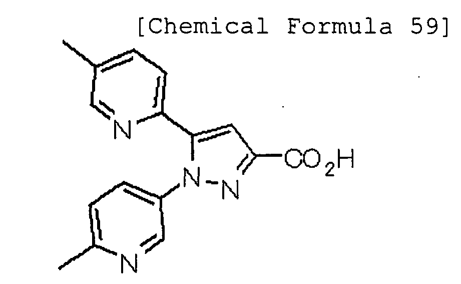

### 1) 1-(6-Methyl-3-pyridyl)-5-(5-methyl-2-pyridyl)-1H-pyrazole-3-carboxylic acid ethyl ester

A solution of 4-(5-methyl-2-pyridyl)-2, 4-dioxobutanoic acid ethyl ester (1.98 g) of Reference Example 15-(3) and 5-hydrazino-2-methylpyridine (1.04 g) of Reference Example 7 in ethanol (40 mL) was heated to reflux for 1 hour. Acetic acid (2.41 mL) was added to the reaction solution, which was then heated to reflux for 17 hours. Concentrated hydrochloric acid (1.3 mL) was added to the reaction solution, and the mixture was heated to reflux for 1.5 hours. After air cooling, a saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (hexane-ethyl acetate), to obtain 1-(6-methyl-3-pyridyl)-5-(5-methyl-2-pyridyl)-1H-pyrazole-3-carboxylic acid ethyl ester (0.887 g, 33%) as a solid.
¹H-NMR(400MHz, CDCl₃)δ: 1.43(3H, t, J=7.2Hz), 2.34(3H, s), 2.59(3H, s), 4.46(2H, q, J=7.2Hz), 7.20-7.29(3H, m), 7.49-7.51(1H, m), 7.73(1H, dd, J=8.3, 2.7Hz), 8.33-8.37(2H, m).
FAB-MSm/z: 323(M+H⁺).

### 2) Title compound

The title compound (0.752 g, 93%) was obtained as a solid by the same method as that in Reference Example 4, Method B-(4), using 1-(6-methyl-3-pyridyl)-5-(5-methyl-2-pyridyl)-1H-pyrazole-3-carboxylic acid ethyl ester (0.882 g).
¹H-NMR(400MHz, DMSO-d₆)δ: 2.30(3H, s), 2.53(3H, s), 7.28(1H, s), 7.33(1H, d, J=8.3Hz), 7.58-7.60(1H, m), 7.65-7.71(2H, m), 8.28(1H, m), 8.36(1H, d, J=2.7Hz), 13.08(1H, br s).
FAB-MSm/z: 295(M+H⁺).

[Reference Example 49] 1-(6-Methoxy-3-pyridyl)-5-(1-methyl-1H-1, 2, 4-triazol-3-yl)-1H-pyrazole-3-carboxylic acid ethyl ester

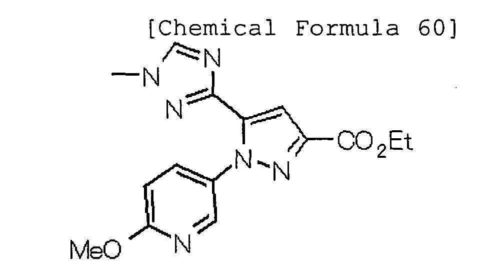

### 1) 4-(1-Methyl-5-phenylthio-1H-1, 2, 4-triazol-3-yl)-2, 4-dioxobutanoic acid ethyl ester

4-(1-Methyl-5-phenylthio-1H-1, 2, 4-triazol-3-yl)-2, 4-dioxobutanoic acid ethyl ester (1.62 g, 82%) was obtained as a solid by the same method as that in Reference Example 8-(3), using, under an argon atmosphere, 3-acetyl 1-methyl-5-phenylthio-1H-1, 2, 4-triazole (1.37 g, S. Ohta, et al. Chem. Pharm. Bull., 1993, 41(7), 1226-1231), diethyl oxalate (1.60 mL) and lithium bis(trimethylsilyl)amide (a 1.0 M solution in tetrahydrofuran, 7.05 mL).
¹H-NMR(400MHz, CDCl₃)δ: 1.38-1.43(3H, m), 3.93(3H, s), 4.39(2H, q, J=7.2Hz), 7.25-7.46(5H, m).
ESI-MSm/z: 334(M+H)⁺.

### 2) 1-(6-Methoxy-3-pyridyl)-5-(1-methyl-5-phenylthio-1H-1, 2, 4-triazol-3-yl)-1H-pyrazole-3-carboxylic acid ethyl ester

1 M Hydrochloric acid-ethanol (1.98 mL) was added to a solution of 4-(1-methyl-5-phenylthio-1H-1, 2, 4-triazol-3-yl)-2, 4-dioxobutanoic acid ethyl ester (439 mg) and 5-hydrazino-2-methoxypyridine (183 mg) of Reference Example 2 at room temperature, and the resultant mixture was heated to reflux for 5 hours. After air cooling, water and chloroform were added to the reaction solution, and the mixture was partitioned. The organic layer was dried over anhydrous sodium sulfate. After separation by filtration, a residue obtained by evaporating the solvent under reduced pressure was purified by silica gel thin layer chromatography (ethyl acetate-hexane), to obtain 1-(6-methoxy-3-pyridyl)-5-(1-methyl-5-phenylthio-1H-1, 2, 4-triazol-3-yl)-1H-pyrazole-3-carboxylic acid ethyl ester (169 mg, 29%) as a solid.
ESI-MSm/z: 437(M+H)⁺.

### 3) Title compound

A suspension of 1-(6-methoxy-3-pyridyl)-5-(1-methyl-5-phenylthio-1H-1, 2, 4-triazol-3-yl)-1H-pyrazole-3-carboxylic acid ethyl ester (165 mg) and Raney nickel (1.0 g, used after washing with ethanol) in ethanol (20 mL) was heated to reflux for 2 hours. After air cooling, the reaction solution was filtered, Raney nickel (1.7 g, used after washing with ethanol) and ethanol (30 mL) were further added to the filtrate, and the mixture was heated to reflux for 30 minutes. After air cooling, the mixture was separated by filtration, and a residue obtained by evaporating the solvent of the filtrate under reduced pressure was purified by silica gel thin layer chromatography (hexane-ethyl acetate), to obtain the title compound (68 mg, 55%) as a solid.
ESI-MSm/z: 329(M+H)⁺.

[Reference Example 50] 5-(5-Methoxyoxazol-2-yl)-1-(6-methyl-3-pyridyl)-1H-pyrazole-3-carboxylic acid

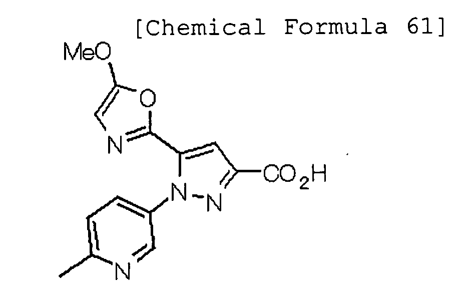

### 1) N-Methoxycarbonylmethyloxamic acid methyl ester

N-Methoxycarbonylmethyloxamic acid methyl ester (19.86 g, quantitative) was obtained as an oily product according to the method of E. Menta, et al., (J. Heterocyclic Chem., 1995, 32, 1693), using glycine methyl ester hydrochloride (12.59 g), dimethyl oxalate (23.65 g), triethylamine (14.0 mL) and methanol (110 mL).
¹H-NMR(400MHz, CDCl₃)δ: 3.79(3H, s), 3.92(3H, s), 4.14(2H, d, J=5.6Hz), 7.55(1H, br).
ESI-MSm/z: 176(M+H)⁺.

### 2) 5-Methoxyoxazole-2-carboxylic acid methyl ester

5-Methoxyoxazole-2-carboxylic acid methyl ester (10.26 g, 65%) was obtained as a solid according to the method of E. Menta, et al., (J. Heterocyclic Chem., 1995, 32, 1693), using N-methoxycarbonylmethyloxamic acid methyl ester (19.86 g), phosphorous pentoxide (72.4 g) and acetonitrile (300 mL).
¹H-NMR(400MHz, CDCl₃)δ: 3.96(3H, s), 4.00(3H, s), 6.36(1H, s).
ESI-MSm/z: 158(M+H)⁺.

### 3) 5-Methoxyoxazole-2-carboxylic acid

Lithium hydroxide monohydrate (3.072 g) was added to a mixed solution of 5-methoxyoxazole-2-carboxylic acid methyl ester (10.26 g) in tetrahydrofuran (150 mL), methanol (75 mL), and water (75 mL) at room temperature, and the resultant mixture was stirred for 2 hours. The reaction solution was made weakly acidic (PH 4) by adding 1 M hydrochloric acid-ethanol solution, and then most of the organic solvent was evaporated under reduced pressure. Chloroform-methanol (10: 1) was added to a residue thus obtained, and the mixture was partitioned. Further, the aqueous layer was extracted with chloroform-methanol (10: 1), and the organic layers were combined and dried over anhydrous sodium sulfate. After separation by filtration, the solvent was evaporated under reduced pressure, and 5-methoxyoxazole-2-carboxylic acid [1.06 g, 11%; ¹H-NMR (400 MHz, DMSO-d₆) δ: 3.91 (3H, s), 6.45 (1H, s). EI-MSm/z: 143 (M⁺)] was obtained as a solid. The organic solvent in the aqueous layer was further evaporated under reduced pressure, and the precipitated solid was filtered, combined with the previously obtained solid, to obtain a crude product of 5-methoxy oxazol-2-carboxylic acid (15.63 g).

### 4) 5-Methoxyoxazole-2-carboxylic acid N-methoxy-N-methylamide

To a mixed solution of 5-methoxyoxazole-2-carboxylic acid (15.63 g) in dichloromethane (400 mL), acetonitrile (100 mL) and N, N-dimethylformamide (100 mL), 1-hydroxybenzotriazole (9.69 g), 3-(3-dimethylaminopropyl)-1-ethylcarbodiimide hydrochloride (27.50 g), triethylamine (46 mL) and N, O-dimethylhydroxylamine hydrochloride (12.79 g) were added at room temperature, and the resultant mixture was stirred for 19 hours. The solvent of the reaction solution was evaporated under reduced pressure, water (300 mL), 1-hydroxybenzotriazole (4.84 g), 3-(3-dimethylaminopropyl)-1-ethylcarbodiimide hydrochloride (8.97 g), triethylamine (23 mL) and N, O-dimethylhydroxylamine hydrochloride (6.98 g) were added to a residue obtained above at room temperature, and the mixture was stirred for 3 days. Ethyl acetate was added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (acetone-chloroform), to obtain 5-methoxyoxazole-2-carboxylic acid N-methoxy-N-methylamide (0.89 g, 7.3%) as an oily product.
¹H-NMR(400MHz, CDCl₃)δ: 3.46(3H, br), 3.85(3H, s), 3.98(3H, s), 6.29 (1H, s).
ESI-MSm/z: 187(M+H)⁺.

### 5) 2-Acetyl-5-methoxyoxazole

2-Acetyl-5-methoxyoxazole (0.258 g, 39%) was obtained as a solid by the same method as that in Reference Example 8-(2), using 5-methoxyoxazole-2-carboxylic acid N-methoxy-N-methylamide (0.89 g) and methyllithium (a 0.98 M solution in diethyl ether, 8.0 mL).
¹H-NMR(400MHz, CDCl₃)δ: 2.56(3H, s), 4.02(3H, s), 6.34(1H, s) .
EI-MSm/z: 141(M⁺).

### 6) 4-(5-Methoxyoxazol-2-yl)-2, 4-dioxobutanoic acid methyl ester

Under cooling to -78°C, lithium bis(trimethylsilyl)amide (a 1.0 M solution in tetrahydrofuran, 2.0 mL) was added dropwise to a solution of 2-acetyl-5-methoxyoxazole (0.258 g) and dimethyl oxalate (0.435 g) in tetrahydrofuran (12 mL), and the resultant mixture was stirred for 20 minutes, and then stirred for 1.5 hours at 0°C. Water and diethyl ether were added to the reaction solution, and the mixture was partitioned. The aqueous layer was made weakly acidic (pH 4) using an aqueous 1 N hydrochloric acid solution, and extracted with chloroform. The organic layer was washed with saturated saline, and then dried over anhydrous sodium sulfate. After separation by filtration, the solvent was evaporated under reduced pressure, and 4-(5-methoxyoxazol-2-yl)-2, 4-dioxobutanoic acid methyl ester (0.350 g, 84%) was obtained as a solid.
¹H-NMR(400MHz, CDCl₃)δ: 3.93(3H, s), 4.07(3H, s), 6.47(1H, br s), 7.21(1H, br s).
ESI-MSm/z: 228(M+H)⁺.

### 7) 5-(5-Methoxyoxazol-2-yl)-1-(6-methyl-3-pyridyl)-1H-pyrazole-3-carboxylic acid methyl ester

A solution of 4-(5-methoxyoxazol-2-yl)-2, 4-dioxobutanoic acid methyl ester (0.350 g) and 5-hydrazino 2-methyl pyridine (0.219 g) of Reference Example 7 in methanol (15 mL) was heated to reflux for 30 minutes. After air cooling, acetic acid (0.352 mL) was added to the reaction solution, and the mixture was heated to reflux for 16 hours. After air cooling, the solvent of the reaction solution was evaporated under reduced pressure, a saturated aqueous solution of sodium hydrogen carbonate and chloroform-methanol (20: 1) were added to a residue thus obtained, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain 4, 5-dihydro-5-hydroxy-5-(5-methoxyoxazol-2-yl)-1-(6-methyl-3-pyridyl)-1H-pyrazole-3-carboxylic acid methyl ester (85.6 mg) as an oily product. [ESI-MSm/z: 333 (M+H)⁺.]
To a solution of this 4, 5-dihydro-5-hydroxy-1H-pyrazole-3-carboxylic acid methyl ester product (85.6 mg) in dichloromethane (3.0 mL), triethylamine (0.0898 mL), methanesulfonyl chloride (0.0399 mL) and 4-(dimethylamino)pyridine (4.1 mg) were added at room temperature, and the mixture was stirred for 1 hour. Water and chloroform were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel thin layer chromatography (acetone-chloroform), to obtain 5-(5-methoxyoxazol-2-yl)-1-(6-methyl-3-pyridyl)-1H-pyrazole-3-carboxylic acid methyl ester (25.2 mg, 5.2%) as a solid.
¹H-NMR(400MHz, CDCl₃)δ: 2.65(3H, s), 3.89(3H, s), 3.97(3H, s), 6.12(1H, s), 7.28(1H, d, J=8.3Hz), 7.37(1H, s), 7.78(1H, dd, J=8.3, 2.4Hz), 8.63(1H, d, J=2.4Hz).
FAB-MSm/z: 315(M+H)⁺.

### 8) Title compound

The title compound (14.9 mg, 62%) was obtained as a solid by the same method as that in Reference Example 31-(5), using 5-(5-methoxyoxazol-2-yl)-1-(6-methyl-3-pyridyl)-1H-pyrazole-3-carboxylic acid methyl ester (25.2 mg) and lithium hydroxide monohydrate (3.8 mg).
¹H-NMR(400MHz, DMSO-d₆)δ: 2.56(3H, s), 3.87(3H, s), 6.41(1H, s), 7.27(1H, s), 7.42(1H, d, J=8.3Hz), 7.90(1H, dd, J=8.3, 2.7Hz), 8.59(1H, d, J=2.7Hz).
FAB-MSm/z: 301(M+H)⁺.

[Reference Example 51] 1-(6-Methoxy-3-pyridyl)-5-(2-methyl-1H-imidazol-4-yl)-1H-pyrazole-3-carboxylic acid ethyl ester

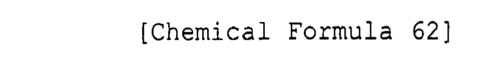

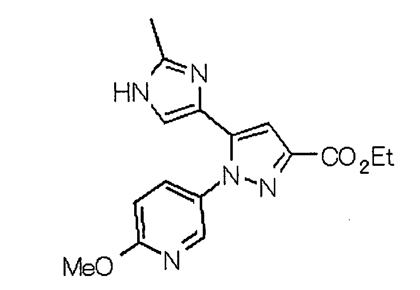

### 1) 1-{2-Methyl-1-[(4-methylphenyl)sulfonyl]-1H-imidazol-4-yl}-1-ethanone

To a solution of 1-(2-methyl-1H-imidazol-4-yl)-1-ethanone (1.00 g, L.A. Reiter, J. Org. Chem., 1987, 52, 2714-2726) in dichloromethane (20 mL), triethylamine (1.67 mL) and 4-methylbenzenesulfonyl chloride (1.83 g) were added at room temperature, and the resultant mixture was stirred for 1.5 days. Ethyl acetate and water were added to the reaction solution, and the mixture was partitioned. The organic layer was dried over anhydrous sodium sulfate. After separation by filtration, a residue obtained by evaporating the solvent under reduced pressure was purified by silica gel column chromatography (methanol-chloroform), to obtain 1-{2-methyl-1-[(4-methylphenyl)sulfonyl]-1H-imidazol-4-yl}-1-ethanone (2.11 g, 94%) as an oily product.
¹H-NMR(400MHz, CDCl₃)δ: 2.47(3H, s), 2.49(3H, s), 2.54(3H, s), 7.40(2H, d, J=8.3Hz), 7.82(2H, d, J=8.3Hz), 8.02(1H, s).
ESI-MSm/z: 279(M+H)⁺.

### 2) Title compound

Under cooling to -78°C, lithium bis(trimethylsilyl)amide (a 1.0 M solution in tetrahydrofuran, 7.78 mL) was added to a solution of 1-{2-methyl-1-[(4-methylphenyl)sulfonyl]-1H-imidazol-4-yl}-1-ethanone (1.97 g) in tetrahydrofuran (7 mL), and the resultant mixture was stirred for 35 minutes. Diethyl oxalate (1.44 mL) was added dropwise to the reaction solution, and then the mixture was gradually warmed to room temperature and stirred for 4.5 hours. To this reaction solution, a solution of 5-hydrazino-2-methoxypyridine (0.771 g) of Reference Example 2 in ethanol (35 mL) and a 1 M hydrochloric acid-ethanol solution (7.78 mL) were added, and the mixture was heated to reflux for 13 hours. After air cooling, ethyl acetate and a saturated aqueous solution of sodium hydrogen carbonate were added to the reaction solution, and the mixture was partitioned. The organic layer was dried over anhydrous sodium sulfate. After separation by filtration, a residue obtained by evaporating the solvent under reduced pressure was purified by silica gel column chromatography (methanol-ethyl acetate), to obtain the title compound (0.353 g, 15%) as an amorphous form.
¹H-NMR(400MHz, CDCl₃)δ: 1.40(3H, t, J=7.1Hz), 2.39(3H, s), 3.97(3H, s), 4.42(2H, q, J=7.1Hz), 6.44(1H, s), 6.80(1H, d, J=8.8Hz), 7.19(1H, br s), 7.68(1H, dd, J=8.8, 2.7Hz), 8.24(1H, d, J=2.7Hz).
ESI-MSm/z: 328(M+H)⁺.

[Reference Example 52] 5-(3-Carbamoyl-1H-pyrrol-1-yl)-1-(6-methoxy-3-pyridyl)-1H-pyrazole-3-carboxylic acid ethyl ester

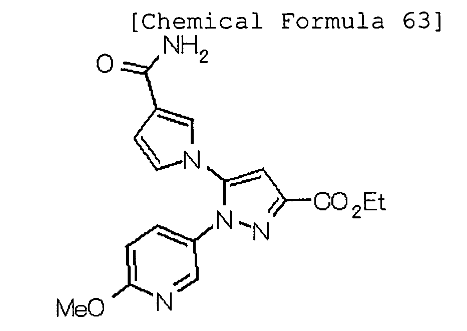

### 1) 5-(3-Carboxy-1H-pyrrol-1-yl)-1-(6-methoxy-3-pyridyl)-1H-pyrazole-3-carboxylic acid ethyl ester

Under cooling to 0°C, sodium chlorite (0.543 g) was added to a mixed solution of 5-(3-formyl-1H-pyrrol-1-yl)-1-(6-methoxy-3-pyridyl)-1H-pyrazol 3-carboxylic acid ethyl ester (0.680 g) of Reference Example 38 and amidesulfuric acid (0.583 g) in dioxane (10 mL), acetonitrile (20 mL) and water (10 mL), and the resultant mixture was gradually warmed to room temperature and stirred for 21.5 hours. The reaction solvent was evaporated under reduced pressure, ethyl acetate and water were added to a residue thus formed, and the mixture was partitioned. The organic layer was dried over anhydrous sodium sulfate. After separation by filtration, the solvent was evaporated under reduced pressure, diethyl ether was added to a residue thus obtained, and the precipitated solidwas filtered, to obtain 5-(3-carboxy-1H-pyrrol-1-yl)-1-(6-methoxy-3-pyridyl)-1H-pyrazole-3-carboxylic acid ethyl ester (0.191 g, 26%).
¹H-NMR(400MHz, CDCl₃)δ: 1.43 (3H, t, J=7.1Hz), 3. 95 (3H, s), 4.46(2H, q, J=7.2Hz), 6.63-6.64(1H, m), 6.73-6.74(1H, m), 6.76(1H, d, J=9.0Hz), 7.00(1H, s), 7.37-7.38(1H, m), 7.48(1H, dd, J=8.9, 2.7Hz), 8.02(1H, d, J=2.7Hz).
ESI-MSm/z: 357(M+H)⁺.

### 2) Title compound

The title compound (0.123 g, 64%) was obtained as a solid by the same method as that in Reference Example 8-(1), using 5-(3-carboxy-1H-pyrrol-1-yl)-1-(6-methoxy-3-pyridyl)-1H-pyrazole-3-carboxylic acid ethyl ester (0.191 g) and ammonium chloride (0.143 g).
¹H-NMR(400MHz, CDCl₃)δ: 1. 43 (3H, t, J=7.1Hz), 3. 94 (3H, s), 4.46(2H, q, J=7.1Hz), 5.50(2H, br s), 6.51(1H, dd, J=2.9, 1.7Hz), 6.64(1H, dd, J=2.9, 2.2Hz), 6.75(1H, d, J=9.3Hz), 6.98(1H, s), 7.26-7.27(1H, m), 7.47(1H, dd, J=8.9, 2.8Hz), 8.01(1H, d, J=2.4Hz).
ESI-MSm/z: 356(M+H)⁺.

[Reference Example 53] 5-(5-tert-Butoxycarbonylamino-2-pyrazinyl)-1-(6-methoxy-3-pyridazinyl)-1H-pyrazole-3-carboxylic acid

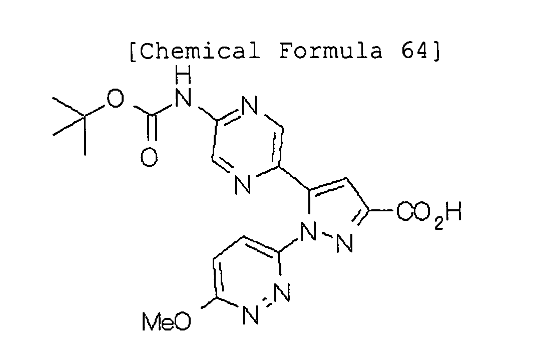

### 1) 1-(6-Methoxy-3-pyridazinyl)-5-(5-methyl-2-pyrazinyl)-1H-pyrazole-3-carboxylic acid methyl ester

Under an argon atmosphere and cooling to 0°C, (trimethylsilyl)diazomethane (a 2.0 M solution in hexane, 6.72 mL) was added dropwise to a mixed suspension of 1-(6-methoxy-3-pyridazinyl)-5-(5-methyl-2-pyrazinyl)-1H-pyrazole-3-carboxylic acid (3.50 g) of Reference Example 47 in dichloromethane (70 mL) and methanol (35 mL), and the resultant mixture was stirred for 1.5 hours. Further, (trimethylsilyl)diazomethane (a 2.0 M solution in hexane, 6.72 mL) was added dropwise to the reaction solution, and the mixture was stirred for 2 hours. A residue obtained by evaporating the solvent of the reaction solution under reduced pressure was purified by silica gel column chromatography (ethyl acetate-chloroform), to obtain 1-(6-methoxy-3-pyridazinyl)-5-(5-methyl-2-pyrazinyl)-1H-pyrazole-3-carboxylic acid methyl ester (3.41 g, 93%) as a solid.
¹H-NMR(400MHz, CDCl₃)δ: 2.59(3H, s), 4.00(3H, s), 4.11(3H, s), 7.18(1H, d, J=9.3Hz), 7.27-7.29(1H, m), 7.98-8.01(1H, m), 8.29(1H, m), 8.71(1H, d, J=1.5Hz).
EI-MSm/z: 326(M⁺).

### 2) 5-(5-Carboxy-2-pyrazinyl)-1-(6-methoxy-3-pyridazinyl)-1H-pyrazole-3-carboxylic acid methyl ester

5-(5-Carboxy-2-pyrazinyl)-1-(6-methoxy-3-pyridazinyl)-1H-pyrazole-3-carboxylic acid methyl ester (2.57 g, 69%) was obtained as a solid by the same method as that in Reference Example 42-(1), using 1-(6-methoxy-3-pyridazinyl)-5-(5-methyl-2-pyrazinyl)-1H-pyrazole-3-carboxylic acid methyl ester (3.40 g) and selenium dioxide (4.62 g).
¹H-NMR(400MHz, DMSO-d₆)δ: 3.92(3H, s), 4.05(3H, s), 7.52(1H, d, J=9.3Hz), 7.77(1H, s), 8.10(1H, d, J=9.3Hz), 8.98(1H, d, J=1.5Hz), 9.21(1H, d, J=1.5Hz).
EI-MSm/z: 356(M⁺).

### 3) 5-(5-tert-Butoxycarbonylamino-2-pyrazinyl)-1-(6-methoxy-3-pyridazinyl)-1H-pyrazole-3-carboxylic acid methyl ester

5-(5-tert-Butoxycarbonylamino-2-pyrazinyl)-1-(6-methoxy-3-pyridazinyl)-1H-pyrazole-3-carboxylic acid methyl ester (0.886 g, 29%) was obtained as a solid by the same method as that in Reference Example 42-(2), using 5-(5-carboxy-2-pyrazinyl)-1-(6-methoxy-3-pyridazinyl)-1H-pyrazole-3-carboxylic acid (2.56 g), diphenylphosphorylazide (1.70 mL) and tert-butanol (1.51 mL).
¹H-NMR(400MHz, CDCl₃)δ: 1.54(9H, s), 4.00(3H, s), 4.12(3H, s), 7.17(1H, d, J=9.3Hz), 7.24-7.27(1H, m), 7.57(1H, s), 7.95(1H, d, J=9.3Hz), 8.48(1H, d, J=1.5Hz), 9.11(1H, d, J=1.5Hz).
EI-MSm/z: 427(M⁺).

### 4) Title compound

An aqueous 1 N sodium hydroxide solution (5.15 mL) was added to a mixed suspension of 5-(5-tert-butoxycarbonylamino-2-pyrazinyl)-1-(6-methoxy-3-pyridazinyl)-1H-pyrazole-3-carboxylic acid methyl ester (0.880 g) in tetrahydrofuran (18 mL) and methanol (18 mL) at room temperature, and the resultant mixture was stirred for 4 hours. An aqueous 1 N hydrochloric acid solution (5.15 mL) was added to the reaction solution to neutralize, and water (250 mL) was further added to the reaction solution. The precipitated solid was filtered, and the title compound (0.732 g, 86%) was obtained as a solid.
¹H-NMR(400MHz, DMSO-d₆)δ: 1.48(9H, s), 4.05(3H, m), 7.44(1H, m), 7.49(1H, dd, J=9.3, 1.0Hz), 8.02(1H, dd, J=9.3, 1.0Hz), 8.71(1H, m), 8.82(1H, m), 10.39(1H, s).
EI-MSm/z: 413(M⁺).

[Reference Example 54] (2R)-2-Fluoromethylpyrrolidine hydrochloride

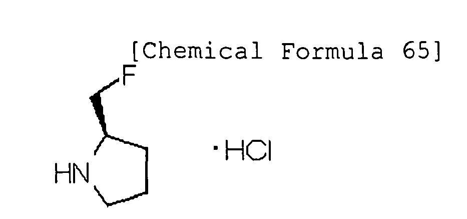

### 1) (2R)-N-Benzyloxycarbonyl-2-hydroxymethylpyrrolidine

Borane dimethylsulfide (3.23 mL) was added to a solution of N-benzyloxycarbonyl-D-proline (4.02 g) in tetrahydrofuran (50 mL) at room temperature, and the resultant mixture heated to reflux for 2 hours. Under ice cooling, an aqueous 1 N hydrochloric acid solution was added to the reaction solution to treat excessive borane dimethylsulfide, then chloroform was added to the reaction solution, which was then partitioned. The organic layer was dried over anhydrous sodium sulfate. After separation by filtration, a residue obtained by evaporating the solvent under reduced pressure was purified by silica gel column chromatography (hexane-ethyl acetate), to obtain (2R)-N-benzyloxycarbonyl-2-hydroxymethylpyrrolidine (3.68 g, 97%) as an oily product.
¹H-NMR(400MHz, CDCl₃)δ: 1.57-2.05(4H, m), 3. 36-3. 68 (4H, m), 4.01(1H, br s), 4.39(1H, br s), 5.15(2H, s), 7.32(5H, m).
ESI-MSm/z: 236(M+H)⁺.

### 2) (2R)-N-Benzyloxycarbonyl-2-(p-toluenesulfonyloxymethyl)pyrrolidine

p-Toluenesulfonyl chloride (3.52 g) was added to a solution of (2R)-N-benzyloxycarbonyl-2-hydroxymethylpyrrolidine (3.62 g) in pyridine (70 mL) at room temperature, and the resultant mixture was stirred overnight. Water and diethyl ether were added to the reaction solution, and the mixture was partitioned. The organic layer was washed with an aqueous 1 N hydrochloric acid solution, and dried over anhydrous sodium sulfate. After separation by filtration, a residue obtained by evaporating the solvent under reduced pressure was purified by silica gel column chromatography (ethyl acetate-hexane), to obtain (2R)-N-benzyloxycarbonyl-2-(p-toluenesulfonyloxymethyl)pyrrolidine (1.82 g, 30%) as an oily product.
¹H-NMR(400MHz, CDCl₃)δ: 1.81-2.40(4H, m), 2.42(3H, br s), 3.37(2H, s), 3.90-4.20(3H, m), 4.96-5.10(2H, m), 7.26-7.78(9H, m).

### 3) (2R)-N-Benzyloxycarbonyl-2-fluoropyrrolidine

A solution of (2R)-N-benzyloxycarbonyl-2-(p-toluenesulfonyloxymethyl) pyrrolidine (1.82 g) and tetrabutylammonium fluoride trihydrate (2.95 g) in acetonitrile (50 mL) was stirred overnight at an external temperature of 75°C. After air cooling, water and ethyl acetate were added to the reaction solution, and the mixture was partitioned. The organic layer was washed with water and saturated saline, and then dried over anhydrous sodium sulfate. After separation by filtration, a residue obtained by evaporating the solvent under reduced pressure was purified by silica gel column chromatography (ethyl acetate-hexane) to obtain (2R)-N-benzyloxycarbonyl-2-fluoropyrrolidine (705 mg, 64%) as an oily product.
¹H-NMR(400MHz, CDCl₃)δ: 1.87-2.04(4H, m), 3.46(2H, s), 4.03-4.61(3H, m), 5.14(2H, dd, J=19.8, 12.5Hz), 7.31-7.37(5H, m).

### 4) Title compound

At room temperature, a suspension of (2R)-N-benzyloxycarbonyl-2-fluoropyrrolidine (699 mg), 10% palladium-carbon (300 mg) and concentrated hydrochloric acid (1 mL) in methanol (30 mL) was stirred overnight underf a hydrogen atmosphere. The reaction suspension was separated by filtration, the solvent of the filtrate was evaporated under reduced pressure, and a residue thus obtained was solidified from methanol-diethyl ether, to obtain the title compound (358 mg, 87%) as a solid.
¹H-NMR(400MHz, DMSO-d₆)δ: 1.58-3.22 (6H, m), 3.76-3.80(1H, m), 4.55-4.70(2H, m), 9.58(2H, br s).

[Reference Example 55] 1-(6-Methoxypyridazin-3-yl)-5-(1-methyl-1H-pyrrol-3-yl)-1H-pyrazole-3-carboxylic acid

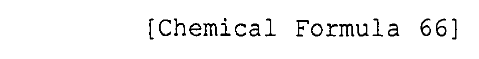

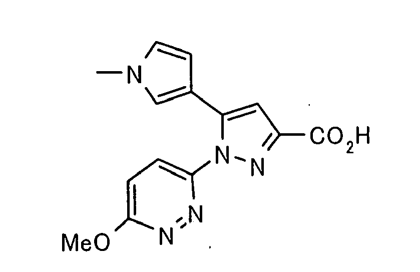

### 1) 1-(6-Chloropyridazin-3-yl)-5-(1-methyl-1H-pyrrol-3-yl)-1H-pyrazole-3-carboxylic acid methyl ester

Under cooling to -78°C, lithium bis(trimethylsilyl)amide (a 1.0 M solution in tetrahydrofuran, 27.8 mL) was added to a solution of 3-acetyl-1-methyl-1H-pyrrole (3.00 mL) in tetrahydrofuran (30 mL), and the resultant mixture was stirred for 45 minutes. A solution of dimethyl oxalate (4.48 g) in tetrahydrofuran (15 mL) was added to the reaction solution, and the mixture was stirred for 1 hour at room temperature. 3-Chloro-6-hydrazinopyridazine (4.40 g), concentrated hydrochloric acid (2.08 mL), and methanol (150 mL) were added to the reaction solution, and then the mixture was heated to reflux for 16 hours. Furthermore, concentrated hydrochloric acid (1.04 mL) was added to the reaction solution, and the mixture was heated to reflux for 3 hours. After air cooling, the solvent of the reaction solution was evaporated under reduced pressure, a saturated aqueous solution of sodium hydrogen carbonate, water, and ethyl acetate were added to a residue thus obtained, and the precipitated solid was filtered, to obtain 1-(6-chloropyridazin-3-yl)-5-(1-methyl-1H-pyrrol-3-yl)-1H-pyrazole-3-carboxylic acid methyl ester (2.83 g, 35%).
¹H-NMR(400MHz, DMSO-d₆)δ: 3.57 (3H, s), 3.85(3H, s), 5.94(1H, t, J=1.9Hz), 6.69(1H, t, J=1.9Hz), 6.89(1H, br), 7.00(1H, s), 8.10(1H, d, J=8.0Hz), 8.20(1H, d, J=8.0Hz).
EI-MSm/z: 317(M⁺) for ³⁵Cl.

### 2) Title compound

To a suspension of 1-(6-chloropyridazin-3-yl)-5-(1-methyl-1H-pyrrol-3-yl)-1H-pyrazole-3-carboxylic acid methyl ester (2.80 g) of the above in methanol (50 mL) and tetrahydrofuran (50 mL), sodium methoxide (1.43 g) was added at room temperature, and the resultant mixture was heated to reflux for 4 hours. After air cooling, an aqueous 1 M sodium hydroxide solution (18.0 mL) was added, and the mixture was stirred overnight. The solvent of the reaction solution was evaporated under reduced pressure, water and diethyl ether were added to a residue thus obtained, and the precipitate was separated by filtration. The solid precipitated by adding an aqueous 1 M hydrochloric acid solution to the solvent of the filtrate was filtered, and thus the title compound (1.02 g, 39%) was obtained.
¹H-NMR(400MHz, DMSO-d₆)δ: 3.55(3H, s), 4.11(3H, s), 5.82-5.86(1H, m), 6.67(1H, t, J=2.4Hz), 6.76(1H, t, J=2.4Hz), 6.88(1H, s), 7.50(1H, d, J=9.1Hz), 7.84(1H, d, J=9.1Hz).
EI-MSm/z: 299(M⁺).
[Reference Example 56] 1-Ethyl-2-oxopiperazine hydrochloride

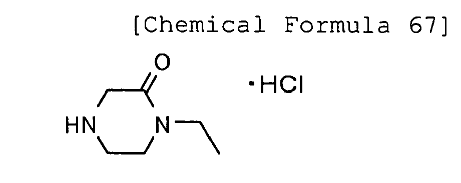

### 1) 3-Oxopiperazine-1-carboxylic acid tert-butyl ester

To a mixed solution of piperazin-2-one (5.07 g) in tetrahydrofuran (50 mL) and methanol (50 mL), triethylamine (7.76 mL) and di-tert-butyl dicarbonate ester (12.17 g) were added at room temperature, and the resultant mixture was stirred for 4 hours. The solvent of the reaction solution was evaporated under reduced pressure, diethyl ether was added to a residue thus obtained, and the precipitated solid was filtered, to obtain 3-oxopiperazine-1-carboxylic acid tert-butyl ester (9.36 g, 92%).
¹H-NMR(400MHz, DMSO-d₆)δ: 1.40(9H, s), 3.15(2H, br), 3.45(2H, br), 3.81(2H, br), 8.03(1H, br).
ESI-MSm/z: 201(M+H)⁺.

### 2) 4-Ethyl-3-oxopiperazine-1-carboxylic acid tert-butyl ester

To a solution of 3-oxopiperazine-1-carboxylic acid tert-butyl ester (9.36 g) of the above in N, N-dimethylformamide (105 mL), sodium hydride (1.361 g) was added at 0°C, and the resultant mixture was stirred for 25 minutes. Ethyl iodide (4.48 mL) was added to the reaction solution, and the mixture was stirred for 15 hours at 40°C. Cold water and ethyl acetate were added to the reaction solution, and the mixture was partitioned. Furthermore, the aqueous layer was extracted with ethyl acetate, and the organic layers were combined and washed with saturated saline and dried over anhydrous sodium sulfate. After separation by filtration, a residue obtained by evaporating the solvent under reduced pressure was purified by silica gel column chromatography (chloroform-acetone), to obtain 4-ethyl-3-oxopiperazine-1-carboxylic acid tert-butyl ester (8.69 g, 81%) as a pale yellow transparent oil.
¹H-NMR(400MHz, CDCl₃)δ: 1.15(3H, t, J=7.1Hz), 1.46(9H, s), 3.33(2H, t, J=5.4Hz), 3.46(2H, q, J=7.1Hz), 3.64(2H, t, J=5.4Hz), 4.06(2H, s).
EI-MSm/z: 228(M⁺).

### 3) Title compound

To a solution of 4-ethyl-3-oxopiperazine-1-carboxylic acid tert-butyl ester (8.69 g) of the above in dichloromethane (170 mL), a 4 M hydrochloric acid-dioxane solution (60 mL) was added at 0°C, and the resultant mixture was stirred for 4 hours at room temperature. The solvent of the reaction solution was evaporated under reduced pressure, and the title compound (5.88 g, 94%) was obtained as an oily product.
¹H-NMR(400MHz, DMSO-d₆)δ: 1.04(3H, t, J=7.1Hz), 3.29-3.39(4H, m), 3.53(2H, t like, J=5.6Hz), 3.63(2H, br), 10. 00 (2H, br).
FAB-MSm/z: 129(M+H)⁺.

[Reference Example 57] 1-(6-Methoxypyridazin-3-yl)-5-(1H-pyrrol-3-yl)-1H-pyrazole-3-carboxylic acid

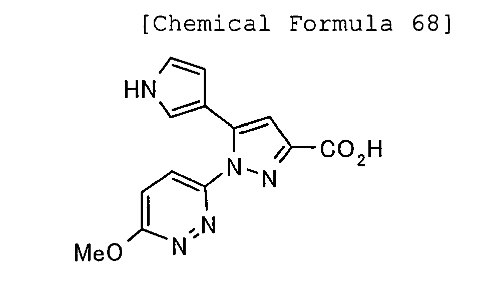

### 1) 2, 4-Dioxo-4-(1-phenylsulfonyl-1H-pyrrol-3-yl)butanoic acid methyl ester

Under cooling to -78°C, lithium bis(trimethylsilyl)amide (a 1.0 M solution in tetrahydrofuran, 9.85 mL) was added to a solution of 3-acetyl-1-phenylsulfonyl-1H-pyrrole (2.23 g) in tetrahydrofuran (10 mL), and the resultant mixture was stirred for 45 minutes. A solution of dimethyl oxalate (1.60 g) in tetrahydrofuran (5.0 mL) was added to the reaction solution, and the mixture was stirred for 22 hours at room temperature. The solvent of the reaction solution was evaporated under reduced pressure, water, an aqueous 1 M hydrochloric acid solution and ethyl acetate were added to a residue thus obtained, and the mixture was partitioned. Further, the aqueous layer was extracted with ethyl acetate, and the organic layers were combined and dried over anhydrous sodium sulfate. After separation by filtration, the solvent was evaporated under reduced pressure, and 2, 4-dioxo-4-(1-phenylsulfonyl-1H-pyrrol-3-yl)butanoic acid methyl ester (1.76 g, 59%) was obtained as a solid.
¹H-NMR(400MHz, DMSO-d₆)δ: 3.83(3H, s), 6.82(1H, br), 7.00(1H, br), 7.52(1H, br), 7.69(2H, t, J=7.6Hz), 7.80(1H, t, J=7.6Hz), 8.11(2H, d, J=7.6Hz), 8.63(1H, br).
ESI-MSm/z: 336(M+H)⁺.

### 2) 1-(6-Chloropyridazin-3-yl)-5-(1-phenylsulfonyl-1H-pyrrol-3-yl)-1H-pyrazole-3-carboxylic acid methyl ester

To a solution of 2, 4-dioxo-4-(1-phenylsulfonyl-1H-pyrrol-3-yl)butanoic acid methyl ester (1.76 g) of the above in methanol (30 mL), 3-chloro-6-hydrazinopyridazine (0.759 g) was added at room temperature, and the resultant mixture was heated to reflux overnight. After air cooling, concentrated hydrochloric acid (1.0 mL) was added to the reaction solution, and the mixture was heated to reflux for 3 hours. After air cooling, the solvent of the reaction solution was evaporated under reduced pressure, a saturated aqueous solution of sodium hydrogen carbonate, water, and ethyl acetate were added to a residue thus obtained, and the mixture was partitioned. Further, the aqueous layer was extracted with ethyl acetate, and the organic layers were combined and dried over anhydrous sodium sulfate. After separation by filtration, a residue obtained by evaporating the solvent under reduced pressure was purified by silica gel column chromatography (dichloromethane-diethyl ether), to obtain a mixture containing 1-(6-chloropyridazin-3-yl)-5-(1-phenylsulfonyl-1H-pyrrol-3-yl)-1H-pyrazole-3-carboxylic acid methyl ester and 1-(6-methoxypyridazin-3-yl)-5-(1-phenylsulfonyl-1H-pyrrol-3-yl)-1H-pyrazole-3-carboxylic acid methyl ester (1.85 g, ratio 8: 1) as an amorphous material.
¹H-NMR(400MHz, CDCl₃)δ: 3.96(3H, s), 6.38-6.41(1H, m), 7.00(1H, s), 7.12-7.17(1H, m), 7.50-7.71(5H, m), 7.90(2H, d, J=7.3Hz), 8.03(1H, d, J=9.0Hz).
ESI-MSm/z: 444(M+H)⁺.

### 3) Title compound

To a mixed solution of a mixture containing 1-(6-chloropyridazin-3-yl)-5-(1-phenylsulfonyl-1H-pyrrol-3-yl)-1H-pyrazole-3-carboxylic acid methyl ester and 1-(6-methoxypyridazin-3-yl)-5-(1-phenylsulfonyl-1H-pyrrol-3-yl)-1H-pyrazole-3-carboxylic acid methyl ester (1.85 g) of the above in methanol (30 mL) and tetrahydrofuran (50 mL), sodium methoxide (0.676 g) was added at room temperature, and the mixture was stirred for 4 hours. An aqueous 1 M sodium hydroxide solution (9.0 mL) was added to the reaction solution, and the mixture was stirred overnight. An aqueous 1 M hydrochloric acid solution was added to the reaction solution to neutralize it, and the solid precipitated by evaporating the solvent of the reaction solution was filtered, to obtain the title compound (1.20 g, 80% in two processes).
¹H-NMR(400MHz, DMSO-d₆)δ: 4.43(3H, s), 6.22(1H, t, J=1.7Hz), 7.01-7.07(1H, m), 7.09-7.15(1H, m), 7.25(1H, s), 7.81(1H, d, J=9.2Hz), 8.18(1H, d, J=9.2Hz), 11.38(1H, br).
FAB-MSm/z: 286(M+H)⁺.

[Reference Example 58] 5-(1-Ethyl-1H-pyrrol-3-yl)-1-(6-methylpyridin-3-yl)-1H-pyrazole-3-carboxylic acid

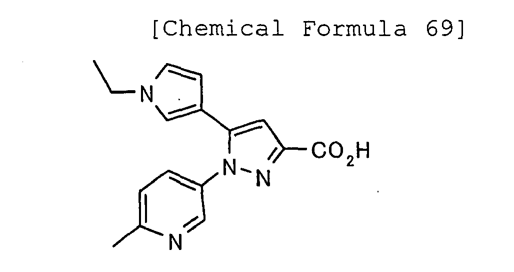

### 1) 2, 4-Dioxo-4-(1-phenylsulfonyl-1H-pyrrol-3-yl)butanoic acid ethyl ester

Under cooling to -78°C, lithium bis(trimethylsilyl)amide (a 1.0 M solution in tetrahydrofuran, 46.3 mL) was added to a solution of 3-acetyl-1-phenylsulfonyl-1H-pyrrole (10.7 g) in tetrahydrofuran (40 mL), and the resultant mixture was stirred for 45 minutes. Diethyl oxalate (8.60 mL) was added to the reaction solution, and then the mixture was stirred for 22 hours at room temperature. The solvent of the reaction solution was evaporated under reduced pressure, water, an aqueous 1 M hydrochloric acid solution, and ethyl acetate were added to a residue thus obtained, and the mixture was partitioned. Further, the aqueous layer was extracted with ethyl acetate, and the organic layers were combined and dried over anhydrous sodium sulfate. After separation by filtration, the solvent was evaporated under reduced pressure, and 2, 4-dioxo-4-(1-phenylsulfonyl-1H-pyrrol-3-yl)butanoic acid ethyl ester (12.4 g, 84%) was obtained as a solid.
ESI-MSm/z: 350(M+H)⁺.

### 2) 1-(6-Methylpyridin-3-yl)-5-(1-phenylsulfonyl-1H-pyrrol-3-yl)-1H-pyrazole-3-carboxylic acid ethyl ester

A solution of 2, 4-dioxo-4-(1-phenylsulfonyl-1H-pyrrol-3-yl)butanoic acid ethyl ester (1.525 g) of the above and 5-hydrazino-2-methylpyridine (678 mg) of Reference Example 7 in ethanol (40 mL) was heated to reflux for 30 minutes. After air cooling, concentrated hydrochloric acid (0.0674 mL) was added to the reaction solution, and the mixture was heated to reflux for 20 hours. After air cooling, the solvent was evaporated under reduced pressure, a saturated aqueous solution of sodium hydrogen carbonate and chloroform were added to a residue thus obtained, and the mixture was partitioned. Further, the aqueous layer was extracted with chloroform, and the organic layers were combined, washed with saturated saline and then dried over anhydrous sodium sulfate. After separation by filtration, a residue obtained by evaporating the solvent under reduced pressure was purified by silica gel column chromatography (chloroform-acetone), to obtain 1-(6-methylpyridin-3-yl)-5-(1-phenylsulfonyl-1H-pyrrol-3-yl)-1H-pyrazole-3-carboxylic acid ethyl ester (0.96 g, 50%) as an oily product.
¹H-NMR(400MHz, CDCl₃)δ: 1.40(3H, t, J=7.1Hz), 2.64(3H, s), 4.43(2H, q, J=7.1Hz), 6.05-6.08(1H, m), 6.94-7.00(2H, m), 7.08-7.13(1H, m), 7.21(1H, d, J=8.8Hz), 7.51-7.70(4H, m), 7.78-7.82(2H, m), 8.46(1H, d, J=2.7Hz).
FAB-MSm/z: 437(M+H)⁺.

### 3) 1-(6-Methylpyridin-3-yl)-5-(1H-pyrrol-3-yl)-1H-pyrazole-3-carboxylic acid

To a solution of 1-(6-methyl-3-pyridyl)-5-(1-phenylsulfonyl-1H-pyrrol-3-yl)-1H-pyrazole-3-carboxylic acid ethyl ester (0.96 g) of the above in ethanol (15 mL), an aqueous 1 M sodium hydroxide solution (15 mL) was added at room temperature, and the resultant mixture was stirred for 16 hours. An aqueous 1 M hydrochloric acid solution was added to the reaction solution to neutralize it, and the solid precipitated by evaporating the solvent of the reaction solution under reduced pressure was filtered, to obtain 1-(6-methylpyridin-3-yl)-5-(1H-pyrrol-3-yl)-1H-pyrazole-3-carboxylic acid (0.487 g, 83%).
¹H-NMR(400MHz, DMSO-d₆)δ: 2.56(3H, s), 5.83-5.86(1H, m), 6.67-6.75(2H, m), 6.89(1H, s), 7.42(1H, d, J=8.3Hz), 7.77(1H, dd, J=2.7, 8.3Hz), 8.47(1H, d, J=2.7Hz), 11.06(1H, br).
FAB-MSm/z: 269(M+H)⁺.

### 4) 1-(6-Methylpyridin-3-yl)-5-(1H-pyrrol-3-yl)-1H-pyrazole-3-carboxylic acid ethyl ester

To a solution of 1-(6-methylpyridin-3-yl)-5-(1-phenylsulfonyl-1H-pyrrol-3-yl)-1H-pyrazole-3-carboxylic acid ethyl ester (0.955 g) of the above in ethanol (20 mL), a 20% sodium ethoxide-ethanol solution (2.88 g) was added at room temperature, and the resultant mixture was stirred for 1 hour. Under cooling to 0°C, a 1 M hydrochloric acid-ethanol solution was added to the reaction solution, and the solvent of the reaction solution was evaporated under reduced pressure. Then, chloroform and a saturated aqueous solution of sodium hydrogen carbonate were added to a residue thus obtained, and the mixture was partitioned. Further, the aqueous layer was extracted with chloroform, and the organic layers were then combined, washed with saturated saline and dried over anhydrous sodium sulfate. After separation by filtration, a residue obtained by evaporating the solvent under reduced pressure was purified by silica gel column chromatography (chloroform-acetone), to obtain 1-(6-methyl pyridin-3-yl)-5-(1H-pyrrol-3-yl)-1H-pyrazole-3-carboxylic acid ethyl ester (0.26 g, 40%) as a solid.
¹H-NMR(400MHz, CDCl₃)δ: 1.41(3H, t, J=7.1Hz), 2.62(3H, s), 4.44(2H, q, J=7.1Hz), 6.00-6.05(1H, m), 6.56-6.62(1H, m), 6.70-6.75(1H, m), 6.95(1H, s), 7.22(1H, d, J=8.3Hz), 7.70(1H, dd, J=2.7, 8.3Hz), 8.35(1H, br), 8.56(1H, d, J=2.7Hz).
FAB-MSm/z: 297(M+H)⁺.

### 5) 5-(1-Ethyl-1H-pyrrol-3-yl)-1-(6-methylpyridin-3-yl)-1H-pyrazole-3-carboxylic acid ethyl ester

To a solution of 1-(6-methylpyridin-3-yl)-5-(1H-pyrrol-3-yl)-1H-pyrazole-3-carboxylic acid ethyl ester (0.254 g) of the above in N, N-dimethylformamide (7.0 mL), sodium hydride (60%, 43.9 mg) and ethyl iodide (0.0824 mL) were added at 0°C, and then the resultant mixture was stirred for 1.5 hours at room temperature. A saturated aqueous ammonium chloride solution and ethyl acetate were added to the reaction solution, and the mixture was partitioned. Further, the aqueous layer was extracted with ethyl acetate, and the organic layers were then combined, washed with saturated saline and dried over anhydrous sodium sulfate. After separation by filtration, a residue obtained by evaporating the solvent under reduced pressure was purified by silica gel thin layer chromatography (chloroform-acetone), to obtain 5-(1-ethyl-1H-pyrrol-3-yl)-1-(6-methylpyridin-3-yl)-1H-pyrazole-3-carboxylic acid ethyl ester (0.277 g, 99%) as an oily product.
¹H-NMR(400MHz, CDCl₃)δ: 1.37(3H, t, J=7.3Hz), 1.41(3H, t, J=7.1Hz), 2.62(3H, s), 3.85(2H, q, J=7.3Hz), 4.43(2H, q, J=7.1Hz), 5.85-5.89(1H, m), 6.49(1H, t, J=2.0Hz), 6.55(1H, t, J=2.0Hz), 6.91(1H, s), 7.23(1H, d, J=8.3Hz), 7.71(1H, dd, J=2.4, 8.3Hz), 8.57(1H, d, J=2.4Hz).
FAB-MSm/z: 325(M+H)⁺.

### 6) Title compound

To a mixed solution of 5-(1-ethyl-1H-pyrrol-3-yl)-1-(6-methylpyridin-3-yl)-1H-pyrazole-3-carboxylic acid ethyl ester (0.277 g) of the above in methanol (3.0 mL) and water (1.8 mL), lithium hydroxide monohydrate (40.1 mg) was added at room temperature, and the resultant mixture was stirred for 2 hours. The solvent of the reaction solution was evaporated under reduced pressure, an aqueous 1 M hydrochloric acid solution was added to a residue thus obtained, and the precipitated solid was filtered, to obtain the title compound (0.193 g, 76%).
¹H-NMR(400MHz, DMSO-d₆)δ: 1.19(3H, t, J=7.3Hz), 2.49(3H, s), 3.77(2H, q, J=7.3Hz), 5.63-5.66(1H, m), 6.68(1H, t like, J=2.7Hz), 6.75(1H, t like, J=2.2Hz), 6.79(1H, s), 7.34(1H, d, J=8.3Hz), 7.70(1H, dd, J=2.7, 8.3Hz), 8.40(1H, d like, J=2.7Hz).
FAB-MSm/z: 297(M+H)⁺.

[Reference Example 59] 5-(5-Cyanopyridin-2-yl)-1-(6-methoxypyridazin-3-yl)-1H-pyrazole-3-carboxylic acid

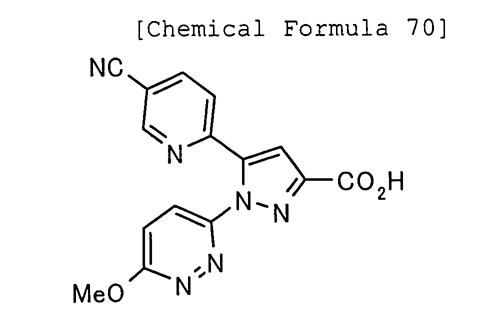

### 1) 2-Acetyl-5-cyanopyridine

Ammonium peroxodisulfate (10.3 g) was slowly added to a mixture of 3-cyanopyridine (3.12 g), pyruvic acid (6.23 mL) and silver nitrate (1.27 g) in dichloromethane (150 mL) and water (150 mL) at room temperature. Under ice cooling, sulfuric acid (3.2 mL) was slowly added to the reaction solution, and then the mixture was stirred for 1.5 hours at 40°C. Under ice cooling, the reaction solution was alkalinized by adding an aqueous 1 M sodium hydroxide solution, and then extracted with dichloromethane. The organic solvent was dried over anhydrous sodium sulfate. After separation by filtration, a residue obtained by evaporating the solvent under reduced pressure was purified by silica gel column chromatography (hexane-ethyl acetate), to obtain 2-acetyl-5-cyanopyridine (903 mg, 21%) as a solid.
¹H-NMR(400MHz, CDCl₃)δ: 2.75(3H, s), 8.13-8.16(2H, m), 8.95(1H, d, J=1.2Hz).

### 2) 4-(5-Cyanopyridin-2-yl)-2, 4-dioxobutanoic acid methyl ester

Under an argon atmosphere and cooling to -78°C, to a solution of 2-acetyl-5-cyanopyridine (5.10 g) of the above in anhydrous tetrahydrofuran (250 mL), lithium bis(trimethylsilyl)amide (a 1.0 M solution in tetrahydrofuran, 38.4 mL) was added, and the resultant mixture was stirred for 35 minutes. A solution of dimethyl oxalate (6.18 g) in tetrahydrofuran (100 mL) was added to the reaction solution, and the mixture was stirred for 5 minutes, and then stirred for 75 minutes at room temperature. Ice and an aqueous 1 M hydrochloric acid solution were added to the reaction solution, and the mixture was extracted with ethyl acetate. The organic layer was washed with saturated saline, and then dried over anhydrous sodium sulfate. After separation by filtration, the solvent was evaporated under reduced pressure, hexane was added to a residue thus obtained, and a precipitate was obtained as 4-(5-cyanopyridin-2-yl)-2, 4-dioxobutanoic acid methyl ester (5.325 g, 66%).
ESI-MSm/z: 233(M+H)⁺.

### 3) 1-(6-Chloropyridazin-3-yl)-5-(5-cyanopyridin-2-yl)-1H-pyrazolcarboxylic acid methyl ester

A solution of 4-(5-cyanopyridin-2-yl)-2, 4-dioxobutanoic acid methyl ester (2.32 g) of the above and 3-chloro-6-hydrazinopyridazine (1.44 g) in methanol (100 mL) was heated to reflux overnight. Acetic acid (2 mL) was added to the reaction solution, and the mixture was heated to reflux for 2 hours. Furthermore, concentrated hydrochloric acid (1 mL) was added to the reaction solution, and the mixture was heated to reflux for 1.5 hours. After air cooling, a precipitated solid was separated by filtration, and the obtained solid was purified by silica gel column chromatography (chloroform-methanol), to obtain 1-(6-chloropyridazin-3-yl)-5-(5-cyanopyridin-2-yl)-1H-pyrazolcarboxylic acid methyl ester (2.94 g, 86%) as a solid.
¹H-NMR(400MHz, CDCl₃)δ: 4.01(3H, s), 7.32(1H, s), 7.73-7.77(2H, m), 8.07(1H, dd, J=8.2, 2.1Hz), 8.14(1H, d, J=9.0Hz), 8.66-8.67(1H, m).
ESI-MSm/z: 341(M+H)⁺.

### 4) 5-(5-Cyanopyridin-2-yl)-1-(6-methoxypyridazin-3-yl)-1H-pyrazole-3-carboxylic acid methyl ester

Under an argon atmosphere, a solution of the 1-(6-chloropyridazin-3-yl)-5-(5-cyanopyridin-2-yl)-1H-pyrazol-carboxylic acid methyl ester (1.19 g) and sodium methoxide (189 mg) in methanol (70 mL) was heated to reflux for 20 minutes. An aqueous 1 M hydrochloric acid solution and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (dichloromethane-acetone) and silica gel thin layer chromatography (dichloromethane-acetone), to obtain 5-(5-cyanopyridin-2-yl)-1-(6-methoxypyridazin-3-yl)-1H-pyrazole-3-carboxylic acid methyl ester (501 mg, 42%) as a solid.
¹H-NMR(400MHz, CDCl₃)δ: 4.00(3H, s), 4.12(3H, s), 7.19(1H, d, J=9.3Hz), 7.32(1H, s), 7.71(1H, d, J=8.3Hz), 7.98(1H, d, J=9.3Hz), 8.03(1H, dd, J=8.3, 2.2Hz), 8.66(1H, s).

### 5) Title compound

To a mixed solution of 5-(5-cyanopyridin-2-yl)-1-(6-methoxypyridazin-3-yl)-1H-pyrazole-3-carboxylic acid methyl ester (251 mg) in tetrahydrofuran (50 mL) and water (15 mL), lithium hydroxide monohydrate (32 mg) was added at room temperature, and the resultant mixture was stirred overnight. An aqueous 1 M hydrochloric acid solution (750µl), a mixed solvent of chloroform-water (10:1) and water were added to the reaction solution, and the mixture was partitioned. The organic solvent was dried over anhydrous sodium sulfate. After separation by filtration, the solvent was evaporated under reduced pressure, and the title compound (183 mg, 76%) was obtained as a solid.
ESI-MSm/z: 323(M+H)⁺.

[Reference Example 60] 5-(1-Methyl-1H-imidazol-4-yl)-1-(6-methylpyridin-3-yl)-1H-pyrazole-3-carboxylic acid

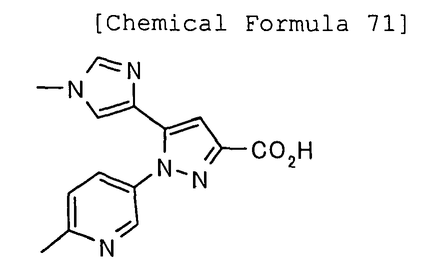

### 1) 5-(1-Methyl-1H-imidazol-4-yl)-1-(6-methylpyridin-3-yl)-1H-pyrazole-3-carboxylic acid ethyl ester

Under an argon atmosphere and cooling to -78°C, lithium bis(trimethylsilyl)amide (a 1.0 M solution in tetrahydrofuran, 6.65 mL) was added dropwise to a solution of 4-acetyl-1-methyl-1H-imidazole (0.75 g) in tetrahydrofuran (40 mL), and the resultant mixture was stirred for 30 minutes. Diethyl oxalate (1.64 mL) was added to the reaction solution, and the mixture was stirred for 90 minutes at room temperature. Water and diethyl ether were added to the reaction solution, and the mixture was partitioned. An aqueous 1 M hydrochloric acid solution (6.70 mL) was added to the aqueous layer, which was then extracted with a mixed solvent of chloroform-methanol (10: 1). The organic layer was dried over anhydrous sodium sulfate. After separation by filtration, a residue obtained by evaporating the solvent under reduced pressure and 5-hydrazino-2-methylpyridine (676 mg) of Reference Example 7 were dissolved in a 1 M hydrochloric acid-ethanol solution (40 mL), and then the solution was heated to reflux for 45 minutes. After air cooling, a saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-methanol), to obtain 5-(1-methyl-1H-imidazol-4-yl)-1-(6-methylpyridin-3-yl)-1H-pyrazole-3-carboxylic acid ethyl ester (705 mg, 45%) as a red brown solid.
¹H-NMR(400MHz, CDCl₃)δ: 1.41(3H, t, J=7.2Hz), 2.63(3H, s), 3.64(3H, s), 4.44(2H, q, J=7.1Hz), 6.64(1H, s), 7.15(1H, s), 7.26(1H, d, J=8.3Hz), 7.40(1H, s), 7.78(1H, dd, J=8.3, 2.7Hz), 8.54(1H, d, J=2.7Hz).
ESI-MS m/z: 312(M+H)⁺.

### 2) Title compound

The title compound (444 mg, 70%) was obtained as a solid by the same method as that in Reference Example 59-(5), using 5-(1-methyl-1H-imidazol-4-yl)-1-(6-methylpyridin-3-yl)-1H-pyrazole-3-carboxylic acid ethyl ester (694 mg) of the above and lithium hydroxide dihydrate (112 mg).
¹H-NMR(400MHz, DMSO-d₆)δ: 2.54(3H, s), 3.61(3H, s), 6.96(1H, s), 7.17(1H, s), 7.37(1H, d, J=8.3Hz), 7.59(1H, s), 7.77(1H, dd, J=8.3, 2.7Hz), 8.47(1H, d, J=2.4Hz).
ESI-MSm/z: 284(M+H)⁺.

[Reference Example 61] 1-(6-Methoxy-3-pyridyl)-5-(1-methyl-1H-pyrazol-4-yl)-1H-pyrazole-3-carboxylic acid

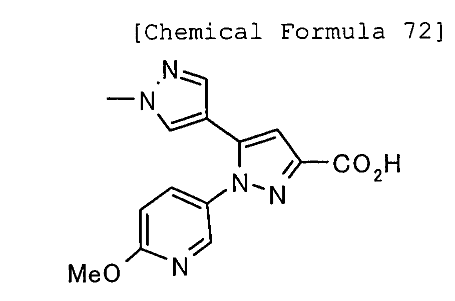

### 1) 1-Methyl-1H-pyrazole-4-carbaldehyde

Under an argon atmosphere, phosphorus oxychloride (65.3 mL) was added dropwise to N, N-dimethylformamide (54.2 mL) at 0°C over 30 minutes, and then the resultant mixture was stirred for 1 hour at room temperature. The reaction solution was stirred for 10 minutes at 80°C, and then 1-methylpyrazole (25.0 g) was added dropwise thereto over 30 minutes. Furthermore, the reaction solution was stirred for 1 hour at 85°C, for 3 hours at 100°C, and for 1 hour at 115°C. After air cooling, the reaction solution was added to ice water (1 L), and stirred for 20 hours. An aqueous 1 M sodium hydroxide solution (2 L) and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-methanol), to obtain 1-methyl-1H-pyrazole-4-carbaldehyde (22.1 g, 66%) as an oily product.
¹H-NMR(400MHz, CDCl₃)δ: 3.97(3H, s), 7.91(1H, s), 7.96(1H, s), 9.85(1H, s).
ESI-MSm/z: 111(M+H)⁺.

### 2) 1-(1-Methyl-1H-pyrazol-4-yl)ethanol

Under an argon atmosphere and cooling to -78°C, to a solution of 1-methyl-1H-pyrazole-4-carbaldehyde (22.0 g) of the above in tetrahydrofuran (220 mL), magnesium methyl bromide (a 0.84 M solution in tetrahydrofuran, 250 mL) was added dropwise over 50 minutes, and then the resultant mixture was stirred for 20 minutes. The reaction solution was stirred for 50 minutes at 0°C, and then water and chloroform were added to the reaction solution, and stirred. The insoluble of the reaction solution was separated by filtration using Celite. Water and chloroform were added to the filtrate, and the mixture was partitioned. The organic layer was dried over anhydrous sodium sulfate. After separation by filtration, a residue obtained by evaporating the solvent under reduced pressure was purified by silica gel column chromatography (methanol-chloroform), to obtain 1-(1-methyl-1H-pyrazol-4-yl)ethanol (20.2 g, 80%) as an oily product.
¹H-NMR(400MHz, CDCl₃)δ: 1.49-1.51(3H, m), 3.87(3H, s), 4.87-4.92(1H, m), 7.33(1H, s), 7.44(1H, s).
ESI-MSm/z: 127(M+H)⁺.

### 3) 1-(1-Methyl-1H-pyrazol-4-yl)ethanone

Under an argon atmosphere, to a solution of the 1-(1-methyl-1H-pyrazol-4-yl)ethanol (20.2 g) in dichloromethane (202 mL), manganese(IV) oxide (active, 209 g) was added at 0°C. After stirring the mixture for 14 hours at room temperature, the insoluble matter in the reaction solution was separated by filtration, and the solvent of the obtained filtrate was evaporated under reduced pressure, to obtain 1-(1-methyl-1H-pyrazol-4-yl)ethanone (18.1 g, 91%) as a solid.
¹H-NMR(400MHz, CDCl₃)δ: 2.42(3H, s), 3.94(3H, s), 7.86(1H, s), 7.89(1H, s).
ESI-MSm/z: 125(M+H)⁺.

### 4) 4-(1-Methyl-1H-pyrazol-4-yl)-2, 4-dioxobutanoic acid ethyl ester

Under an argon atmosphere, diethyl oxalate (17.5 mL) was added to a solution of sodium ethoxide (8.77 g) in ethanol (80 mL) at room temperature, and the mixture was stirred for 10 minutes. A solution of 1-(1-methyl-1H-pyrazol-4-yl)ethanone (8.00 g) in ethanol (80 mL) was added to the reaction solution, and the mixture was stirred for 90 minutes. Water and diethyl ether were added to the reaction solution, and the mixture was partitioned. A saturated aqueous ammonium chloride solution was added to the aqueous layer, and the mixture was extracted with chloroform. The organic layer was dried over anhydrous sodium sulfate. After separation by filtration, the solvent was evaporated under reduced pressure, to obtain 4-(1-methyl-1H-pyrazol-4-yl)-2, 4-dioxobutanoic acid ethyl ester (11.4 g, 79%) as a solid.
¹H-NMR(400MHz, CDCl₃)δ: 1.39-1.42(3H, m), 3.98(3H, s), 4.36-4.41(2H, m), 6.69(1H, s), 7.98(2H, m).
EI-MSm/z: 224(M⁺).

### 5) 1-(6-Methoxy-3-pyridyl)-5-(1-methyl-1H-pyrazol-4-yl)-1H-pyrazole-3-carboxylic acid ethyl ester

Under an argon atmosphere, a solution of 4-(1-methyl-1H-pyrazol-4-yl)-2, 4-dioxobutanoic acid ethyl ester (8.55 g) of the above and 5-hydrazino-2-methoxypyridine (5.31 g) of Reference Example 2 in ethanol (171 mL) was heated to reflux for 30 minutes. Acetic acid (10.9 mL) was added to the reaction solution, and the mixture was heated to reflux for 13 hours. After air cooling, saturated sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 1-(6-methoxy-3-pyridyl)-5-(1-methyl-1H-pyrazol-4-yl)-1H-pyrazole-3-carboxylic acid ethyl ester (11.0 g, 88%) as a solid.
¹H-NMR(400MHz, CDCl₃)δ: 1.42(3H, t, J=7.2Hz), 3.86(3H, s), 3.99(3H, s), 4.44(2H, q, J=7.2Hz), 6.82(1H, dd, J=8.8, 0.7Hz), 6.96(1H, s), 7.16(1H, s), 7.30(1H, m), 7.63(1H, dd, J=8.8, 2.7Hz), 8.21-8.22(1H, m).
EI-MSm/z: 327(M⁺).

### 6) Title compound

To a mixed solution of 1-(6-methoxy-3-pyridyl)-5-(1-methyl-1H-pyrazol-4-yl)-1H-pyrazole-3-carboxylic acid ethyl ester (11.0 g) of the above in tetrahydrofuran (110 mL) and methanol (110 mL), an aqueous 1 M sodium hydroxide solution (84.0 mL) was added at room temperature, and the resultant mixture was stirred for 2 hours. The reaction solution was neutralized by adding an aqueous 1 M hydrochloric acid solution (84.0 mL), and water and chloroform were added to the reaction solution, which was then partitioned. The solvent of the organic layer was evaporated under reduced pressure, diethyl ether was added to a residue thus obtained, and the precipitated solid was filtered, to obtain the title compound (8.84 g, 88%).
¹H-NMR(400MHz, DMSO-d₆)δ: 3.79(3H, s), 3.93(3H, s), 6.98(1H, d, J=8.8Hz), 7.01(1H, s), 7.33(1H, s), 7.66(1H, s), 7.80-7.83(1H, m), 8.27(1H, d, J=2.7Hz), 12.93(1H, br).
EI-MSm/z: 299(M⁺).

[Reference Example 62] 1-(6-Methoxy-3-pyridyl)-5-(thiazol-5-yl)-1H-pyrazole-3-carboxylic acid

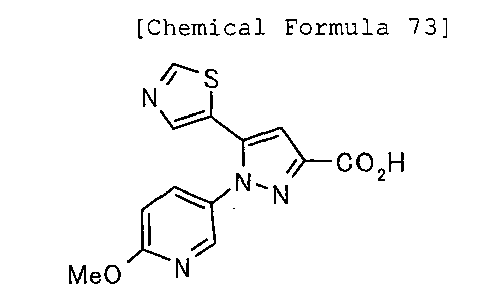

### 1) 5-Acetylthiazole

Under an argon atmosphere, manganese(IV) oxide (active, 5.72 g) was added to a solution of 1-(5-thiazolyl)ethanol (D.S. Noyce, et al., J. Org. Chem., 1973, 38, 3316; 1.70 g) in dichloromethane (34 mL) at room temperature, and the mixture was stirred for 1.5 hours. Further, manganese(IV) oxide (active, 5.72 g) was added thereto, and the mixture was stirred for 2 hours. Further, manganese(IV) oxide (active, 5.72 g) was added thereto, and the mixture was stirred for 3.5 hours. The insoluble in the reaction solution was separated by filtration, and the solvent of the obtained filtrate was evaporated under reduced pressure, to obtain 5-acetylthiazole (1.17 g, 70%) as a solid.
¹H-NMR(400MHz, CDCl₃)δ: 2.63(3H, m), 8.43(1H, s), 9.01(1H, s).
EI-MSm/z: 127(M⁺).

### 2) 4-(Thiazol-5-yl)-2, 4-dioxobutanoic acid ethyl ester

Under an argon atmosphere and cooling to -78°C, to a solution of the 5-acetylthiazole (1.15 g) in tetrahydrofuran (23 mL), lithium bis(trimethylsilyl)amide (a 1.0 M solution in tetrahydrofuran, 9.95 mL) was added dropwise, and the resultant mixture was stirred for 1 hour. Diethyl oxalate (2.46 mL) was added dropwise to the reaction solution, and the mixture was stirred for 15 minutes at -78°C, for 45 minutes at 0°C, and for 3.5 hours at room temperature. Water and diethyl ether were added to the reaction solution, and the mixture was partitioned. A saturated aqueous ammonium chloride solution was added to the aqueous layer, which was then extracted chloroform. The organic layer was dried over anhydrous sodium sulfate. After separation by filtration, the solvent was evaporated under reduced pressure, and 4-(thiazol-5-yl)-2, 4-dioxobutanoic acid ethyl ester (1.64 g, 80%) was obtained as a solid.
¹H-NMR(400MHz, CDCl₃)δ: 1.40-1.44(3H, m), 4.39-4.44(2H, m), 6.91(1H, s), 8.57(1H, s), 9.07(1H, s).
EI-MSm/z: 227(M⁺).

### 3) 1-(6-Methoxy-3-pyridyl)-5-(thiazol-5-yl)-1H-pyrazole-3-carboxylic acid ethyl ester

1-(6-Methoxy-3-pyridyl)-5-(thiazol-5-yl)-1H-pyrazole-3-carboxylic acid ethyl ester (1.16 g, 49%) was obtained as a solid by the same method as that in Reference Example 61-(5), using 4-(thiazol-5-yl)-2, 4-dioxobutanoic acid ethyl ester (1.63 g) of the above and 5-hydrazino-2-methoxypyridine (1.10 g) of Reference Example 2 under an argon atmosphere.
¹H-NMR(400MHz, CDCl₃)δ: 1.43(3H, t, J=7.2Hz), 3.99(3H, m), 4.46(2H, q, J=7.2Hz), 6.81-6.84(1H, m), 7.17(1H, m), 7.60-7.63(1H, m), 7.79(1H, s), 8.19(1H, d, J=2.7Hz), 8.78(1H, s).
EI-MSm/z: 330(M⁺).

### 4) Title compound

The title compound (0.710 g, 68%) was obtained as a solid by the same method as that in Reference Example 61-(6), using 1-(6-methoxy-3-pyridyl)-5-(thiazol-5-yl)-1H-pyrazole-3-carboxylic acid ethyl ester (1.15 g) of the above.
¹H-NMR(400MHz, DMSO-d₆)δ: 3.94(3H, s), 7.00(1H, d, J=8.8Hz), 7.32(1H, s), 7.85(1H, dd, J=8.8, 2.7Hz), 8.12(1H, s), 8.31(1H, d, J=2.7Hz), 9.08(1H, s), 13.10(1H, br).
EI-MSm/z: 302(M⁺).

[Reference Example 63] 1-(6-Methoxy-3-pyridyl)-5-(thiazol-2-yl)-1H-pyrazole-3-carboxylic acid

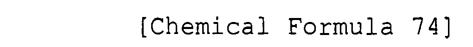

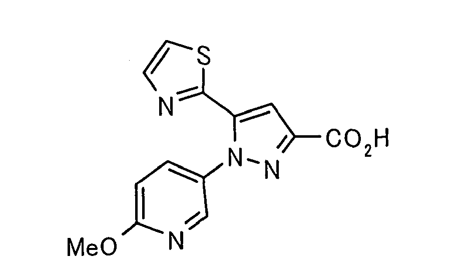

### 1) 4-(Thiazol-2-yl)-2, 4-dioxobutanoic acid ethyl ester

4-(Thiazol-2-yl)-2, 4-dioxobutanoic acid ethyl ester (3.33 g, 74%) was obtained as a solid by the same method as in Reference Example 62-(2), using 2-acetylthiazole (2.50 g), lithium bis(trimethylsilyl)amide (a 1.0 M solution in tetrahydrofuran, 21.6 mL), and diethyl oxalate (5.34 mL) under an argon atmosphere.
¹H-NMR(400MHz, CDCl₃)δ: 1.39-1.43(3H, m), 4.38-4.43(2H, m), 7.41(1H, s), 7.77-7.78(1H, m), 8.08(1H, d, J=2.9Hz).
EI-MSm/z: 227(M⁺).

2) 1-(6-Methoxy-3-pyridyl)-5-(thizol-2-yl)-1H-pyrazole-3-carboxylic acid ethyl ester

1-(6-Methoxy-3-pyridyl)-5-(thiazol-2-yl)-1H-pyrazole-3-carboxylic acid ethyl ester (1.22 g, 25%) was obtained as a solid by the same method as that in Reference Example 61-(5), using 4-(thiazol-2-yl)-2, 4-dioxobutanoic acid ethyl ester (3.32 g) of the above and 5-hydrazino-2-methoxypyridine (2.24) of Reference Example 2 under an argon atmosphere.
¹H-NMR(400MHz, CDCl₃)δ: 1.41-1.45(3H, m), 3.99(3H, s), 4.43-4.49(2H, m), 6.83(1H, dd, J=8.8, 0.7Hz), 7.36-7.37(1H, m), 7.42(1H, m), 7.70(1H, dd, J=8.8, 2.7Hz), 7.81-7.82(1H, m), 8.26(1H, d, J=2.7Hz).
FAB-MSm/z: 331(M+H)⁺.

### 3) Title compound

The title compound (0.951 g, 86%) was obtained as a solid by the same method as that in Reference Example 61-(6), using 1-(6-methoxy-3-pyridyl)-5-(thiazol-2-yl)-1H-pyrazole-3-carboxylic acid ethyl ester (1.21 g) of the above.
¹H-NMR(400MHz, DMSO-d₆)δ: 3.94(3H, s), 7.00(1H, dd, J=8.8, 0.5Hz), 7.39(1H, s), 7.84-7.93(3H, m), 8.36(1H, dd, J=2.7, 0.5Hz), 13.21(1H, br).
FAB-MSm/z: 303(M+H)⁺.

[Reference Example 64] 1-(6-Methoxy-3-pyridyl)-5-(thaizol-4-yl)-1H-pyrazole-3-carboxylic acid

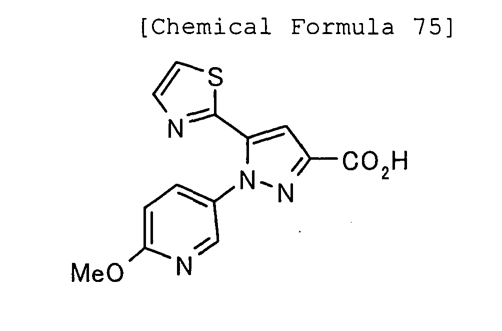

### 1) (4-Thiazolyl)methanol

An aqueous 1 M sodium hydroxide solution (278 mL) was added to 4-(chloromethyl)thiazole hydrochloride (47.2 g), and the resultant mixture was heated to reflux for 75 minutes. After air cooling, 1 M sodium hydroxide (278 mL) was further added thereto, and the mixture was extracted with chloroform. The organic layer was dried over anhydrous sodium sulfate. After separation by filtration, a residue obtained by evaporating the solvent under reduced pressure was purified by silica gel column chromatography (chloroform-methanol), to obtain (4-thiazolyl)methanol (21.8 g, 68%) as an oily product.
¹H-NMR(400MHz, CDCl₃)δ: 3.41(1H, s), 4.83(2H, m), 7.27-7.28(1H, m), 8.81(1H, m).
FAB-MSm/z: 116(M+H)⁺.

### 2) 4-Formylthaizole

Under an argon atmosphere, to a solution of (4-thiazolyl)methanol (21.8 g) of the above in dichloromethane (218 mL), manganese(IV) oxide (active, 247 g) was added at 0°C, and the resultant mixture was stirred for 21 hours. The insoluble in the reaction solution was separated by filtration, and the solvent of the obtained filtrate was evaporated under reduced pressure, to obtain 4-formylthiazole (6.91 g, 32%) as a solid.
¹H-NMR(400MHz, CDCl₃)δ: 8.27(1H, d, J=2.0Hz), 8.93(1H, d, J=2.0Hz), 10.14 (1H, s).
FAB-MSm/z: 114(M+H)⁺.

### 3) 1-(4-Thiazolyl)ethanol

Under an argon atmosphere, to a solution of 4-formylthiazole (6.90 g) of the above in tetrahydrofuran (138 mL), magnesium methyl bromide (a 0.89 M solution in tetrahydrofuran, 72.0 mL) was added dropwise for 30 minutes under cooling to -78°C, and the resultant mixture was stirred for 30 minutes, and then stirred for 150 minutes at 0°C. Water was added to the reaction solution, and the insoluble matter in the reaction solution was separated by filtration using Celite. Water and chloroform were added to the obtained filtrate, and the mixture was partitioned. The organic layer was dried over anhydrous sodium sulfate. After separation by filtration, a residue obtained by evaporating the solvent under reduced pressure was purified by silica gel column chromatography (chloroform-methanol), to obtain 1-(4-thiazolyl)ethanol (7.46 g, 95%) as an oily product.
¹H-NMR(400MHz, CDCl₃)δ: 1.60-1.61(3H, m), 2.73(1H, s), 5.06-5.10(1H, m), 7.22-7.23(1H, m), 8.80(1H, d, J=2.0Hz).
FAB-MSm/z: 130(M+H)⁺.

### 4) 4-Acetylthiazole

Under an argon atmosphere, to a solution of 1-(4-thiazolyl)ethanol (7.45 g) of the above in dichloromethane (149 mL), manganese(IV) oxide (active, 75.2 g) was added at 0°C, and the resultant mixture was stirred for 6 hours. The insoluble in the reaction solution was separated by filtration, and the solvent of the obtained filtrate was evaporated under reduced pressure, to obtain 4-acetylthiazole (6.94 g, 95%) as a solid.
¹H-NMR(400MHz, CDCl₃)δ: 2.71(3H, s), 8.21-8.22(1H, m), 8.84(1H, m).
ESI-MSm/z: 128(M+H)⁺.

### 5) 4-(Thiazol-4-yl)-2, 4-dioxobutanoic acid ethyl ester

Under an argon atmosphere, diethyl oxalate (7.37 mL) was added to a solution of sodium ethoxide (3.69 g) in ethanol (35 mL) at room temperature, and the resultant mixture was stirred for 10 minutes. A solution of 4-acetylthiazole (3.45 g) of the above in ethanol (35 mL) was added to the reaction solution, and the mixture was stirred for 3 hours. Water and diethyl ether were added to the reaction solution, and the mixture was partitioned. A saturated aqueous ammonium chloride solution was added to the aqueous layer, which was then extracted with chloroform. The organic layer was dried over anhydrous sodium sulfate. After separation by filtration, the solvent was evaporated under reduced pressure, and 4-(thiazol-4-yl)-2, 4-dioxobutanoic acid ethyl ester (5.27 g, 85%) was obtained as a solid.
¹H-NMR(400MHz, CDCl₃)δ: 1.41(3H, t, J=7.2Hz), 4.40(2H, q, J=7.2Hz), 7.41(1H, s), 8.33-8.34(1H, m), 8.89(1H, m), 14.69(1H, br).
EI-MSm/z: 227(M⁺).

### 6) 1-(6-Methoxy-3-pyridyl)-5-(thiazol-4-yl)-1H-pyrazole-3-carboxylic acid ethyl ester

1-(6-Methoxy-3-pyridyl)-5-(thiazol-4-yl)-1H-pyrazole-3-carboxylic acid ethyl ester (5.89 g, 77%) was obtained as a solid by the same method as that in Reference Example 61-(5), using 4-(thiazol-4-yl)-2, 4-dioxobutanoic acid ethyl ester (5.26 g) of the above and 5-hydrazino-2-methoxypyridine (3.22 g) of Reference Example 2 under an argon atmosphere.
¹H-NMR(400MHz, CDCl₃)δ: 1.41-1.44(3H, m), 3.98(3H, s), 4.43-4.48(2H, m), 6.82(1H, d, J=8.8Hz), 7.15(1H, d, J=2.0Hz), 7.31(1H, s), 7.69(1H, dd, J=8.8, 2.7Hz), 8.18(1H, d, J=2.7Hz), 8.79(1H, d, J=2.0Hz).
EI-MSm/z: 330(M⁺).

### 7) Title compound

The title compound (5.16 g, 96%) was obtained as a solid by the same method as that in Reference Example 61-(6), using 1-(6-methoxy-3-pyridyl)-5-(thiazol-4-yl)-1H-pyrazole-3-carboxylic acid ethyl ester (5.88) of the above.
¹H-NMR(400MHz, DMSO-d₆)δ: 3.91(3H, s), 6.94(1H, d, J=8.8Hz), 7.27(1H, s), 7.79(1H, dd, J=8.8, 2.7Hz), 7.85(1H, m), 8.22(1H, d, J=2.7Hz), 9.11-9.12(1H, m), 13.07(1H, br).
EI-MSm/z: 302(M⁺).

[Reference Example 65] 1-(6-Methoxy-3-pyridyl)-5-(6-methyl-3-pyridyl)-1H-pyrazole-3-carboxylic acid

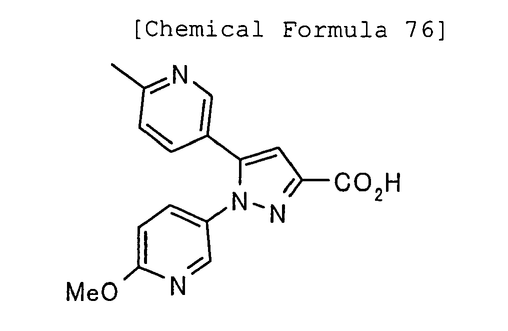

### 1) 6-Methylpyridine-3-carboxylic acid N-methoxy-N-methylamide

6-Methylpyridine-3-carboxylic acid N-methoxy-N-methylamide (17.5 g, 67%) was obtained as an oily product by the same method as that in Reference Example 31-(1), using 6-methylnicotinic acid (20.0 g) and N, O-dimethylhydroxylamine hydrochloride (14.2 g).
¹H-NMR(400MHz, CDCl₃)δ: 2.60(3H, s), 3.38(3H, s), 3.56(3H, s), 7.21(1H, d, J=8.1Hz), 7.93-7.95(1H, m), 8.86(1H, d, J=2.0Hz).
EI-MSm/z: 180(M⁺).

### 2) 1-(6-Methyl-3-pyridyl)-1-ethanone

1-(6-Methyl-3-pyridyl)-1-ethanone (12.3 g, 94%) was obtained as an oily product by the same method as that in Reference Example 8-(2), using 6-methylpyridine-3-carboxylic acid N-methoxy-N-methylamide (17.5 g) of the above and methyllithium (a 0.98 M solution in diethyl ether, 104 mL).
¹H-NMR(400MHz, CDCl₃)δ: 2.61(3H, s), 2.62(3H, s), 7.25-7.27(1H, m), 8.11-8.13(1H, m), 9.04(1H, d, J=2.2Hz).
ESI-MSm/z: 136(M+H)⁺.

### 3) 4-(6-Methyl-3-pyridyl)-2, 4-dioxobutanoic acid ethyl ester

4-(6-Methyl-3-pyridyl)-2, 4-dioxobutanoic acid ethyl ester (5.08 g, 47%) was obtained as a solid by the same method as that in Reference Example 8-(3), using 1-(6-methyl-3-pyridyl)-1-ethanone (6.29 g) of the above, lithium bis(trimethylsilyl)amide (a 1.0 M solution in tetrahydrofuran, 51.2 mL) and diethyl oxalate (12.6 mL).
¹H-NMR(400MHz, CDCl₃)δ: 1.39-1.42(3H, m), 2.65(3H, s), 4.37-4.42(2H, m), 7.03(1H, s), 7.30(1H, d, J=8.3Hz), 8.13-8.16(1H, m), 9.07(1H, d, J=2.2Hz).
EI-MSm/z: 235(M⁺).

### 4) 1-(6-Methoxy-3-pyridyl)-5-(6-methyl-3-pyridyl)-1H-pyrazole-3-carboxylic acid ethyl ester

Under an argon atmosphere, a solution of 4-(6-methyl-3-pyridyl)-2, 4-dioxobutanoic acid ethyl ester (5.06 g) of the above and 5-hydrazino-2-methoxypyridine (2.99 g) of Reference Example 2 in ethanol (101 mL) was heated to reflux for 30 minutes, and then acetic acid (6.16 mL) was added to the reaction solution, which was then heated to reflux for 42 hours. After air cooling, saturated sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 1-(6-methoxy-3-pyridyl)-5-(6-methyl-3-pyridyl)-1H-pyrazole-3-carboxylic acid ethyl ester (4.68 g, 64%) as an oily product.
¹H-NMR(400MHz, CDCl₃)δ: 1.41-1.44(3H, m), 2.57 (3H, s), 3.94(3H, s), 4.43-4.49(2H, m), 6.77(1H, d, J=8.8Hz), 7.08(1H, s), 7.13(1H, d, J=8.1Hz), 7.39(1H, dd, J=8.1, 2.2Hz), 7.59(1H, dd, J=8.8, 2.7Hz), 8.08(1H, d, J=2.7Hz), 8.41(1H, d, J=2.2Hz).
EI-MSm/z: 338(M⁺).

### 5) Title compound

To a mixed solution of 1-(6-methoxy-3-pyridyl)-5-(6-methyl-3-pyridyl)-1H-pyrazole-3-carboxylic acid ethyl ester (1.10 g) of the above in tetrahydrofuran (22 mL) and methanol (22 mL), an aqueous 1 M sodium hydroxide solution (8.13 mL) was added at room temperature, and the resultant mixture was stirred for 4.5 hours. The reaction solution was neutralized by adding an aqueous 1 M hydrochloric acid solution (8.13 mL), water and chloroform were added to the reaction solution, which was then partitioned. The organic layer was dried over anhydrous sodium sulfate. After separation by filtration, the solvent was evaporated under reduced pressure, and the solid generated by adding diethyl ether to a residue thus obtained was filtered, to obtain the title compound (0.873 g, 86%) as a solid.
¹H-NMR(400MHz, DMSO-d₆)δ: 2.47(3H, s), 3.89(3H, s), 6.92(1H, d, J=9.0Hz), 7.16(1H, s), 7.26(1H, d, J=8.3Hz), 7.53-7.56(1H, m), 7.74-7.77(1H, m), 8.17(1H, d, J=2.7Hz), 8.40(1H, d, J=2.2Hz), 13.05(1H, br).
EI-MSm/z: 310(M⁺).

[Reference Example 66] 5-(6-tert-Butoxycarbonylamino-3-pyridyl)-1-(6-methoxy-3-pyridyl)-1H-pyrazole-3-carboxylic acid

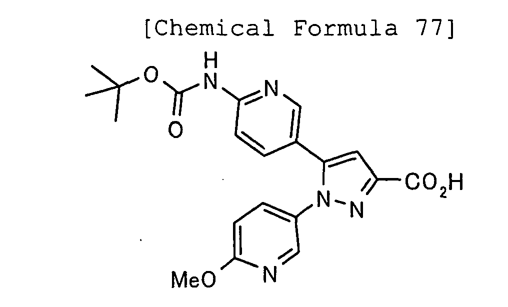

### 1) 5-(6-Carboxy-3-pyridyl)-1-(6-methoxy-3-pyridyl)-1H-pyrazole-3-carboxylic acid ethyl ester

5-(6-Carboxy-3-pyridyl)-1-(6-methoxy-3-pyridyl)-1H-pyrazole-3-carboxylic acid ethyl ester (3.86 g, quantitative) was obtained as an amorphous material by the same method as that in Reference Example 42-(1), using 1-(6-methoxy-3-pyridyl)-5-(6-methyl-3-pyridyl)-1H-pyrazole-3-carboxylic acid ethyl ester (3.45 g) of Reference Example 65-(4) and selenium dioxide (4.53 g).
¹H-NMR(400MHz, DMSO-d₆)δ: 1.34(3H, t, J=7.1Hz), 3.90(3H, s), 4.36(2H, q, J=7.1Hz), 6.95(1H, d, J=8.8Hz), 7.36-7.40(1H, m), 7.79-7.87(2H, m), 8.03(1H, d, J=8.1Hz), 8.22(1H, d, J=2.7Hz), 8.65(1H, m).
EI-MSm/z: 368(M⁺).

### 2) 5-(6-tert-Butoxycarbonylamino-3-pyridyl)-1-(6-methoxy-3-pyridyl)-1H-pyrazole-3-carboxylic acid ethyl ester

5-(6-tert-Butoxycarbonylamino-3-pyridyl)-1-(6-methoxy-3-pyridyl)-1H-pyrazole-3-carboxylic acid ethyl ester (2.56 g, 57%) was obtained as a solid by the same method as that in Reference Example 42-(2), using 5-(6-carboxy 3-pyridyl)-1-(6-methoxy-3-pyridyl)-1H-pyrazole-3-carboxylic acid ethyl ester (3.84 g) of the above, diphenylphosphorylazide (2.42 mL) and tert-butanol (2.15 mL).
¹H-NMR(400MHz, CDCl₃)δ: 1.41-1.45(3H, m), 1.52(9H, s), 3.95(3H, s), 4.43-4.49(2H, m), 6.75-6.78(1H, m), 7.05(1H, s), 7.37-7.46(2H, m), 7.58(1H, dd, J=8.8, 2.7Hz), 7.95(1H, d, J=8.8Hz), 8.10(1H, d, J=2.2Hz), 8.18-8.19(1H, m).
EI-MSm/z: 439(M⁺).

### 3) Title compound

The title compound (1.75 g, 73%) was obtained as a solid by the same method as that in Reference Example 4, Method B-(4), using 5-(6-tert-butoxycarbonylamino-3-pyridyl)-1-(6-methoxy-3-pyridyl)-1H-pyrazole-3-carboxylic acid ethyl ester (2.55 g) of the above.
¹H-NMR(400MHz, DMSO-d₆)δ: 1. 47 (9H, s), 3.89(3H, s), 6.92(1H, d, J=8.8Hz), 7.12 (1H, s), 7.59(1H, dd, J=8.8, 2.4Hz), 7.73-7.78(2H, m), 8.16-8.18(2H, m), 9.95(1H, s), 13.01(1H, br).
EI-MSm/z: 411(M⁺).

[Reference Example 67] 1-(6-Methoxypyridazin-3-yl)-5-(6-methyl-3-pyridyl)-1H-pyrazole-3-carboxylic acid

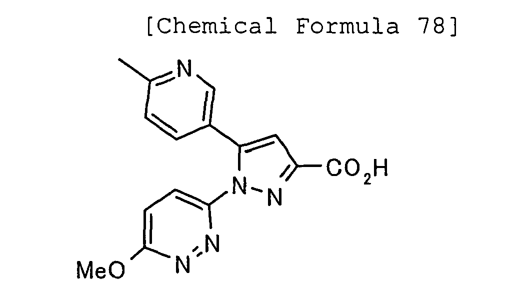

### 1) 4-(6-Methyl-3-pyridyl)-2, 4-dioxobutanoic acid methyl ester

4-(6-Methyl-3-pyridyl)-2, 4-dioxobutanoic acid methyl ester (5.63 g, 57%) was obtained as a solid by the same method as that in Reference Example 8-(3), using 1-(6-methyl-3-pyridyl)-1-ethanone (6.00 g) of Reference Example 65-(2), lithium bis(trimethylsilyl)amide (a 1.0 M solution in tetrahydrofuran, 48.8 mL) and dimethyl oxalate (10.5 g).
¹H-NMR(400MHz, CDCl₃)δ: 2.66(3H, s), 3.96(3H, s), 7.05(1H, s), 7.31(1H, d, J=8.3Hz), 8.15-8.17(1H, m), 9.08(1H, d, J=2.2Hz).
EI-MSm/z: 221(M⁺).

### 2) 1-(6-Chloro-3-pyridazinyl)-5-(6-methyl-3-pyridyl)-1H-pyrazole-3-carboxylic acid methyl ester

1-(6-Chloro-3-pyridazinyl)-5-(6-methyl-3-pyridyl)-1H-pyrazole-3-carboxylic acid methyl ester (4.82 g, 58%) was obtained as a solid by the same method as that in Reference Example 4, Method B-(3), using 4-(6-methyl-3-pyridyl)-2, 4-dioxobutanoic acid methyl ester (5.62 g) of the above and 3-chloro-6-hydrazinopyridazine (3.67 g).
¹H-NMR(400MHz, CDCl₃)δ: 2.60(3H, s), 4.01(3H, m), 7.07-7.08(1H, m), 7.21(1H, d, J=8.1Hz), 7.64-7.67(1H, m), 7.69(1H, dd, J=9.0, 1.0Hz), 8.19(1H, dd, J=9.0, 1.0Hz), 8.44-8.45(1H, m).
FAB-MSm/z: 330(M+H)⁺.

### 3) Title compound

Under an argon atmosphere, to a suspension of 1-(6-chloro-3-pyridazinyl)-5-(6-methyl-3-pyridyl)-1H-pyrazole-3-carboxylic acid methyl ester (4.81 g) of the above in methanol (96 mL), sodium methoxide (2.36 g) was added at room temperature, and the resultant mixture was heated to reflux for 100 minutes. After air cooling, water (96 mL) was added to the reaction solution, and the mixture was stirred for 30 minutes. An aqueous 1 M hydrochloric acid solution (29.2 mL) was added to the reaction solution, and the precipitated solid was filtered, to obtain the title compound (4.40 g, 97%).
¹H-NMR(400MHz, DMSO-d₆)δ: 2.49(3H, s), 4.03(3H, s), 7.18(1H, s), 7.26(1H, d, J=7.8Hz), 7.51(1H, d, J=9.3Hz), 7.60-7.62(1H, m), 8.05(1H, d, J=9.3Hz), 8.42(1H, m), 13.22(1H, br).
FAB-MSm/z: 312(M+H)⁺.

[Reference Example 68] 5-(6-tert-Butoxycarbonylamino-3-pyridyl)-1-(6-methoxypyridazine-3-yl)-1H-pyrazole-3-carboxylic acid

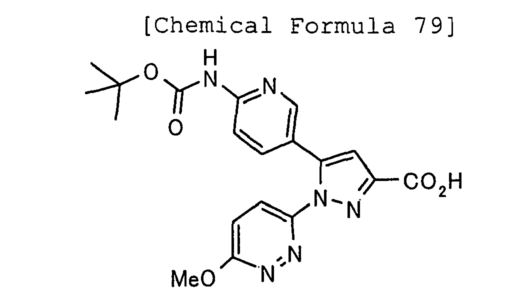

### 1) 1-(6-Methoxy-3-pyridazinyl)-5-(6-methyl-3-pyridyl)-1H-pyrazole-3-carboxylic acid methyl ester

Under an argon atmosphere, to a mixed suspension of 1-(6-methoxy-3-pyridazinyl)-5-(6-methyl-3-pyridyl)-1H-pyrazole-3-carboxylic acid (3.11 g) of Reference Example 67 in methanol (31 mL) and dichloromethane (62 mL), (trimethylsilyl)diazomethane (a 2.0 M solution in hexane, 12.0 mL) was added at 0°C, and the resultant mixture was stirred for 90 minutes, and then stirred for 22 hours at room temperature. A residue obtained by evaporating the solvent of the reaction solution under reduced pressure was purified by silica gel column chromatography (chloroform-acetone), to obtain 1-(6-methoxy-3-pyridazinyl)-5-(6-methyl-3-pyridyl)-1H-pyrazole-3-carboxylic acid methyl ester (3.35 g, quantitative) as a solid.
¹H-NMR(400MHz, CDCl₃)δ: 2.59(3H, s), 4.00(3H, s), 4.12(3H, s), 7.08(1H, s), 7.14-7.21(2H, m), 7.63(1H, dd, J=7.9, 2.3Hz), 7.96-7.98(1H, m), 8.41(1H, dd, J=2.3, 0.6Hz).
FAB-MSm/z: 326(M+H)⁺.

### 2) 5-(6-Carboxy-3-pyridyl)-1-(6-methoxy-3-pyridazinyl)-1H-pyrazole-3-carboxylic acid methyl ester

5-(6-Carboxy-3-pyridyl)-1-(6-methoxy-3-pyridazinyl)-1H-pyrazole-3-carboxylic acid methyl ester (2.72 g, 76%) was obtained as a solid by the same method as that in Reference Example 42-(1), using 1-(6-methoxy-3-pyridazinyl)-5-(6-methyl-3-pyridyl)-1H-pyrazole-3-carboxylic acid methyl ester (3.27 g) of the above and selenium dioxide (4.46 g).
¹H-NMR(400MHz, DMSO-d₆)δ: 3.90(3H, s), 4.02(3H, s), 7.43(1H, s), 7.54(1H, d, J=9.3Hz), 7.93-7.96(1H, m), 8.03-8.05(1H, m), 8.13(1H, d, J=9.3Hz), 8.70(1H, d, J=2.0Hz), 13.35(1H, s).
FAB-MSm/z: 356(M+H)⁺.

### 3) 5-(6-tert-Butoxycarbonylamino-3-pyridyl)-1-(6-methoxy-3-pyridazinyl)-1H-pyrazole-3-carboxylic acid methyl ester

5-(6-tert-Butoxycarbonylamino-3-pyridyl)-1-(6-methoxy-3-pyridazinyl)-1H-pyrazole-3-carboxylic acid methyl ester (1.75 g, 54%) was obtained as an amorphous material by the same method as that in Reference Example 42-(2), using 5-(6-carboxy-3-pyridyl)-1-(6-methoxypyridazin-3-yl)-1H-pyrazole-3-carboxylic acid methyl ester (2.70 g) of the above, diphenylphosphorylazide (1.80 mL) and tert-butanol (1.60 mL).
¹H-NMR(400MHz, CDCl₃)δ: 1.52(9H, s), 3.99(3H, s), 4.12(3H, s), 7.04(1H, s), 7.14(1H, d, J=9.3Hz), 7.61-7.64(1H, m), 7.92-7.99(3H, m), 8.27(1H, d, J=2.2Hz).
EI-MSm/z: 426(M⁺).

### 4) 5-(6-tert-Butoxycarbonylamino-3-pyridyl)-1-(6-methoxy-3-pyridazinyl)-1H-pyrazole-3-carboxylic acid

The title compound (1.32 g, 79%) was obtained as a solid by the same method as that in Reference Example 4, Method B-(4), using 5-(6-tert-butoxycarbonylamino-3-pyridyl)-1-(6-methoxy-3-pyridazinyl)-1H-pyrazole-3-carboxylic acid methyl ester (1.74 g) of the above.
¹H-NMR(400MHz, DMSO-d₆)δ: 1.48(9H, s), 4.04(3H, s), 7.16(1H, s), 7.50(1H, d, J=9.3Hz), 7.64-7.66(1H, m), 7.77-7.79(1H, m), 8.04(1H, d, J=9.3Hz), 8.23-8.24(1H, m), 9.94(1H, s), 13.12(1H, br).
FAB-MSm/z: 413(M+H)⁺.

[Reference Example 69] 5-(1H-Imidazol-4-yl)-1-(6-methoxy-3-pyridyl)-1H-pyrazole-3-carboxylic acid

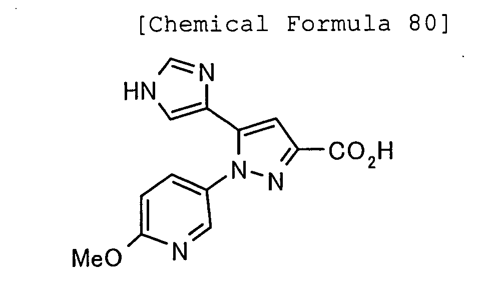

### 1) 4-(1-Triphenylmethyl-1H-imidazol-4-yl)-2, 4-dioxobutanoic acid ethyl ester

4-(1-Triphenylmethyl-1H-imidazol-4-yl)-2, 4-dioxobutanoic acid ethyl ester (3.26 g, quantitative) was obtained as an oily product by the same method as that in Reference Example 8-(3), using 4-acetyl-1-triphenylmethyl-1H-imidazole (N. Matsunaga, et al., Bioorg. Med. Chem., 2004, 12, 2251, 2.48 g), lithium bis(trimethylsilyl)amide (a 1.0 M solution in tetrahydrofuran, 7.74 mL) and diethyl oxalate (1.91 mL).
¹H-NMR(400MHz, CDCl₃)δ: 1.36-1.40(3H, m), 4.34-4.39(2H, m), 6.92(1H, m), 7.11-7.17(6H, m), 7.26-7.39(9H, m), 7.51(1H, m), 7.70(1H, m).

### 2) 5-(1H-Imidazol-4-yl)-1-(6-methoxy-3-pyridyl)-1H-pyrazole-3-carboxylic acid ethyl ester

A solution of 4-(1-triphenylmethyl-1H-imidazol-4-yl)-2, 4-dioxobutanoic acid ethyl ester (3.25 g) of the above and 5-hydrazino-2-methoxypyridine (0.979 g) of Reference Example 2 in ethanol (65 mL) was heated to reflux for 49 hours. After air cooling, water and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 1-(6-methoxy-3-pyridyl)-5-(1-triphenylmethyl-1H-imidazol-4-yl)-1H-pyrazole-3-carboxylic acid ethyl ester (0.865 g) as an amorphous material. Further, from a different elution fraction, 5-(1H-imidazol-4-yl)-1-(6-methoxy-3-pyridyl)-1H-pyrazole-3-carboxylic acid ethyl ester (0.367 g) was obtained as an oily product.
Under an argon atmosphere, trifluoroacetic acid (8.5 mL) was added to a solution of 1-(6-methoxy-3-pyridyl)-5-(1-triphenylmethyl-1H-imidazol-4-yl)-1H-pyrazole-3-carboxylic acid ethyl ester (0.860 g) in dichloromethane (17 mL) at room temperature, and the resultant mixture was stirred for 3.5 hours. A saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 5-(1H-imidazol-4-yl)-1-(6-methoxy-3-pyridyl)-1H-pyrazole-3-carboxylic acid ethyl ester (0.385 g) as a solid. The portion obtained above was combined to yield 5-(1H-imidazol-4-yl)-1-(6-methoxy-3-pyridyl)-1H-pyrazole-3-carboxylic acid ethyl ester (0.752 g, 19%) was obtained.
¹H-NMR(400MHz, CDCl₃)δ: 1.39-1.43(3H, m), 3.97(3H, s), 4.41-4.46(2H, m), 6.72(1H, d, J=1.0Hz), 6.81(1H, d, J=8.8Hz), 7.20(1H, s), 7.63(1H, d, J=1.0Hz), 7.69(1H, dd, J=8.8, 2.7Hz), 8.23(1H, d, J=2.7Hz).
EI-MSm/z: 313(M⁺).

### 3) Title compound

The title compound (0.449 g, 66%) was obtained as a solid by the same method as that in Reference Example 4, Method B-(4), using 5-(1H-imidazol-4-yl)-1-(6-methoxy-3-pyridyl)-1H-pyrazole-3-carboxylic acid ethyl ester (0.742 g) of the above.
¹H-NMR(400MHz, DMSO-d₆)δ: 3.90(3H, s), 6.76(1H, s), 6.83-6.89(2H, m), 7.64(1H, m), 7.74(1H, dd, J=8.8, 2.9Hz), 8.21-8.22(1H, m), 12.50(1H, br).
EI-MSm/z: 285(M⁺).

[Reference Example 70] 1-(6-Methoxy-3-pyridyl)-5-(1H-pyrazol-4-yl)-1H-pyrazole-3-carboxylic acid

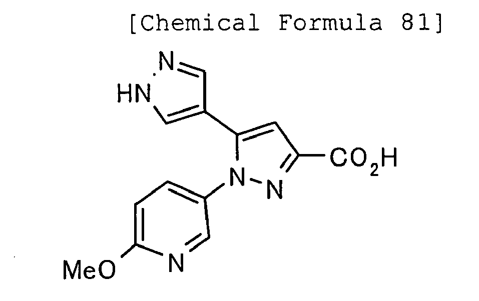

### 1) 1-Triphenylmethyl-1H-pyrazole-4-carboxylic acid ethyl ester

Under an argon atmosphere, to a solution of 1H-pyrazole-4-carboxylic acid ethyl ester (W. Holzer, et al., J. Heterocyclic Chem., 1993, 30, 865; 9.62 g) and triethylamine (9.57 mL) in N, N-dimethylformamide (96 mL), triphenylmethyl bromide (22.2 g) was added at room temperature, and the resultant mixture was stirred for 3 hours. Water and ethyl acetate were added to the reaction solution, and the mixture was partitioned. The organic layer was dried over anhydrous sodium sulfate. After separation by filtration, the solvent was evaporated under reduced pressure, and the solid generated by adding diisopropyl ether to a residue thus obtained was filtered, to obtain 1-triphenylmethyl-1H-pyrazole-4-carboxylic acid ethyl ester (16.1 g, 61%).
¹H-NMR(400MHz, CDCl₃)δ: 1.30-1.34(3H, m), 4.24-4.30(2H, m), 7.11-7.15(6H, m), 7.31-7.34(9H, m), 7.93(1H, s), 8.04(1H, s).
EI-MSm/z: 382(M⁺).

### 2) 1-Triphenylmethyl-1H-pyrazole-4-carboxylic acid

To a mixed solution of 1-triphenylmethyl-1H-pyrazole-4-carboxylic acid ethyl ester (16.1 g) in tetrahydrofuran (161 mL) and methanol (161 mL), an aqueous 1 M sodium hydroxide solution (105 mL) was added at room temperature, and the resultant mixture was stirred for 3.5 hours. The reaction solution was neutralized by adding an aqueous 1 M hydrochloric acid solution (105 mL) to the reaction solution, and the precipitated solid was filtered, to obtain a mixture (15.4 g) containing 1-triphenylmethyl-1H-pyrazole-4-carboxylic acid methyl ester as the main component. To a solution of this mixture (15.4 g) containing 1-triphenylmethyl-1H-pyrazole-4-carboxylic acid methyl ester as the main component in tetrahydrofuran (200 mL), a solution of lithium hydroxide monohydrate (2.12 g) in water (100 mL) was added dropwise at room temperature, and the mixture was stirred for 2 hours at room temperature, for 2 hours at 60°C, and for 2 hours at 90°C. After air cooling, the reaction solution was neutralized by adding an aqueous 1 M hydrochloric acid solution (50.5 mL) at 0°C, water and a mixed solvent of chloroform-methanol (10: 1) were added, and the mixture was partitioned. The organic layer was dried over anhydrous sodium sulfate. After separation by filtration, the solvent was evaporated under reduced pressure, and 1-triphenylmethyl-1H-pyrazole-4-carboxylic acid (14.3 g, 96%) was obtained as a solid.
¹H-NMR(400MHz, DMSO-d₆)δ: 7.05-7.08(6H, m), 7.38-7.41(9H, m), 7.72(1H, s), 8.01(1H, s), 12.54(1H, br).
EI-MSm/z: 354(M⁺).

### 3) 1-Triphenylmethyl-1H-pyrazole-4-carboxylic acid N-methoxy-N-methylamide

1-Triphenylmethyl-1H-pyrazole-4-carboxylic acid N-methoxy-N-methylamide (14.4 g, 90%) was obtained as a solid by the same method as that in Reference Example 8-(1), using 1-triphenylmethyl-1H-pyrazole-4-carboxylic acid (14.3 g) of the above and N, O-dimethylhydroxylamine hydrochloride (4.33 g).
¹H-NMR(400MHz, CDCl₃)δ: 3.29(3H, s), 3.62(3H, s), 7.13-7.18(6H, m), 7.28-7.33(9H, m), 7.97(1H, s), 8.11(1H, s).
EI-MSm/z: 397(M⁺).

### 4) 4-Acetyl-1-triphenylmethyl-1H-pyrazole

4-Acetyl-1-triphenylmethyl-1H-pyrazole (11.7 g, 91%) was obtained as a solid by the same method as that in Reference Example 8-(2), using 1-triphenylmethyl-1H-pyrazole-4-carboxylic acid N-methoxy-N-methylamide (14.4 g) of the above and methyllithium (a 0.98 M solution in diethyl ether, 38.8 mL).
¹H-NMR(400MHz, CDCl₃)δ: 2.40(3H, s), 7.11-7.15(6H, m), 7.29-7.35(9H, m), 7.93(1H, d, J=0.7Hz), 8.04(1H, d, J=0.7Hz).
EI-MSm/z: 352(M⁺).

### 5) 4-(1-Triphenylmethyl-1H-pyrazol-4-yl)-2, 4-dioxobutanoic acid ethyl ester

4-(1-Triphenylmethyl-1H-pyrazol-4-yl)-2, 4-dioxobutanoic acid ethyl ester (6.03 g, 80%) was obtained as a solid by the same method as that in Reference Example 8-(3), using 4-acetyl-1-triphenylmethyl-1H-pyrazole (5.85 g) of the above, lithium bis(trimethylsilyl)amide (a 1.0 M solution in tetrahydrofuran, 18.3 mL) and diethyl oxalate (4.51 mL).
¹H-NMR(400MHz, CDCl₃)δ: 1.37-1.41(3H, m), 4.34-4.40(2H, m), 6.65(1H, m), 7.12-7.16(6H, m), 7.31-7.36(9H, m), 8.02(1H, s), 8.12(1H, s).
EI-MSm/z: 452(M⁺).

### 6) 1-(6-Methoxy-3-pyridyl)-5-(1H-pyrazol-4-yl)-1H-pyrazole-3-carboxylic acid ethyl ester

A solution of 4-(1-triphenylmethyl-1H-pyrazol-4-yl)-2, 4-dioxobutanoic acid ethyl ester (6.02 g) of the above and 5-hydrazino-2-methoxypyridine (2.04 g) of Reference Example 2 in ethanol (120 mL) was heated to reflux for 38 hours. After air cooling, a residue obtained by evaporating the solvent of the reaction solution under reduced pressure was purified by silica gel column chromatography (chloroform-acetone), to obtain 1-(6-methoxy-3-pyridyl)-5-(1-triphenylmethyl-1H-pyrazol-4-yl)-1H-pyrazole-3-carboxylic acid ethyl ester (2.24 g) as an amorphous material. Further, from a different elution fraction, 1-(6-methoxy-3-pyridyl)-5-(1H-pyrazol-4-yl)-1H-pyrazole-3-carboxylic acid ethyl ester (1.17 g) was obtained as an amorphous material.
Under an argon atmosphere, to a solution of 1-(6-methoxy-3-pyridyl)-5-(1-triphenylmethyl-1H-pyrazol-4-yl)-1H-pyrazole-3-carboxylic acid ethyl ester (2.23 g) of the above in dichloromethane (44 mL), trifluoroacetic acid (22 mL) was added at room temperature, and the resultant mixture was stirred for 2 hours. A saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 1-(6-methoxy-3-pyridyl)-5-(1H-pyrazol-4-yl)-1H-pyrazole-3-carboxylic acid ethyl ester (0.830 g) as an amorphous material. This was combined with the portion obtained above, and 1-(6-methoxy-3-pyridyl)-5-(1H-pyrazol-4-yl)-1H-pyrazole-3-carboxylic acid ethyl ester (2.00 g, 27%) was obtained.
¹H-NMR(400MHz, CDCl₃)δ: 1.40-1.44(3H, m), 3.98(3H, m), 4.42-4.47(2H, m), 6.82(1H, d, J=8.8Hz), 7.01(1H, m), 7.42(2H, m), 7.62-7.65(1H, m), 8.21(1H, d, J=2.7Hz).
EI-MSm/z: 313(M⁺).

### 7) Title compound

The title compound (1.29 g, 71%) was obtained as a solid by the same method as that in Reference Example 4, Method B-(4), using 1-(6-methoxy-3-pyridyl)-5-(1H-pyrazol-4-yl)-1H-pyrazole-3-carboxylic acid ethyl ester (2.00 g) of the above.
¹H-NMR(400MHz, DMSO-d₆)δ: 3.93(3H, s), 6.98(1H, d, J=8.8Hz), 7.04(1H, m), 7.53(2H, br), 7.80-7.83(1H, m), 8.26(1H, d, J=2.7Hz), 13.00(1H, br).
EI-MSm/z: 285(M⁺).

[Reference Example 71] Piperidine-2-carboxylic acid amide

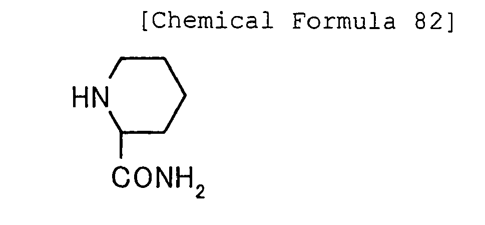

Concentrated aqueous ammonia (3 mL) and triethylamine (2 mL) were added to a solution of N-benzyloxycarbonylpiperidine-2-carboxylic acid (2.0 g), 1-hydroxybenzotriazole (1.6 g), 1-(3-dimethylaminopropyl)-3-ethylcarbodiimide hydrochloride (2.3 g) in dichloromethane (20 mL) at room temperature, and the resultant mixture was stirred for 3 days. Water and dichloromethane were added to the reaction solution, and the mixture was partitioned. The organic layer was dried over anhydrous magnesium sulfate. After separation by filtration, to a solution in methanol (30 mL) of a residue obtained by evaporating the solvent under reduced pressure, 10% palladium-carbon (1 g, 50% wet) was added, and the resultant mixture was stirred for 20 hours in the presence of hydrogen. After separating the catalyst from the reaction solution by filtration, the solvent of the filtrate was evaporated under reduced pressure, and the title compound (970 mg, quantitative) was obtained as a solid.
ESI-MSm/z: 128(M⁺).

[Reference Example 72] (3S)-Piperidin-3-ylcarbamide acid tert-butyl ester

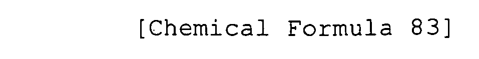

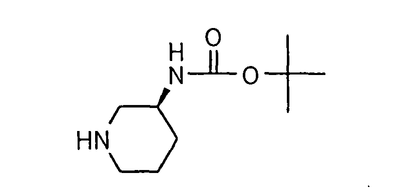

### 1) (3S)-1-Benzylpiperidin-3-ylcarbamide acid tert-butyl ester

Potassium carbonate (1.1 g) and di-tert-butoxydicarbonate (829 mg) were added to a solution of (S)-(+)-1-benzyl-3-aminopiperidine dihydrochloride (1.0 g) in N, N-dimethylformamide (10 mL) at 0°C, and the resultant mixture was stirred for 30 minutes, and stirred for 19.5 hours at room temperature. Water and ethyl acetate were added to the reaction solution, and the mixture was partitioned. The organic layer was dried over anhydrous magnesium sulfate. After separation by filtration, the solvent was evaporated under reduced pressure, and (3S)-1-benzylpiperidin-3-ylcarbamide acid tert-butyl ester (1.0 g, 91%) was obtained as a solid.
¹H-NMR(400MHz, CDCl₃)δ: 1.44(9H, s), 1.51-1.59(4H, m), 2.24-2.50(4H, m), 3.47(2H, s), 3.76(1H, br), 4.99(1H, br), 7.30(5H,m).
ESI-MSm/z: 291(M+H)⁺.

### 2) Title compound

To a solution of (3S)-1-benzylpiperidin-3-ylcarbamide acid tert-butyl ester (1.0 g) of the above in methanol (15 mL), 5% palladium-carbon (200 mg) was added, and the resultant mixture was stirred for 20 hours at room temperature in the presence of hydrogen. The reaction solution was filtered using Celite, and the solvent of the filtrate was evaporated under reduced pressure, to obtain the title compound (663 mg, 96%) as a solid.
¹H-NMR(400MHz, CDCl3)δ: 1.44(9H, s), 1.67-1.84(4H, m), 2.53(1H, br), 2.67(1H, br), 2.81(1H, br), 3.06(1H, m), 3.57(1H, br), 4.83(1H, br).
ESI-MSm/z: 201(M+H)⁺.

[Reference Example 73] 1-(6-Methoxy-3-pyridazinyl)-5-(thiazol-4-yl)-1H-pyrazole-3-carboxylic acid

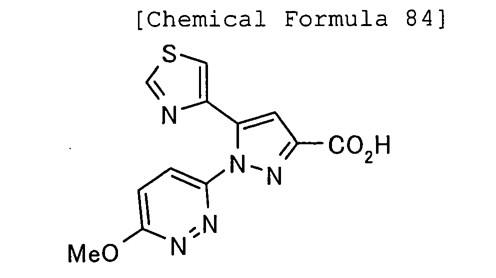

### 1) 4-(Thiazol-4-yl)-2, 4-dioxobutanoic acid methyl ester

4-(Thiazol-4-yl)-2, 4-dioxobutanoic acid methyl ester (3.67 g, 63%) was obtained as a solid by the same method as that in Reference Example 4, Method B-(1), using 4-acetylthiazole (3.46 g) of Reference Example 64-(4), sodium methoxide (2.94 g) and dimethyl oxalate (6.43 g).
¹H-NMR(400MHz, CDCl₃)δ: 3.94(3H, s), 7.42(1H, s), 8.34(1H, d, J=2.0Hz), 8.89(1H, d, J=2.0Hz).
EI-MSm/z: 213(M⁺).

### 2) Title compound

Under an argon atmosphere, a suspension of 4-(thiazol-4-yl)-2, 4-dioxobutanoic acid methyl ester (3.66 g) of the above and 3-chloro-6-hydrazinopyridazine (2.48 g) in methanol (73 mL) was heated to reflux for 1 hour, then acetic acid (4.91 mL) was added thereto, and the mixture was heated to reflux for 69 hours. After air cooling, saturated sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain a mixture (4.41 g) of 1-(6-chloro-3-pyridazinyl)-5-(thiazol-4-yl)-1H-pyrazole-3-carboxylic acid methyl ester and 1-(6-methoxy-3-pyridazinyl)-5-(thiazol-4-yl)-1H-pyrazole-3-carboxylic acid methyl ester. To a suspension of this mixture of 1-(6-chloro-3-pyridazinyl)-5-(thiazol-4-yl)-1H-pyrazole-3-carboxylic acid methyl ester and 1-(6-methoxy-3-pyridazinyl)-5-(thiazol-4-yl)-1H-pyrazole-3-carboxylic acid methyl ester in methanol (88 mL), sodium methoxide (2.78 g) was added at room temperature, and the mixture was heated to reflux for 85 minutes under an argon atmosphere. After air cooling, water (88 mL) was added, and the mixture was stirred for 45 minutes at room temperature. An aqueous 1 M hydrochloric acid solution (51.5 mL) was added to the reaction solution, and the mixture was stirred. The precipitated solid was filtered, and the title compound (3.88 g, 74%) was obtained.
¹H-NMR(400MHz, DMSO-d₆)δ: 4.07(3H, s), 7.32-7.33(1H, m), 7.49-7.52(1H, m), 7.97-8.00(1H, m), 8.06-8.07(1H, m), 9.04(1H, m), 13.22(1H, br).
EI-MSm/z: 303(M⁺).

[Reference Example 74] 1, 4-Oxazepane hydrochloride

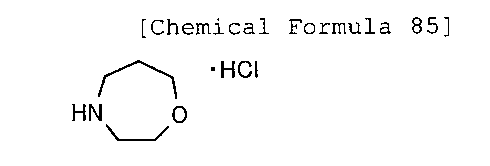

### 1) 1, 4-Oxazepan-5-one

Under ice cooling, sodium azide (17.8 g) was added to a solution of tetrahydro-4H-pyran-4-one (9.80 g) in concentrated hydrochloric acid (50 mL) over 40 minutes, and the resultant mixture was stirred for 30 minutes, and then stirred for 16 hours at room temperature. Under ice cooling, sodium carbonate was added to the reaction solution to adjust pH from 8 to 9, then chloroform was added, and the mixture was partitioned. The organic layer was washed with saturated saline, and then dried over anhydrous magnesium sulfate. After separation by filtration, the solvent was evaporated under reduced pressure, and 1, 4-oxazepan-5-one (5.34 g, 47.4%) was obtained as a solid.
¹H-NMR(300MHz, CDCl₃)δ: 2.70-2.74(2H, m), 3.32-3.37(2H, m), 3.75-3.83(4H, m), 6.31(1H, br s).
FAB-MSm/z: 116(M+H)⁺.

### 2) 1, 4-Oxazepane-4-carboxylic acid tert-butyl ester

Under an argon atmosphere and ice cooling, 1, 4-oxazepan-5-one (3.041 g) of the above was added to a borane-tetrahydrofuran complex (a 1.0 M tetrahydrofuran solution, 40 mL) over 30 minutes, and the resultant mixture was stirred for 30 minutes at room temperature. Further, the reaction solution was heated to reflux for 2.5 hours. After air cooling, a 4 M hydrochloric acid-dioxane solution (25 mL) and methanol (12 mL) were added to the reaction solution, and the mixture was heated to reflux for 1 hour. After air cooling, an aqueous 1 M sodium hydroxide solution (80 mL) was added to the reaction solution, a solution of di-tert-butoxydicarbonate (8.849 g) in tetrahydrofuran (25 mL), and methanol (20 mL) were added thereto at room temperature, and the mixture was stirred for 17 hours. Water and chloroform were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (hexane-ethyl acetate), to obtain 1, 4-oxazepane-4-carboxylic acid tert-butyl ester (2.68 g, 50%) as an oily product.
¹H-NMR(400MHz, CDCl₃)δ: 1.46(9H, s), 1.82-1.95(2H, m), 3.45-3.58(4H, m), 3.66-3.77(4H, m).

### 3) Title compound

To a solution of 1, 4-oxazepane-4-carboxylic acid tert-butyl ester (0.468 g) in dichloromethane (9.2 mL), a 4 M hydrochloric acid-dioxane solution (4.6 mL) was added at 0°C, and the resultant mixture was stirred for 0.5 hour at room temperature. The solvent of the reaction solution was evaporated under reduced pressure, and the title compound (0.263 g, 82%) was obtained as a solid.
¹H-NMR(400MHz, CDCl₃)δ: 2.22-2.33(2H, m), 3.27-3.43(4H, m), 3.82-3.90(2H, m), 3.92-4.01(2H, m), 9.89(1H, br).
ESI-MSm/z: 102(M+H)⁺.

[Reference Example 75] [(1-Piperidin-2-yl)cyclopropyl]carbamide acid tert-butyl ester

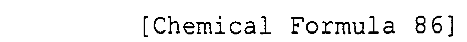

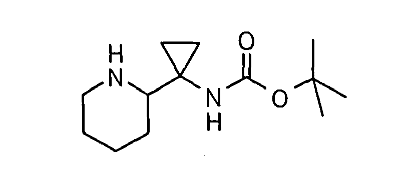

### 1) 2-(1-Aminocyclopropyl)pyridine

Under an argon atmosphere, titanium chlorotriisopropoxide (10.3 mL) was added to a solution of 2-cyanopyridine (3.75 g) in tetrahydrofuran (200 mL) at room temperature, and the resultant mixture was stirred for 7 minutes. Ethyl magnesium bromide (a 0.97 M solution in tetrahydrofuran, 86 mL) was added dropwise over 5 minutes to the reaction solution at room temperature, and then the mixture was stirred for 55 minutes. A boron trifluoride-diethyl ether complex (10.9 mL) was added to the reaction solution at room temperature, and the mixture was stirred for 55 minutes. The reaction liquid was alkalinized by adding an aqueous 1 M sodium hydroxide solution, and then extracted with chloroform. The organic layer was dried over anhydrous sodium sulfate. After separation by filtration, a residue obtained by evaporating the solvent under reduced pressure was purified by silica gel column chromatography (chloroform-methanol), to obtain 2-(1-aminocyclopropyl)pyridine (2.554 g, 53%) as an oily product.
ESI-MSm/z: 135(M+H)⁺.

### 2) [(1-Pyridin-2-yl)cyclopropyl]carbamide acid tert-butyl ester

To a solution of 2-(1-aminocyclopropyl)pyridine (513 mg) of the above and di-tert-butyldicarbonate ester (1.25 g) in dichloromethane (50 mL), triethylamine (1.06 mL) was added at room temperature, and the resultant mixture was stirred for 3 days. A residue obtained by evaporating the reaction solvent was purified by silica gel column chromatography (hexane-ethyl acetate), to obtain [(1-pyridin-2-yl)cyclopropyl]carbamide acid tert-butyl ester (563 mg, 63%) as a solid.
ESI-MSm/z: 235(M+H)⁺.

### 3) Title compound

A suspension of [(1-pyridin-2-yl)cyclopropyl]carbamide acid tert-butyl ester (277 mg), 5% rhodium-alumina (200 mg), acetic acid (1 mL) and ethanol (10 mL) was stirred for 2 hours at room temperature under a hydrogen atmosphere (6 atmosphere). The reaction mixture was filtered with Celite, and then the solvent was evaporated under reduced pressure. To a residue thus formed, a saturated aqueous solution of sodium hydrogen carbonate and a mixed solvent of chloroform-methanol (10: 1) were added, and the mixture was partitioned. The organic layer was dried over anhydrous sodium sulfate. After separation by filtration, the solvent was evaporated under reduced pressure, and the title compound (297 mg, quantitative) was obtained as a solid.
ESI-MSm/z: 241(M+H)⁺.

[Reference Example 76] 5-(5-Benzyloxy-2-pyridyl)-1-(6-methoxy-3-pyridyl)-1H-pyrazole-3-carboxylic acid

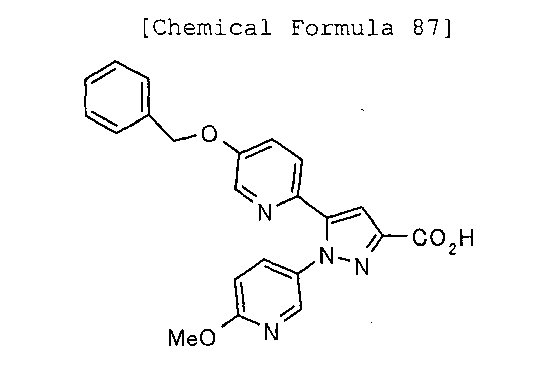

### 1) 5-Benzyloxy-2-methylpyridine

Benzyl bromide (10.9 mL) was added to a solution of 3-hydroxy-6-methylpyridine (10.0 g) and potassium carbonate (38.0) in acetonitrile (200 mL) at room temperature, and the resultant mixture was stirred for 12 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-benzyloxy-2-methylpyridine (4.14 g, 23%) as an oily product.
¹H-NMR(400MHz, CDCl₃)δ: 2.48(3H, s), 5.08(2H, s), 7.05(1H, d, J=8.5Hz), 7.16(1H, dd, J=8.5, 2.9Hz), 7.31-7.43(5H, m), 8.26(1H, d, J=2.9Hz).
EI-MS m/z : 199(M⁺).

### 2) 1-(5-Benzyloxy-2-pyridyl)ethanone

To a solution of 5-benzyloxy-2-methylpyridine (4.13 g) in pyridine (83 mL), selenium dioxide (9.20 g) was added at room temperature, and the resultant mixture was heated to reflux for 61 hours. After air cooling, water and chloroform were added to the reaction solution, and the mixture was partitioned. The organic layer was dried over anhydrous magnesium sulfate. After separation by filtration, the solvent was evaporated under reduced pressure. To a solution of a residue thus obtained, N, O-dimethylhydroxylamine hydrochloride (2.22 g), 1-ethyl-3-(3-dimethylaminopropyl)carbodiimide hydrochloride (4.37 g), and 1-hydroxybenzotriazole (3.08 g) in N, N-dimethylformamide (95 mL), triethylamine (6.35 mL) was added at room temperature, and the mixture was stirred for 61 hours. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-benzyloxypyridine-2-carboxylic acid methoxymethylamide (3.75 g, 66%) as an oily product. (FAB-MSm/z: 273 (M+H)⁺.)
Undern an argon atmosphere and cooling to 0°C, methyllithium (a 1.10 M solution in diethyl ether, 13.7 mL) was added dropwise to a solution of 5-benzyloxypyridine-2-carboxylic acid methoxymethylamide (3.74 g) in tetrahydrofuran (75 mL), and the resultant mixture was stirred for 40 minutes. Water and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 1-(5-benzyloxy-2-pyridyl)ethanone (1.47 g, 47%) as an oily product.
¹H-NMR(400MHz, CDCl₃)δ: 2.67(3H, s), 5.18(2H, s), 7.30-7.45(6H, m), 8.03(1H, d, J=8.8Hz), 8.39(1H, d, J=2.7Hz).
EI-MS m/z : 227(M⁺).

### 3) 4-(5-Benzyloxy-2-pyridyl)-2, 4-dioxobutanoic acid ethyl ester

Under an argon atmosphere, a solution of diethyl oxalate (1.75 mL) and 1-(5-benzyloxy-2-pyridyl)ethanone (1.46 g) of the above in ethanol (15 mL) was added to a solution of sodium ethoxide (0.874 g) in ethanol (15 mL), and the mixture was stirred for 7 hours at room temperature, and stirred for 1 hour at 60°C. After air cooling, sodium ethoxide (0.874 g) and diethyl oxalate (1.75 mL) were further added to the reaction solution, and the mixture was stirred for 1 hour at 60°C. After air cooling, water was added to the reaction solution, and the mixture was washed with diethyl ether. Subsequently, a saturated aqueous ammonium chloride solution and chloroform were added to the aqueous layer, and the mixture was partitioned. The organic layer was dried over anhydrous sodium sulfate. After separation by filtration, the solvent was evaporated under reduced pressure, and 4-(5-benzyloxy-2-pyridyl)-2, 4-dioxobutanoic acid ethyl ester (1.38 g, 66%) was obtained as a solid.
¹H-NMR(400MHz, CDCl₃)δ: 1.38-1.42(3H, m), 4.35-4.42(2H, m), 5.20(2H, s), 7.35-7.44(6H, m), 7.59(1H, s), 8.14(1H, d, J=8.8Hz), 8.44(1H, d, J=2.7Hz).
EI-MSm/z: 327(M⁺).

### 4) 5-(5-Benzyloxy-2-pyridyl)-1-(6-methoxy-3-pyridyl)-1H-pyrazole-3-carboxylic acid ethyl ester

To a solution of 4-(5-benzyloxy-2-pyridyl)-2, 4-dioxobutanoic acid ethyl ester (1.37 g) of the above and 5-hydrazino-2-methoxypyridine (0.699 g) of Reference Example 2 in ethanol (27 mL), acetic acid (0.958 mL) was added, and the resultant mixture was heated to reflux for 12 hours. After air cooling, a saturated aqueous solution of sodium hydrogen carbonate and ethyl acetate were added to the reaction solution, and the mixture was partitioned. The organic layer was dried over anhydrous sodium sulfate. After separation by filtration, a residue obtained by evaporating the solvent under reduced pressure was purified by silica gel column chromatography (ethyl acetate-hexane), to obtain 5-(5-benzyloxy-2-pyridyl)-1-(6-methoxy-3-pyridyl)-1H-pyrazole-3-carboxylic acid ethyl ester (1.50 g, 83%) as a solid.
¹H-NMR(400MHz, CDCl₃)δ: 1.42(3H, t, J=7.1Hz), 3.95(3H, s), 4.45(2H, q, J=7.1Hz), 5.10(2H, s), 6.76(1H, d, J=8.8Hz), 7.18-7.42(8H, m), 7.66(1H, dd, J=8.8, 2.7Hz), 8.10(1H, d, J=2.7Hz), 8.28(1H, d, J=2.7Hz).
FAB-MSm/z: 431(M+H)⁺.

### 5) Title compound

1N Sodium hydroxide (8.65 mL) was added to a mixed solution of 5-(5-benzyloxy-2-pyridyl)-1-(6-methoxy-3-pyridyl)-1H-pyrazole-3-carboxylic acid ethyl ester (1.49 g) in methanol (30 mL) and tetrahydrofuran (30 mL) at room temperature, and the resultant mixture was stirred for 90 minutes. The solvent of the reaction solution was evaporated under reduced pressure, and a residue thus obtained was dissolved in water and chloroform. An aqueous 1M hydrochloric acid solution (8.65 mL) was added to the mixture, which was then partitioned. The organic layer was dried over anhydrous sodium sulfate. After separation by filtration, the solvent was evaporated under reduced pressure, and the title compound (1.27 g, 91%) was obtained as a solid.
¹H-NMR(400MHz, CDCl₃)δ: 3.95(3H, s), 5.11(2H, s), 6.75-6.78(1H, m), 7.22-7.41(8H, m), 7.66(1H, dd, J=8.8, 2.7Hz), 8.11(1H, dd, J=2.7, 0.7Hz), 8.30(1H, dd, J=2.7, 0.7Hz).
EI-MSm/z: 402(M⁺).

[Reference Example 77] 1-(6-Methoxy-3-pyridazinyl)-5-(1-methyl-1H-pyrazol-4-yl)-1H-pyrazole-3-carboxylic acid

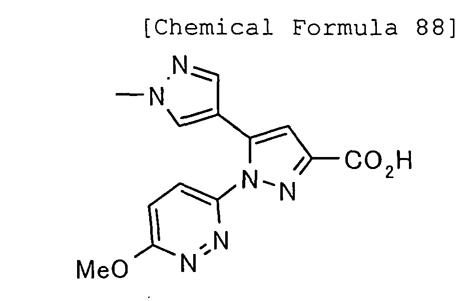

### 1) 4-(1-Methyl-1H-pyrazol-4-yl)-2, 4-dioxobutanoic acid methyl ester

4-(1-Methyl-1H-pyrazol-4-yl)-2, 4-dioxobutanoic acid methyl ester (3.48 g, 69%) was obtained as a solid by the same method as that in Reference Example 4, Method B-(1), using 1-(1-methyl-1H-pyrazol-4-yl)ethanone (3.00 g) of Reference Example 61-(3) and sodium methoxide (2.61 g).
¹H-NMR(400MHz, DMSO-d₆)δ: 3.84(3H, s), 3.91(3H, s), 6.82(1H, s), 8.14(1H, s), 8.66(1H, s).
EI-MSm/z: 210(M⁺).

### 2) 1-(6-Chloro-3-pyridazinyl)-5-(1-methyl-1H-pyrazol-4-yl)-1H-pyrazole-3-carboxylic acid methyl ester

Under an argon atmosphere, a suspension of 4-(1-methyl-1H-pyrazol-4-yl)-2, 4-dioxobutanoic acid methyl ester (3.46 g) of the above and 3-chloro-6-hydrazinopyridazine (2.38 g) in methanol (69 mL) was heated to reflux for 1 hour. Acetic acid (4.71 mL) was added to the reaction solution, and the mixture was heated to reflux for 64 hours. After air cooling, a saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-acetone), to obtain 1-(6-chloro-3-pyridazinyl)-5-(1-methyl-1H-pyrazol-4-yl)-1H-pyrazole-3-carboxylic acid methyl ester (4.30 g, 82%) as a solid.
¹H-NMR(400MHz, CDCl₃)δ: 3.94(3H, s), 3.99(3H, s), 7.03(1H, s), 7.64(1H, s), 7.68-7.70(1H, m), 7.96(1H, s), 8.07-8.10(1H, m).
EI-MSm/z: 318(M⁺).

### 3) Title compound

Under an argon atmosphere, to a suspension of 1-(6-chloro-3-pyridazinyl)-5-(1-methyl-1H-pyrazol-4-yl)-1H-pyrazole-3-carboxylic acid methyl ester (4.29 g) in methanol (86 mL), sodium methoxide (2.18 g) was added at room temperature, and the resultant mixture was heated to reflux for 4 hours. After air cooling, water (86 mL) was added, and the mixture was stirred for 1 hour. An aqueous 1 M hydrochloric acid solution (40.4 mL) was added to the reaction solution and stirred, and the precipitated solid thus generated was filtered to obtain the title compound (3.35 g, 83%).
¹H-NMR(400MHz, DMSO-d₆)δ: 3.81(3H, s), 4.12(3H, s), 7.07(1H, s), 7.48(1H, s), 7.52(1H, d, J=9.3Hz), 7.78(1H, s), 7.95(1H, d, J=9.3Hz), 13.10(1H, br).
EI-MSm/z: 300(M⁺).

[Example 1] 4-[5-(5-Methoxy-2-pyridyl)-1-(6-methyl-3-pyridyl)-1H-pyrazole-3-carbonyl]morpholine

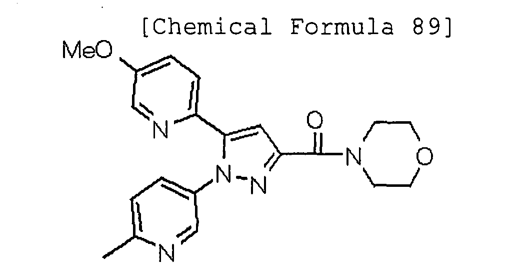

Morpholine (84.2 µL) and triethylamine (98.8 µL) were added to a solution of 5-(5-methoxy-2-pyridyl)-1-(6-methyl-3-pyridyl)-1H-pyrazole-3-carboxylic acid (0.200 g) of Reference Example 9, 3-(3-dimethylaminopropyl)-1-ethylcarbodiimide hydrochloride (0.136 g) and 1-hydroxybenzotriazole (95.8 mg) in N, N-dimethylformamide (4 mL) at room temperature, and the resultant mixture was stirred for 14 hours. Water and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (methanol-chloroform), to obtain the title compound (0.186 g, 76%) as a solid.
¹H-NMR(400MHz, CDCl₃)δ: 2.59(3H, s), 3.73-3.84(6H, m), 3.87(3H, s), 4.14(2H, m), 7.07(1H, s), 7.18-7.22(2H, m), 7.37(1H, d, J=8.5Hz), 7.59-7.62(1H, m), 8.19(1H, d, J=2.9Hz), 8.39(1H, d, J=2.4Hz).
EI-MSm/z: 379(M⁺).
Elemental analysis: as C₂₀H₂₁N₅O₃
Theoretical value: C, 63.31; H, 5.58; N, 18.46.
Measured value: C, 63.42; H, 5.62; N, 18.51.

[Example 2] 1-[5-(5-Methyl-2-pyrazinyl)-1-(6-methyl-3-pyridyl)-1H-pyrazole-3-carbonyl]-4, 4-difluoropiperidine

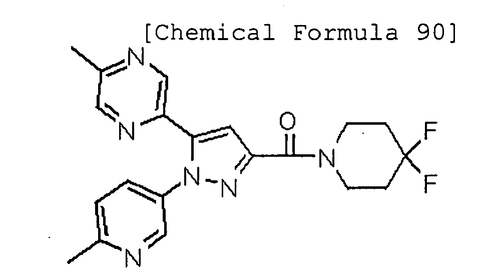

The title compound (0.252 g, 82%) was obtained as a solid by the same method as that in Example 1, using 5-(5-methyl-2-pyrazinyl)-1-(6-methyl-3-pyridyl)-1H-pyrazole-3-carboxylic acid (0.228 g) of Reference Example 8 and 4, 4-difluoropiperidine hydrochloride (0.146 g) of Reference Example 18.
¹H-NMR(400MHz, CDCl₃)δ: 2.10(4H, m), 2.58(3H, s), 2.61(3H, s), 3.93(2H, m), 4.20(2H, m), 7.22-7.24(2H, m), 7.61-7.63(1H, m), 8.34(1H, m), 8.41(1H, d, J=2.4Hz), 8.61(1H, d, J=1.5Hz).
EI-MSm/z: 398(M⁺).

[Example 3] 1-[5-(5-Methyl-2-pyrazinyl)-1-(6-methyl-3-pyridyl)-1H-pyrazole-3-carbonyl]-4-methoxypiperidine

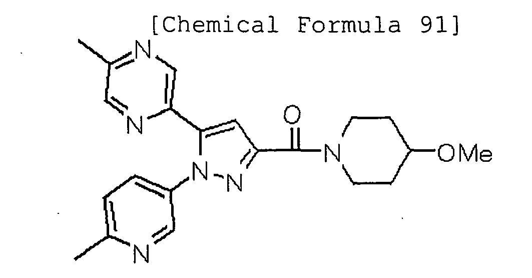

The title compound (0.244 g, 80%) was obtained as a solid by the same method as that in Example 1, using 5-(5-methyl-2-pyrazinyl)-1-(6-methyl-3-pyridyl)-1H-pyrazole-3-carboxylic acid (0.228 g) of Reference Example 8 and 4-methoxypiperidine hydrochloride (0.142 g) of Reference Example 23.
¹H-NMR(400MHz, CDCl₃)δ: 1.69(2H, m), 1.95(2H, m), 2.58(3H, s), 2.61(3H, s), 3.38-3.39(3H, m), 3.48-3.58(2H, m), 3.74-3.78(1H, m), 4.09(1H, m), 4.28(1H, m), 7.18(1H, s), 7.22(1H, d, J=8.3Hz), 7.62-7.65(1H, m), 8.34(1H, s), 8.41(1H, d, J=2.4Hz), 8.61(1H, s).
EI-MSm/z: 392(M⁺).

[Example 4] 1-[5-(5-Methoxy-2-pyridyl)-1-(6-methyl-3-pyridyl)-1H-pyrazole-3-carbonyl]-4-methoxypiperidine

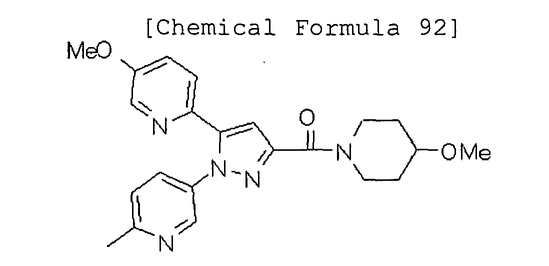

The title compound (0.208 g, 79%) was obtained as a solid by the same method as that in Example 1, using 5-(5-methoxy-2-pyridyl)-1-(6-methyl-3-pyridyl)-1H-pyrazole-3-carboxylic acid (0.200 g) of Reference Example 9 and 4-methoxypiperidine hydrochloride (0.119 g) of Reference Example 23.
¹H-NMR(400MHz, CDCl₃)δ: 1.68(2H, m), 1.94(2H, m), 2.59(3H, s), 3.38(3H, s), 3.47-3.55(2H, m), 3.68-3.76(1H, m), 3.87(3H, s), 4.09(1H, m), 4.25(1H, m), 7.00(1H, s), 7.17-7.21(2H, m), 7.36-7.38(1H, m), 7.62(1H, dd, J=8.3, 2.7Hz), 8.19(1H, d, J=2.9Hz), 8.39(1H, d, J=2.7Hz).
EI-MSm/z: 407(M⁺).
Elemental analysis: as C₂₂H₂₅N₅O₃
Theoretical value: C, 64.85; H, 6.18; N, 17.19.
Measured value: C, 64.85; H, 6.09; N, 17.28.

[Example 5] 1-[1-(6-Methoxy-3-pyridazinyl)-5-(2-pyridyl)-1H-pyrazole-3-carbonyl]hexahydropyridazine

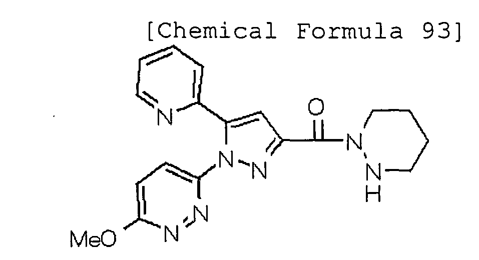

3-(3-Dimethylaminopropyl)-1-ethylcarbodiimide hydrochloride (0.864 g), 1-hydroxybenzotriazole (0.309 g), triethylamine (1.20 mL) and hexahydropyridazine hydrochloride (0.292 g) of Reference Example 20 were added to a solution of 1-(6-methoxy-3-pyridazinyl)-5-(2-pyridyl)-1H-pyrazole-3-carboxylic acid (0.610 g) of Reference Example 4 in dichloromethane (20 mL) at room temperature, and the resultant mixture was stirred for 21 hours. Water and chloroform were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain the title compound (0.360 g, 48%) as a solid.
¹H-NMR(400MHz, CDCl₃)δ: 1.67-1.87(4H, m), 2.96-3.07(2H, m), 3.85(2H×1/3, br), 4.11(3H, s), 4.23(2Hx2/3, br), 6.66(1H×1/3, s), 7.10-7.27(3H, m), 7.10-7.27(1H×2/3, m), 7.51-7.62(1H, m), 7.65-7.82(2H, m), 8.41(1H, br).
ESI-MSm/z: 366(M+H)⁺.

[Example 6] 1-[1-(6-Methoxy-3-pyridazinyl)-5-(2-pyridyl)-1H-pyrazole-3-carbonyl]-2-methylpyrazolidine

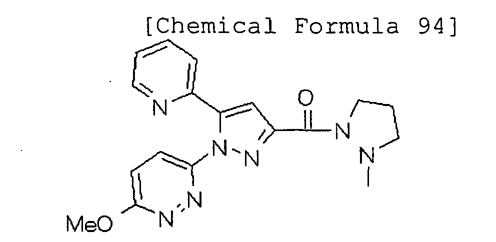

### 1) 2-[1-(6-Methoxy-3-pyridazinyl)-5-(2-pyridyl)-1H-pyrazole-3-carbonyl]pyrazolidine-1-carboxylic acid tert-butyl ester

2-[1-(6-Methoxy-3-pyridazinyl)-5-(2-pyridyl)-1H-pyrazole-3-carbonyl]pyrazolidine-1-carboxylic acid tert-butyl ester (1.31 g, 86%) was obtained as an amorphous form by the same method as that in Example 5, using 1-(6-methoxy-3-pyridazinyl)-5-(2-pyridyl)-1H-pyrazole-3-carboxylic acid (1.003 g) of Reference Example 4 and pyrazolidine-1-carboxylic acid tert-butyl ester (0.910 g, Y. Endo, et al., Bioorg. Med. Chem., 2002, 10, 953).
¹H-NMR(400MHz, CDCl₃)δ: 1.40(9H, br s), 2.05-2.22(2H, br), 3.15-3.60(2H, br), 4.05-4.35(2H, br), 4.09(3H, s), 7.12(1H, d, J=9.3Hz), 7.15-7.25(2H, m), 7.55(1H, br d, J=7.8Hz), 7.73(1H, dt, J=7.8, 1.7Hz), 7.90-8.00(1H, br), 8.41(1H, brd J=4.1Hz).
FAB-MSm/z: 452(M+H)⁺.

### 2) 1-[1-(6-Methoxy-3-pyridazinyl)-5-(2-pyridyl)-1H-pyrazole-3-carbonyl]pyrazolidine

A 4N hydrochloric acid-dioxane solution (10 mL) was added to a solution of 2-[1-(6-methoxy-3-pyridazinyl)-5-(2-pyridyl)-1H-pyrazole-3-carbonyl]pyrazolidine-1-carboxylic acid tert-butyl ester (1.31 g) in dichloromethane (30 mL), and the resultant mixture was stirred for 1 hour. Diethyl ether was added to the reaction solution, and the precipitated solid was filtered. To this solid, chloroform and a saturated aqueous solution of sodium hydrogen carbonate were added, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain 1-[1-(6-methoxy-3-pyridazinyl)-5-(2-pyridyl)-1H-pyrazole-3-carbonyl]pyrazolidine (0.591 g, 59%) as a solid.
¹H-NMR(400MHz, CDCl₃)δ: 2.05-2.30(2H, m), 3.11-3.26(2H, m), 3.75-3.86(1H, m), 4.10(3H, s), 4.12-4.20(1H, m), 4.60-4.70(1H×2/3, br), 5.45-5.53(1H×1/3, br), 7.11(1H×2/3, s), 7.13(1H×1/3, s), 7.16-7.34(2H, m), 7.52-7.96(3H, m), 8.35-8.45(1H, br).
ESI-MSm/z: 352(M+H)⁺.

### 3) Title compound

Sodium hydride (55% in oil, 63.6 mg) was added to a solution of 1-[1-(6-methoxy-3-pyridazinyl)-5-(2-pyridyl)-1H-pyrazole-3-carbonyl]pyrazolidine (0.419 g) in N, N-dimethylformamide (8.0 mL) at 0°C, and the resultant mixture was stirred for 20 minutes. Methyl iodide (0.111 mL) was added to the reaction solution, and the mixture was stirred for 1 hour. Water and ethyl acetate were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain the title compound (0.190 g, 59%) as a solid.
¹H-NMR(400MHz,CDCl₃)δ: 1.95-2.33(2H, br), 2.65(3H×2/3, br s), 2.75(3H×1/3, brs), 3.02-3.18(2H, m), 3.80-3.95(2H×2/3, br), 4.09(3H, s), 4.15-4.25(2H×1/3, br), 7.10-7.40(3H, m), 7.52-7.80(2H, m), 7.74(1H×2/3, d, J=7.3Hz), 8.35-8.50(1H, br).
ESI-MSm/z: 366(M+H)⁺.

[Example 7] 1-[1-(6-Methoxy-3-pyridyl)-5-(2-pyridyl)-1H-pyrazole-3-carbonyl]-2-formylpyrazolidine

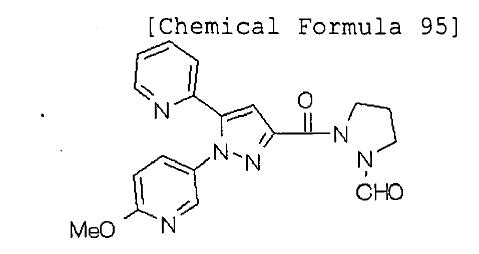

### 1) 1-[1-(6-Methoxy-3-pyridyl)-5-(2-pyridyl)-1H-pyrazole-3-carbonyl]pyrazolidine-1-carboxylic acid tert-butyl ester

2-[1-(6-Methoxy-3-pyridyl)-5-(2-pyridyl)-1H-pyrazole-3-carbonyl]pyrazolidine-1-carboxylic acid tert-butyl ester (1.33 g, 82%) was obtained as an amorphous form by the same method as that in Example 5, using 1-(6-methoxy-3-pyridyl)-5-(2-pyridyl)-1H-pyrazole-3-carboxylic acid (1.073 g) of Reference Example 3 and pyrazolidine-1-carboxylic acid tert-butyl ester (0.967 g, Y. Endo, et al., Bioorg. Med. Chem., 2002, 10, 953).
¹H-NMR(400MHz, CDCl₃)δ: 1.41(9H, br s), 2.05-2.20(2H, m), 3.15-3.60(2H, br), 3.94(3H, s), 4.00-4.50(2H, br), 6.74(1H, d, J=8.7Hz), 7.15-7.43(3H, m), 7.64(1H, dd, J=8.7, 2.7Hz), 7.67(1H, dt, J=7.8, 1.5Hz), 8.09(1H, d like, J=1.5Hz), 8.50(1H, d like, J=4.9Hz).
FAB-MSm/z: 451(M+H)⁺.

### 2) 1-[1-(6-Methoxy-3-pyridyl)-5-(2-pyridyl)-1H-pyrazole-3-carbonyl]pyrazolidine

1-[1-(6-Methoxy-3-pyridyl)-5-(2-pyridyl)-1H-pyrazole-3-carbonyl]pyrazolidine (0.895 g, 87%) was obtained as an amorphous form by the same method as that in Example 6-(2), using 2-[1-(6-methoxy-3-pyridyl)-5-(2-pyridyl)-1H-pyrazole-3-carbonyl]pyrazolidine-1-carboxylic acid tert-butyl ester (1.33 g).
¹H-NMR(400MHz, CDCl₃)δ: 2.08-2.30(2H, m), 3.10-3.25(2H, m), 3.76-3.81(2H×2/3, m), 3.95(3H, s), 4.10-4.23(2H×1/3, m), 4.76(1H×2/3, br), 5.46(1H×1/3, br), 6.75(1H, d like, J=8.8Hz), 7.17-7.76(5H, m), 8.13(1H, d like, J=2.2Hz), 8.50(1H, br).
FAB-MSm/z: 351(M+H)⁺.

### 3) Title compound

4-(Dimethylamino)pyridine (0.212 g) and trifluoromethanesulfonic anhydride (0.220 mL) were added to a solution of 1-[1-(6-methoxy-3-pyridyl)-5-(2-pyridyl)-1H-pyrazole-3-carbonyl]pyrazolidine (0.306 g) in N, N-dimethylformamide (5.0 mL) at 0°C, and the resultant mixture was stirred for 1 hour. An aqueous sodium hydrogen carbonate solution and ethyl acetate were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain the title compound (0.196 g, 59%) as a solid.
¹H-NMR(400MHz, CDCl₃)δ: 2.13-2.25(2H, m), 3. 66-3.77 (2H, m), 3.95(3H, s), 4.07-4.18(2H, br), 6.76(1H, d, J=9.0Hz), 7.23-7.34(2H, m), 7.41(1H, d, J=7.8Hz), 7.60(1H, dd, J=9.0, 2.7Hz), 7.72(1H, dt, J=7.8, 2.0Hz), 8.10(1H, d, J=2.0Hz), 8.52(1H, br d, J=4.9Hz), 8.59(1H, br s).ESI-MSm/z: 379(M+H)⁺.
Elemental analysis: as C₁₉H₁₈N₆O₃·0.25H₂O
Theoretical value: C, 59.60; H, 4.87; N, 21.95.
Measured value: C, 59.52; H, 4.77; N, 21.85.

[Example 8] 1-[1-(6-Methoxy-3-pyridyl)-5-(2-pyridyl)-1H-pyrazole-3-carbonyl]-2-methanesulfonylpyrazolidine

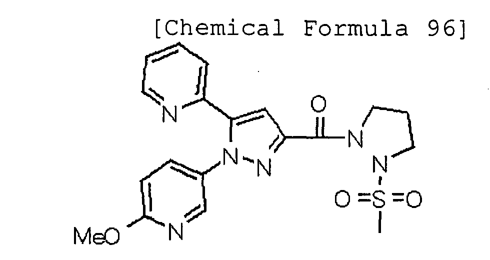

Triethylamine (0.168 mL) and methanesulfonyl chloride (0.0749 mL) were added to a solution of 1-[1-(6-methoxy-3-pyridyl)-5-(2-pyridyl)-1H-pyrazole-3-carbonyl]pyrazolidine (0.282 g) of Example 7-(2) in dichloromethane (5.0 mL) at room temperature, and the resultant mixture was stirred for 3 hours. Water and chloroform were added to the reaction solution, and the mixture was partitioned. The organic layer was washed with saturated saline, and then dried over anhydrous sodium sulfate. After separation by filtration, a residue obtained by evaporating the solvent under reduced pressure was purified by silica gel column chromatography (methanol-chloroform), to obtain the title compound (0.230 g, 67%) as a solid.
¹H-NMR(400MHz, CDCl₃)δ: 2.22-2.36(2H, m), 3.19(3H, s), 3.75(2H, br), 3.96(3H, s), 4.28(2H, br), 6.77(1H, d, J=8.8Hz), 7.21-7.28(1H, m), 7.30(1H, s), 7.41(1H, d like, J=7.8Hz), 7.61(1H, dd, J=8.8, 2.7Hz), 7.71(1H, dt, J=7.8, 1.7Hz), 8.12(1H, d, J=2.7Hz), 8.52(1H, d like, J=4.9Hz).
ESI-MSm/z: 429(M+H)⁺.
Elemental analysis: as C₁₉H₂₀N₆O₄S
Theoretical value: C, 53.26; H, 4.70; N, 19.61; S, 7.48.
Measured value: C, 53.19; H, 4.68; N, 19.48; S, 7.51.

[Example 9] 1-[1-(6-Methoxy-3-pyridazinyl)-5-(2-pyrazinyl)-1H-pyrazole-3-carbonyl]-4, 4-difluoropiperidine

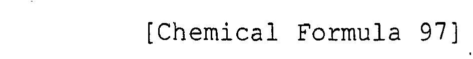

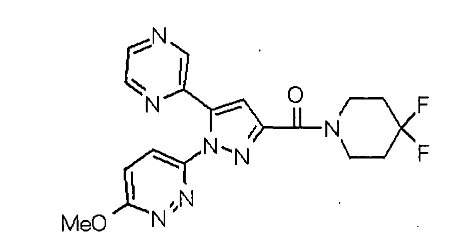

The title compound (0.265 g, 84%) was obtained as a solid by the same method as that in Example 1, using 1-(6-methoxy-3-pyridazinyl)-5-(2-pyrazinyl)-1H-pyrazole-3-carboxylic acid (0.232 g) of Reference Example 12 and 4, 4-difluoropiperidine hydrochloride (0.222 g) of Reference Example 18.
¹H-NMR(400MHz, CDCl₃)δ: 2.06-2.14(4H, m), 3.92-3.96 (2H, m), 4.12(3H, s), 4.14-4.18(2H, m), 7.19(1H, d, J=9.3Hz), 7.23(1H, s), 7.85(1H, d, J=9.3Hz), 8.42-8.43(1H, m), 8.53(1H, d, J=2.4Hz), 8.83-8.84(1H, m).
ESI-MSm/z: 402(M+H)⁺.
Elemental analysis: as C₁₈H₁₇F₂N₇O₂
Theoretical value: C, 53.86; H, 4.27; N, 24.43; F, 9.47.
Measured value: C, 53.57; H, 4.22; N, 24.39; F, 9.17.

[Example 10] 1-[1-(6-Methoxy-3-pyridyl)-5-(1H-pyrrol-1-yl)-1H-pyrazole-3-carbonyl]-4-methyl-3-oxopiperazine

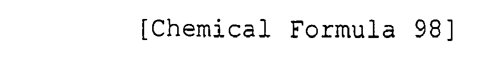

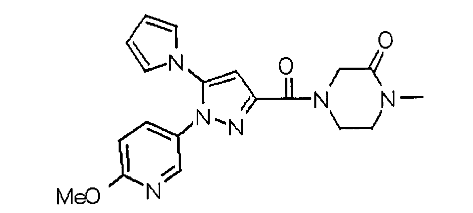

The title compound (0.239 g, 80%) was obtained as a solid by the same method as that in Example 1, using 1-(6-methoxy-3-pyridyl)-5-(1H-pyrrol-1-yl)-1H-pyrazole-3-carboxylic acid (0.221 g) of Reference Example 11 and 1-methylpiperazin-2-one hydrochloride (0.180 g) of Reference Example 21.
¹H-NMR(400MHz, CDCl₃)δ: 3.03(3H, s), 3.45-3.52(2H, m), 3.95(3H, s), 4.03-4.06(1H, m), 4.42-4.46(2H, m), 4.83(1H, br s), 6.31(2H, t, J=2.1Hz), 6.66(2H, t, J=2.2Hz), 6.71(1/2H, s), 6.73(1/2H, s), 6.86(1/2H, br s), 6.91(1/2H, br s), 7.18-7.23(1/2H, m), 7.32-7.35(1/2H, m), 7.96-7.98(1/2H, m), 8.05-8.07(1/2H, m).
ESI-MSm/z: 381(M+H)⁺.
Elemental analysis: as C₁₉H₂₀N₆O₃
Theoretical value: C, 59.99; H, 5.30; N, 22.09.
Measured value: C, 59.97; H, 5.23; N, 22.23.

[Example 11] 1-[1-(6-Methoxy-3-pyridyl)-5-(1H-pyrrol-2-yl)-1H-pyrazole-3-carbonyl]-4-methyl-3-oxopiperazine

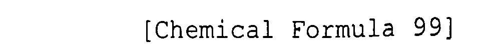

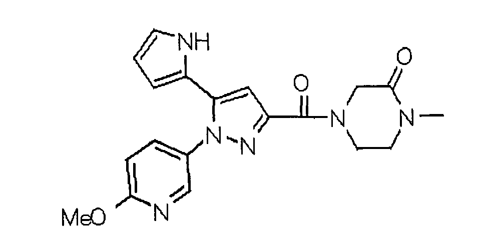

The title compound (0.246 g, 82%) was obtained as a solid by the same method as that in Example 1, using 1-(6-methoxy-3-pyridyl)-5-(1H-pyrrol-2-yl)-1H-pyrazole-3-carboxylic acid (0.222 g) of Reference Example 5 and 1-methylpiperazin-2-one hydrochloride (0.212 g) of Reference Example 21.
¹H-NMR(400MHz, DMSO-d₆)δ: 2.87(3H, s), 3.38-3.42(2H, m), 3.89(1H, br s), 3.93(3H, s), 4.18(1H, br s), 4.25-4.28(1H, m), 4.67(1H, br s), 5.54(1H, br s), 5.99-6.01(1H, m), 6.88-6.90(1H, m), 6.97-7.04(2H, m), 7.81(1H, dd, J=8.8, 2..7Hz), 8.27-8.30(1H, m), 11.42(1H, br s).
ESI-MSm/z: 381(M+H)⁺.

[Example 12] 1-[1-(6-Methoxy-3-pyridazinyl)-5-(1H-pyrrol-2-yl)-1H-pyrazole-3-carbonyl]-4-methylpiperazine

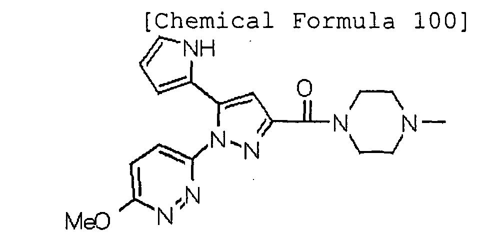

The title compound (0.197 g, 68%) was obtained as a solid by the same method as that in Example 1, using 1-(6-methoxy-3-pyridazinyl)-5-(1H-pyrrol-2-yl)-1H-pyrazole-3-carboxylic acid (0.222 g) of Reference Example 10 and N-methylpiperazine (0.156 mL).
¹H-NMR(400MHz, CDCl₃)δ: 2.33(3H, s), 2.42-2.45(2H, m), 2.49-2.52(2H, m), 3.83-3.86(2H, m), 3.94-3.97(2H, m), 4.19(3H, s), 6.28-6.31(1H, m), 6.61-6.64(1H, m), 6.94(1H, s), 6.96-6.98(1H, m), 7.19(1H, d, J=9.3Hz), 8.00(1H, d, J=9.5Hz), 11.56(1H, br s).
ESI-MSm/z: 368(M+H)⁺.
Elemental analysis: as C₁₈H₂₁N₇O₂
Theoretical value: C, 58.84;H, 5.76;N, 26.69.
Measured value: C, 58.54;H, 5.71;N, 26.82.

[Example 13] 4-[1-(6-Methoxy-3-pyridyl)-5-(1H-pyrrol-1-yl)-1H-pyrazole-3-carbonyl]morpholine

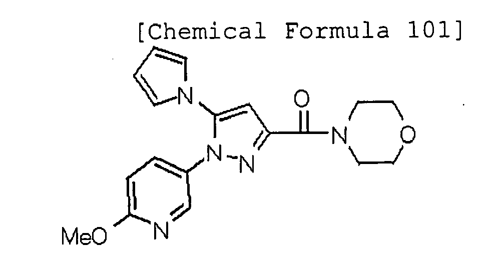

The title compound (0.227 g, 82%) was obtained as a solid by the same method as in Example 1, using 1-(6-methoxy-3-pyridyl)-5-(1H-pyrrol-1-yl)-1H-pyrazole-3-carboxylic acid (0.221 g) of Reference Example 11 and morpholine (0.123 mL).
¹H-NMR(400MHz, CDCl₃)δ: 3.74-3.84(6H, m), 3.94(3H, s), 4.17-4.20(2H, m), 6.29-6.31(2H, m), 6.66-6.67(2H, m), 6.71(1H, d, J=8.8Hz), 6.86(1H, s), 7.25(1H, dd, J=8.8, 2.7Hz), 8.02(1H, d, J=2.9Hz).
ESI-MSm/z: 354(M+H)⁺.
Elemental analysis: as C₁₈H₁₉N₅O₃
Theoretical value: C, 61.18; H, 5.42; N, 19.82.
Measured value: C, 61.23; H, 5.46; N, 19.70.

### [Example 14] 1-[1-(6-Methoxy-3-pyridazinyl)-5-(1H-pyrrol-2-yl)-1H-pyrazole-3-carbonyl]-4-methoxypiperidine

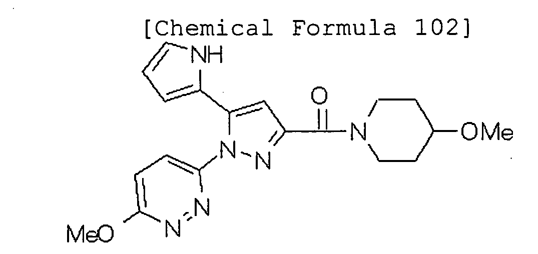

The title compound (0.177 g, 59%) was obtained as a solid by the same method as that in Example 1, using 1-(6-methoxy-3-pyridazinyl)-5-(1H-pyrrol-2-yl)-1H-pyrazole-3-carboxylic acid (0.222 g) of Reference Example 10 and 4-methoxypiperidine hydrochloride (0.216 g) of Reference Example 23.
¹H-NMR(400MHz, CDCl₃)δ: 1. 61-1. 75 (2H, m), 1. 86-2. 00 (2H, m), 3.38(3H, s), 3.47-3.65(3H, m), 4.05-4.16(2H, m), 4.19(3H, s), 6.28-6.31(1H, m), 6.61-6.64(1H, m), 6.91(1H, s), 6.96-6.98(1H, m), 7.18(1H, d, J=9.5Hz), 8.01(1H, d, J=9.3Hz), 11.60(1H, br s).
ESI-MSm/z: 383(M+H)⁺.
Elemental analysis: as C₁₉H₂₂N₆O₃
Theoretical value: C, 59.67; H, 5.80; N, 21.98.
Measured value: C, 59.44; H, 5.74; N, 22.03.

[Example 15] 4-[1-(6-Methoxy-3-pyridazinyl)-5-(1H-pyrrol-2-yl)-1H-pyrazole-3-carbonyl]morpholine

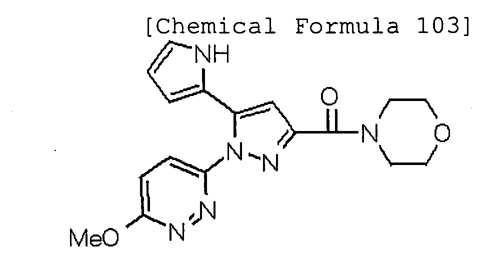

The title compound (0.197 g, 68%) was obtained as a solid by the same method as that in Example 1, using 1-(6-methoxy-3-pyridazinyl)-5-(1H-pyrrol-2-yl)-1H-pyrazole-3-carboxylic acid (0.222 g) of Reference Example 10 and morpholine (0.123 mL).
¹H-NMR(400MHz, CDCl₃)δ: 3.70-3.73 (2H, m), 3.79-3.84 (4H, m), 4.00-4.03(2H, m), 4.20(3H, s), 6.29-6.31(1H, m), 6.62-6.64(1H, m), 6.96-6.98(2H, m), 7.20(1H, d, J=9.5Hz), 7.97(1H, d, J=9.5Hz), 11.53(1H, br s).
ESI-MSm/z: 355(M+H)⁺.

[Example 16] 4-[1-(6-Methyl-3-pyridyl)-5-(1-methyl-1H-pyrrol-3-yl)-1H-pyrazole-3-carbonyl]morpholine

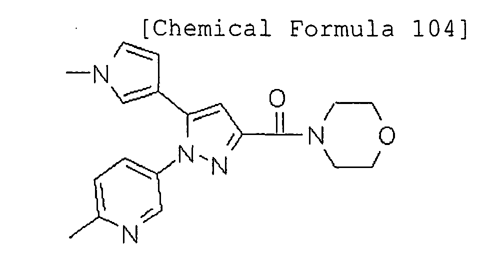

The title compound (0.228 g, 71%) was obtained as a solid by the same method as that in Example 5, using 1-(6-methyl-3-pyridyl)-5-(1-methyl-1H-pyrrol-3-yl)-1H-pyrazole-3-carboxylic acid (0.260 g) of Reference Example 14 and morpholine (0.200 mL).
¹H-NMR(400MHz, CDCl₃)δ: 2.62(3H, s), 3.60(3H, s), 3.67-3.86(6H, m), 4.10-4.20(2H, m), 5.86-5.90(1H, m), 6.47-6.53(2H, m), 6.79(1H, s), 7.21(1H, d, J=8.1Hz), 7.64(1H, dd, J=8.1, 2.7Hz), 8.57(1H, d, J=2.7Hz).
ESI-MSm/z: 352(M+H)⁺.

[Example 17] 1-[1-(6-Hydroxy-3-pyridazinyl)-5-(2-pyridyl)-1H-pyrazole-3-carbonyl]-4-methylpiperazine

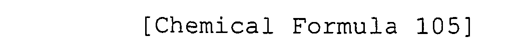

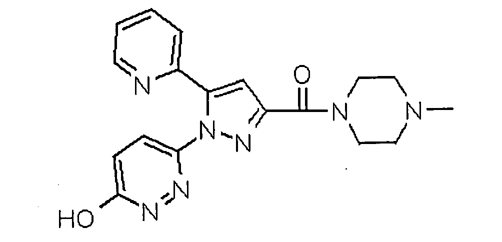

### 1) 1-(6-Hydroxy-3-pyridazinyl)-5-(2-pyridyl)-1H-pyrazole-3-carboxylic acid hydrochloride

A solution of 1-(6-methoxy-3-pyridazinyl)-5-(2-pyridyl)-1H-pyrazole-3-carboxylic acid (0.60 g) of Reference Example 4 in an aqueous 1 N hydrochloric acid solution (12 mL) was heated to reflux for 4 hours. After air cooling, the solvent of the reaction solution was evaporated under reduced pressure, and 1-(6-hydroxy-3-pyridazinyl)-5-(2-pyridyl)-1H-pyrazole-3-carboxylic acid hydrochloride (0.641 g, 96%) was obtained as a solid.
¹H-NMR(400MHz, DMSO-d₆)δ: 7.04-7.06(1H, m), 7.37-7.40(2H, m), 7.70(1H, d, J=9.8Hz), 7.86-7.95(2H, m), 8.49(1H, d, J=4.6Hz), 13.01(1H, br s).
EI-MSm/z: 283(M⁺).

### 2) Title compound

The title compound (0.149 g, 50%) was obtained as a solid by the same method as that in Example 1, using 1-(6-hydroxy-3-pyridazinyl)-5-(2-pyridyl)-1H-pyrazole-3-carboxylic acid hydrochloride (0.250 g) and N-methylpiperazine (0.104 mL).
¹H-NMR(400MHz, CDCl₃)δ: 2.33(3H, s), 2.45-2.52(4H, m), 3.81(2H, m), 4.04(2H, m), 7.04(1H, d, J=10.0Hz), 7.10(1H, s), 7.24-7.27(1H, m), 7.58-7.62(2H, m), 7.75-7.79(1H, m), 8.47-8.48(1H, m), 11.23(1H, br s).
EI-MSm/z: 365(M⁺).

[Example 18] 1-[1-(6-Methyl-3-pyridazinyl)-5-(5-methyl-2-pyridyl)-1H-pyrazole-3-carbonyl]-4, 4-difluoropiperidine

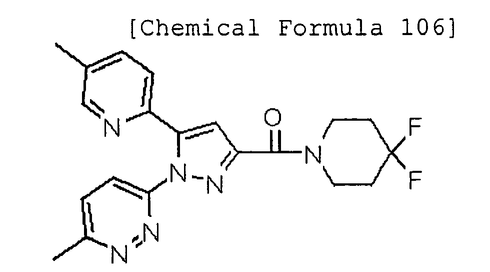

The title compound (0.236 g, 70%) was obtained as a solid by the same method as that in Example 1, using 1-(6-methyl-3-pyridazinyl)-5-(5-methyl-2-pyridyl)-1H-pyrazole-3-carboxylic acid (0.250 g) of Reference Example 15 and 4, 4-difluoropiperidine hydrochloride (0.160 g) of Reference Example 18.
¹H-NMR(400MHz, CDCl₃)δ: 2.09-2.10(4H, m), 2.33(3H, s), 2.74(3H, s), 3.92-3.94(2H, m), 4.15-4.17(2H, m), 7.08(1H, s), 7.47-7.57(3H, m), 7.82(1H, d, J=8.8Hz), 8.23(1H, m).
EI-MSm/z: 398(M⁺).
Elemental analysis: as C₂₀H₂₀F₂N₆O
Theoretical value: C, 60.29; H, 5.06; N, 21.09; F, 9.54.
Measured value: C, 60.11; H, 4.97; N, 20.95; F, 9.40.

[Example 19] 1-[1-(6-Methoxy-3-pyridazinyl)-5-(2-pyridyl)-1H-pyrazole-3-carbonyl]-4-methylpiperidine

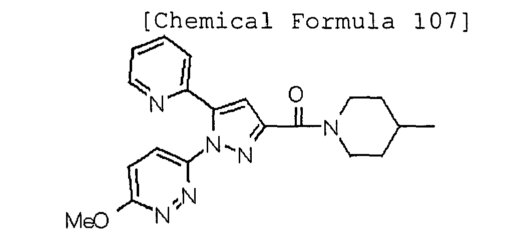

The title compound (0.289 g, 90%) was obtained as a solid by the same method as that in Example 1, using 1-(6-methoxy-3-pyridazinyl)-5-(2-pyridyl)-1H-pyrazole-3-carboxylic acid (0.250 g) of Reference Example 4 and 4-methylpiperidine (0.119 mL).
¹H-NMR(400MHz, CDCl₃)δ: 0.98(3H, d, J=6.1Hz), 1.19-1.28(2H, m), 1.60-1.76(3H, m), 2.76-2.84(1H, m), 3.11-3.17(1H, m), 4.10(3H, s), 4.62-4.74(2H, m), 7.04(1H, s), 7.12-7.14(1H, m), 7.20-7.24(1H, m), 7.56-7.58(1H, m), 7.72-7.76(1H, m), 7.83(1H, d, J=9.3Hz), 8.41-8.43(1H, m).
EI-MSm/z: 378(M⁺).
Elemental analysis: as C₂₀H₂₂N₆O₂·0.25H₂O
Theoretical value: C, 62.73; H, 5.92; N, 21.95.
Measured value: C, 62.92; H, 5.79; N, 21.97.

### [Example 20] 1-[1-(6-Methoxy-3-pyridyl)-5-(2-pyridyl)-1H-pyrazole-3-carbonyl]-4-methoxypiperidine

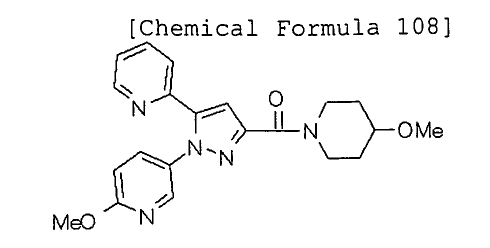

The title compound (0.302 g, 90%) was obtained as a solid by the same method as that in Example 1, using 1-(6-methoxy-3-pyridyl)-5-(2-pyridyl)-1H-pyrazole-3-carboxylic acid (0.250 g) of Reference Example 3 and 4-methoxypiperidine hydrochloride (0.142 g) of Reference Example 23.
¹H-NMR(400MHz, CDCl₃)δ: 1.67(2H, m), 1. 94 (2H, m), 3. 37 (3H, s), 3.47-3.55(2H, m), 3.71-3.76(1H, m), 3.95(3H, s), 4.09(1H, m), 4.27(1H, m), 6.75(1H, d, J=8.8Hz), 7.09(1H, s), 7.21-7.25(1H, m), 7.40-7.42(1H, m), 7.59(1H, dd, J=8.8, 2.7Hz), 7.68-7.72(1H, m), 8.11(1H, d, J=2.7Hz), 8.51-8.53(1H, m).
EI-MSm/z: 393(M⁺).
Elemental analysis: as C₂₁H₂₃N₅O₃·0.25H₂O
Theoretical value: C, 63.38; H, 5.95; N, 17.60.
Measured value: C, 63.53; H, 5.83; N, 17.69.

[Example 21] (3R)-1-[1-(6-Methoxy-3-pyridyl)-5-(2-pyridyl)-1H-pyrazole-3-carbonyl]-3-fluoropiperidine

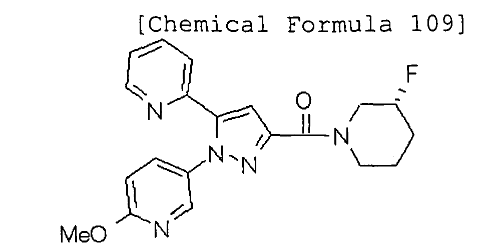

The title compound (271 mg, 66%) was obtained as a solid by the same method as that in Example 5, using 1-(6-methoxy-3-pyridyl)-5-(2-pyridyl)-1H-pyrazole-3-carboxylic acid (318 mg) of Reference Example 3 and (3R)-3-fluoropiperidine hydrochloride (150 mg) of Reference Example 26.
¹H-NMR(400MHz, CDCl₃)δ: 1.59(1H, br s), 2.00(3H, br s), 3.53(0.5H, s), 3.93-4.13(6H, m), 4.33(0.5H, s), 4.70(1H, d, J=46.6Hz), 6.76(1H, d, J=8.8Hz), 7.13(1H, s), 7.22-7.26(1H, m), 7.47(1H, d, J=8.0Hz), 7.56(1H, d, J=32.0Hz), 7.71(1H, td, J=7.8, 1.2Hz), 8.12(1H, d, J=2.4Hz), 8.52(1H, d, J=4.9Hz).
ESI-MSm/z: 382(M+H)⁺.
Elemental analysis: as C₂₀H₂₀FN₅O₂
Theoretical value: C, 62.98; H, 5.29; N, 18.36.
Measured value: C, 62.70; H, 5.22; N, 18.12.

[Example 22] (3R)-1-[1-(6-Methoxy-3-pyridazinyl)-5-(2-pyridyl)-1H-pyrazole-3-carbonyl]-3-fluoropiperidine

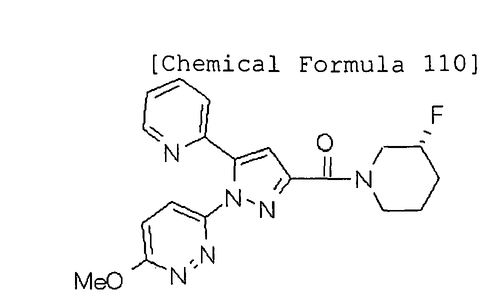

The title compound (268 mg, 70%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and (3R)-3-fluoropiperidine hydrochloride (140 mg) of Reference Example 26.
¹H-NMR(400MHz, CDCl₃)δ: 1.64(1H, br s), 2.00(3H, br s), 3.50-4.30(4H, m), 4.10(3H, s), 4.72(1H, dd, J=46.1, 25.1Hz), 7.10-7.15(2H, m), 7.20-7.23(1H, m), 7.59(1H, br s), 7.72-7.85(2H, m), 8.40-8.42(1H, m).
ESI-MSm/z: 383(M+H)⁺.

[Example 23] (2S)-1-[1-(6-Methoxy-3-pyridazinyl)-5-(2-pyridyl)-1H-pyrazole-3-carbonyl]-2-trifluoromethylpyrrolidine

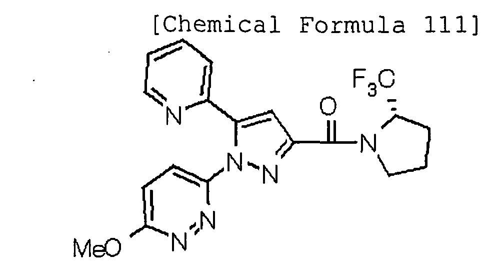

The title compound (240 mg, 45%) was obtained as a solid by the same method as that in Example 5, using 1-(6-methoxy-3-pyridazinyl)-5-(2-pyridyl)-1H-pyrazole-3-carboxylic acid (378 mg) of Reference Example 4 and (2S)-2-trifluoromethylpyrrolidine (177 mg).
¹H-NMR(400MHz, CDCl₃)δ: 2.05-2.25(4H, m), 3.72-6.05(3H, m), 4.11(3H, s), 7.14(1H, d, J=9.0Hz), 7.20-7.30(2H, m), 7.57(1H, br s), 7.62-7.81(2H, m), 8.40(1H, d, J=4.6Hz).
ESI-MSm/z: 419(M+H)⁺.
Elemental analysis: as C₁₉H₁₇F₃N₆O₂
Theoretical value: C, 54.55; H, 4.10; N, 20.09.
Measured value: C, 54.32; H, 4.04; N, 20.05.

### [Example 24] (3S)-1-[1-(6-Methoxy-3-pyridyl)-5-(2-pyridyl)-1H-pyrazole-3-carbonyl]-3-fluoropyrrolidine

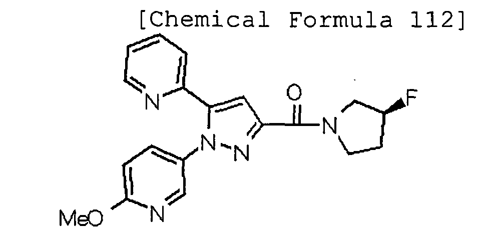

The title compound (69 mg, 19%) was obtained as a solid by the same method as that in Example 5, using 1-(6-methoxy-3-pyridyl)-5-(2-pyridyl)-1H-pyrazole-3-carboxylic acid (318 mg) of Reference Example 3 and (3S)-3-fluoropyrrolidine hydrochloride (126 mg) of Reference Example 24.
¹H-NMR(400MHz, CDCl₃)δ: 2.01-2.09(1H, m), 2.28-2.35(1H, m), 3.76-4.20(3H, m), 3.96(3H, s), 4.44-4.49(1H, m), 5.32(1H, dd, J=52.6, 4.3Hz), 6.76(1H, d, J=8.8Hz), 7.22-7.28(2H, m), 7.46(1H, dd, J=7.9, 2.8Hz), 7.57-7.61(1H, m), 7.72(1H, td, J=7.7, 1.7Hz), 8.14(1H, d, J=2.7Hz), 8.51(1H, t, J=2.4Hz).
ESI-MSm/z: 368(M+H)⁺.
Elemental analysis: as C₁₉H₁₈FN₅O₂·0.25H₂O
Theoretical value: C, 61.37; H, 5.01; N, 18.83.
Measured value: C, 61.51; H, 4.74; N, 18.95.

[Example 25] (2S, 5R)-1-[1-(6-Methoxy-3-pyridyl)-5-(2-pyridyl)-1H-pyrazole-3-carbonyl]-2-methoxymethyl-5-methylpyrrolidine

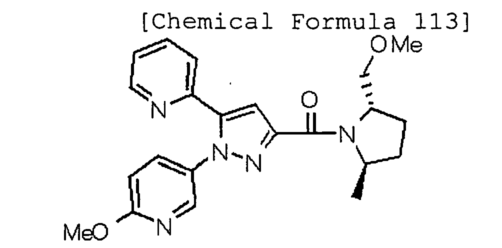

The title compound (158 mg, 30%) was obtained as an oily product by the same method as that in Example 5, using 1-(6-methoxy-3-pyridyl)-5-(2-pyridyl)-1H-pyrazole-3-carboxylic acid (385 mg) of Reference Example 3 and (2S, 5R)-2-methoxymethyl-5-methylpyrrolidine hydrochloride (215 mg) of Reference Example 29.
¹H-NMR(400MHz, CDCl₃)δ: 1.12 and 1.31(3H, d, J=6.3Hz), 1.55-2.30(3H, m), 3.24-5.25(6H, m), 3.38 and 3.40(3H, s), 3.95 and 3.96(3H, s), 6.73 and 6.77(1H, s), 7.22-7.24(1H, m), 7.47-7.49(1H, m), 7.57-7.61(1H, m), 7.70-7.74(1H, m), 8.09-8.13(1H, m), 8.49-8.51(1H, m).
ESI-MSm/z: 408(M+H)⁺.

[Example 26] 1-[1-(6-Methoxy-3-pyridazinyl)-5-(2-pyridyl)-1H-pyrazole-3-carbonyl]-2, 5-dimethylpyrrolidine

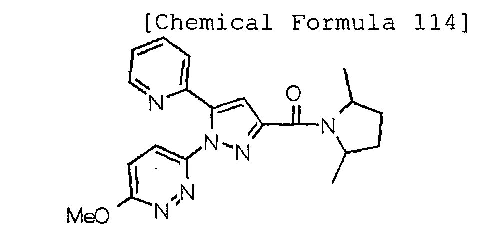

The title compound (227 mg, 61%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and 2, 5-dimethylpyrrolidine (100 mg).
¹H-NMR(400MHz, CDCl₃)δ: 1.10-1.52(6H, m), 1.57-1.74(2H, m), 1.96-2.35(2H, m), 4.11(3H, s), 4.35-5.16(2H, m), 7.12-7.23(3H, m), 7.59-7.62(1H, m), 7.73-7.83(2H, m), 8.40(1H, d, J=3.2Hz).
ESI-MSm/z: 379(M+H)⁺.
Elemental analysis: as C₂₀H₂₂N₆O₂
Theoretical value: C, 63.48; H, 5.86; N, 22.21.
Measured value: C, 63.23; H, 5.85; N, 22.20.

[Example 27] 1-[1-(6-Methoxy-3-pyridazinyl)-5-(2-pyridyl)-1H-pyrazole-3-carbonyl]-2-methylpyrrolidine

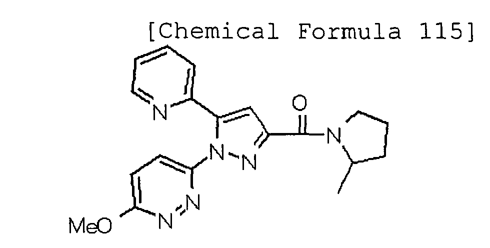

The title compound (153 mg, 42%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and 2-methylpyrrolidine (106 µL).
¹H-NMR(400MHz, CDCl₃)δ: 1.24(3×1/3H, d, J=6.3Hz), 1.35(3×2/3H, d, J=6.3Hz), 1.61-2.11(4H, m), 3.70-4.10(2H, m), 4.10(3H, s), 4.44(1×2/3H, dd, J=10.5, 6.3Hz), 5.00(1×1/3H, t, J=6.3Hz), 7.11-7.15(1H, m), 7.19-7.23(2H, m), 7.59-7.62(1H, m), 7.75(1H, t, J=7.7Hz), 7.81(1H, d, J=9.3Hz), 8.40(1H, t, J=3.7Hz).
ESI-MSm/z: 365(M+H)⁺.
Elemental analysis: as C₁₉H₂₀N₆O₂
Theoretical value: C, 62.62; H, 5.53; N, 23.06.
Measured value: C, 62.33; H, 5.52; N, 22.96.

[Example 28] 1-[1-(6-Methoxy-3-pyridyl)-5-(2-pyridyl)-1H-pyrazole-3-carbonyl]-2, 5-dimethylpyrrolidine

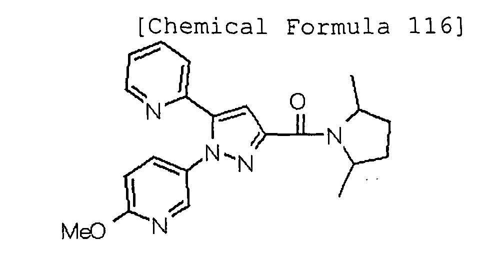

The title compound (105 mg, 28%) was obtained as a solid by the same method as that in Example 5, using 1-(6-methoxy-3-pyridyl)-5-(2-pyridyl)-1H-pyrazole-3-carboxylic acid (296 mg) of Reference Example 3 and 2, 5-dimethylpyrrolidine (100 mg).
¹H-NMR(400MHz, CDCl₃)δ: 1.10-1.45(6H, m), 1.60-2.15(4H, m), 3.96(3H, s), 4.33(1H, s), 4.85(1H, s), 6.74(1H, d, J=8.8Hz), 7.20-7.26(2H, m), 7.47(1H, d, J=7.8Hz), 7.57(1H, dd, J=8.8, 2.7Hz), 7.72(1H, td, J=7.8, 1.6Hz), 8.14(1H, d, J=2.7Hz), 8.50(1H, d, J=4.6Hz).
ESI-MSm/z: 378(M+H)⁺.
Elemental analysis: as C₂₁H₂₃N₅O₂
Theoretical value: C, 66.83; H, 6.14; N, 18.55.
Measured value: C, 66.57; H, 6.13; N, 18.55.

[Example 29] 1-[1-(6-Methoxy-3-pyridyl)-5-(2-pyridyl)-1H-pyrazole-3-carbonyl]-2-methylpyrrolidine

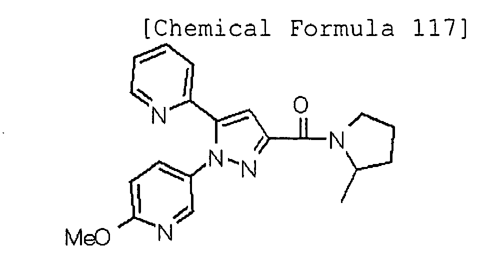

The title compound (219 mg, 60%) was obtained as a solid by the same method as that in Example 5, using 1-(6-methoxy-3-pyridyl)-5-(2-pyridyl)-1H-pyrazole-3-carboxylic acid (296 mg) of Reference Example 3 and 2-methylpyrrolidine (106 µL).
¹H-NMR(400MHz, CDCl₃)δ: 1.23(3×1/3H, d, J=6.3Hz), 1.34(3×2/3H, d, J=6.3Hz), 1.61-2.32(3H, m), 3.70-4.53(3H, m), 3.95(3×2/3H, s), 3.96(3×1/3H, s), 4.44(1×2/3H, dd, J=10.7, 6.3Hz), 5.04(1×1/3H, br s), 6.74(1H, d, J=8.8Hz), 7.20-7.23(2H, m), 7.47(1H, t, J=8.1Hz), 7.56-7.61(1H, m), 7.69-7.74(1H, m), 8.14(1H, q, J=2.9Hz), 8.50(1H, d, J=4.9Hz).
ESI-MSm/z: 364(M+H)⁺.
Elemental analysis: as C₂₀H₂₁N₅O₂
Theoretical value: C, 66.10; H, 5.82; N, 19.27.
Measured value: C, 66.09; H, 5.76; N, 19.34.

[Example 30] 1-[1-(6-Methoxy-3-pyridyl)-5-(4-pyrimidinyl)-1H-pyrazole-3-carbonyl]-4-methylpiperazine

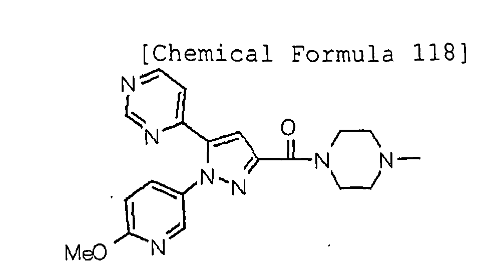

The title compound (51 mg, 76%) was obtained as a solid by the same method as that in Example 5, using 1-(6-methoxy-3-pyridyl)-5-(4-pyrimidinyl)-1H-pyrazole-3-carboxylic acid (50 mg) of Reference Example 16 and N-methylpiperazine (28 µL).
¹H-NMR(400MHz, CDCl₃)δ: 2.33(3H, s), 2.43-2.55(4H, m), 3.80-3.90(2H, m), 3.99(3H, s), 4.06-4.13(2H, m), 6.82(1H, d, J=8.8Hz), 7.34(1H, s), 7.39(1H, dd, J=5.1, 1.2Hz), 7.61(1H, dd, J=8.8, 2.7Hz), 8.15(1H, d, J=2.7Hz), 8.75(1H, d, J=5.1Hz), 9.09(1H, d, J=1.2Hz).
ESI-MSm/z: 380(M+H)⁺.

[Example 31] (2S)-1-[1-(6-Methoxy-3-pyridazinyl)-5-(2-pyridyl)-1H-pyrazole-3-carbonyl]-2-fluoromethylpyrrolidine

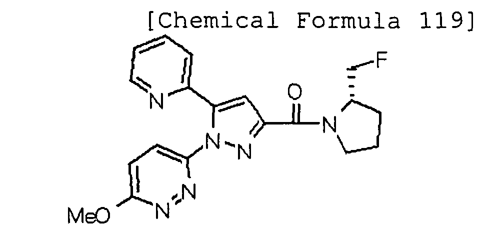

The title compound (245 mg, 64%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and (2S)-2-fluoromethylpyrrolidine hydrochloride (140 mg) of Reference Example 27.
¹H-NMR(400MHz, CDCl₃)δ: 1.92-2.08(4H, m), 3.71-4.82(5H, m), 4.11(3H, s), 7.12-7.16(1H, m), 7.20-7.27(2H, m), 7.59-7.63(1H, m), 7.73-7.82(2H, m), 8.39-8.42(1H, m).
ESI-MSm/z: 383(M+H)⁺.

[Example 32] 1-[5-(5-Methyl-2-pyrazinyl)-1-(6-methyl-3-pyridyl)-1H-pyrazole-3-carbonyl]-4-fluoropiperidine

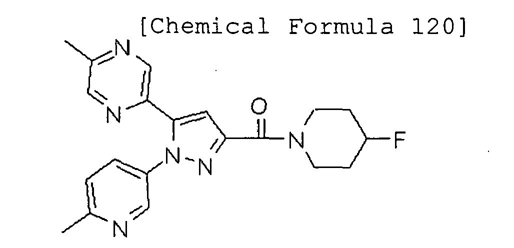

The title compound (0.225 g, 76%) was obtained as a solid by the same method as that in Example 1, using 5-(5-methyl-2-pyrazinyl)-1-(6-methyl-3-pyridyl)-1H-pyrazole-3-carboxylic acid (0.230 g) of Reference Example 8 and 4-fluoropiperidine hydrochloride (0.141 g) of Reference Example 19.
¹H-NMR(400MHz, CDCl₃)δ: 1.93-2.00(4H, m), 2.58(3H, s), 2.62(3H, s), 3.69-3.72(1H, m), 3.97-4.10(2H, m), 4.22-4.26(1H, m), 4.87-5.01(1H, m), 7.20-7.24(2H, m), 7.63(1H, dd, J=8.3, 2.7Hz), 8.34(1H, m), 8.42(1H, d, J=2.7Hz), 8.62(1H, d, J=1.5Hz).
EI-MSm/z: 380(M⁺).

[Example 33] 4-[1-(6-Methoxy-3-pyridyl)-5-(1H-pyrazol-3-yl)-1H-pyrazole-3-carbonyl]morpholine

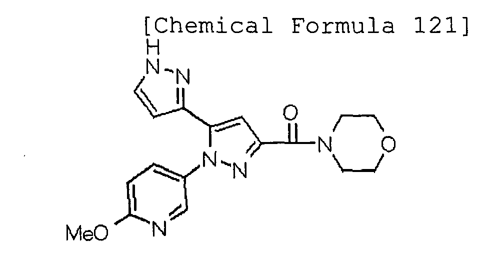

The title compound (0.241 g, 51%) was obtained as a solid by the same method as that in Example 1, using 1-(6-methoxy-3-pyridyl)-5-(1H-pyrazol-3-yl)-1H-pyrazole-3-carboxylic acid (0.370 g) of Reference Example 13 and morpholine (0.203 mL).
¹H-NMR(400MHz, CDCl₃)δ: 3.72-3.85(6H, m), 3. 98 (3H, s), 4.15-4.18(2H, m), 6.13(1H, d, J=2.2Hz), 6.80(1H, dd, J=8.8, 0.5Hz), 7.28(1H, s), 7.54(1H, d, J=2.4Hz), 7.63(1H, dd, J=8.8, 2.7Hz), 8.23-8.24(1H, m), 10.89(1H, br s).
ESI-MSm/z: 355(M+H)⁺.

[Example 34] 1-[1-(6-Methoxy-3-pyridyl)-5-(2-pyridyl)-1H-pyrazole-3-carbonyl]-3-methoxypiperidine

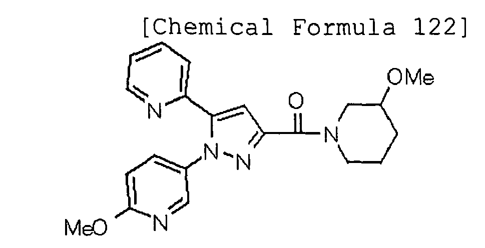

The title compound (285 mg, 86%) was obtained as a solid by the same method as that in Example 1, using 1-(6-methoxy-3-pyridyl)-5-(2-pyridyl)-1H-pyrazole-3-carboxylic acid (250 mg) of Reference Example 3 and 3-methoxypiperidine hydrochloride (166 mg) of Reference Example 28.
¹H-NMR(400MHz, CDCl₃)δ: 1.59-1.63(2H, m), 1.85(1H, m), 2.03(1H, m), 3.31(3/2H, s), 3.35-3.51(3H, m), 3.45(3/2H, s), 3.95-3.95(3H, m), 4.19-4.45(2H, m), 6.75(1H, d, J=8.8Hz), 7.10-7.14(1H, m), 7.22-7.25(1H, m), 7.40-7.45(1H, m), 7.59(1H, dd, J=8.8, 2.7Hz), 7.73-7.69(1H, m), 8.11(1H, s), 8.51(1H, s).
EI-MSm/z: 393(M⁺).
Elemental analysis: as C₂₁H₂₁N₅O₃
Theoretical value: C, 64.11; H, 5.89; N, 17.80.
Measured value: C, 63.89; H, 5.87; N, 17.63.

[Example 35] 4-[1-(6-Methoxy-3-pyridyl)-5-(1H-pyrrol-3-yl)-1H-pyrazole-3-carbonyl]morpholine

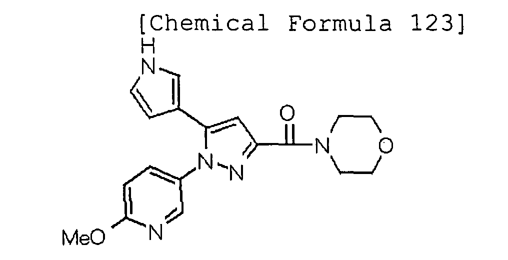

The title compound (230 mg, 84%) was obtained as a foamy material by the same method as that in Example 1, using 1-(6-methoxy-3-pyridyl)-5-(1H-pyrrol-3-yl)-1H-pyrazole-3-carboxylic acid (200 mg) of Reference Example 30 and morpholine (184 µL).
¹H-NMR(400MHz, CDCl₃)δ: 3.68-3.83 (6H, m), 3.98(3H, s), 4.16(2H, br s), 6.03-6.6(1H, m), 6.62-6.65(1H, m), 6.71-6.75(1H, m), 6.79(1H, d, J=8.8Hz), 6.84(1H, s), 7.61(1H, dd, J=8.8, 2.7Hz), 8.24(1H, d, J=2.7Hz), 8.41(1H, br s).
FAB-MSm/z: 354(M+H)⁺.

[Example 36] 1-[1-(6-Methoxy-3-pyridyl)-5-(1-methyl-1H-pyrazol-3-yl)-1H-pyrazole-3-carbonyl]-4-methyl-3-oxopiperazine

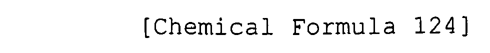

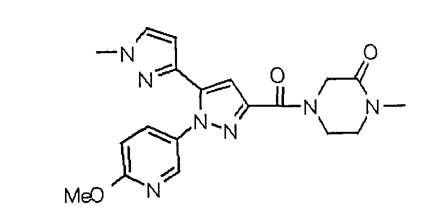

The title compound (0.094 g, 59%) was obtained as an amorphous material by the same method as that in Example 5, using 1-(6-methoxy-3-pyridyl)-5-(1-methyl-1H-pyrazol-3-yl)-1H-pyrazole-3-carboxylic acid (0.119 g) of Reference Example 31 and 1-methylpiperazin-2-one (0.139 g) of Reference Example 46.
¹H-NMR(400MHz, CDCl₃)δ: 3.01(3H, s), 3.42-3.50(2H, m), 3.87(3H, s), 3.98(3H, s), 3.95-4.10(1H+1H×1/2, m), 4.36-4.47(1H+1H×1/2, m), 4.80(1H, br), 5.95-6.06(1H, m), 6.78(1H×1/2, s), 6.80(1H×1/2, s), 7.09(1H, br), 7.30(1H, br), 7.59-7.65(1H, m), 8.21(1H, br).
ESI-MSm/z: 396(M+H)⁺.

[Example 37] 4-[1-(6-Methoxy-3-pyridyl)-5-(1-methyl-1H-pyrazol-3-yl)-1H-pyrazole-3-carbonyl]morpholine

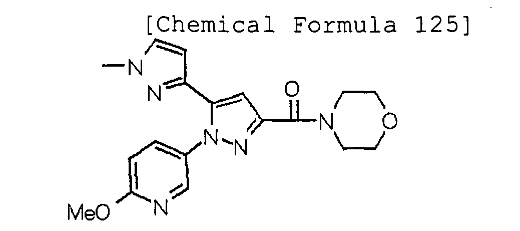

The title compound (0.225 g, 81%) was obtained as a solid by the same method as that in Example 5, using 1-(6-methoxy-3-pyridyl)-5-(1-methyl-1H-pyrazol-3-yl)-1H-pyrazole-3-carboxylic acid (0.225 g) of Reference Example 31 and morpholine (0.164 mL).
¹H-NMR(400MHz, CDCl₃)δ: 3.66-3.87(6H, m), 3.87 (3H, s), 3.97(3H, s), 4.06-4.15(2H, m), 5.99(1H, d, J=2.4Hz), 6.78(1H, d, J=8.8Hz), 7.04(1H, s), 7.29(1H, d, J=2.4Hz), 7.63(H, dd, J=8.8, 3.0Hz), 8.21(1H, d, J=3.0Hz).
ESI-MSm/z: 369(M+H)⁺.

[Example 38] 1-[1-(6-Methyl-3-pyridyl)-5-(1-methyl-1H-pyrrol-3-yl)-1H-pyrazole-3-carbonyl]-4-methyl-3-oxopiperazine

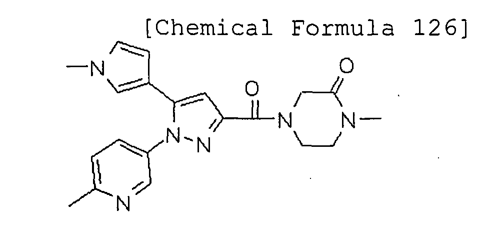

The title compound (0.225 g, 67%) was obtained as an amorphous material by the same method as that in Example 5, using 1-(6-methyl-3-pyridyl)-5-(1-methyl-1H-pyrrol-3-yl)-1H-pyrazole-3-carboxylic acid (0.251 g) of Reference Example 14 and 1-methyl piperazin-2-one (0.329 g) of Reference Example 46.
¹H-NMR(400MHz, CDCl₃)δ: 2.60(3H, s), 2.98(3H, s), 3.38-3.48(2H+1H×1/2, m), 3.58(3H, s), 4.00(1H, br), 4.39(1H+1H×1/2, br), 4.78(1H, br), 5.86(1H, br), 6.44-6.52(2H, m), 6.78(1H×1/2, s), 6.83(1H×1/2, s), 7.16-7.25(1H, m), 7.55-7.70(1H, m), 8.52(1H×1/2, br), 8.57(1H×1/2, br).
ESI-MSm/z: 379(M+H)⁺.

[Example 39] (2S)-1-[1-(6-Methoxy-3-pyridyl)-5-(1H-pyrrol-3-yl)-1H-pyrazole-3-carbonyl]-2-carbamoylpyrrolidine

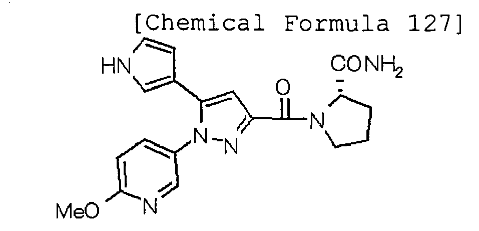

The title compound (0.109 g, 32%) was obtained as a solid by the same method as that in Example 1, using 1-(6-methoxy-3-pyridyl)-5-(1H-pyrrol-3-yl)-1H-pyrazole-3-carboxylic acid (0.250 g) of Reference Example 30 and L-proline amide (0.120 g).
¹H-NMR(400MHz, DMSO-d₆)δ: 1.77-2.09(4H, m), 3.58(1H, m), 3.90(3H, s), 3.96(1H, m), 4.40(0.5H, dd, J=8.42, 3.85Hz), 5.12(0.5H, J=8.54, 2.87Hz), 5.87(1H, d, J=2.87Hz), 6.72(1H, m), 6.81(1H, s), 6.93(1H, dd, J=20.4, 8.73Hz), 7.33(1H, s), 7.75(1H, ddd, J=21.9, 8.73, 2.7Hz), 8.24(1H, m), 11.05(1H, br).
FAB-MSm/z: 381(M+H)⁺.

[Example 40] 4-[1-(6-Methoxy-3-pyridyl)-5-(1-methyl-1H-pyrrol-3-yl)-1H-pyrazole-3-carbonyl]morpholine

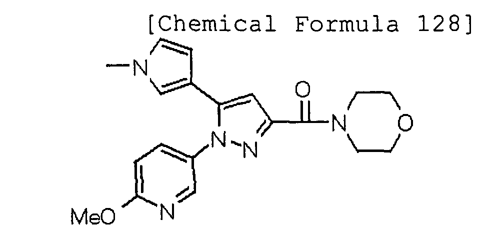

The title compound (0.162 g, 53%) was obtained as a solid by the same method as that in Example 1, using 1-(6-methoxy-3-pyridyl)-5-(1-methyl-1H-pyrrol-3-yl)-1H-pyrazole-3-carboxylic acid (0.250 g) of Reference Example 32 and morpholine (110 µL).
¹H-NMR(400MHz, CDCl₃)δ: 3.60(3H, s), 3.72-3.79(6H, br), 3.99(3H, s), 4.15(2H, br), 5.90(1H, dd, J=2.69, 1.83Hz), 6.47(1H, t, J=1.83Hz), 6.51(1H, t, J=2.56Hz), 6.78(1H, d, J=8.79Hz), 6.79(1H, s), 7.61(1H, dd, J=8.79, 2.69Hz), 8.24(1H, d, J=2.69Hz).
FAB-MSm/z: 368(M+H)⁺.

[Example 41] 1-[1-(6-Methoxy-3-pyridazinyl)-5-(1H-pyrrol-3-yl)-1H-pyrazole-3-carbonyl]morpholine

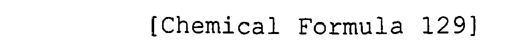

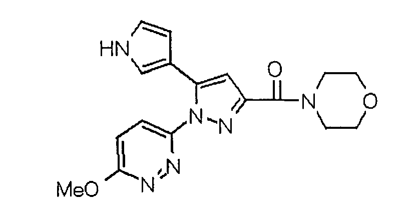

The title compound (0.135 g, 42%) was obtained as a solid by the same method as that in Example 1, using 1-(6-methoxy-3-pyridazinyl)-5-(1H-pyrrol-3-yl)-1H-pyrazole-3-carboxylic acid (0.250 g) of Reference Example 33 and morpholine (115 µL).
¹H-NMR(400MHz, CDCl₃)δ: 3.71(2H, br), 3.80(4H, br), 4.10(2H, br), 4.19(3H, s), 6.19(1H, dd, J=4.15, 2.44Hz), 6.75(1H, dd, J=4.88, 2.19Hz), 6.85(1H, s), 7.03(1H, m), 7.08(1H, d, J=9.28Hz), 7.55(1H, d, J=9.28Hz).
FAB-MSm/z: 355(M+H)⁺.

[Example 42] 1-[1-(6-Methyl-3-pyridazinyl)-5-(1-methyl-1H-pyrrol-3-yl)-1H-pyrazole-3-carbonyl]-4, 4-difluoropiperidine

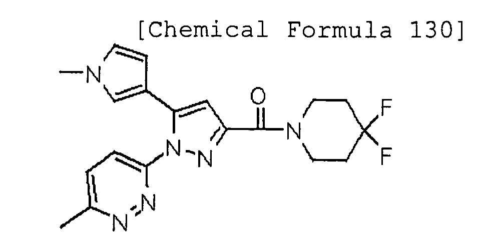

The title compound (0.295 g, 88%) was obtained as a solid by the same method as that in Example 1, using 1-(6-methyl-3-pyridazinyl)-5-(1-methyl-1H-pyrrol-3-yl)-1H-pyrazole-3-carboxylic acid (0.245 g) of Reference Example 36 and 4, 4-difluoropiperidine hydrochloride (0.217 g) of Reference Example 18.
¹H-NMR(400MHz, CDCl₃)δ: 1.97-2.15(4H, m), 2.79(3H, s), 3.63(3H, s), 3.91(2H, br), 4.13(2H, br), 6.11-6.15(1H, m), 6.51-6.57(1H, m), 6.81(1H, s), 6.92-6.97(1H, m), 7.46(1H, d, J=8.8Hz), 7.61(1H, d, J=8.8Hz).
ESI-MSm/z: 387(M+H)⁺.

[Example 43] 1-[5-(1H-Imidazol-2-yl)-1-(6-methoxy-3-pyridyl)-1H-pyrazole-3-carbonyl]-4-methoxypiperidine

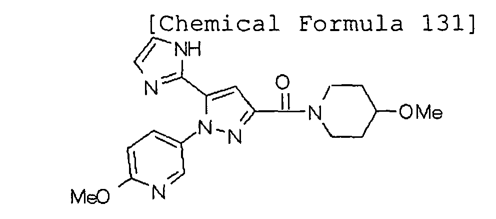

An aqueous 1 N sodium hydroxide solution (1.00 mL) was added to a mixed suspension of 5-(1H-imidazol-2-yl)-1-(6-methoxy-3-pyridyl)-1H-pyrazole-3-carboxylic acid ethyl ester (0.210 g) of Reference Example 43 in ethanol (5 mL) and tetrahydrofuran (2.5 mL), and the resultant mixture was stirred for 5 hours at room temperature. Further, an aqueous 1 N sodium hydroxide solution (1.00 mL) was added to the reaction solution, and the mixture was stirred for 20.5 hours. An aqueous 1 N hydrochloric acid solution (2.00 mL) was added to the reaction solution, and the reaction solvent was evaporated under reduced pressure, to obtain 5-(1H-imidazol-2-yl)-1-(6-methoxy-3-pyridyl)-1H-pyrazole-3-carboxylic acid. Using this carboxylic acid and 4-methoxypiperidine hydrochloride (0.218 g) of Reference Example 23, the title compound (73.7 mg, 28%) was obtained as a solid by the same method as that in Example 1.
¹H-NMR(400MHz, CDCl₃)δ: 1.64-1.77(2H, m), 1.87-1.99(2H, m), 3.38(3H, s), 3.49-3.62(2H, m), 3.78-3.86(1H, m), 3.93-4.03(1H, m), 3.97(3H, s), 4.21-4.28(1H, m), 6.77(1H, dd, J=8.8, 0.5Hz), 7.10(2H, br s), 7.16(1H, s), 7.64(1H, dd, J=8.8, 2.7Hz), 8.26(1H, dd, J=2.7, 0.5Hz), 11.05(1H, br s).
ESI-MSm/z: 383(M+H)⁺.

[Example 44] 1-[5-(5-Cyano-2-pyridyl)-1-(6-methyl-3-pyridyl)-1H-pyrazole-3-carbonyl]-4, 4-difluoropiperidine

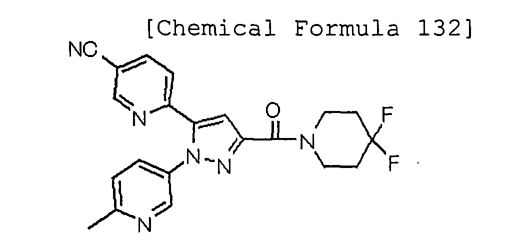

The title compound (0.827 g, 84%) was obtained as a solid by the same method as that in Example 1, using 5-(5-cyano-2-pyridyl)-1-(6-methyl-3-pyridyl)-1H-pyrazole-3-carboxylic acid (0.740 g) of Reference Example 37 and 4, 4-difluoropiperidine hydrochloride (0.458 g) of Reference Example 18.
¹H-NMR(400MHz, CDCl₃)δ: 2.08-2.10(4H, m), 2.64(3H, s), 3.93(2H, m), 4.20(2H, m), 7.25-7.27(1H, m), 7.32(1H, s), 7.59-7.62(2H, m), 8.00(1H, dd, J=8.2, 2.1Hz), 8.41(1H, d, J=2.4Hz), 8.70-8.71(1H, m).
EI-MSm/z: 408(M⁺).

### [Example 45] 1-[5-(5-Carbamoyl-2-pyridyl)-1-(6-methyl-3-pyridyl)-1H-pyrazole-3-carbonyl]-4, 4-difluoropiperidine

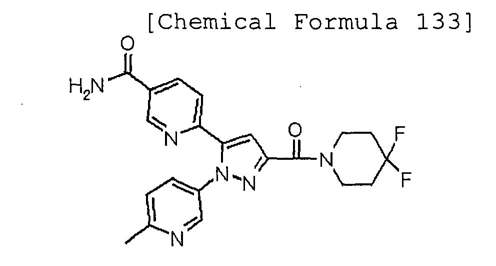

An aqueous 1 N sodium hydroxide solution (5.02 mL) was added to a mixed solution of 1-[5-(5-cyano-2-pyridyl)-1-(6-methyl-3-pyridyl)-1H-pyrazole-3-carbonyl]-4, 4-difluoropiperidine (0.410 g) of Example 44 in methanol (8.2 mL) and tetrahydrofuran (8.2 mL) at room temperature, and the resultant mixture was heated to reflux for 15 minutes. After air cooling, water and a mixed solvent of chloroform-methanol (10: 1 (v/v)) were added to the reaction solution, which was then partitioned. A residue obtained by evaporating the solvent of the organic layer under reduced pressure was purified by silica gel column chromatography (methanol-chloroform), to obtain the title compound (0.237 g, 55%) as a solid.
¹H-NMR(400MHz, DMSO-d₆)δ: 2.07(4H, m), 3.32(3H, s), 3.79(2H, m), 4.06(2H, m), 7.34-7.36(2H, m), 7.63(1H, br s), 7.69-7.71(1H, m), 7.83-7.85(1H, m), 8.15(1H, br s), 8.27-8.30(1H, m), 8.43(1H, d, J=2.4Hz), 8.85-8.86(1H, m).
EI-MSm/z: 426(M⁺).

[Example 46] 1-[1-(6-Methoxy-3-pyridyl)-5-(2-pyrazinyl)-1H-pyrazole-3-carbonyl]-4, 4-difluoropiperidine

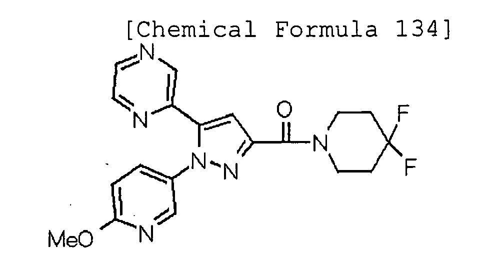

The title compound (242 mg, 77%) was obtained as a solid by the same method as that in Example 1, using 1-(6-methoxy-3-pyridyl)-5-(2-pyrazinyl)-1H-pyrazole-3-carboxylic acid (0.231 g) of Reference Example 6 and 4, 4-difluoropiperidine hydrochloride (0.222 g) of Reference Example 18.
¹H-NMR(400MHz, CDCl₃)δ: 2.05-2.14(4H, m), 3.91-3.95(2H, m), 3.97(3H, s), 4.19-4.22(2H, m), 6.80(1H, dd, J=8.8, 0.5Hz), 7.29(1H, s), 7.59(1H, dd, J=8.8, 2.7Hz), 8.13(1H, dd, J=2.7, 0.7Hz), 8.48(1H, dd, J=2.4, 1.5Hz), 8.52(1H, d, J=2.4Hz), 8.73(1H, d, J=1.5Hz).
ESI-MSm/z: 401(M+H)⁺.
Elemental analysis: as C₁₉H₁₈F₂N₆O₂
Theoretical value: C, 57.00; H, 4.53; N, 20.99; F, 9.49.
Measured value: C, 56.78; H, 4.40; N, 20.92; F, 9.36.

[Example 47] 1-[1-(6-Methoxy-3-pyridyl)-5-(2-pyrazinyl)-1H-pyrazole-3-carbonyl]-4-methoxypiperidine

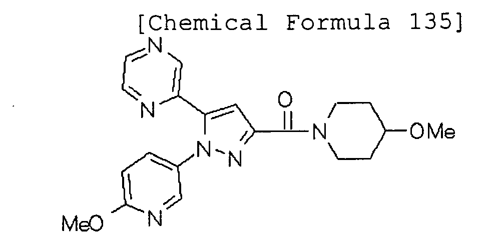

The title compound (243 mg, 78%) was obtained as a solid by the same method as that in Example 1, using 1-(6-methoxy-3-pyridyl)-5-(2-pyrazinyl)-1H-pyrazole-3-carboxylic acid (0.231 g) of Reference Example 6 and 4-methoxypiperidine hydrochloride (0.218 g) of Reference Example 23.
¹H-NMR(400MHz, CDCl₃)δ: 1.63-1.75(2H, m), 1.89-2.00(2H, m), 3.38(3H, s), 3.48-3.59(2H, m), 3.72-3.79(1H, m), 3.97(3H, s), 4.06-4.12(1H, m), 4.25-4.31(1H, m), 6.79(1H, dd, J=8.8, 0.5Hz), 7.23(1H, s), 7.61(1H, dd, J=8.8, 2.7Hz), 8.12(1H, dd, J=2.7, 0.5Hz), 8.48(1H, dd, J=2.4, 1.5Hz), 8.51(1H, d, J=2.4Hz), 8.73(1H, d, J=1.5Hz).
ESI-MSm/z: 395(M+H)⁺.
Elemental analysis: as C₂₀H₂₂N₆O₃
Theoretical value: C, 60.90; H, 5.62; N, 21.31.
Measured value: C, 60.69; H, 5.60; N, 21.12.

[Example 48] 1-[1-(6-Methoxy-3-pyridyl)-5-(1-methyl-1H-imidazol-4-yl)-1H-pyrazole-3-carbonyl]-4, 4-difluoropiperidine

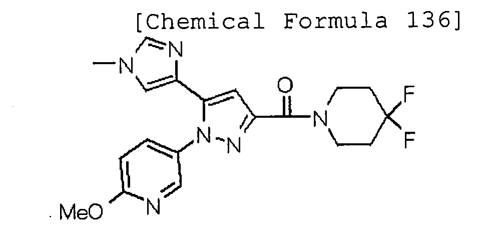

The title compound (182 mg, 74%) was obtained as a solid by the same method as that in Example 5, using 1-(6-methoxy-3-pyridyl)-5-(1-methyl-1H-imidazol-4-yl)-1H-pyrazole-3-carboxylic acid (183 mg) of Reference Example 34 and 4, 4-difluoropiperidine hydrochloride (116 mg) of Reference Example 18.
¹H-NMR(400MHz, CDCl₃)δ: 2.04(4H, br s), 3.66(3H, s), 3.90(2H, s), 3.98(3H, s), 4.15(2H, s), 6.69(1H, s), 6.80(1H, d, J=8.8Hz), 7.00(1H, s), 7.41(1H, s), 7.68(1H,
dd, J=8.8, 2.7Hz), 8.24(1H, d, J=2.7Hz).
ESI-MSm/z: 403(M+H)⁺.

[Example 49] 4-[5-(3-Hydroxymethyl-1H-pyrrol-1-yl)-1-(6-methoxy-3-pyridyl)-1H-pyrazole-3-carbonyl]morpholine

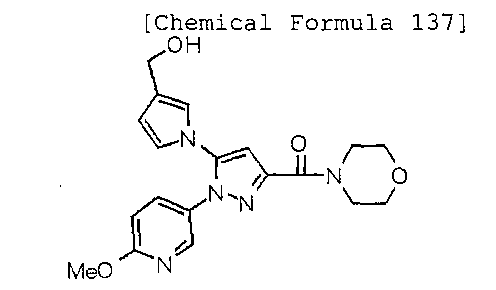

Under cooling to 0°C, sodium borohydride (36.7 mg) was added to a mixed solution of 5-(3-formyl-1H-pyrrol-1-yl)-1-(6-methoxy-3-pyridyl)-1H-pyrazole-3-carboxylic acid ethyl ester (0.607 g) of Reference Example 38 in methanol (20 mL) and dichloromethane (10 mL), and the resultant mixture was stirred for 35 minutes, and then stirred for 21 hours at room temperature. Dichloromethane and a saturated aqueous ammonium chloride solution were added to the reaction solution, and the mixture was partitioned. The organic layer was dried over anhydrous sodium sulfate. After separation by filtration, the solvent was evaporated under reduced pressure, and 5-(3-hydroxymethyl-1H-pyrrol-1-yl)-1-(6-methoxy-3-pyridyl)-1H-pyrazole-3-carboxylic acid ethyl ester was obtained. This ethyl ester product was dissolved in methanol (5 mL), an aqueous 1 N sodium hydroxide solution (5.5 mL) was added thereto, and the mixture was stirred for 20.5 hours at room temperature. Diethyl ether and an aqueous 1 N hydrochloric acid solution were added to the reaction solution, and the mixture was partitioned. Further, sodium chloride was added to the aqueous layer to saturation, and the aqueous layer was extracted with ethyl acetate. The organic layers were combined and dried over anhydrous sodium sulfate. After separation by filtration, the solvent was evaporated under reduced pressure, and 5-(3-hydroxymethyl-1H-pyrrol-1-yl)-1-(6-methoxy-3-pyridyl)-1H-pyrazole-3-carboxylic acid (0.595 g, quantitative) was obtained as an amorphous material. Using this carboxylic acid (0.298 g) and morpholine (0.140 mL), the title compound (217 mg, 63%) was obtained as an amorphous material by the same method as that in Example 1.
¹H-NMR(400MHz, CDCl₃)δ: 3.74-3.83(6H, m), 3.95(3H, s), 4.16-4.19(2H, m), 4.55(2H, s), 6.33(1H, dd, J=2.9, 1.7Hz), 6.62(1H, dd, J=2.7, 2.4Hz), 6.66-6.67(1H, m), 6.73(1H, d, J=8.8Hz), 6.84(1H, s), 7.32(1H, dd, J=9.0, 2.7Hz), 8.01(1H, d, J=2.7Hz).
ESI-MSm/z: 384(M+H)⁺.

[Example 50] 1-[1-(6-Methoxy-3-pyridazinyl)-5-(2-pyridyl)-1H-pyrazole-3-carbonyl]-3, 3-difluoropyrrolidine

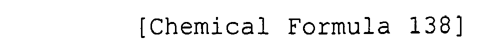

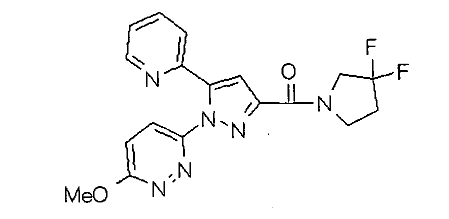

The title compound (311 mg, 80%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and 3, 3-difluoropyrrolidine hydrochloride (144 mg) of Reference Example 44.
¹H-NMR(400MHz, CDCl₃)δ: 2.35-2.52(2H, m), 3.95(1H, t, J=7.6Hz), 4.04(1H, t, J=13.1Hz), 4.12(3H, s), 4.33(1H, t, J=7.4Hz), 4.42(1H, t, J=12.9Hz), 7.15(1H, t, J=9.8Hz), 7.22(1H, dd, J=7.6, 4.9Hz), 7.27(1H, s), 7.62(1H, d, J=7.8Hz), 7.73-7.79(2H, m), 8.40(1H, d, J=4.9Hz).
ESI-MSm/z: 387(M+H)⁺.

[Example 51] 1-[1-(6-Methoxy-3-pyridazinyl)-5-(5-methyl-2-pyridyl)-1H-pyrazole-3-carbonyl]-4-methoxypiperidine

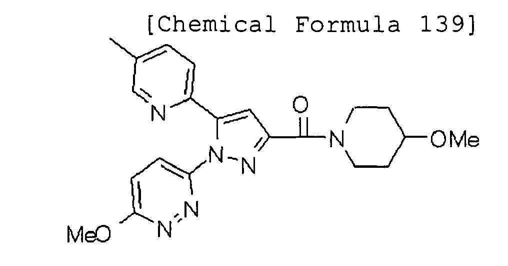

The title compound (236 mg, 36%) was obtained as a solid by the same method as that in Example 5, using 1-(6-methoxy-3-pyridazinyl)-5-(5-methyl-2-pyridyl)-1H-pyrazole-3-carboxylic acid (500 mg) of Reference Example 39 and 4-methoxypiperidine hydrochloride (372 mg) of Reference Example 23.
¹H-NMR(400MHz, CDCl₃)δ: 1.60(2H, br), 1.96(2H, br), 2.33(3H, s), 3.38(3H, s), 3.51(2H, br), 3.71(1H, br), 4.09(4H, br), 4.24(1H, br), 7.02(1H, s), 7.12(1H, d, J=9.16Hz), 7.45(1H, d, J=7.94Hz), 7.54(1H, m), 7.81(1H, d, J=9.16Hz), 8.25(1H, s).
FAB-MSm/z: 409(M+H)⁺.

[Example 52] 4-[1-(6-Methoxy-3-pyridazinyl)-5-(5-methyl-2-pyridyl)-1H-pyrazole-3-carbonyl]morpholine

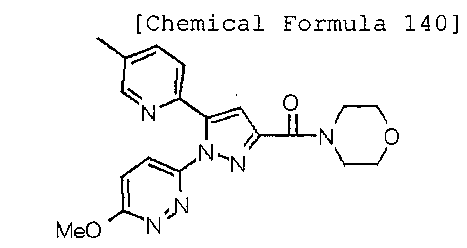

The title compound (279 mg, 45%) was obtained as a solid by the same method as that in Example 5, using 1-(6-methoxy-3-pyridazinyl)-5-(5-methyl-2-pyridyl)-1H-pyrazole-3-carboxylic acid (500 mg) of Reference Example 39 and morpholine (210 µL).
¹H-NMR(400MHz, CDCl₃)δ: 2.33(3H, s), 3.72(2H, br), 3.81-3.83(4H, br), 4.12(5H, br), 7.10(1H, s), 7.12(1H, d, J=9.16Hz), 7.47(1H, d, J=7.94Hz), 7.54(1H, dd, J=7.94, 1.83Hz), 7.75(1H, d, J=9.16Hz), 8.23(1H, d, J=1.83Hz).
FAB-MSm/z: 381(M+H)⁺.

[Example 53] 1-[1-(6-Methoxy-3-pyridazinyl)-5-(5-methyl-2-pyridyl)-1H-pyrazole-3-carbonyl]-4-methylpiperazine

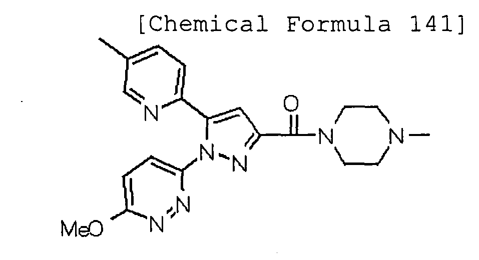

The title compound (220 mg, 34%) was obtained as a solid by the same method as that in Example 5, using 1-(6-methoxy-3-pyridazinyl)-5-(5-methyl-2-pyridyl)-1H-pyrazole-3-carboxylic acid (500 mg) of Reference Example 39 and N-methylpiperazine (270 µL).
¹H-NMR(400MHz, CDCl₃)δ: 2.33(6H, s), 2.44(2H, br), 2.51(2H, br), 3.85(2H, br), 4.07(2H, br), 4.12(3H, s), 7.06(1H, s), 7.12(1H, d, J=9.28Hz), 7.46(1H, d, J=7.94Hz), 7.54(1H, d, J=7.94Hz), 7.79(1H, d, J=9.28Hz), 8.24(1H, s).
FAB-MSm/z: 394(M+H)⁺.

[Example 54] 1-[1-(6-Methyl-3-pyridyl)-5-(4-methyl-2-pyridyl)-1H-pyrazole-3-carbonyl]-4-methoxypiperidine

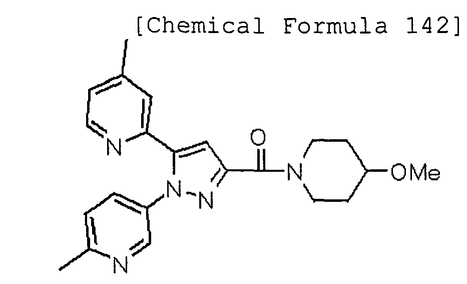

The title compound (219 mg, 54%) was obtained as an amorphous form by the same method as that in Example 5, using 1-(6-methyl-3-pyridyl)-5-(4-methyl-2-pyridyl)-1H-pyrazole-3-carboxylic acid (300 mg) of Reference Example 40 and 4-methoxypiperidine hydrochloride (227 mg) of Reference Example 23.
¹H-NMR(400MHz, CDCl₃)δ: 1.69(2H, brs), 1.94(2H, brs), 2.36(3H, s), 2.58(3H, s), 3.38(3H, s), 3.50(2H, m), 3.74(1H, m), 4.09(1H, brs), 4.26(1H, br s), 7.05(1H, s), 7.07(1H, s), 7.17(1H, d, J=8.18Hz), 7.30(1H, s), 7.62(1H, dd, J=8.18, 2.56Hz), 8.34(1H, d, J=5.01Hz), 8.39(1H, d, H=2.56Hz).
FAB-MSm/z: 392(M+H)⁺.

[Example 55] 4-[1-(6-Methoxy-3-pyridyl)-5-(5-methyl-2-pyrazinyl)-1H-pyrazole-3-carbonyl]morpholine

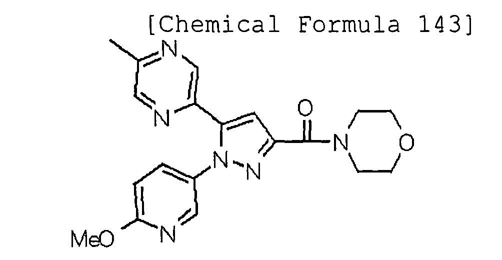

The title compound (0.174 g, 56%) was obtained as a solid by the same method as that in Example 1, using 1-(6-methoxy-3-pyridyl)-5-(5-methyl-2-pyrazinyl)-1H-pyrazole-3-carboxylic acid (0.250 g) of Reference Example 41 and morpholine (0.105 mL).
¹H-NMR(400MHz, CDCl₃)δ: 2.58(3H, s), 3.74-3.83(6H, m), 3.97(3H, s), 4.15-4.17(2H, m), 6.79(1H, d, J=8.8Hz), 7.24(1H, s), 7.59-7.62(1H, m), 8.12(1H, d, J=2.4Hz), 8.36(1H, d, J=1.5Hz), 8.59(1H, d, J=1.5Hz).
EI-MSm/z: 380(M⁺).
Elemental analysis: as C₁₉H₂₀N₆O₃·0.25H₂O
Theoretical value: C, 59.29; H, 5.37; N, 21.83.
Measured value: C, 59.33; H, 5.21; N, 21.73.

[Example 56] 1-[5-(5-Amino-2-pyrazinyl)-1-(6-methoxy-3-pyridyl)-1H-pyrazole-3-carbonyl]-4, 4-difluoropiperidine

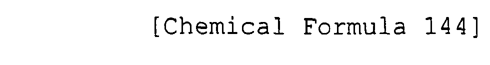

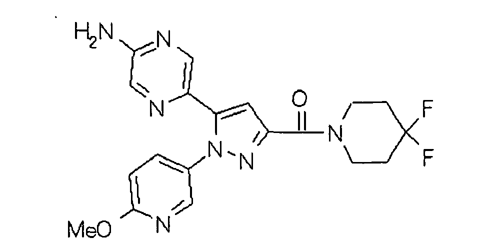

### 1) 1-[5-(5-tert-Butoxycarbonylamino-2-pyrazinyl)-1-(6-methoxy-3-pyridyl)-1H-pyrazole-3-carbonyl]-4, 4-difluoropiperidine

1-[5-(5-tert-Butoxycarbonylamino-2-pyrazinyl)-1-(6-methoxy-3-pyridyl)-1H-pyrazole-3-carbonyl]-4, 4-difluoropiperidine (0.306 g, 84%) was obtained as a solid by the same method as that in Example 1, using 5-(5-tert-butoxycarbonylamino-2-pyrazinyl)-1-(6-methoxy-3-pyridyl)-1H-pyrazole-3-carboxylic acid (0.290 g) of Reference Example 42 and 4, 4-difluoropiperidine hydrochloride (0.133 g) of Reference Example 18.
¹H-NMR(400MHz, CDCl₃)δ: 1.54(9H, s), 2.08(4H, m), 3.92(2H, m), 3.97(3H, s), 4.20(2H, m), 6.78(1H, dd, J=8.8, 0.7Hz), 7.17(1H, s), 7.40(1H, s), 7.60(1H, dd, J=8.8, 2.9Hz), 8.11-8.12(1H, m), 8.33(1H, d, J=1.5Hz), 9.17(1H, d, J=1.5Hz).
EI-MSm/z: 515(M⁺).

### 2) Title compound

Trifluoroacetic acid (3 mL) was added to a solution of 1-[5-(5-tert-butoxycarbonylamino-2-pyrazinyl)-1-(6-methoxy-3-pyridyl)-1H-pyrazole-3-carbonyl]-4, 4-difluoropiperidine (0.300 g) in dichloromethane (6 mL) at room temperature, and the resultant mixture was stirred for 105 minutes. A saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (methanol-chloroform), to obtain the title compound (0.189 g, 77%) as a solid.
¹H-NMR(400MHz, CDCl₃)δ: 2.08(4H, m), 3.92(2H, m), 3.96(3H, s), 4.19(2H, m), 4.78(2H, br s), 6.78(1H, d, J=8.8Hz), 7.06(1H, s), 7.59(1H, dd, J=8.8, 2.7Hz), 7.87(1H, d, J=1.5Hz), 8.10(1H, d, J=1.5Hz), 8.14(1H, d, J=2.7Hz).
EI-MSm/z: 415(M⁺).
Elemental analysis: as C₁₉H₁₉F₂N₇O₂·0.25H₂O
Theoretical value: C, 54.35;H, 4.68;N, 23.35;F, 9.05.
Measured value: C, 54.61;H, 4.50;N, 23.24;F, 9.31.

[Example 57] 4-[5-(5-Amino-2-pyrazinyl)-1-(6-methoxy-3-pyridyl)-1H-pyrazole-3-carbonyl]morpholine

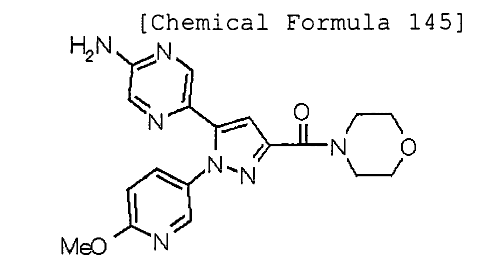

### 1) 4-[5-(5-tert-Butoxycarbonylamino-2-pyrazinyl)-1-(6-methoxy-3-pyridyl)-1H-pyrazole-3-carbonyl]morpholine

4-[5-(5-tert-Butoxycarbonylamino-2-pyrazinyl)-1-(6-methoxy-3-pyridyl)-1H-pyrazole-3-carbonyl]morpholine (0.282 g, 83%) was obtained as a solid by the same method as that in Example 1, using 5-(5-tert-butoxycarbonylamino-2-pyrazinyl)-1-(6-methoxy-3-pyridyl)-1H-pyrazole-3-carboxylic acid (0.290 g) of Reference Example 42 and morpholine (0.0919 mL).
¹H-NMR(400MHz, CDCl₃)δ: 1.54(9H, s), 3.74-3.83(6H, m), 3.97(3H, s), 4.16(2H, m), 6.78(1H, dd, J=8.8, 0.7Hz), 7.17(1H, s), 7.44(1H, s), 7.60(1H, dd, J=8.8, 2.7Hz), 8.11-8.12(1H, m), 8.33(1H, d, J=1.5Hz), 9.17(1H, d, J=1.5Hz).
EI-MSm/z: 481(M⁺).

### 2) Title compound

The title compound (0.186 g, 84%) was obtained as a solid by the same method as that in Example 56-(2), using 4-[5-(5-tert-butoxycarbonylamino-2-pyrazinyl)-1-(6-methoxy-3-pyridyl)-1H-pyrazole-3-carbonyl]morpholine (0.276 g).
¹H-NMR(400MHz, CDCl₃)δ: 3.73-3.82(6H, m), 3.96(3H, s), 4.14(2H, m), 4.79(2H, br s), 6.76-6.78(1H, m), 7.07(1H, s), 7.59(1H, dd, J=8.8, 2.7Hz), 7.87(1H, d, J=1.5Hz), 8.10(1H, d, J=1.5Hz), 8.13-8.14(1H, m).
EI-MSm/z: 381(M⁺).
Elemental analysis: as C₁₈H₁₉N₇O₃·0.25 H₂O
Theoretical value: C, 56.02;H, 5.09;N, 25.41.
Measured value: C, 56.14;H, 4.95;N, 25.12.

[Example 58] 1-[1-(6-Methoxy-3-pyridazinyl)-5-(1H-pyrrol-3-yl)-1H-pyrazole-3-carbonyl]-4, 4-difluoropiperidine

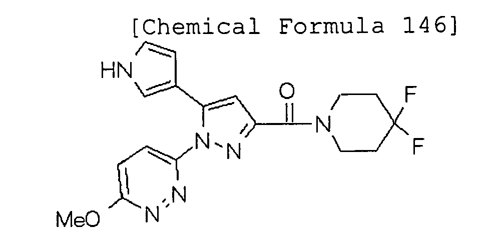

The title compound (117 mg, 43%) was obtained as a solid by the same method as that in Example 1, using 1-(6-methoxy-3-pyridazinyl)-5-(1H-pyrrol-3-yl)-1H-pyrazole-3-carboxylic acid (200 mg) of Reference Example 33 and 4, 4-difluoropiperidine hydrochloride (133 mg) of Reference Example 18.
¹H-NMR(400MHz, CDCl₃)δ: 2.07(4H, m), 3.91(2H, m), 4.15(2H, m), 4.19(3H, s), 6.20(1H, dt, J=2.69, 1.59Hz), 6.75(1H, dd, J=4.76, 2.69Hz), 6.85(1H, s), 7.04(1H, dt, J=2.81, 1.83Hz), 7.09(1H, d, J=9.28Hz), 7.55(1H, d, J=9.28Hz), 8.34(1H, br).
FAB-MSm/z: 389(M+H)⁺.

[Example 59] 1-[1-(6-Methoxy-3-pyridazinyl)-5-(1H-pyrrol-3-yl)-1H-pyrazole-3-carbonyl]-4-methoxypiperidine

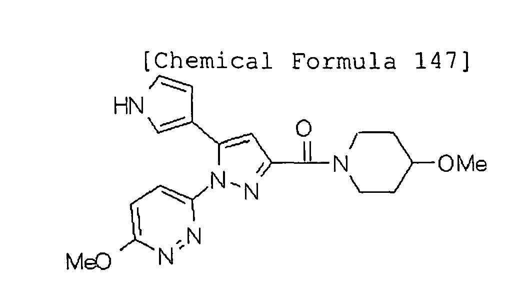

The title compound (117 mg, 66%) was obtained as an amorphous form by the same method as that in Example 1, using 1-(6-methoxy-3-pyridazinyl)-5-(1H-pyrrol-3-yl)-1H-pyrazole-3-carboxylic acid (200 mg) of Reference Example 33 and 4-methoxypiperidine hydrochloride (128 mg) of Reference Example 23.
¹H-NMR(400MHz, CDCl₃)δ: 1.66(2H, m), 1.93(2H, m), 3.37(3H, s), 3.50(2H, m), 3.67(1H, m), 4.10(1H, m), 4.18(3H, s), 4.24(1H, m), 6.19(1H, d, J=1.47Hz), 6.74(1H, dd, J=4.76, 2.69Hz), 6.78(1H, s), 7.04(1H, s), 7.08(1H, d, J=9.28Hz), 7.60(1H, d, J=9.28Hz), 8.52(1H, br).
FAB-MSm/z: 383(M+H)⁺.

[Example 60] 1-[1-(6-Methoxy-3-pyridyl)-5-(1-methyl-1H-pyrrol-3-yl)-1H-pyrazole-3-carbonyl]-4-methyl-3-oxopiperazine

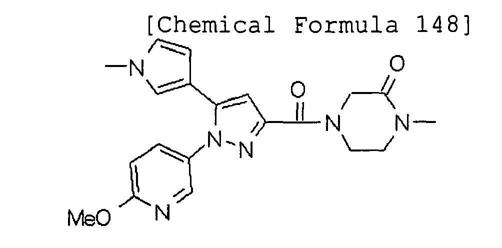

The title compound (159 mg, 45%) was obtained as an amorphous form by the same method as that in Example 1, using 1-(6-methoxy-3-pyridyl)-5-(1-methyl-1H-pyrrol-3-yl)-1H-pyrazole-3-carboxylic acid (250 mg) of Reference Example 32 and 1-methylpiperazin-2-one (115 mg) of Reference Example 46.
¹H-NMR(400MHz, CDCl₃)δ: 3.01(3H, s), 3.46(2H, t, J=5.49Hz), 3.60(3H, s), 3.99(3H, s), 4.02(1.5H, m), 4.42(1.5H, br), 4.81(1H, br), 5.91(1H, s), 6.46(1H, br), 6.51(1H, t, J=2.44Hz), 6.80(2H, m), 7.64(1H, m), 8.24(1H, s).
FAB-MSm/z: 395(M+H)⁺.

[Example 61] 4-[5-(2-Methyloxazol-4-yl)-1-(6-methyl-3-pyridyl)-1H-pyrazole-3-carbonyl]morpholine

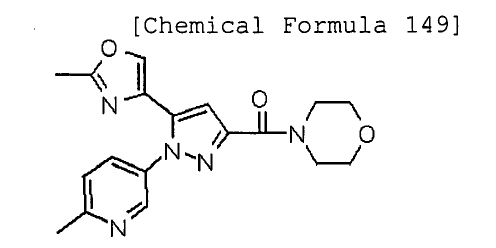

The title compound (7.4 mg, 0.2%) was obtained as an oily product by the same method as that in Example 1, using 5-(2-methyloxazol-4-yl)-1-(6-methyl-3-pyridyl)-1H-pyrazole-3-carboxylic acid (95.9 mg) of Reference Example 45 and morpholine (0.0673 mL).
¹H-NMR(400MHz, CDCl₃)δ: 2.44(3H, s), 2.64(3H, s), 3.65-3.85(6H, m), 4.08(2H, br), 7.08(1H, s), 7.23-7.32(2H, m), 7.70(1H, dd, J=8.3, 2.7Hz), 8.56(1H, d, J=2.7Hz).
FAB-MSm/z: 354(M+H)⁺.

[Example 62] (2S)-1-[5-(1-Methyl-1H-pyrrol-3-yl)-1-(6-methyl-3-pyridazinyl)-1H-pyrazole-3-carbonyl]-2-fluoromethylpyrrolidine

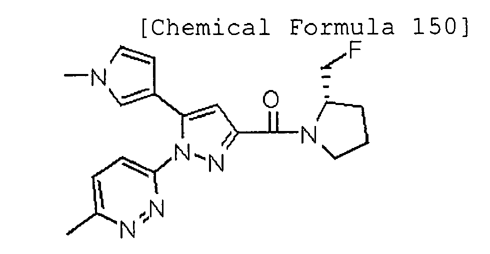

The title compound (0.203 g, 64%) was obtained as an amorphous form by the same method as that in Example 1, using 5-(1-methyl-1H-pyrrol-3-yl)-1-(6-methyl-3-pyridazinyl)-1H-pyrazole-3-carboxylic acid (0.244 g) of Reference Example 36 and (2S)-2-fluoromethylpyrrolidine hydrochloride (0.180 g) of Reference Example 27.
¹H-NMR(400MHz, CDCl₃)δ: 1.85-2.19(4H, m), 2.78(3H, s), 3.60-3.82(1H×2/3, m), 3.63(3H, s), 3.96-4.07(1H+1H×1/3, m), 4.32-4.81(2H+1H×2/3, m), 5.07-5.22(1H×1/3, m), 6.12-6.18(1H, m), 6.53-6.57(1H, m), 6.90-7.03(2H, m), 7.42-7.50(1H, m), 7.64-7.69(1H, m).
ESI-MSm/z: 369(M+H)⁺.

[Example 63] 1-[5-(1-Methyl-1H-pyrrol-3-yl)-1-(6-methyl-3-pyridazinyl)-1H-pyrazole-3-carbonyl]-3, 3-difluoroazetidine

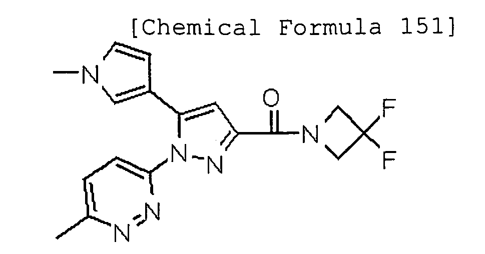

The title compound (0.243 g, 78%) was obtained as a solid by the same method as that in Example 1, using 5-(1-methyl-1H-pyrrol-3-yl)-1-(6-methyl-3-pyridazinyl)-1H-pyrazole-3-carboxylic acid (0.248 g) of Reference Example 36 and 3, 3-difluoroazetidine hydrochloride (0.173 g; C.W. Robert, et al., MERCK SHARP & DOHME LIMITED, WO00/47582).
¹H-NMR(400MHz, CDCl₃)δ: 2.80(3H, s), 3.62(3H, s), 4.51(2H, t, J=12.0Hz), 4.91(2H, t, J=12.0Hz), 6.05-6.12(1H, m), 6.50-6.54(1H, m), 6.90-6.96(1H, m), 6.97(1H, s), 7.47(1H, d, J=8.8Hz), 7.61(1H, d, J=8.8Hz).
FAB-MSm/z: 359(M+H)⁺.

[Example 64] 1-[1-(6-Methoxy-3-pyridazinyl)-5-(2-pyridyl)-1H-pyrazole-3-carbonyl]-3, 3-difluoroazetidine

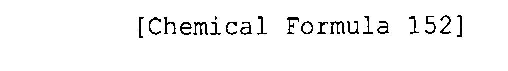

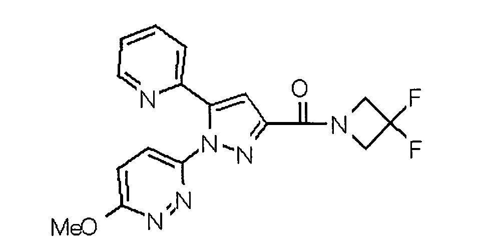

The title compound (0.232 g, 73%) was obtained as a solid by the same method as that in Example 1, using 1-(6-methoxy-3-pyridazinyl)-5-(2-pyridyl)-1H-pyrazole-3-carboxylic acid (0.254 g) of Reference Example 4 and 3, 3-difluoroazetidine hydrochloride (0.168 g; C.W. Robert, et al., MERCK SHARP & DOHME LIMITED, WO00/47582).
¹H-NMR(400MHz, CDCl₃)δ: 4.12(3H, s), 4.54(2H, t, J=11.9Hz), 4.94(2H, t, J=11.9Hz), 7.15(1H, d, J=9.0Hz), 7.20-7.35(2H, m), 7.61(1H, d like, J=7.1Hz), 7.73-7.82(2H, m), 8.38(1H, d like, J=4.9Hz).
FAB-MSm/z: 373(M+H)⁺.

[Example 65] 1-[1-(6-Methoxy-3-pyridyl)-5-(2-pyrazinyl)-1H-pyrazole-3-carbonyl]-4-methyl-3-oxopiperazine

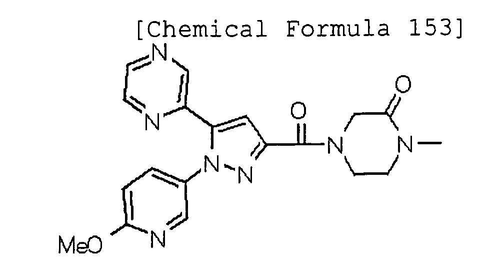

The title compound (0.252 g, 78%) was obtained as a solid by the same method as that in Example 1, using 1-(6-methoxy-3-pyridyl)-5-(2-pyrazinyl)-1H-pyrazole-3-carboxylic acid (0.231 g) of Reference Example 6 and 1-methyl piperazin-2-one (0.267 g) of Reference Example 46.
¹H-NMR(400MHz, CDCl₃)δ: 3.03(3H, s), 3.46-3.50(2H, m), 3.97(3H, s), 4.04-4.07(1H, m), 4.41-4.45(2H, m), 4.84(1H, br s), 6.80(1H, d, J=8.8Hz), 7.31-7.36(1H, m), 7.56-7.65(1H, m), 8.09-8.15(1H, m), 8.47-8.52(2H, m), 8.72-8.75(1H, m).
ESI-MSm/z: 394(M+H)⁺.

[Example 66] 1-[1-(6-Methoxy-3-pyridyl)-5-(1H-pyrazol-3-yl)-1H-pyrazole-3-carbonyl]-4-methoxypiperidine

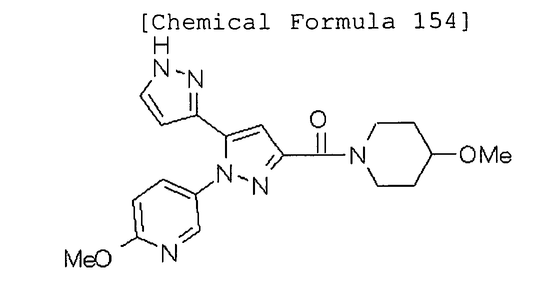

The title compound (0.222 g, 43%) was obtained as an amorphous form by the same method as that in Example 1, using 1-(6-methoxy-3-pyridyl)-5-(1H-pyrazol-3-yl)-1H-pyrazole-3-carboxylic acid (0.370 g) of Reference Example 13 and 4-methoxypiperidine hydrochloride (0.359 g) of Reference Example 23.
¹H-NMR(400MHz, CDCl₃)δ: 1.61-1.74(2H, m), 1.89-2.00(2H, m), 3.38(3H, s), 3.47-3.58(2H, m), 3.72-3.79(1H, m), 3.97(3H, s), 4.07-4.13(1H, m), 4.27-4.33(1H, m), 6.12(1H, d, J=2.2Hz), 6.79(1H, d, J=8.8Hz), 7.19(1H, s), 7.53(1H, d, J=2.4Hz), 7.64(1H, dd, J=8.8, 2.7Hz), 8.24(1H, d, J=2.4Hz).
ESI-MSm/z: 383(M+H)⁺.

[Example 67] 1-[1-(6-Methoxy-3-pyridazinyl)-5-(5-methyl-2-pyrazinyl)-1H-pyrazole-3-carbonyl]-4, 4-difluoropiperidine

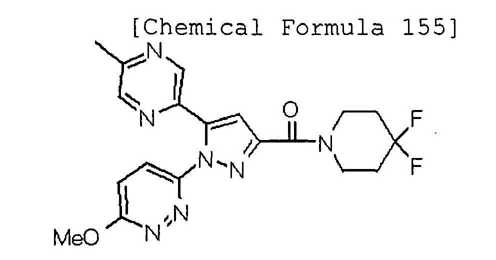

The title compound (0.314 g, 79%) was obtained as a solid by the same method as that in Example 1, using 1-(6-methoxy-3-pyridazinyl)-5-(5-methyl-2-pyrazinyl)-1H-pyrazole-3-carboxylic acid (0.30 g) of Reference Example 47 and 4, 4-difluoropiperidine hydrochloride (0.182 g) of Reference Example 18.
¹H-NMR(400MHz, CDCl₃)δ: 2.08-2.09(4H, m), 2.59(3H, s), 3.92-3.94(2H, m), 4.09-4.19(2H, m), 4.12(3H, s), 7.17-7.19(2H, m), 7.84(1H, d, J=9.3Hz), 8.29-8.30(1H, m), 8.70(1H, d, J=1.5Hz).
EI-MSm/z: 415(M⁺).

[Example 68] 4-[1-(6-Methyl-3-pyridyl)-5-(5-methyl-2-pyridyl)-1H-pyrazole-3-carbonyl]morpholine

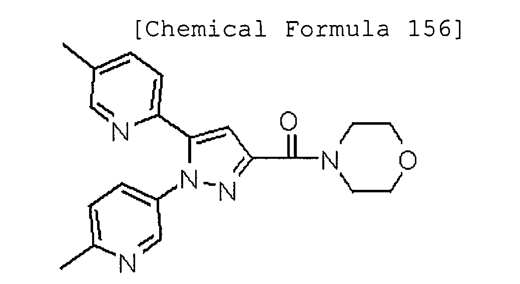

The title compound (0.228 g, 75%) was obtained as a solid by the same method as that in Example 1, using 1-(6-methyl-3-pyridyl)-5-(5-methyl-2-pyridyl)-1H-pyrazole-3-carboxylic acid (0.245 g) of Reference Example 48 and morpholine (0.109 mL).
¹H-NMR(400MHz, CDCl₃)δ: 2.34(3H, s), 2.59(3H, s), 3.73-3.83(6H, m), 4.14(2H, m), 7.11(1H, s), 7.19(1H, d, J=8.4Hz), 7.33(1H, d, J=8.0Hz), 7.51-7.53(1H, m), 7.61-7.64(1H, m), 8.33-8.38(2H, m).
ESI-MSm/z: 364(M+H)⁺.

[Example 69] 1-[1-(6-Methoxy-3-pyridyl)-5-(1-methyl-1H-1, 2, 4-triazol-3-yl)-1H-pyrazole-3-carbonyl]-4, 4-difluoropiperidine

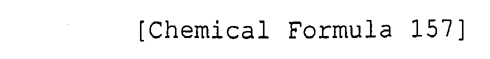

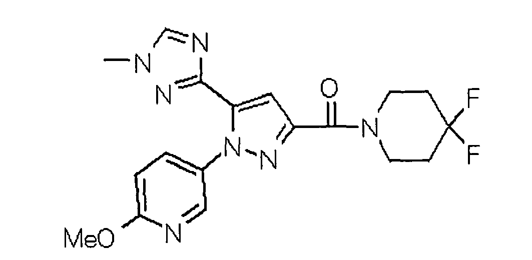

Lithium hydroxide monohydrate (26 mg) was added to a mixed solution of 1-(6-methoxy-3-pyridyl)-5-(1-methyl-1H-1, 2, 4-triazol-3-yl)-1H-pyrazole-3-carboxylic acid ethyl ester (68 mg) of Reference Example 49 in tetrahydrofuran (10 mL) and water (5 mL) at room temperature, and the resultant mixture was stirred overnight. The solvent of the reaction solution was evaporated under reduced pressure, and a lithium salt of 1-(6-methoxy-3-pyridyl)-5-(1-methyl-1H-1, 2, 4-triazol-3-yl)-1H-pyrazole-3-carboxylic acid was obtained. A mixed solution of this lithium salt, 4, 4-difluoropiperidine hydrochloride (65 mg), 1-hydroxybenzotriazole (28 mg) of Reference Example 18, triethylamine (144µl) and 3-(3-dimethylaminopropyl)-1-ethylcarbodiimide hydrochloride (79 mg) in dichloromethane (20 mL) and N, N-dimethylformamide (10 mL) was stirred overnight at room temperature. A residue obtained by evaporating the solvent of the reaction solution under reduced pressure was purified by silica gel thin layer chromatography (methanol-chloroform), to obtain the title compound (46 mg, 55%) as an amorphous form.
¹H-NMR(400MHz, CDCl₃)δ: 2.08 (4H, br s), 3.90 (5H, s), 3.98(3H, s), 4.16(2H, s), 6.81(1H, d, J=8.5Hz), 7.30(1H, s), 7.70(1H, dd, J=8.9, 2.6Hz), 7.99(1H, s), 8.23(1H, d, J=2.7Hz).
ESI-MSm/z: 404(M+H)⁺.

[Example 70] (2S)-1-[1-(6-Methoxy-3-pyridyl)-5-(2-pyrazinyl)-1H-pyrazole-3-carbonyl]-2-fluoromethylpyrrolidine

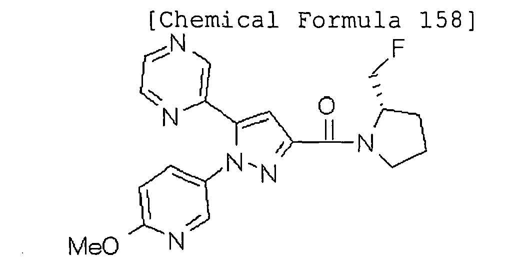

The title compound (140 mg, 37%) was obtained as a solid by the same method as that in Example 5, using 1-(6-methoxy-3-pyridyl)-5-(2-pyrazinyl)-1H-pyrazole-3-carboxylic acid (297 mg) of Reference Example 6 and (2S)-2-fluoromethylpyrrolidine hydrochloride (140 mg) of Reference Example 27.
¹H-NMR(400MHz, CDCl₃)δ: 1.59-2.08(4H, m), 3.70-5.19(5H, m), 3.97(3H, s), 6.77 and 6.80(combining 1H, s), 7.37 and 7.40(combining 1H, s), 7.57-7.62(1H, m), 8.11-8.14(1H, m), 8.46-8.52(2H, m), 8.75 and 8.77(combining 1H, s).
ESI-MSm/z: 383(M+H)⁺.

[Example 71] (2S)-1-[1-(6-Methoxy-3-pyridazinyl)-5-(2-pyrazinyl)-1H-pyrazole-3-carbonyl]-2-fluoromethylpyrrolidine

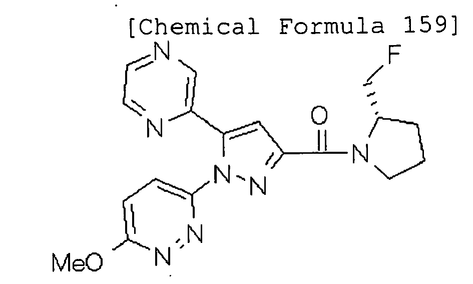

The title compound (166 mg, 43%) was obtained as a solid by the same method as that in Example 5, using 1-(6-methoxy-3-pyridazinyl)-5-(2-pyrazinyl)-1H-pyrazole-3-carboxylic acid (298 mg) of Reference Example 12 and (2S)-2-fluoromethylpyrrolidine hydrochloride (140 mg) of Reference Example 27.
¹H-NMR(400MHz, CDCl₃)δ: 1.95-2.17(4H, m), 3.72-5.20(5H, m), 4.11(3H, s), 7.17 and 7.19(combining 1H, d, J=9.2Hz), 7.32 and 7.37(combining 1H, s), 7.87 and 7.89(combining 1H, d, J=9.2Hz), 8.42-8.44(1H, m), 8.52-8.54(1H, m), 8.85 and 8.86(combining 1H, d, J=1.2Hz).
ESI-MSm/z: 384(M+H)⁺.

[Example 72] 1-[1-(6-Methoxy-3-pyridyl)-5-(1H-pyrrol-3-yl)-1H-pyrazole-3-carbonyl]-4-methoxypiperidine

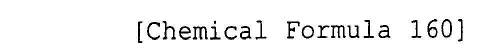

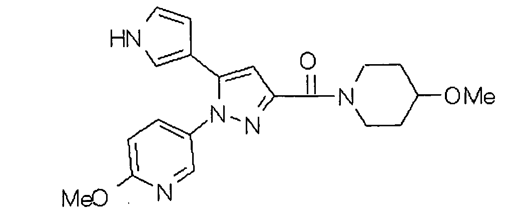

The title compound (130 mg, 48%) was obtained as an amorphous form by the same method as that in Example 1, using 1-(6-methoxy-3-pyridyl)-5-(1H-pyrrol-3-yl)-1H-pyrazole-3-carboxylic acid (200 mg) of Reference Example 32 and 4-methoxypiperidine hydrochloride (160 mg) of Reference Example 23.
¹H-NMR(400MHz, CDCl₃)δ: 1.67(2H, m), 1.94(2H, m), 3.38(3H, s), 3.49(2H, m), 3.72(1H, m), 3.98(3H, s), 4.10(1H, br), 4.29(1H, br), 6.04(1H, dt, J=2.69, 1.59Hz), 6.64(1H, dt, J=2.69, 1.83Hz), 6.73(1H, dd, J=4.26, 2.69Hz), 6.77(1H, s), 6.79(1H, d, J=8.79Hz), 7.61(1H, dd, J=8.79, 2.69Hz), 8.24(1H, d, J=2.08Hz), 8.39(1H, br).
FAB-MSm/z: 382(M+H)⁺.

[Example 73] 1-[1-(6-Methoxy-3-pyridyl)-5-(1-methyl-1H-imidazol-4-yl)-1H-pyrazole-3-carbonyl]-4-methoxypiperidine

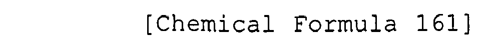

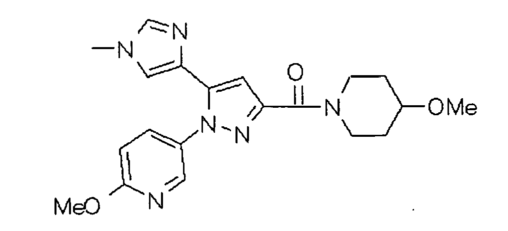

The title compound (133 mg, 67%) was obtained as a solid by the same method as that in Example 5, using 1-(6-methoxy-3-pyridyl)-5-(1-methyl-1H-imidazol-4-yl)-1H-pyrazole-3-carboxylic acid (150 mg) of Reference Example 34 and 4-methoxypiperidine hydrochloride (91 mg) of Reference Example 23.
¹H-NMR(400MHz, CDCl₃)δ: 1.65(2H, br s), 1.93(2H, br s), 3.36(3H, s), 3.46-3.50(2H, m), 3.65(4H, s), 3.97(3H, s), 4.09(1H, br s), 4.23(1H, br s), 6.68(1H, d, J=1.2Hz), 6.79(1H, d, J=8.8Hz), 6.93(1H, s), 7.40(1H, s), 7.70(1H, dd, J=8.8, 2.7Hz), 8.24(1H, d, J=2.7Hz).
ESI-MSm/z: 397(M+H)⁺.

[Example 74] 1-[1-(6-Methoxy-3-pyridazinyl)-5-(1H-pyrrol-3-yl)-1H-pyrazole-3-carbonyl]-4-methylpiperazine

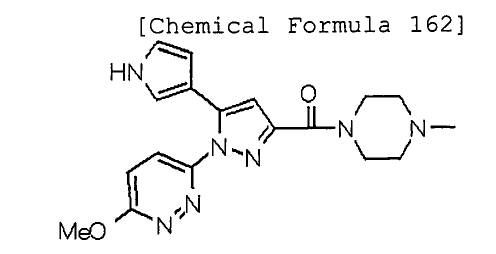

The title compound (86 mg, 30%) was obtained as an amorphous form by the same method as that in Example 1, using 1-(6-methoxy-3-pyridazinyl)-5-(1H-pyrrol-3-yl)-1H-pyrazole-3-carboxylic acid (200 mg) of Reference Example 33 and N-methyl piperazine (116 µL).
¹H-NMR(400MHz, CDCl₃)δ: 2.32(3H, s), 2.43(2H, br), 2.50(2H, br), 3.84(2H, br), 4.05(2H, br), 4.18(3H, s), 6.19(1H, s), 6.74(1H, dd, J=4.64, 2.69Hz), 6.81(1H, s), 7.03(1H, s), 7.08(1H, d, J=9.16Hz), 7.58(1H, d, J=9.16Hz), 8.45(1H, br).
FAB-MSm/z: 368(M+H)⁺.

[Example 75] 4-[5-(3-Carbamoyl-1H-pyrrol-1-yl)-1-(6-methoxy-3-pyridyl)-1H-pyrazole-3-carbonyl]morpholine

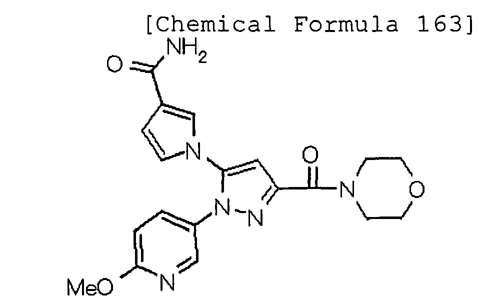

An aqueous 1 N sodium hydroxide solution (1.00 mL) was added to a mixed solution of 5-(3-carbamoyl-1H-pyrrol-1-yl)-1-(6-methoxy-3-pyridyl)-1H-pyrazole-3-carboxylic acid ethyl ester (0.123 g) of Reference Example 52 in ethanol (5 mL) and tetrahydrofuran (5 mL) at room temperature, and the resultant mixture was stirred for 5.5 hours. An aqueous 1 N hydrochloric acid solution (1.00 mL) was added to the reaction solution, and then reaction solvent was evaporated under reduced pressure, to obtain 5-(3-carbamoyl-1H-pyrrol-1-yl)-1-(6-methoxy-3-pyridyl)-1H-pyrazole-3-carboxylic acid. Using this carboxylic acid and morpholine (55.0 µL), the title compound (98.0 mg, 69%) was obtained as a solid by the same method as that in Example 1. ¹H-NMR(400MHz, CDCl₃)δ: 3.74-3.83(6H, m), 3.95(3H, s), 4.16-4.19(2H, m), 5.55(2H, br s), 6.53(1H, dd, J=2.9, 1.7Hz), 6.66(1H, dd, J=3.1, 2.3Hz), 6.74(1H, d, J=8.8Hz), 6.91(1H, s), 7.26-7.27(1H, m), 7.32(1H, dd, J=8.9, 2.8Hz), 8.02(1H, d, J=2.4Hz).
ESI-MSm/z: 397(M+H)⁺.

[Example 76] 1-[1-(6-Methoxy-3-pyridyl)-5-(1H-pyrrol-3-yl)-1H-pyrazole-3-carbonyl]-2-carbamoylpyrazolidine

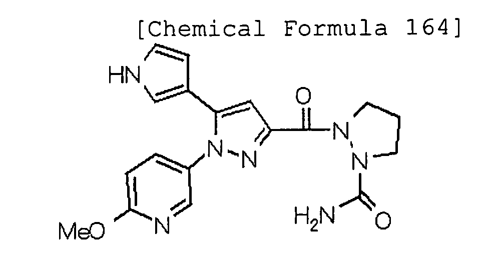

### 1) 1-[1-(6-Methoxy-3-pyridyl)-5-(1H-pyrrol-3-yl)-1H-pyrazole-3-carbonyl]-2-tert-butoxycarbonylpyrazolidine

1-[1-(6-Methoxy-3-pyridyl)-5-(1H-pyrrol-3-yl)-1H-pyrazole-3-carbonyl]-2-tert-butoxycarbonylpyrazolidine (566 mg, 82%) was obtained as a solid by the same method as that in Example 1, using 1-(6-methoxy-3-pyridyl)-5-(1H-pyrrol-3-yl)-1H-pyrazole-3-carboxylic acid (0.444 g) of Reference Example 30 and pyrazolidine-1-carboxylic acid tert-butyl ester (0.403 g; Y. Endo, et al., Bioorg. Med. Chem., 2002, 10, 953).
¹H-NMR(400MHz, CDCl₃)δ: 1.42(9H, s), 2.11(2H, br s), 3.25(1H, br s), 3.49(1H, br s), 3.97(3H, s), 4.10(1H, br s), 4.35(1H, br s), 6.02-6.04(1H, m), 6.60(1H, br s), 6.71-6.73(1H, m), 6.77(1H, d, J=8.5Hz), 6.88(1H, br s), 7.64(1H, dd, J=8.8, 2.7Hz), 8.23(1H, d, J=2.2Hz), 8.38(1H, br s).
ESI-MSm/z: 439(M+H)⁺.

### 2) 1-[1-(6-Methoxy-3-pyridyl)-5-(1H-pyrrol-3-yl)-1H-pyrazole-3-carbonyl]pyrazolidine

1-[1-(6-Methoxy-3-pyridyl)-5-(1H-pyrrol-3-yl)-1H-pyrazole-3-carbonyl]pyrazolidine (378 mg, 89%) was obtained as an amorphous form by the same method as that in Example 6-(2), using 1-[1-(6-methoxy-3-pyridyl)-5-(1H-pyrrol-3-yl)-1H-pyrazole-3-carbonyl]-2-tert-butoxycarbonylpyrazolidine (0.549 g).
¹H-NMR(400MHz, CDCl₃)δ: 2.14(1H, br s), 2.23(1H, br s), 3.16-3.20(2H, m), 3.80(1H, br s), 3.97(3H, s), 4.14(1H, br s), 4.92(1H, br s), 6.04-6.06(1H, m), 6.63(1H, br s), 6.71-6.73(1H, m), 6.77(1H, d, J=8.8Hz), 6.95(1H, s), 7.62-7.66(1H, m), 8.26(1H, d, J=2.2Hz), 8.33(1H, br s).
ESI-MSm/z: 339(M+H)⁺.

### 3) Title compound

Trimethylsilyl isocyanate (1.77 mL) was added to a solution of 1-[1-(6-methoxy-3-pyridyl)-5-(1H-pyrrol-3-yl)-1H-pyrazole-3-carbonyl]pyrazolidine (0.378 g) in 1, 4-dioxane (2.5 mL) at room temperature, and the resultant mixture was stirred in a sealed tube for 3 hours at an external temperature of 115°C. After air cooling, methanol was added to the reaction solution, and a residue obtained by evaporating the reaction solvent under reduced pressure was purified by silica gel column chromatography (methanol-dichloromethane), to obtain the title compound (280 mg, 65%) as a solid.
¹H-NMR(400MHz, DMSO-d₆)δ: 1.93-2.00(2H, m), 3.30(2H, br s), 3.92(3H, s), 5.86-5.88(2H, m), 6.65-6.66(2H, m), 6.73-6.75(1H, m), 6.81(1H, s), 6.94(1H, d, J=8.5Hz), 7.75(1H, dd, J=8.8, 2.7Hz), 8.22(1H, d, J=2.2Hz), 11.05(1H, br s).ESI-MSm/z: 382(M+H)⁺.

[Example 77] 1-[1-(6-Methoxy-3-pyridyl)-5-(1H-pyrazol-3-yl)-1H-pyrazole-3-carbonyl]-4-methyl-3-oxopiperazine

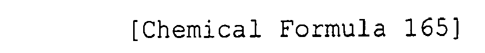

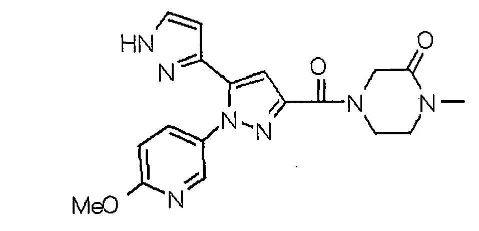

The title compound (217 mg, 38%) was obtained as an amorphous form by the same method as that in Example 1, using 1-(6-methoxy-3-pyridyl)-5-(1H-pyrazol-3-yl)-1H-pyrazole-3-carboxylic acid (0.393 g) of Reference Example 13 and 1-methyl piperazin-2-one (0.469 g) of Reference Example 46.
¹H-NMR (400MHz, CDCl₃)δ: 3.02(3H, s), 3.46-3.49 (2H, m), 3.98(3H, s), 4.03-4.07(1H, m), 4.44(2H, br s), 4.84(1H, br s), 6.15(1H, br s), 6.80(1H, d, J=9.0Hz), 7.23 (1/2H, br s), 7.31(1/2H, br s), 7.54(1H, br s), 7.60-7.67(1H, m), 8.20-8.26(1H, m).
ESI-MSm/z: 382(M+H)⁺.

[Example 78] 1-[5-(2-Methyl-1H-imidazol-4-yl)-1-(6-methoxy-3-pyridyl)-1H-pyrazole-3-carbonyl]-4-methoxypiperidine

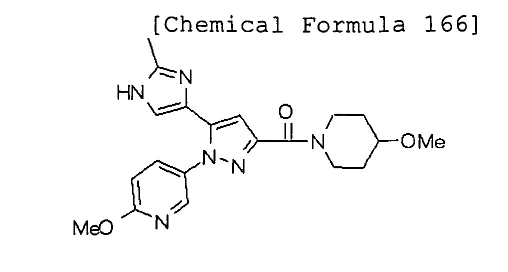

An aqueous 1 N sodium hydroxide solution (3.50 mL) was added to a solution of 5-(2-methyl-1H-imidazol-4-yl)-1-(6-methoxy-3-pyridyl)-1H-pyrazole-3-carboxylic acid ethyl ester (0.353 g) of Reference Example 51 in ethanol (10 mL) at room temperature, and the resultant mixture was stirred for 3 hours. An aqueous 1 N hydrochloric acid solution (3.50 mL) was added to the reaction solution, and the solvent of the reaction solution was evaporated under reduced pressure, to obtain 5-(2-methyl-1H-imidazol-4-yl)-1-(6-methoxy-3-pyridyl)-1H-pyrazole-3-carboxylic acid. Using this carboxylic acid product and 4-methoxypiperidine hydrochloride (0.293 g) of Reference Example 23, the title compound (0.193 g, 45%) was obtained as an amorphous form by the same method as that in Example 1.
¹H-NMR(400MHz, CDCl₃)δ: 1.60-1.73(2H, m), 1.86-1.98(2H, m), 2.35(3H, s), 3.37(3H, s), 3.46-3.57(2H, m), 3.65-3.72(1H, m), 3.96(3H, s), 4.02-4.08(1H, m), 4.15-4.22(1H, m), 6.49(1H, s), 6.78(1H, d, J=8.8Hz), 6.89(1H, s), 7.62(1H, dd, J=8.8, 2.7Hz), 8.21(1H, d, J=2.7Hz).
ESI-MSm/z: 397(M+H)⁺.

[Example 79] 1-[5-(5-Amino-2-pyrazinyl)-1-(6-methoxy-3-pyridyl)-1H-pyrazole-3-carbonyl]-4-fluoropiperidine

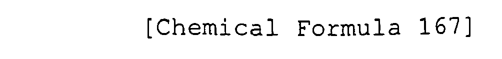

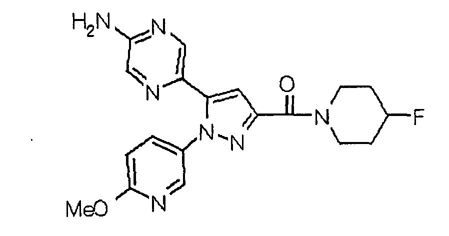

### 1) 1-[5-(5-tert-Butoxycarbonylamino-2-pyrazinyl)-1-(6-methoxy-3-pyridyl)-1H-pyrazole-3-carbonyl]-4-fluoropiperidine

1-[5-(5-tert-Butoxycarbonylamino-2-pyrazinyl)-1-(6-methoxy-3-pyridyl)-1H-pyrazole-3-carbonyl]-4-fluoropiperidine (0.230 g, 95%) was obtained as a solid by the same method as that in Example 1, using 5-(5-tert-butoxycarbonylamino-2-pyrazinyl)-1-(6-methoxy-3-pyridyl)-1H-pyrazole-3-carboxylic acid (0.200 g) of Reference Example 42 and 4-fluoropiperidine hydrochloride (0.135 g) of Reference Example 19.
¹H-NMR(400MHz, CDCl₃)δ: 1.54(9H, s), 1. 92-1. 99 (4H, m), 3.70-4.08(3H, m), 3.97(3H, s), 4.21-4.25(1H, m), 4.86-4.99(1H, m), 6.77(1H, d, J=8.8Hz), 7.13(1H, s), 7.45(1H, s), 7.60(1H, dd, J=8.8, 2.7Hz), 8.12(1H, d, J=2.7Hz), 8.33(1H, d, J=1.5Hz), 9.17(1H, d, J=1.5Hz).
EI-MSm/z: 497(M⁺).

### 2) Title compound

The title compound (0.117 g, 65%) was obtained as a solid by the same method as that in Example 56-(2), using 1-[5-(5-tert-butoxycarbonylamino-2-pyrazinyl)-1-(6-methoxy-3-pyridyl)-1H-pyrazole-3-carbonyl]-4-fluoropiperidine (0.225 g).
¹H-NMR(400MHz, CDCl₃)δ: 1.92-1.99(4H, m), 3.70(1H, m), 3.92-4.07(2H, m), 3.96(3H, s), 4.20-4.23(1H, m), 4.81(2H, br s), 4.85-4.99(1H, m), 6.77(1H, d, J=8.8Hz), 7.03(1H, s), 7.60(1H, dd, J=8.8, 2.7Hz), 7.88(1H, s), 8.10(1H, s), 8.14(1H, d, J=2.7Hz).
EI-MSm/z: 397(M⁺).

[Example 80] 1-[5-(5-Amino-2-pyrazinyl)-1-(6-methoxy-3-pyridyl)-1H-pyrazole-3-carbonyl]-4-methoxypiperidine

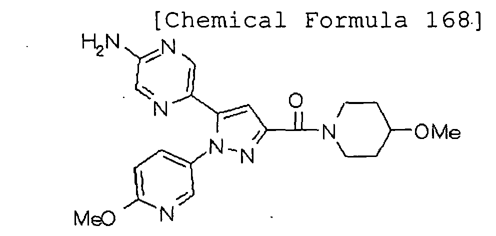

### 1) 1-[5-(5-tert-Butoxycarbonylamino-2-pyrazinyl)-1-(6-methoxy-3-pyridyl)-1H-pyrazole-3-carbonyl]-4-methoxypiperidine

1-[5-(5-tert-Butoxycarbonylamino-2-pyrazinyl)-1-(6-methoxy-3-pyridyl)-1H-pyrazole-3-carbonyl]-4-methoxypiperidine (0.211 g, 85%) was obtained as a solid by the same method as that in Example 1, using 5-(5-tert-butoxycarbonylamino-2-pyrazinyl)-1-(6-methoxy-3-pyridyl)-1H-pyrazole-3-carboxylic acid (0.200 g) of Reference Example 42 and 4-methoxypiperidine hydrochloride (0.147 g) of Reference Example 23.
¹H-NMR(400MHz, CDCl₃)δ: 1.54(9H, s), 1.69-1.86(4H, m), 3.38(3H, s), 3.47-3.57(2H, m), 3.72-3.78(1H, m), 3.96(3H, s), 4.09(1H, m), 4.26(1H, m), 6.77(1H, d, J=8.8Hz), 7.11(1H, s), 7.38(1H, s), 7.61(1H, dd, J=8.8, 2.7Hz), 8.11-8.12(1H, m), 8.32(1H, d, J=1.5Hz), 9.16(1H, d, J=1.5Hz).
EI-MSm/z: 509(M⁺).

### 2) Title compound

The title compound (0.125 g, 75%) was obtained as a solid by the same method as that in Example 56-(2), using 1-[5-(5-tert-butoxycarbonylamino-2-pyrazinyl)-1-(6-methoxy-3-pyridyl)-1H-pyrazole-3-carbonyl]-4-methoxypiperidine (0.206 g).
¹H-NMR(400MHz, CDCl₃)δ: 1.67-1.94(4H, m), 3.38(3H, s), 3.45-3.55(2H, m), 3.71-3.75(1H, m), 3.95(3H, s), 4.09(1H, m), 4.28(1H, m), 4.81(2H, br s), 6.77(1H, d, J=8.8Hz), 7.00(1H, s), 7.61(1H, dd, J=8.8, 2.7Hz), 7.87-7.88(1H, m), 8.09(1H, d, J=1.5Hz), 8.14(1H, d, J=2.7Hz).
EI-MSm/z: 409(M⁺).

[Example 81] 1-[5-(5-Amino-2-pyrazinyl)-1-(6-methoxy-3-pyridyl)-1H-pyrazole-3-carbonyl]-4-methylpiperazine

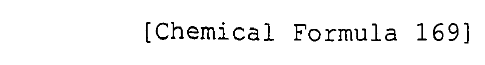

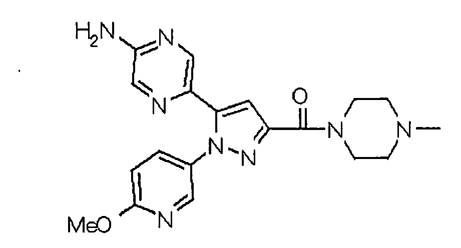

### 1) 1-[5-(5-tert-Butoxycarbonylamino-2-pyrazinyl)-1-(6-methoxy-3-pyridyl)-1H-pyrazole-3-carbonyl]-4-methylpiperazine

1-[5-(5-tert-Butoxycarbonylamino-2-pyrazinyl)-1-(6-methoxy-3-pyridyl)-1H-pyrazole-3-carbonyl]-4-methylpiperazine (0.146 g, 61%) was obtained as a solid by the same method as that in Example 1, using 5-(5-tert-butoxycarbonylamino-2-pyrazinyl)-1-(6-methoxy-3-pyridyl)-1H-pyrazole-3-carboxylic acid (0.200 g) of Reference Example 42 and N-methylpiperazine (0.108 mL).
¹H-NMR(400MHz, CDCl₃)δ: 1.54(9H, s), 2.34(3H, s), 2.48-2.53(4H, m), 3.86(2H, m), 3.96(3H, s), 4.12(2H, m), 6.77(1H, d, J=8.8Hz), 7.14(1H, s), 7.39(1H, s), 7.61(1H, dd, J=8.8, 2.7Hz), 8.11(1H, d, J=2.7Hz), 8.32(1H, d, J=1.5Hz), 9.16(1H, d, J=1.5Hz).
EI-MSm/z: 494(M⁺).

### 2) Title compound

The title compound (0.0799 g, 71%) was obtained as a solid by the same method as that in Example 56-(2), using 1-[5-(5-tert-butoxycarbonylamino-2-pyrazinyl)-1-(6-methoxy-3-pyridyl)-1H-pyrazole-3-carbonyl]-4-methylpiperazine (0.141 g).
¹H-NMR(400MHz, CDCl₃)δ: 2.33(3H, s), 2.46-2.51(4H, m), 3.85(2H, m), 3.95(3H, s), 4.09(2H, m), 4.76(2H, s), 6.75-6.77(1H, m), 7.02(1H, s), 7.60(1H, dd, J=8.8, 2.7Hz), 7.86(1H, d, J=1.5Hz), 8.09(1H, d, J=1.5Hz), 8.12-8.13(1H, m).
EI-MSm/z: 394(M⁺).

[Example 82] 1-[5-(5-Amino-2-pyrazinyl)-1-(6-methoxy-3-pyridazinyl)-1H-pyrazole-3-carbonyl]-4, 4-difluoropiperidine

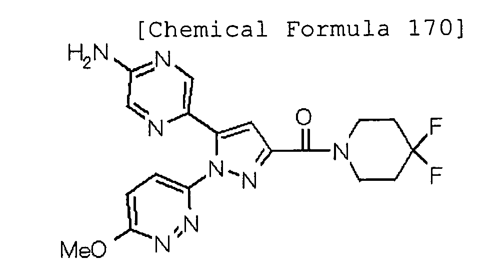

### 1) 1-[5-(5-tert-Butoxycarbonylamino-2-pyrazinyl)-1-(6-methoxy-3-pyridazinyl)-1H-pyrazole-3-carbonyl]-4, 4-difluoropiperidine

1-[5-(5-tert-Butoxycarbonylamino-2-pyrazinyl)-1-(6-methoxy-3-pyridyl)-1H-pyrazole-3-carbonyl]-4, 4-difluoropiperidine (0.400 g, 92%) was obtained as a solid by the same method as that in Example 1, using 5-(5-tert-butoxycarbonylamino-2-pyrazinyl)-1-(6-methoxy-3-pyridazinyl)-1H-pyrazole-3-carboxylic acid (0.350 g) of Reference Example 53 and 4, 4-difluoropiperidine hydrochloride (0.200 g) of Reference Example 18.
¹H-NMR(400MHz, CDCl₃)δ: 1.54(9H, s), 2.09(4H, m), 3.93(2H, m), 4.13(3H, s), 4.17(2H, m), 7.14(1H, s), 7.17(1H, d, J=9.3Hz), 7.67(1H, s), 7.80(1H, d, J=9.3Hz), 8.49(1H, d, J=1.5Hz), 9.11(1H, m).
EI-MSm/z: 516(M⁺).

### 2) Title compound

The title compound (0.268 g, 85%) was obtained as a solid by the same method as that in Example 56-(2), using 1-[5-(5-tert-butoxycarbonylamino-2-pyrazinyl)-1-(6-methoxy-3-pyridyl)-1H-pyrazole-3-carbonyl]-4, 4-difluoropiperidine (0.390 g).
¹H-NMR(400MHz, CDCl₃)δ: 2.01-2.10(4H, m), 3.78(2H, m), 4.06(2H, m), 4.06(3H, s), 6.74(2H, s), 7.09(1H, s), 7.44(1H, d, J=9.2Hz), 7.65(1H, m), 7.94(1H, d, J=9.2Hz), 8.24(1H, m).
ESI-MSm/z: 417(M+H⁺).

[Example 83] 1-[1-(6-Methyl-3-pyridyl)-5-(5-methyl-2-pyridyl)-1H-pyrazole-3-carbonyl]-4-methyl-3-oxopiperazine

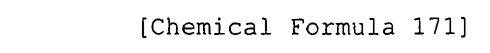

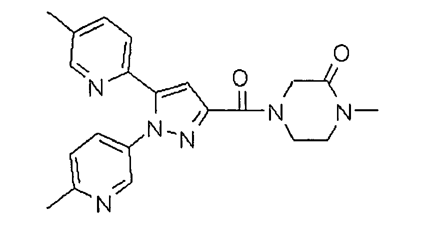

The title compound (0.156 g, 48%) was obtained as a solid by the same method as that in Example 1, using 1-(6-methyl-3-pyridyl)-5-(5-methyl-2-pyridyl)-1H-pyrazole-3-carboxylic acid (0.245 g) of Reference Example 48 and 1-methylpiperazin-2-one (0.285 g) of Reference Example 46.
¹H-NMR(400MHz, CDCl₃)δ: 2.34(3H, s), 2.60(3H, s), 3.02(3H, s), 3.48(2H, m), 4.05(1H, m), 4.42-4.44(2H, m), 4.84(1H, m), 7.15-7.36(3H, m), 7.51-7.72(2H, m), 8.33-8.43(2H, m).
FAB-MSm/z: 391(M+H⁺).

[Example 84] (2S)-1-[1-(6-Methyl-3-pyridyl)-5-(5-methyl-2-pyridyl)-1H-pyrazole-3-carbonyl]-2-carbamoylpyrrolidine

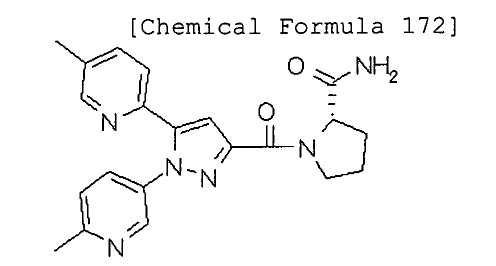

The title compound (0.182 g, 56%) was obtained as a solid by the same method as that in Example 1, using 1-(6-methyl-3-pyridyl)-5-(5-methyl-2-pyridyl)-1H-pyrazole-3-carboxylic acid (0.245 g) of Reference Example 48 and L-proline amide (0.190 g).
¹H-NMR(400MHz, CDCl₃)δ: 1.97-2.46(4H, m), 2.34(3H, s), 2.57-2.60(3H, m), 3.84-4.20(2H, m), 4.86-5.30(1H, m), 5.46-5.53(1H, m), 6.22-7.04(1H, m), 7.19-7.24(2H, m), 7.35-7.37(1H, m), 7.52-7.55(1H, m), 7.63-7.72(1H, m), 8.25-8.40(2H, m).
ESI-MSm/z: 391(M+H⁺).

[Example 85] 4-[1-(6-Methoxy-3-pyridazinyl)-5-(5-methyl-2-pyrazinyl)-1H-pyrazole-3-carbonyl]morpholine

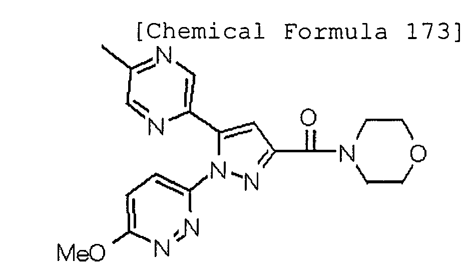

The title compound (0.157 g, 51%) was obtained as a solid by the same method as that in Example 1, using 1-(6-methoxy-3-pyridazinyl)-5-(5-methyl-2-pyrazinyl)-1H-pyrazole-3-carboxylic acid (0.250 g) of Reference Example 47 and morpholine (0.105 mL).
¹H-NMR(400MHz, CDCl₃)δ: 2.58(3H, s), 3.72-3.84(6H, m), 4.09-4.10(2H, m), 4.12(3H, s), 7.16-7.19(2H, m), 7.84(1H,
d, J=9.2Hz), 8.29(1H, m), 8.70-8.71(1H, m).
ESI-MSm/z: 382(M+H⁺) .

[Example 86] 4-[5-(5-Methoxyoxazol-2-yl)-1-(6-methyl-3-pyridyl)-1H-pyrazole-3-carbonyl]morpholine

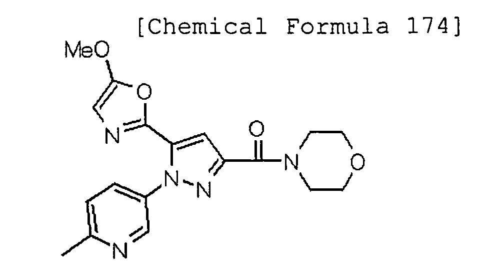

The title compound (9.2 mg, 50%) was obtained as a solid by the same method as that in Example 1, using 5-(5-methoxyoxazol-2-yl)-1-(6-methyl-3-pyridyl)-1H-pyrazole-3-carboxylic acid (14.9 mg) of Reference Example 50 and morpholine (0.0150 mL).
¹H-NMR(400MHz, CDCl₃)δ: 2.65(3H, s), 3.66-3.85(6H, m), 3.90(3H, s), 4.10(2H, br), 6.12(1H, s), 7.25-7.32(2H, m), 7.75(1H, dd, J=8.3, 2.7Hz), 8.63(1H, d, J=2.7Hz).
ESI-MSm/z: 370(M+H)⁺.

[Example 87] (2R)-1-[1-(6-Methoxy-3-pyridazinyl)-5-(2-pyridyl)-1H-pyrazole-3-carbonyl]-2-fluoromethylpyrrolidine

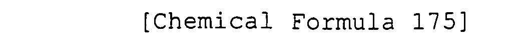

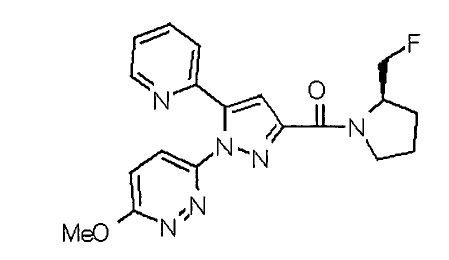

The title compound (240 mg, 63%) was obtained as a solid by the same method as that in Example 5, using 1-(6-methoxy-3-pyridazinyl)-5-(2-pyridyl)-1H-pyrazole-3-carboxylic acid (297 mg) of Reference Example 4 and (2R)-2-fluoromethylpyrrolidine hydrochloride (140 mg) of Reference Example 54.
¹H-NMR(400MHz, CDCl₃)δ: 1.92-2.08(4H, m), 3.71-4.82(5H, m), 4.12(3H, s), 7.14(1H, dd, J=9.3, 5.9Hz), 7.20-7.25(2H, m), 7.59-7.63(1H, m), 7.73-7.82(2H, m), 8.40-8.42(1H, m).
ESI-MSm/z: 383(M+H)⁺.

[Example 88] (2S)-1-[1-(6-Methyl-3-pyridyl)-5-(1-methyl-1H-pyrrol-3-yl)-1H-pyrazole-3-carbonyl]-2-carbamoylpyrrolidine

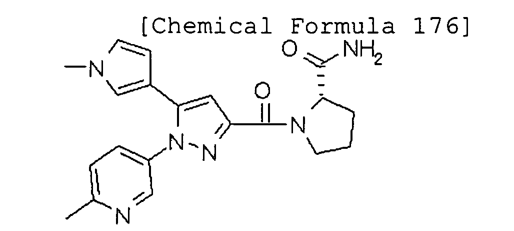

The title compound (127 mg, 34%) was obtained as an amorphous form by the same method as that in Example 1, using 1-(6-methyl-3-pyridyl)-5-(5-methyl-1H-pyrrol-3-yl)-1H-pyrazole-3-carboxylic acid (250 mg) of Reference Example 14 and L-proline amide (121 mg).
¹H-NMR(400MHz, CDCl₃)δ: 1.99-2.10(3H, br), 2.60(3H, s), 3.61(3H, s), 4.10 (1H, m), 4.86(1H, d, J=5.24Hz), 5.38(1H, br), 5.89(1H, t, J=2.08Hz), 6.52(2H, m), 6.91(1H, s), 7.21(1H, d, J=8.30Hz), 7.66(1H, d, J=5.62Hz), 8.59(1H, s).
FAB-MSm/z: 379(M+H)⁺.

[Example 89] 1-[5-[1-(2-Amino)ethyl-1H-pyrrol-3-yl]-1-(6-methylpyridin-3-yl)-1H-pyrazole-3-carbonyl]-4, 4-difluoropiperidine

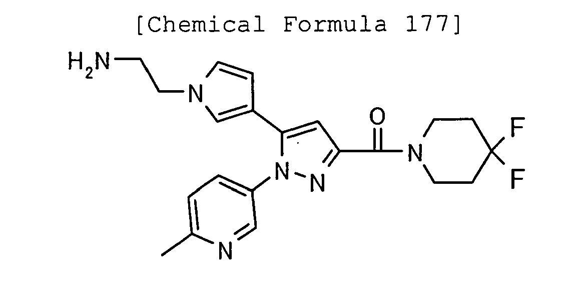

### 1) 4, 4-Difluoro-1-[1-(6-methylpyridin-3-yl)-5-(1H-pyrrol-3-yl)-1H-pyrazole-3-carbonyl]piperidine

4, 4-Difluoro-1-[1-(6-methylpyridin-3-yl)-5-(1H-pyrrol-3-yl)-1H-pyrazole-3-carbonyl]piperidine (0.465 g, quantitative) was obtained as an amorphous material by the same method as that in Example 1, using 1-(6-methylpyridin-3-yl)-5-(1H-pyrrol-3-yl)-1H-pyrazole-3-carboxylic acid (0.335 g) of Reference Example 58-(3) and 4, 4-difluoropiperidine hydrochloride (0.316 g) of Reference Example 18.
¹H-NMR(400MHz, CDCl₃)δ: 2.02-2.15(4H, m), 2.63(3H, s), 3.90(2H, br), 4.19(2H, br), 6.01-6.05(1H, m), 6.66-6.70(1H, m), 6.72-6.77(1H, m), 6.83(1H, s), 7.22(1H, d, J=8.3Hz), 7.65(1H, dd, J=2.5, 8.3Hz), 8.42(1H, br), 8.57(1H, d, J=2.5Hz).
EI-MSm/z: 371(M⁺).

### 2) 1-[5-(1-Allyl-1H-pyrrol-3-yl)-1-(6-methylpyridin-3-yl)-1H-pyrazole-3-carbonyl]-4, 4-difluoropiperidine

Sodium hydride (41.0 mg) was added to a solution of 4, 4-difluoro-1-[1-(6-methylpyridin-3-yl)-5-(1H-pyrrol-3-yl)-1H-pyrazole-3-carbonyl]piperidine (0.465 g) of the above in N, N-dimethylformamide (10 mL) at 0°C, and the resultant mixture was stirred for 10 minutes. Allyl iodide (0.171 mL) was added to the reaction solution, and the mixture was stirred for 1 hour at room temperature. Water and ethyl acetate were added to the reaction solution, and the mixture was partitioned. Further, the aqueous layer was extracted with ethyl acetate, and the organic layers were combined, washed with saturated saline, and then dried over anhydrous sodium sulfate. After separation by filtration, a residue obtained by evaporating the solvent under reduced pressure was purified by silica gel column chromatography (chloroform-methanol), to obtain 1-[5-(1-allyl-1H-pyrrol-3-yl)-1-(6-methylpyridin-3-yl)-1H-pyrazole-3-carbonyl]-4, 4-difluoropiperidine (0.420 g, 82%) as an oily product.
¹H-NMR(400MHz, CDCl₃)δ: 2.00-2.16(4H, m), 2.63(3H, s), 3.90(2H, br), 4.19(2H, br), 4.39-4.45(2H, m), 5.02-5.14(1H, m), 5.18-5.25(1H, m), 5.86-5.97(2H, m), 6.51-6.58(2H, m), 6.79(1H, s), 7.21(1H, d, J=8.3Hz), 7.65(1H, dd, J=2.4, 8.3Hz), 8.57(1H, d, J=2.4Hz).
FAB-MSm/z: 412(M+H)⁺.

### 3) 4, 4-Difluoro-1-[5-[1-(2, 3-dihydroxy)propyl-1H-pyrrol-3-yl]-1-(6-methylpyridin-3-yl)-1H-pyrazole-3-carbonyl]piperidine

N-Methylmorpholine N-oxide (0.477 g) and osmium tetraoxide (37.5 mg) were added to a mixed solution of 1-[5-(1-allyl-1H-pyrrol-3-yl)-1-(6-methylpyridin-3-yl)-1H-pyrazole-3-carbonyl]-4, 4-difluoropiperidine (0.420 g) of the above in tetrahydrofuran (12 mL), methanol (3 mL), and water (4 mL) at room temperature, and the resultant mixture was stirred for 1.5 hours. A saturated aqueous sodium thiosulfate solution was added to the reaction solution, and the mixture was stirred for 20 minutes. The reaction solution was extracted with ethyl acetate, and the aqueous layer was extracted with ethyl acetate. The organic layers were combined, washed with saturated saline, and then dried over anhydrous sodium sulfate. After separation by filtration, a residue obtained by evaporating the solvent under reduced pressure was purified by silica gel column chromatography (chloroform-methanol), to obtain 4, 4-difluoro-1-[5-[1-(2, 3-dihydroxy)propyl-1H-pyrrol-3-yl]-1-(6-methylpyridin-3-yl)-1H-pyrazole-3-carbonyl]piperidine (0.455 g, quantitative) as an oily product.
¹H-NMR(400MHz, CDCl₃)δ: 2.00-2.14 (4H, br), 2.63(3H, s), 3.43(1H, dd, J=5.1, 11.0Hz), 3.61(1H, dd, J=3.7, 11.0Hz), 3.83-3.99(5H, m), 4.17(2H, br), 5.95-6.00(1H, m), 6.51-6.57(1H, m), 6.60-6.65(1H, m), 6.79(1H, s), 7.28(1H, d, J=8.3Hz), 7.78(1H, dd, J=2.5, 8.3 Hz), 8.46(1H, d, J=2.5Hz).
FAB-MSm/z: 446(M+H)⁺.

### 4) 4, 4-Difluoro-1-[5-(1-formylmethyl-1H-pyrrol-3-yl)-1-(6-methylpyridin-3-yl)-1H-pyrazole-3-carbonyl]piperidine

Sodium meta-periodate (0.352 g) was added to a mixed solution of 4, 4-difluoro-1-[5-[1-(2, 3-dihydroxy)propyl-1H-pyrrol-3-yl]-1-(6-methylpyridin-3-yl)-1H-pyrazole-3-carbonyl]piperidine (0.455 g) of the above in tetrahydrofuran (5 mL), methanol (5 mL), and water (5 mL) at room temperature, and the resultant mixture was stirred for 1.5 hours. Cold water and ethyl acetate were added to the reaction solution, and the mixture was partitioned. Further, the aqueous layer was extracted with ethyl acetate, and the organic layers were combined, washed with saturated saline, and then dried over anhydrous sodium sulfate. After separation by filtration, the solvent was evaporated under reduced pressure, and 4, 4-difluoro-1-[5-(1-formylmethyl-1H-pyrrol-3-yl)-1-(6-methylpyridin-3-yl)-1H-pyrazole-3-carbonyl]piperidine (0.352 g, 83% from two processes) as an amorphous form.

### 5) 1-[5-[1-(2-Benzylamino)ethyl-1H-pyrrol-3-yl]-1-(6-methylpyridin-3-yl)-1H-pyrazole-3-carbonyl]-4, 4-difluoropiperidine

Benzylamine (0.465 mL), acetic acid (0.243 mL), and sodium cyanoborohydride (0.165 g) were added to a solution of 4, 4-difluoro-1-[5-(1-formylmethyl-1H-pyrrol-3-yl)-1-(6-methylpyridin-3-yl)-1H-pyrazole-3-carbonyl]piperidine (0.352 g) of the above in ethanol (10 mL) at 0°C, and the resultant mixture was stirred for 1.5 hours at room temperature. A saturated aqueous solution of sodium hydrogen carbonate and ethyl acetate were added to the reaction solution, and the mixture was partitioned. Further, the aqueous layer was extracted with ethyl acetate, and the organic layers were combined, washed with saturated saline, and then dried over anhydrous sodium sulfate. After separation by filtration, a residue obtained by evaporating the solvent under reduced pressure was purified by silica gel column chromatography (chloroform-methanol), to obtain 1-[5-[1-(2-benzylamino)ethyl-1H-pyrrol-3-yl]-1-(6-methylpyridin-3-yl)-1H-pyrazole-3-carbonyl]-4, 4-difluoropiperidine (0.412 g, 96%) as an oily product.
¹H-NMR(400MHz, CDCl₃)δ: 2.00-2.15(4H, m), 2.60(3H, s), 2.90(2H, t, J=6.1Hz), 3.74(2H, s), 3.86-3.96(2H, br), 3.92(2H, t, J=6.1Hz), 4.19(2H, br), 5.90-5.96(1H, m), 6.51-6.57(1H, m), 6.58-6.63(1H, m), 6.79(1H, s), 7.15(1H, d, J=8.3Hz), 7.20-7.40(5H, m), 7.61(1H, dd, J=2.4, 8.3Hz), 8.56(1H, d, J=2.4Hz).
FAB-MSm/z: 505(M+H)⁺.

### 6) Title compound

A 1M hydrochloric acid-ethanol solution (0.831 mL) and 20% palladium hydroxide-carbon (0.310 g) were added to a solution of 1-[5-[1-(2-benzylaminoethyl)-1H-pyrrol-3-yl]-1-(6-methylpyridin-3-yl)-1H-pyrazole-3-carbonyl]-4, 4-difluoropiperidine (0.419 g) of the above in methanol (10 mL) at room temperature, and the resultant mixture was stirred for 2 days under a hydrogen atmosphere. After separating the catalyst by filtration, the solids were washed with methanol. To a solution combining the filtrate and the washing solution, a saturated aqueous solution of sodium hydrogen carbonate and a mixture of chloroform-methanol (10: 1) were added, and the mixture was partitioned. Further, the aqueous layer was extracted with a mixture of chloroform-methanol (10: 1), and the organic layers were combined and dried over anhydrous sodium sulfate. After separation by filtration, a residue obtained by evaporating the solvent under reduced pressure was purified by silica gel thin layer chromatography (chloroform-methanol (containing ammonia)), to obtain the title compound (0.154 g, 45%) as an oily product.
¹H-NMR(400MHz, CDCl₃)δ: 1.90-2.15(4H, m), 2.62(3H, s), 3.02(2H, t, J=5.8Hz), 3.86-3.96(4H, m), 4.19(2H, br), 5.91-5.97(1H, m), 6.56-6.65(2H, m), 6.80(1H, s), 7.22(1H, d, J=8.3Hz), 7.65(1H, dd, J=2.5, 8.3Hz), 8.56(1H, d, J=2.5Hz).
FAB-MSm/z: 415(M+H)⁺.

### 7) Hydrochloride of title compound

1M Hydrochloric acid-ethanol (0.237 mL) was added to a solution of the title compound, i.e., 1-[5-[1-(2-aminoethyl)-1H-pyrrol-3-yl]-1-(6-methylpyridin-3-yl)-1H-pyrazole-3-carbonyl]-4, 4-difluoropiperidine (98.4 mg) in chloroform, ethyl ether and hexane at room temperature, and the resultant mixture was stirred. The precipitated solid was filtered, and the title compound (88.0 mg, 82%) was obtained.
¹H-NMR(400MHz, DMSO-d₆)δ: 1. 90-2. 05 (4H, m), 2.49(3H, s), 3.02-3.13(2H, m), 3.27-3.43(2H, m), 3.68(2H, br), 5.76(1H, br), 6.72(2H, br), 6.82(1H, br), 7.38(1H, d, J=8.3Hz), 7.75(1H, dd, J=2.5, 8.3Hz), 7.86(3H, br), 8.39(1H, d, J=2.5Hz).
ESI-MSm/z: 415(M+H)⁺.

[Example 90] 4-[1-(6-Methoxypyridazin-3-yl)-5-[1-methyl-1H-pyrrol-3-yl]-1H-pyrazole-3-carbonyl]morpholine

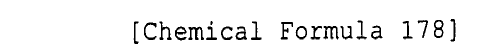

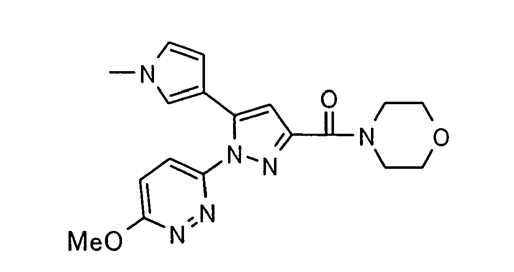

The title compound (0.152 g, 63%) was obtained as a solid by the same method as that in Example 5, using 1-(6-methoxypyridazin-3-yl)-5-(1-methyl-1H-pyrrol-3-yl)-1H-pyrazole-3-carboxylic acid (0.195 g) of Reference Example 55 and morpholine (0.170 mL).
¹H-NMR(400MHz, CDCl₃)δ: 3.62(3H, s), 3.67-3.85(6H, m), 4.09(2H, br), 4.19(3H, s), 6.05(1H, t, J=2.0Hz), 6.52(1H, t, J=2.0Hz), 6.80(1H, s), 6.82(1H, t, J=2.0Hz), 7.07(1H, d, J=9.3Hz), 7.54(1H, d, J=9.3Hz).
ESI-MSm/z: 369(M+H)⁺.

[Example 91] 4-Ethyl-1-[1-(6-methoxypyridin-3-yl)-5-(pyridin-2-yl)-1H-pyrazole-3-carbonyl]-3-oxopiperazine

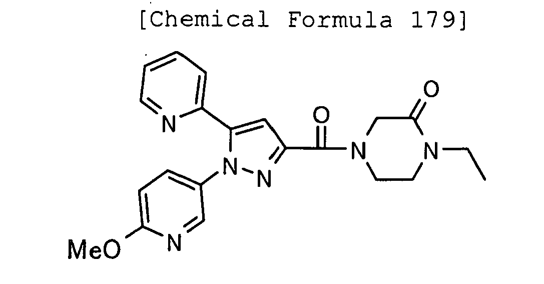

The title compound (0.322 g, 93%) was obtained as an amorphous material by the same method as that in Example 1, using 1-(6-methoxypyridin-3-yl)-5-(pyridin-2-yl)-1H-pyrazole-3-carboxylic acid (0.254 g) of Reference Example 3 and 1-ethyl-2-oxopiperazine hydrochloride (0.201 g) of Reference Example 56.
¹H-NMR(400MHz, CDCl₃)δ: 1.17(3H, t, J=7.1Hz), 3.44-3.56(4H, m), 3.96(3H, s), 4.03(1H, br), 4.37-4.45(2H, m), 4.81(1H, br), 6.74-6.80(1H, m), 7.19-7.30(2H, m), 7.38-7.78(3H, m), 8.09(1H×1/2, br), 8.14(1H×1/2, br), 8.52(1H, d, J=4.4Hz).
ESI-MSm/z: 407(M+H)⁺.

[Example 92] 4-[1-(6-Methoxypyridazin-3-yl)-5-(1H-pyrrol-3-yl)-1H-pyrazole-3-carbonyl]morpholine

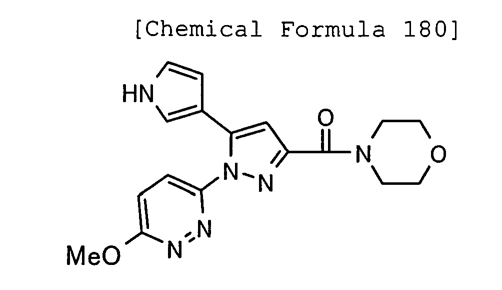

The title compound (0.343 g, 95%) was obtained as a solid by the same method as that in Example 5, using 1-(6-methoxypyridazin-3-yl)-5-(1H-pyrrol-3-yl)-1H-pyrazole-3-carboxylic acid (0.291 g) of Reference Example 57 and morpholine (0.267 mL).
¹H-NMR(400MHz, CDCl₃)δ: 3.67-3.85(6H, m), 4.06-4.12(2H, m), 4.18(3H, s), 6.16-6.20(1H, m), 6.70-6.75(1H, m), 6.84(1H, s), 7.01-7.06(1H, m), 7.08(1H, d, J=9.3Hz), 7.55(1H, d, J=9.3Hz), 8.40(1H, br) .
ESI-MSm/z: 355(M+H)⁺.

[Example 93] 4-[5-(1-Acetyl-1H-pyrrol-3-yl)-1-(6-methoxypyridazin-3-yl)-1H-pyrazole-3-carbonyl]morpholine

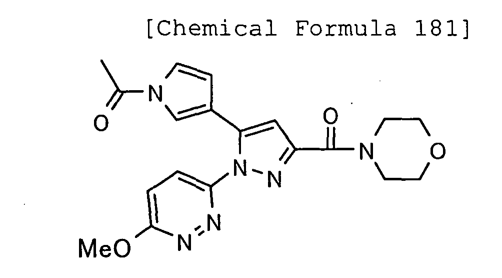

Triethylamine (0.191 mL), acetic anhydride (0.0976 mL), and 4-(dimethylamino)pyridine (13.2 mg) were added to a mixed solution of 4-[1-(6-methoxypyridazin-3-yl)-5-(1H-pyrrol-3-yl)-1H-pyrazole-3-carbonyl]morpholine (0.122 g) of Example 92 in dichloromethane (3.0 mL) and 1, 4-dioxane (5.0 mL) at room temperature, and the resultant mixture was stirred for 19 hours at 70°C. After air cooling, water and chloroform were added to the reaction solution, and the mixture was partitioned. Further, the aqueous layer was extracted with chloroform, and the organic layers were combined, washed with saturated saline, and then dried over anhydrous sodium sulfate. After separation by filtration, a residue obtained by evaporating the solvent under reduced pressure was purified by silica gel thin layer chromatography (chloroform-methanol), to obtain the title compound (90.8 mg, 67%) as a solid.
¹H-NMR(400MHz, DMSO-d₆)δ: 2.53(3H, s), 3.57-3.74(6H, m), 3.90-3.98(2H, m), 4.09(3H, s), 6.17-6.22(1H, m), 7.04(1H, s), 7.41-7.44(1H, m), 7.51(1H, d, J=9.3Hz), 7.58-7.62(1H, m), 7.97(1H, d, J=9.3Hz).
ESI-MSm/z: 397(M+H)⁺.

[Example 94] 4-Methyl-1-[1-(6-methylpyridin-3-yl)-5-(1H-pyrrol-3-yl)-1H-pyrazole-3-carbonyl]-3-oxopiperazine

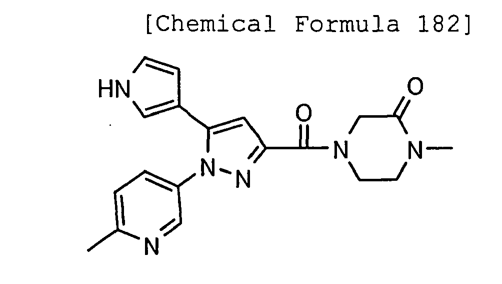

The title compound (0.167 g, 52%) was obtained as an amorphous material by the same method as that in Example 1, using 1-(6-methylpyridin-3-yl)-5-(1H-pyrrol-3-yl)-1H-pyrazole-3-carboxylic acid (0.235 g) of Reference Example 58-(3) and 1-methyl piperazin-2-one (0.299 g) of Reference Example 46.
¹H-NMR(400MHz, CDCl₃)δ: 2.61(3H, s), 3.01(3H, s), 3.40-3.52(2H+1/2×1H, m), 4.02(1H, br), 4.40(1H+1/2×1H, m), 4.81(1H, br), 5.94(1H, br), 6.60-6.72(2H, m), 6.76-6.87(1H, m), 7.22(1H, d, J=8.3Hz), 7.58-7.70(1H, m), 8.53(1/2×1H, br), 8.58(1/2×1H, br), 9.73(1H, br).
ESI-MSm/z: 365(M+H)⁺.

[Example 95] 4-Methoxy-1-[1-(6-methylpyridin-3-yl)-5-(1H-pyrrol-3-yl)-1H-pyrazole-3-carbonyl]piperidine

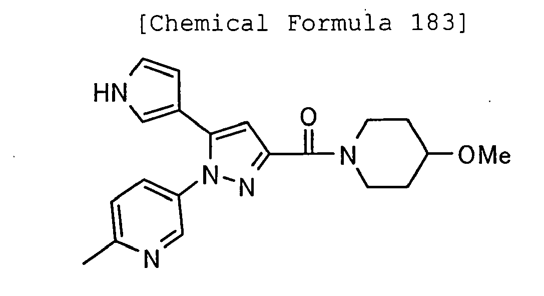

The title compound (0.205 g, 61%) was obtained as an amorphous material by the same method as that in Example 1, using 1-(6-methylpyridin-3-yl)-5-(1H-pyrrol-3-yl)-1H-pyrazole-3-carboxylic acid (0.245 g) of Reference Example 58-(3) and 4-methoxypiperidine hydrochloride (0.204 g) of Reference Example 23.
¹H-NMR(400MHz, CDCl₃)δ: 1.66(2H, br), 1.93(2H, br), 2.60(3H, s), 3.37(3H, s), 3.50(2H, br), 3.72(1H, br), 4.08(1H, br), 4.27(1H, br), 5.95(1H, br), 6.60-6.74(3H, m), 7.19(1H, d, J=8.0Hz), 7.63(1H, dd, J=2.7, 8.0Hz), 8.56(1H, d, J=2.7Hz), 9.46(1H, br).
ESI-MSm/z: 366(M+H)⁺.

[Example 96] 1-[5-(1-Ethyl-1H-pyrrol-3-yl)-1-(6-methylpyridin-3-yl)-1H-pyrazole-3-carbonyl]-4-methyl-3-oxopiperazine

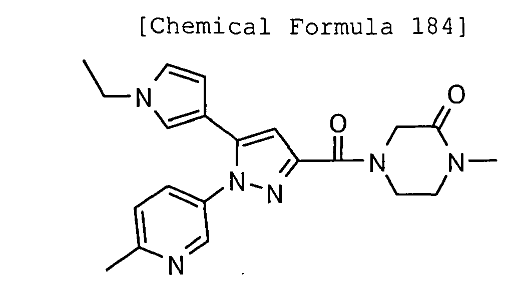

The title compound (0.120 g, 75%) was obtained as an amorphous material by the same method as that in Example 5, using 5-(1-ethyl-1H-pyrrol-3-yl)-1-(6-methylpyridin-3-yl)-1H-pyrazole-3-carboxylic acid (0.120 g) of Reference Example 58 and 1-methylpiperazin-2-one trifluoroacetate (0.131 g) of Reference Example 22.
¹H-NMR(400MHz, CDCl₃)δ: 1.38(3H, t, J=7.4Hz), 2.63(3H, s), 3.01(3H, s), 3.40-3.55(2H+1H×1/3, m), 3.86(2H, q, J=7.4Hz), 4.02(1H, br), 4.42(1H+1H×2/3, m), 4.81(1H, br), 5.88(1H, br), 6.52-6.62(2H, m), 6.81(1H×2/3, br), 6.86(1H×1/3, br), 7.20-7.30(1H, m), 7.61-7.74(1H, m), 8.55(1H×2/3, br), 8.60(1H×1/3, br).
ESI-MSm/z: 393(M+H)⁺.

[Example 97] 4-[1-(6-Methoxypyridin-3-yl)-5-(1-methyl-1H-imidazol-4-yl)-1H-pyrazole-3-carbonyl]morpholine

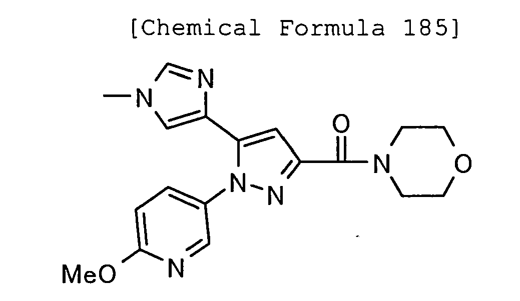

The title compound (99 mg, 79%) was obtained as a solid by the same method as that in Example 5, using 1-(6-methoxypyridin-3-yl)-5-(1-methyl-1H-imidazol-4-yl)-1H-pyrazole-3-carboxylic acid (102 mg) of Reference Example 34 and morpholine (45 µL).
¹H-NMR(400MHz, CDCl₃)δ: 3.66(3H, s), 3.72-3.79(6H, m), 3.98(3H, s), 4.10(2H, s), 6.68(1H, s), 6.80(1H, d, J=8.8Hz), 7.00(1H, s), 7.42(1H, s), 7.68(1H, dd, J=8.8, 2.7Hz), 8.23(1H, d, J=2.7Hz).
ESI-MSm/z: 369(M+H)⁺.

[Example 98] 1-[5-(5-Aminomethylpyridin-2-yl)-1-(6-methylpyridin-3-yl)-1H-pyrazole-3-carbonyl]-4, 4-difluoropiperidine

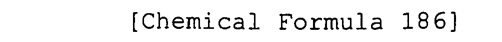

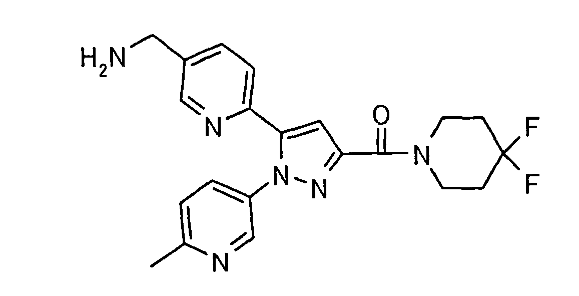

In an autoclave, 1-[5-(5-cyanopyridin-2-yl)-1-(6-methylpyridin-3-yl)-1H-pyrazole-3-carbonyl]-4, 4-difluoropiperidine (1.12 g) of Example 44 and nickel on silica/alumina (up to 65%, 200 mg) were suspended in a 2 M ammonia-ethanol solution (40 mL), and the suspension was stirred at 120°C for 1 hour under a hydrogen atmosphere (8 atmospheres). After air cooling, the reaction solution was filtered using Celite, and then a residue obtained by evaporating the solvent under reduced pressure was purified by silica gel thin layer chromatography (lower layer solvent of chloroform-methanol-water), to obtain the title compound (0.62 g, 55%) as an oily product.
ESI-MSm/z: 413(M+H)⁺.

[Example 99] 1-[5-(5-Hydroxymethylpyridin-2-yl)-1-(6-methylpyridin-3-yl)-1H-pyrazole-3-carbonyl]-4, 4-difluoropiperidine

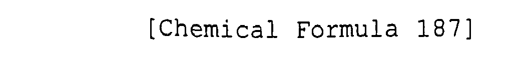

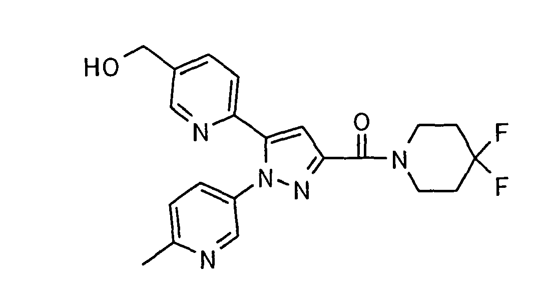

Sodium nitrite (519 mg) was added to a mixed solution of 1-[5-(5-aminomethylpyridin-2-yl)-1-(6-methylpyridin-3-yl)-1H-pyrazole-3-carbonyl]-4, 4-difluoropiperidine (0.62 g) of Example 98 in water (20 mL) and acetic acid (5 mL) at room temperature, and the resultant mixture was stirred for 1.5 hours. The reaction solution was alkalinized by adding a saturated aqueous solution of sodium hydrogen carbonate under ice cooling, and extracted with chloroform. The organic layer was dried over anhydrous sodium sulfate. After separation by filtration, a residue obtained by evaporating the solvent under reduced pressure was purified by silica gel column chromatography (chloroform-methanol), to obtain the title compound (131 mg, 21%) as a solid.
¹H-NMR(400MHz, CDCl₃)δ: 2.09(4H, br s), 2.60(3H, s), 3.92(2H, s), 4.19(2H, s), 4.74(2H, s), 7.14(1H, s), 7.21(1H, d, J=8.3Hz), 7.44(1H, d, J=8.1Hz), 7.62(1H, dd, J=8.3, 2.4Hz), 7.76(1H, d, J=8.1Hz), 8.38(1H, d, J=2.4Hz), 8.47(1H, s).
ESI-MSm/z: 414(M+H)⁺.

[Example 100] 4-[5-(5-Cyanopyridin-2-yl)-1-(6-methoxypyridazin-3-yl)-1H-pyrazole-3-carbonyl]morpholine

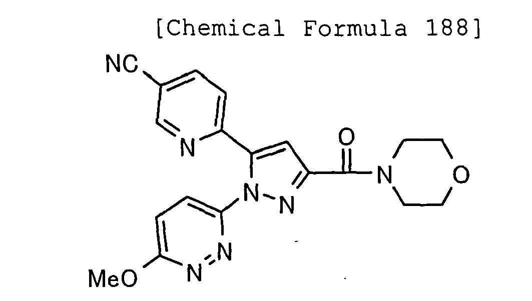

The title compound (186 mg, 86%) was obtained as a solid by the same method as that in Example 5, using 5-(5-cyanopyridin-2-yl)-1-(6-methoxypyridazin-3-yl)-1H-pyrazole-3-carboxylic acid (179 mg) of Reference Example 59 and morpholine (73µl).
¹H-NMR(400MHz, CDCl₃)δ: 3.72-3.83(6H, m), 4.12(5H, s), 7.19(1H, d, J=9.3Hz), 7.24(1H, s), 7.71(1H, d, J=4.2Hz), 7.83(1H, d, J=9.3Hz), 8.02(1H, dd, J=8.2, 2.1Hz), 8.66(1H, d, J=1.2Hz).
ESI-MSm/z: 392(M+H)⁺.

[Example 101] 4-[5-(5-Aminomethylpyridin-2-yl)-1-(6-methoxypyridazin-3-yl)-1H-pyrazole-3-carbonyl]morpholine

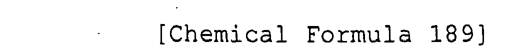

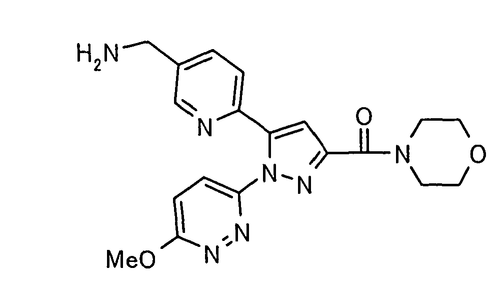

The title compound (162 mg, 88%) was obtained as an oily product by the same method as that in Example 98, using 4-[5-(5-cyanopyridin-2-yl)-1-(6-methoxypyridazin-3-yl)-1H-pyrazole-3-carbonyl]morpholine (182 mg) of Example 100 and nickel on silica/alumina (up to 65%, 100 mg).
ESI-MSm/z: 396(M+H)⁺.

[Example 102] 4-[5-(5-Hydroxymethylpyridin-2-yl)-1-(6-methoxypyridazin-3-yl)-1H-pyrazole-3-carbonyl]morpholine

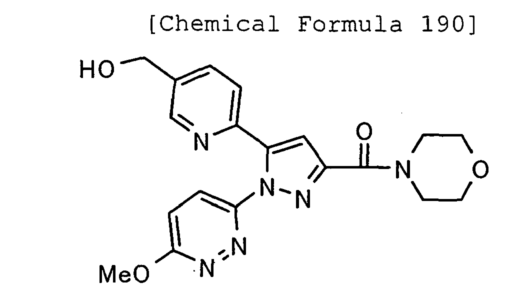

The title compound (97 mg, 60%) was obtained as an amorphous material by the same method as that in Example 99, using 9-[5-(5-aminomethylpyridin-2-yl)-1-(6-methoxypyridazin-3-yl)-1H-pyrazole-3-carbonyl]morpholine (161 mg) of Example 101 and sodium nitrite (84 mg).
ESI-MSm/z: 397(M+H)⁺.

[Example 103] 4-[5-(5-Fluoromethylpyridin-2-yl)-1-(6-methoxypyridazin-3-yl)-1H-pyrazole-3-carbonyl]morpholine

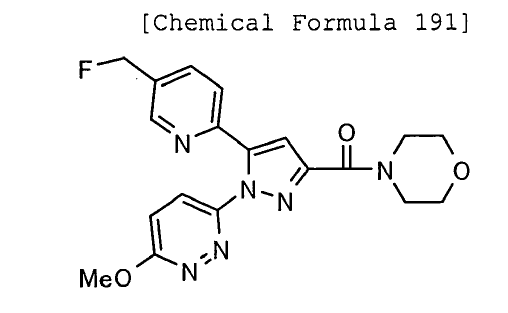

Bis(2-methoxyethyl)aminosulfur trifluoride (53 µL) was added to a solution of 4-[5-(5-hydroxymethylpyridin-2-yl)-1-(6-methoxypyridazin-3-yl)-1H-pyrazole-3-carbonyl]morpholine (96 mg) of Example 102 in dichloromethane (10 mL) under cooling to -15°C, and the resultant mixture was stirred for 20 minutes. The reaction solution was alkalinized by adding a saturated aqueous solution of sodium hydrogen carbonate, and extracted with dichloromethane. The organic layer was dried over anhydrous sodium sulfate. After separation by filtration, a residue obtained by evaporating the solvent under reduced pressure was purified by silica gel thin layer chromatography (chloroform-methanol), to obtain the title compound (25 mg, 24%) as a solid.
¹H-NMR(400MHz, CDCl₃)δ: 3.73-3.85(6H, m), 4.12(5H, s), 5.41(2H, d, J=47.4Hz), 7.15(1H, d, J=9.0Hz), 7.16(1H, s), 7.63(1H, d, J=8.1Hz), 7.76-7.80(2H, m), 8.41(1H, s).
ESI-MSm/z: 399(M+H)⁺.

[Example 104] 1-[5-(1-Methyl-1H-imidazol-4-yl)-1-(6-methylpyridin-3-yl)-1H-pyrazole-3-carbonyl]-4, 4-difluoropiperidine

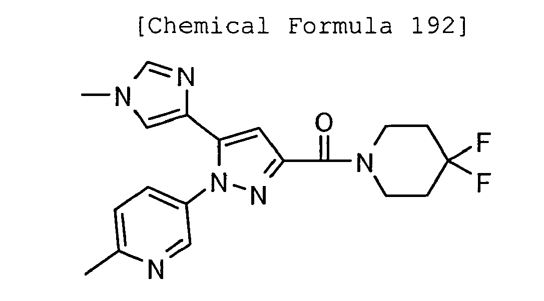

The title compound (115 mg, 84%) was obtained as a solid by the same method as that in Example 5, using 5-(1-methyl-1H-imidazol-4-yl)-1-(6-methylpyridin-3-yl)-1H-pyrazole-3-carboxylic acid (100 mg) of Reference Example 60 and 4, 4-difluoropiperidine hydrochloride (67 mg) of Reference Example 18.
¹H-NMR(400MHz, CDCl₃)δ: 2.06(4H, brs), 2.62(3H, s), 3.67(3H, s), 3.91(2H, s), 4.16(2H, s), 6.78(1H, d, J=1.2Hz), 6.98 (1H, s), 7.24(1H, d, J=8.0Hz), 7.40(1H, d, J=1.0 Hz), 7.73(1H, dd, J=8.2, 2.6Hz), 8.54(1H, d, J=2.7Hz).
ESI-MSm/z: 387(M+H)⁺.

[Example 105] (+)(3S)-4-[1-(6-Methoxypyridazin-3-yl)-5-(2-pyridyl)-1H-pyrazole-3-carbonyl]-3-methylmorpholine

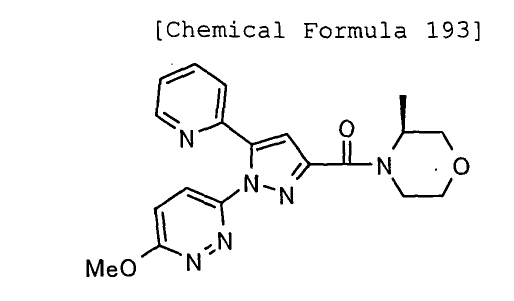

### 1) (5S)-5-Methyl-3-morpholinone

Sodium hydride (55%, 0.409 g) was added to a solution of (2S)-2-amino-1-propanol (0.665 mL) in tetrahydrofuran (100 mL), and the resultant mixture was stirred for 35 minutes. Chloroethyl acetate (0.900 mL) was added to the reaction solution, and the mixture was stirred for 30 minutes, and then heated to reflux for 3 hours. After air cooling, the reaction solution was filtered, and a residue obtained by evaporating the solvent of the filtrate under reduced pressure was purified by silica gel column chromatography (ethyl acetate-n-hexane), to obtain a crude product of (5S)-5-methyl-3-morpholinone (0.170 g, 17%) as an amorphous material.

### 2) (3S)-3-Methylmorpholine

Lithium aluminum hydride (0.121 g) was added to a solution of a crude product of (5S)-5-methyl-3-morpholinone (0.170 g) of the above in tetrahydrofuran (20 mL), and the resultant mixture was heated to reflux for 19 hours. After air cooling, anhydrous magnesium sulfate and methanol were added to the reaction solution, and then the reaction solution was filtered. A 1M hydrochloric acid-ethanol solution (5 mL) was added to the filtrate. The solvent of the reaction solution was evaporated under reduced pressure, and a crude product of (3S)-3-methylmorpholine was obtained.

### 3) Title compound

The title compound (118 mg, 21%) was obtained as a solid by the same method as that in Example 1, using a crude product of (3S)-3-methylmorpholine of the above and 1-(6-methoxy-3-pyridazinyl)-5-(2-pyridyl)-1H-pyrazole-3-carboxylic acid (0.232 g) of Reference Example 4.
¹H-NMR(400MHz, DMSO-d₆)δ: 1.31(3H, brs), 3.14-3.30(1H, m), 3.36-3.45(1H, m), 3.55-3.59(1H, m), 3.67(1H, brs), 3.81-3.94(1H, m), 4.03(3H, s), 4.19-4.40(1H, m), 4.62(1H, brs), 7.26(1H, s), 7.34(1H, ddd, J=7.6, 4.9, 1.0Hz), 7.47(1H, d, J=9.3Hz), 7.77-7.79(1H, m), 7.89(1H, ddd, J=7.8, 7.6, 1.7Hz), 7.98(1H, d, J=9.3Hz), 8.36-8.38(1H, m).
ESI-MSm/z: 381(M+H)⁺.
[α]_{D}²⁵+51.5° (c1.01, CHCl₃).

[Example 106] (-)(2S)-1-[1-(6-Methoxypyridazin-3-yl)-5-(2-pyridyl)-1H-pyrazole-3-carbonyl]-2-methoxymethylpyrrolidine

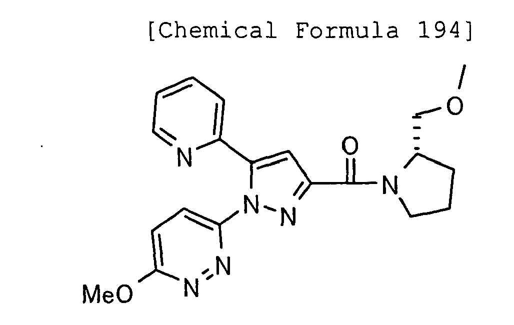

The title compound (247 mg, 80%) was obtained as a solid by the same method as that in Example 1, using 1-(6-methoxy-3-pyridazinyl)-5-(2-pyridyl)-1H-pyrazole-3-carboxylic acid (0.232 g) of Reference Example 4 and (2S)-2-(methoxymethyl)pyrrolidine (0.115 mL).
¹H-NMR(400MHz, CDCl₃)δ: 1.87-2.13(4H, m), 3.28(1H, s), 3.30-3.33(2/3H, m), 3.39(2H, s), 3.54(2/3H, dd, J=9.3, 7.1Hz), 3.63(1/3H, dd, J=9.0, 3.4Hz), 3.69-3.76(4/3H, m), 4.00-4.04(1H, m), 4.10(1H, s), 4.11(2H, s), 4.50-4.55(2/3H, m), 5.05-5.10(1/3H, m), 7.13(1H, d, J=9.3Hz), 7.20-7.25(2H, m), 7.59-7.64(1H, m), 7.73-7.89(2H, m), 8.39-8.43(1H, m).
ESI-MSm/z: 395(M+H)⁺.
[α]_{D}²⁵-83.7° (c0.98, CHCl₃).

[Example 107] (-)(3R)-4-[1-(6-Methoxypyridazin-3-yl)-5-(2-pyridyl)-1H-pyrazole-3-carbonyl]-3-methylmorpholine

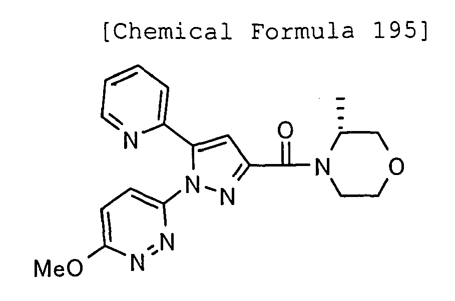

### 1) (5R)-5-Methyl-3-morpholinone

Sodium hydride (55%, 0.820 g) was added to a solution of (2R)-2-amino-1-propanol (1.33 mL) in tetrahydrofuran (200 mL) at room temperature, and the resultant mixture was stirred for 35 minutes. Chloroethyl acetate (1.80 mL) was added to the reaction solution over 5 minutes at room temperature, and the mixture was stirred for 25 minutes, and then heated to reflux for 17.5 hours. After air cooling, the reaction solution was filtered, and a residue obtained by evaporating the solvent of the filtrate under reduced pressure was purified by silica gel column chromatography (ethyl acetate-n-hexane), to obtain a crude product of (5R)-5-methyl-3-morpholinone (0.520 g, 26%) as an oily product.

### 2) (3R)-3-Methylmorpholine

To a solution of the crude product of (5R)-5-methyl-3-morpholinone of the above (0.520 g) in tetrahydrofuran (60 mL), lithium aluminum hydride (0.342 g) was added at room temperature, and the resultant mixture was heated to reflux for 15.5 hours. Magnesium sulfate heptahydrate was added to the reaction solution, and then the reaction solution was filtered. A 1M hydrochloric acid-ethanol solution (15 mL) was added to the filtrate, and then the solvent of the reaction solution was evaporated under reduced pressure, to obtain a crude product of (3R)-3-methylmorpholine.

### 3) Title compound

The title compound (145 mg, 8.4%) was obtained as a solid by the same method as that in Example 1, using the crude product of (3R)-3-methylmorpholine of the above and 1-(6-methoxy-3-pyridazinyl)-5-(2-pyridyl)-1H-pyrazole-3-carboxylic acid (0.464 g) of Reference Example 4.
¹H-NMR(CDCl₃)δ: 1.43(3/2H, brs), 1.45(3/2H, brs), 3.26-3.78(4H, m), 3.86-4.02(1H, m), 4.11(3H, s), 4.84(1H, brs), 7.11(1H, s), 7.13(1H, d, J=9.3Hz), 7.21-7.24(1H, m), 7.57-7.60(1H, m), 7.73-7.79(2H, m), 8.40-8.42(1H, m).
ESI-MSm/z: 381(M+H)⁺.
[α]_{D}²⁵-41.3°(c1.00, CHCl₃).

[Example 108] 4-[1-(6-Methoxy-3-pyridyl)-5-(6-methyl-3-pyridyl)-1H-pyrazole-3-carbonyl]morpholine

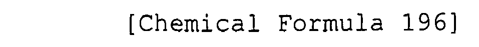

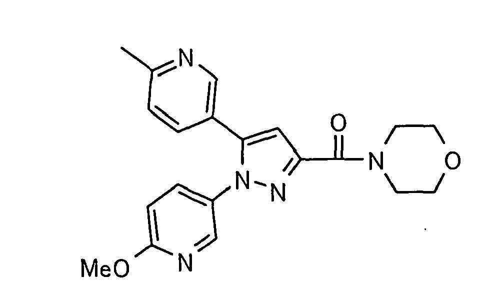

The title compound (0.217 g, 71%) was obtained as a solid by the same method as that in Example 5, using 1-(6-methoxy-3-pyridyl)-5-(6-methyl-3-pyridyl)-1H-pyrazole-3-carboxylic acid (0.250 g) of Reference Example 65 and morpholine (0.105 mL).
¹H-NMR(400MHz, CDCl₃)δ: 2.58(3H, s), 3.75-3.83(6H, m), 3.96(3H, s), 4.18(2H, m), 6.77(1H, d, J=8.8Hz), 7.01(1H, s), 7.16(1H, d, J=8.1Hz), 7.41-7.44(1H, m), 7.49-7.52(1H, m), 8.10(1H, d, J=2.7Hz), 8.42(1H, d, J=2.4Hz).
EI-MSm/z: 379(M⁺).

[Example 109] 4-[5-(6-Amino-3-pyridyl)-1-(6-methoxy-3-pyridyl)-1H-pyrazole-3-carbonyl]morpholine

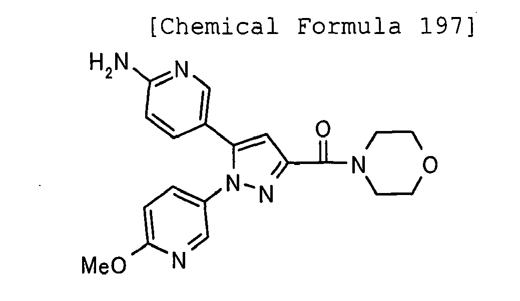

### 1) 4-[5-(6-tert-Butoxycarbonylamino-3-pyridyl)-1-(6-methoxy-3-pyridyl)-1H-pyrazole-3-carbonyl]morpholine

4-[5-(6-tert-Butoxycarbonylamino-3-pyridyl)-1-(6-methoxy-3-pyridyl)-1H-pyrazole-3-carbonyl]morpholine (0.442 g, 90%) was obtained as a solid by the same method as that in Example 1, using 5-(6-tert-butoxycarbonylamino-3-pyridyl)-1-(6-methoxy-3-pyridyl)-1H-pyrazole-3-carboxylic acid (0.420 g) of Reference Example 66 and morpholine (0.133 mL).
¹H-NMR(400MHz, CDCl₃)δ: 1.53(9H, s), 3.75-3.83(6H, m), 3.96(3H, s), 4.18(2H, m), 6.76-6.78(1H, m), 6.97(1H, s), 7.44-7.52(2H, m), 7.73(1H, s), 7.95(1H, d, J=8.8Hz), 8.11-8.12(1H, m), 8.19-8.20(1H, m).
EI-MSm/z: 480(M⁺).

### 2) Title compound

To a solution of 4-[5-(6-tert-butoxycarbonylamino-3-pyridyl)-1-(6-methoxy-3-pyridyl)-1H-pyrazole-3-carbonyl]morpholine (0.434 g) of the above in dichloromethane (8.7 mL), trifluoroacetic acid (4.4 mL) was added at room temperature, and the resultant mixture was stirred for 5 hours. A saturated aqueous solution of sodium hydrogen carbonate and chloroform were added to the reaction solution, and the mixture was partitioned. The organic layer was dried over anhydrous sodium sulfate. After separation by filtration, a residue obtained by evaporating the solvent under reduced pressure was purified by silica gel column chromatography (chloroform-methanol), to obtain the title compound (0.225 g, 65%) as a solid.
¹H-NMR(400MHz, CDCl₃)δ: 3.75-3.83(6H, m), 3.96(3H, s), 4.17(2H, m), 4.72(2H, br), 6.45-6.48(1H, m), 6.77(1H, d, J=8.8Hz), 6.90(1H, s), 7.24-7.27(1H, m), 7.51-7.54(1H, m), 7.99(1H, m), 8.14(1H, d, J=2.7Hz).
EI-MSm/z: 380(M⁺).

[Example 110] 1-[1-(6-Methoxy-3-pyridazinyl)-5-(6-methyl-3-pyridyl)-1H-pyrazole-3-carbonyl]-4, 4-difluoropiperidine

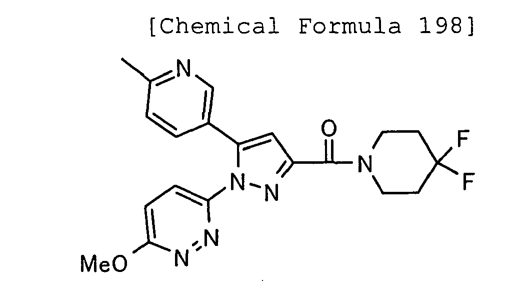

The title compound (0.276 g, 83%) was obtained as a solid by the same method as that in Example 1, using 1-(6-methoxy-3-pyridazinyl)-5-(6-methyl-3-pyridyl)-1H-pyrazole-3-carboxylic acid (0.250 g) of Reference Example 67 and 4, 4-difluoropiperidine hydrochloride (0.152 g) of Reference Example 18.
¹H-NMR(400MHz, CDCl₃)δ: 2. 08-2. 13 (4H, m), 2.60(3H, s), 3.93-3.96(2H, m), 4.13(3H, s), 4.15-4.16(2H, m), 6.97(1H, s), 7.16(1H, d, J=9.3Hz), 7.21(1H, d, J=8.1Hz), 7.63-7.65(1H, m), 7.79(1H, d, J=9.3Hz), 8.42(1H, d, J=2.2Hz).
EI-MSm/z: 414(M⁺).

[Example 111] 1-[5-(6-Amino-3-pyridyl)-1-(6-methoxy-3-pyridazinyl)-1H-pyrazole-3-carbonyl]-4, 4-difluoropiperidine

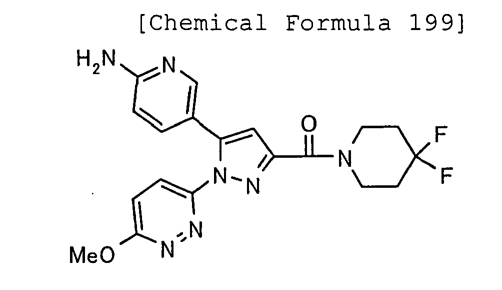

### 1) 1-[5-(6-tert-Butoxycarbonylamino-3-pyridyl)-1-(6-methoxy-3-pyridazinyl)-1H-pyrazole-3-carbonyl]-4, 4-difluoropiperidine

1-[5-(6-tert-Butoxycarbonylamino-3-pyridyl)-1-(6-methoxy-3-pyridazinyl)-1H-pyrazole-3-carbonyl]-4, 4-difluoropiperidine (0.421 g, quantitative) was obtained as a solid by the same method as that in Example 1, using 5-(6-tert-butoxycarbonylamino-3-pyridyl)-1-(6-methoxy-3-pyridazinyl)-1H-pyrazole-3-carboxylic acid (0.330 g) of Reference Example 68 and 4, 4-difluoropiperidine hydrochloride (0.151 g) of Reference Example 18.
¹H-NMR(400MHz, CDCl₃)δ: 1.53(9H, s), 2.07-2.11(4H, m), 3.93(2H, m), 4.13(3H, s), 4.15(2H, m), 6.94(1H, s), 7.13(1H, d, J=9.3Hz), 7.62-7.65(1H, m), 7.73-7.76(2H, m), 7.98(1H, d, J=8.8Hz), 8.25(1H, d, J=2.0Hz).
EI-MSm/z: 515(M⁺).

### 2) Title compound

The title compound (0.265 g, 80%) was obtained as a solid by the same method as that in Example 109-(2), using 1-[5-(6-tert-butoxycarbonylamino-3-pyridyl)-1-(6-methoxy-3-pyridazinyl)-1H-pyrazole-3-carbonyl]-4, 4-difluoropiperidine (0.411 g) of the above.
¹H-NMR(400MHz, CDCl₃)δ: 2.06-2.11(4H, m), 3.92(2H, m), 4.14(3H, s), 4.14(2H, m), 4.78(2H, br), 6.50-6.52(1H, m), 6.88(1H, s), 7.12(1H, d, J=9.3Hz), 7.47-7.50(1H, m), 7.70(1H, d, J=9.3Hz), 8.01(1H, m).
EI-MSm/z: 415(M⁺).

[Example 112] 4-[5-(1H-Imidazol-4-yl)-1-(6-methoxy-3-pyridyl)-1H-pyrazole-3-carbonyl]morpholine

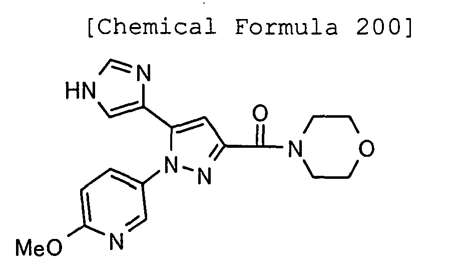

The title compound (0.112 g, 40%) was obtained as a solid by the same method as that in Example 1, using 5-(1H-imidazol-4-yl)-1-(6-methoxy-3-pyridyl)-1H-pyrazole-3-carboxylic acid (0.220 g) of Reference Example 69 and morpholine (0.134 mL).
¹H-NMR(400MHz, CDCl₃)δ: 3.73-3.80(6H, m), 3.97(3H, m), 4.10(2H, m), 6.78-6.80(2H, m), 7.02(1H, s), 7.61-7.64(1H, m), 7.66(1H, s), 8.21(1H, d, J=2.9Hz).
EI-MSm/z: 354 (M⁺).

[Example 113] 1-[5-(1H-Imidazol-4-yl)-1-(6-methoxy-3-pyridyl)-1H-pyrazole-3-carbonyl]-4, 4-difluoropiperidine

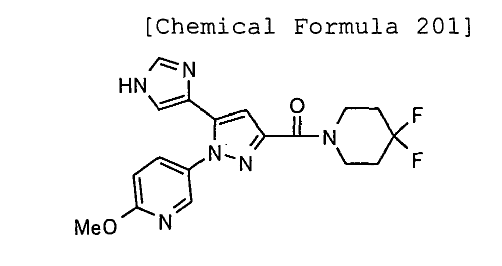

The title compound (0.195 g, 62%) was obtained as a solid by the same method as that in Example 1, using 5-(1H-imidazol-4-yl)-1-(6-methoxy-3-pyridyl)-1H-pyrazole-3-carboxylic acid (0.220 g) of Reference Example 69 and 4, 4-difluoropiperidine hydrochloride (0.243 g) of Reference Example 18.
¹H-NMR(400MHz, DMSO-d₆)δ: 2.00-2.09 (4H, m), 3.76(2H, m), 3.91(3H, s), 4.06(2H, m), 6.92-6.95(2H, m), 7.06(1H, br), 7.70(1H, s), 7.80-7.84(1H, m), 8.28(1H, d, J=2.7Hz).
EI-MSm/z: 388(M⁺).

[Example 114] 4-[1-(6-Methoxy-3-pyridyl)-5-(1H-pyrazol-4-yl)-1H-pyrazole-3-carbonyl]morpholine

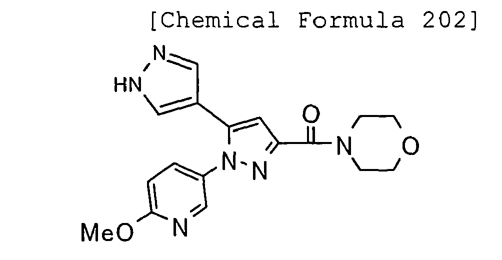

The title compound (0.110 g, 34%) was obtained as a solid by the same method as that in Example 1, using 1-(6-methoxy-3-pyridyl)-5-(1H-pyrazol-4-yl)-1H-pyrazole-3-carboxylic acid (0.250 g) of Reference Example 70 and morpholine (0.153 mL).
¹H-NMR(400MHz, CDCl₃)δ: 3.73-3.82(6H, m), 3.99(3H, s), 4.14-4.17(2H, m), 6.82(1H, d, J=8.8Hz), 6.93(1H, s), 7.46(2H, br), 7.57(1H, dd, J=8.8, 2.7Hz), 8.20(1H, d, J=2.7Hz).
EI-MSm/z: 354(M⁺).

[Example 115] 4-[1-(6-Methoxy-3-pyridyl)-5-(1-methyl-1H-pyrazol-4-yl)-1H-pyrazole-3-carbonyl]morpholine

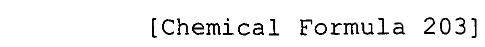

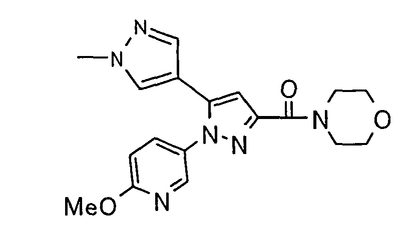

The title compound (1.13 g, 91%) was obtained as a solid by the same method as that in Example 5, using 1-(6-methoxy-3-pyridyl)-5-(1-methyl-1H-pyrazol-4-yl)-1H-pyrazole-3-carboxylic acid (1.00 g) of Reference Example 61 and morpholine (0.349 mL).
¹H-NMR(400MHz, CDCl₃)δ: 3.72-3.81 (6H, m), 3.87(3H, s), 3.99(3H, s), 4.15(2H, m), 6.82(1H, d, J=8.8Hz), 6.86(1H, m), 7.21(1H, s), 7.27-7.28(1H, m), 7.56-7.59(1H, m), 8.21(1H, d, J=2.7Hz).
EI-MSm/z: 368(M⁺).

[Example 116] 4-[1-(6-Methoxy-3-pyridyl)-5-(thiazol-5-yl)-1H-pyrazole-3-carbonyl]morpholine

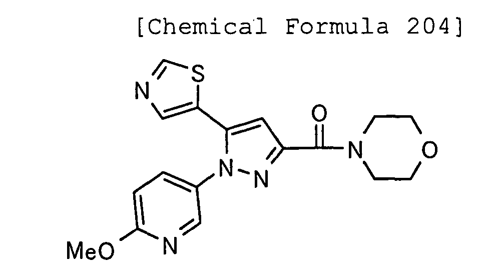

The title compound (0.572 g, 78%) was obtained as a solid by the same method as that in Example 1, using 1-(6-methoxy-3-pyridyl)-5-(thiazol-5-yl)-1H-pyrazole-3-carboxylic acid (0.594 g) of Reference Example 62 and morpholine (0.257 mL).
¹H-NMR(400MHz, CDCl₃)δ: 3.73-3.82(6H, m), 3.99(3H, s), 4.14(2H, m), 6.83(1H, dd, J=8.8, 0.5Hz), 7.09(1H, s), 7.55(1H, dd, J=8.8, 2.7Hz), 7.78(1H, d, J=0.5Hz), 8.17-8.18(1H, m), 8.79(1H, d, J=0.5Hz).
EI-MSm/z: 371(M⁺).

[Example 117] 4-[1-(6-Methoxy-3-pyridyl)-5-(thiazol-2-yl)-1H-pyrazole-3-carbonyl]morpholine

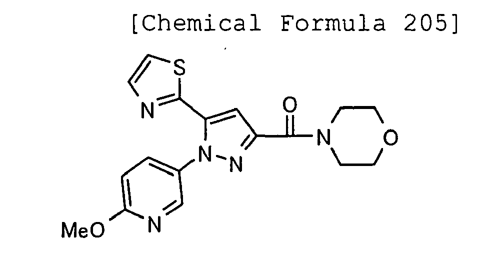

The title compound (0.515 g, 69%) was obtained as a solid by the same method as that in Example 5, using 1-(6-methoxy-3-pyridyl)-5-(thiazol-2-yl)-1H-pyrazole-3-carboxylic acid (0.610 g) of Reference Example 63 and morpholine (0.264 mL).
¹H-NMR(400MHz, CDCl₃)δ: 3.73-3.82(6H, m), 4.00(3H, s), 4.13(2H, m), 6.83(1H, dd, J=8.8, 0.5Hz), 7.31(1H, s), 7.38(1H, d, J=3.2Hz), 7.67(1H, dd, J=8.8, 2.7Hz), 7.80(1H, d, J=3.2Hz), 8.25-8.26(1H, m).
EI-MSm/z: 371(M⁺).

[Example 118] 4-[1-(6-Methoxy-3-pyridyl)-5-(thiazol-4-yl)-1H-pyrazole-3-carbonyl]morpholine

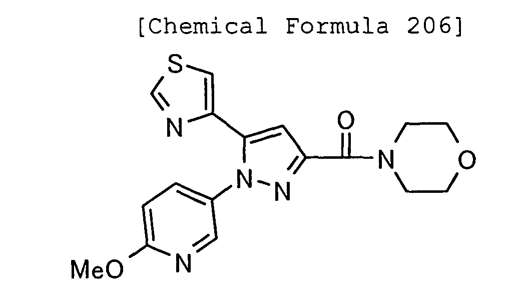

The title compound (0.140 g, 47%) was obtained as a solid by the same method as that in Example 5, using 1-(6-methoxy-3-pyridyl)-5-(thiazol-4-yl)-1H-pyrazole-3-carboxylic acid (0.244 g) of Reference Example 64 and morpholine (0.105 mL).
¹H-NMR(400MHz, CDCl₃)δ: 3.73-3.82(6H, m), 3.98(3H, s), 4.14(2H, m), 6.81(1H, d, J=8.8Hz), 7.17(1H, s), 7.26(1H, d, J=2.0Hz), 7.62(1H, dd, J=8.8, 2.7Hz), 8.17(1H, d, J=2.7Hz), 8.79(1H, d, J=2.0Hz).
EI-MSm/z: 371(M⁺).

[Example 119] 1-[1-(6-Methoxy-3-pyridyl)-5-(1-methyl-1H-pyrazol-4-yl)-1H-pyrazole-3-carbonyl]-4-methoxypiperidine

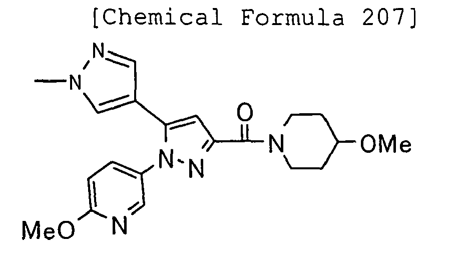

The title compound (0.199 g, 75%) was obtained as a solid by the same method as that in Example 5, using 1-(6-methoxy-3-pyridyl)-5-(1-methyl-1H-pyrazol-4-yl)-1H-pyrazole-3-carboxylic acid (0.200 g) of Reference Example 61 and 4-methoxypiperidine hydrochloride (0.203 g) of Reference Example 23.
¹H-NMR(400MHz, CDCl₃)δ: 1.66-1.93(4H, m), 3.38(3H, s), 3.46-3.52(2H, m), 3.73(1H, m), 3.87(3H, s), 3.99(3H, s), 4.09(1H, m), 4.29(1H, m), 6.80-6.82(2H, m), 7.21(1H, s), 7.28(1H, s), 7.59(1H, dd, J=8.8, 2.7Hz), 8.22(1H, d, J=2.7Hz).
EI-MSm/z: 396(M⁺).

[Example 120] 1-[1-(6-Methoxy-3-pyridyl)-5-(1-methyl-1H-pyrazol-4-yl)-1H-pyrazole-3-carbonyl]-4-methylpiperazine

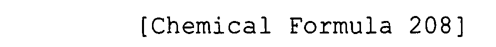

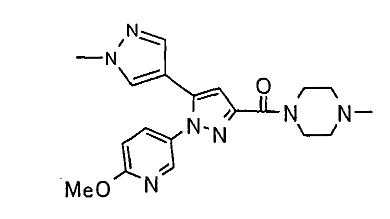

The title compound (0.112 g, 44%) was obtained as a solid by the same method as that in Example 5, using 1-(6-methoxy-3-pyridyl)-5-(1-methyl-1H-pyrazol-4-yl)-1H-pyrazole-3-carboxylic acid (0.200 g) of Reference Example 61 and N-methylpiperazine (0.148 mL).
¹H-NMR(400MHz, CDCl₃)δ: 2.37(3H, s), 2.52(4H, m), 3.84-3.87(2H, m), 3.87(3H, s), 3.99(3H, s), 4.14(2H, m), 6.81(1H, dd, J=8.8, 0.7Hz), 6.84(1H, s), 7.20(1H, s), 7.28(1H, d, J=0.7Hz), 7.58(1H, dd, J=8.8, 2.7Hz), 8.21-8.22(1H, m).
EI-MSm/z: 381(M⁺).

[Example 121] 1-[1-(6-Methoxy-3-pyridyl)-5-(1-methyl-1H-pyrazol-4-yl)-1H-pyrazole-3-carbonyl]-4-methyl-3-oxopiperazine

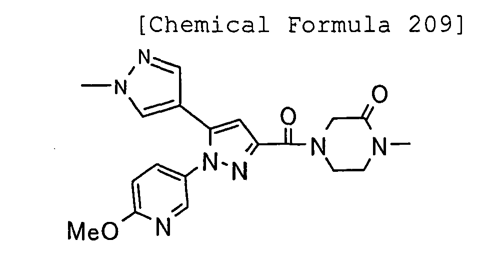

The title compound (92.0 mg, 35%) was obtained as a solid by the same method as that in Example 5, using 1-(6-methoxy-3-pyridyl)-5-(1-methyl-1H-pyrazol-4-yl)-1H-pyrazole-3-carboxylic acid (0.200 g) of Reference Example 61 and 4-methyl-3-oxopiperazine hydrochloride (0.201 g) of Reference Example 21.
¹H-NMR(400MHz, CDCl₃)δ: 3.01(3H, s), 3.45-3.47(2H, m), 3.88(3H, s), 4.00(3H, s), 4.03(1H, m), 4.42(2H, m), 4.80(1H, m), 6.82-6.92(2H, m), 7.19-7.30(2H, m), 7.59-7.61(1H, m), 8.20(1H, m).
EI-MSm/z: 395(M⁺).

[Example 122] 1-[1-(6-Methoxy-3-pyridyl)-5-(1-methyl-1H-pyrazol-4-yl)-1H-pyrazole-3-carbonyl]-2-carbamoylpiperidine

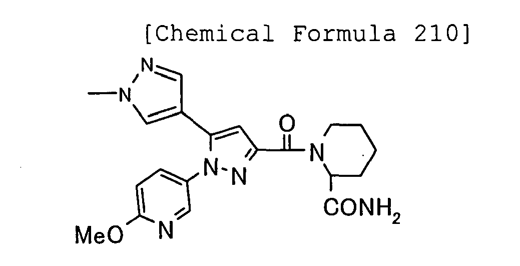

The title compound (0.225 g, 64%) was obtained as a solid by the same method as that in Example 5, using 1-(6-methoxy-3-pyridyl)-5-(1-methyl-1H-pyrazol-4-yl)-1H-pyrazole-3-carboxylic acid (0.250 g) of Reference Example 61 and 2-carbamoylpiperidine (0.118 g) of Reference Example 71.
¹H-NMR(400MHz, CDCl₃)δ: 1.56-1.77(5H, m), 2.29-2.40(1H, m), 2.78-3.20(1H, m), 3.88(3H, s), 3.99(3H, s), 4.69-4.79(1H, m), 5.36-5.48(2H, m), 6.38-7.15(1H, m), 6.81-6.86(2H, m), 7.21-7.23(1H, m), 7.30(1H, s), 7.54-7.61(1H, m), 8.18-8.23(1H, m).
EI-MSm/z: 409(M⁺).

[Example 123] 1-[1-(6-Methoxy-3-pyridyl)-5-(2-pyridyl)-1H-pyrazole-3-carbonyl]hexahydropyridazine

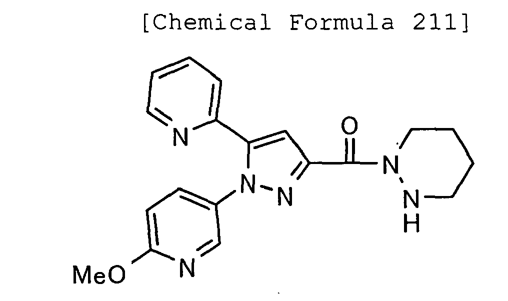

The title compound (1.61 g, 87%) was obtained as an amorphous form by the same method as that in Example 1, using 1-(6-methoxy-3-pyridyl)-5-(2-pyridyl)-1H-pyrazole-3-carboxylic acid (1.495 g) of Reference Example 3 and hexahydropyridazine (0.629 g) of Reference Example 20.
¹H-NMR(400MHz, CDCl₃)δ: 1.60-1.90(4H, m), 2.95-3.10(2H, m), 3.80-3.90(1/3×2H, m), 3.95(2/3×3H, s), 3.97(1/3×3H, s), 4.20-4.27(2/3×2H, m), 6.75(1H, d, J=8.8Hz), 7.17(1H, s), 7.20-7.75(5H, m), 8.12(1H, br), 8.5(1H, br).
FAB-MSm/z: 365(M+H)⁺.

[Example 124] 1-[1-(6-Methoxy-3-pyridazinyl)-5-(1-methyl-1H-pyrrol-3-yl)-1H-pyrazole-3-carbonyl]-4-methyl-3-oxopiperazine

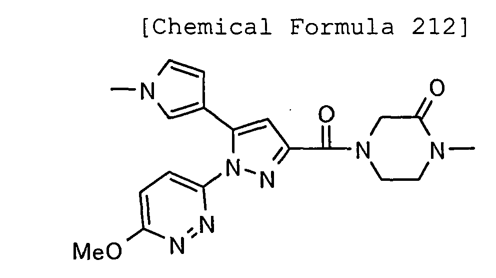

The title compound (0.189 g, 71%) was obtained as an amorphous material by the same method as that in Example 5, using 1-(6-methoxy-3-pyridazinyl)-5-(1-methyl-1H-pyrrol-3-yl)-1H-pyrazole-3-carboxylic acid (0.202 g) of Reference Example 55 and 1-methylpiperazin-2-one hydrochloride (0.160 g) of Reference Example 21.
¹H-NMR(400MHz, CDCl₃)δ: 3.01(3H, s), 3.45(2H, br), 3.62(3H, s), 4.03(1H, br), 4.20(3H, s), 4.34-4.45(2H, m), 4.83(1H, br), 6.00-6.10(1H, m), 6.53(1H, t, J=2.4Hz), 6.79(1H×1/2, br), 6.86(1H, s), 6.90(1H×1/2, br), 7.03-7.15(1H, m), 7.50(1H×1/2, d, J=8.8Hz), 7.65(1H×1/2, d, J=8.8Hz).
ESI-MSm/z: 396(M+H)⁺.

[Example 125] 1-[1-(6-Methoxy-3-pyridyl)-5-(2-pyridyl)-1H-pyrazole-3-carbonyl]-2-(1-aminocyclopropyl)piperidine

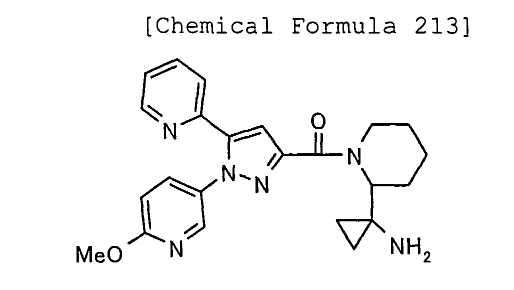

### 1) 1-[1-(6-Methoxypyridin-3-yl)-5-(pyridin-2-yl)-1H-pyrazole-3-carbonyl]-2-[1-(N-tert-butoxycarbonylamino)cyclopropyl]piperidine

1-[1-(6-Methoxypyridin-3-yl)-5-(pyridin-2-yl)-1H-pyrazole-3-carbonyl]-2-[1-(N-tert-butoxycarbonylamino)cyclopropyl]piperidine (308 mg, 96%) was obtained as an amorphous material by the same method as that in Example 5, using 1-(6-methoxypyridin-3-yl)-5-(pyridin-2-yl)-1H-pyrazole-3-carboxylic acid (184 mg) of Reference Example 3 and 2-[1-(N-tert-butoxycarbonylamino)cyclopropyl]piperidine (149 mg) of Reference Example 75.
ESI-MSm/z: 519(M+H)⁺.

### 2) Title compound

To a solution of 1-[1-(6-methoxypyridin-3-yl)-5-(pyridin-2-yl)-1H-pyrazole-3-carbonyl]-2-[1-(N-tert-butoxycarbonylamino)cyclopropyl]piperidine (307 mg) of the above in dichloromethane (20 mL), trifluoroacetic acid (7 mL) was added at room temperature, and the resultant mixture was stirred for 85 minutes. The solvent of the reaction solution was evaporated under reduced pressure, a saturated aqueous solution of sodium hydrogen carbonate and chloroform-methanol (10: 1) were added to a residue thus obtained, and the mixture was partitioned. The organic layer was dried over anhydrous sodium sulfate. After separation by filtration, the solvent was evaporated under reduced pressure, and the title compound (105 mg, 43%) was obtained.
¹H-NMR(400MHz, CDCl₃)δ: 0.85-1.82(11H, m), 2.32-2.35(1H, m), 2.58-2.64(1H, m), 3.10(1H, d, J=11.5), 3.96(3H, s), 6.76(1H, dd, J=8.8, 0.7Hz), 7.20-7.23(2H, m), 7.31(1H, s), 7.42(1H, d, J=7.8Hz), 7.61(1H, dd, J=8.8, 2.7Hz), 7.70(1H, td, J=7.8, 1.9Hz), 8.10(1H, d, J=2.2Hz), 8.48(1H, d, J=4.9Hz).
ESI-MSm/z: 419(M+H)⁺.

[Example 126] 1-[1-(6-Methoxy-3-pyridyl)-5-(thiazol-4-yl)-1H-pyrazole-3-carbonyl]-4-methyl-3-oxopiperazine

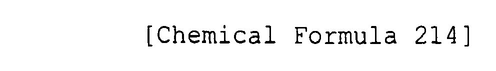

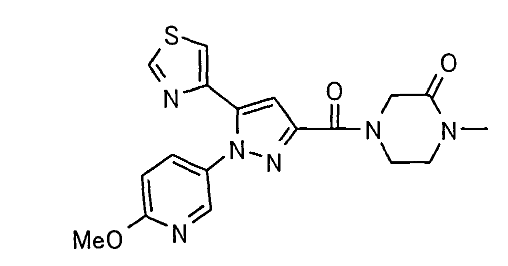

The title compound (0.239 g, 73%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and 1-methylpiperazin-2-one hydrochloride (0.249 g) of Reference Example 21.
¹H-NMR(400MHz, CDCl₃)δ: 3.02(3H, s), 3. 46-3. 49 (2H, m), 3.99(3H, s), 4.04(1H, m), 4.43(2H, m), 4.82(1H, m), 6.82(1H, d, J=8.8Hz), 7.22-7.30(2H, m), 7.59-7.67(1H, m), 8.16-8.19(1H, m), 8.79(1H, d, J=2.0Hz).
EI-MSm/z: 398(M⁺).

[Example 127] 1-[1-(6-Methoxy-3-pyridyl)-5-(thiazol-4-yl)-1H-pyrazole-3-carbonyl]-4-methylpiperazine

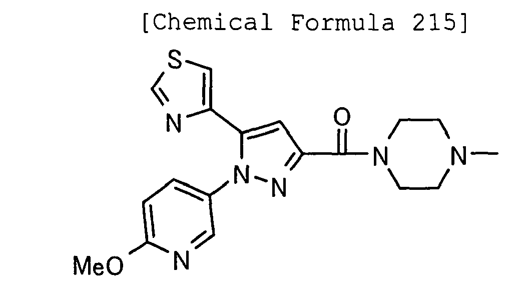

The title compound (0.232 g, 73%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and N-methylpiperazine (0.183 mL).
¹H-NMR(400MHz, CDCl₃)δ: 2.38(3H, s), 2.53-2.58(4H, m), 3.89(2H, m), 3.97(3H, s), 4.14(2H, m), 6.80(1H, dd, J=8.8, 0.5Hz), 7.14(1H, s), 7.25(1H, d, J=2.0Hz), 7.62(1H, dd, J=8.8, 2.9Hz), 8.17-8.18(1H, m), 8.79(1H, d, J=2.0Hz).
EI-MSm/z: 384(M⁺).

[Example 128] 1-[1-(6-Methoxy-3-pyridyl)-5-(thiazol-4-yl)-1H-pyrazole-3-carbonyl]-4-methoxypiperidine

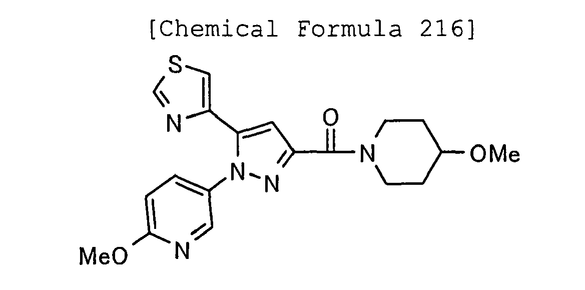

The title compound (0.295 g, 89%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and 4-methoxypiperidine hydrochloride (0.188 g) of Reference Example 23.
¹H-NMR(400MHz, CDCl₃)δ: 1.68-1.94(4H, m), 3.38(3H, s), 3.47-3.55(2H, m), 3.70-3.75(1H, m), 3.98(3H, s), 4.09(1H, m), 4.27(1H, m), 6.79-6.81(1H, m), 7.11(1H, s), 7.25-7.26(1H, m), 7.63(1H, dd, J=8.9, 2.8Hz), 8.17-8.18(1H, m), 8.79(1H, d, J=2.0Hz).
EI-MSm/z: 399(M⁺).

[Example 129] 1-[1-(6-Methoxy-3-pyridyl)-5-(thiazol-4-yl)-1H-pyrazole-3-carbonyl]-2-carbamoylpiperidine

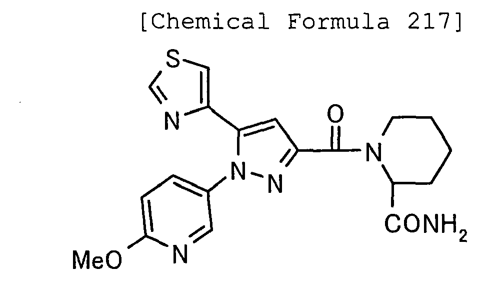

The title compound (0.255 g, 75%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and piperidine-2-carboxylic acid amide (0.138 g) of Reference Example 71.
¹H-NMR(400MHz, CDCl₃)δ: 1.53-1.83(5H, m), 2.30-2.40(1H, m), 2.80-3.21(1H, m), 3.98(3H, s), 4.71-4.79(1H, m), 5.36-5.49(2H, m), 6.38-7.08(1H, m), 6.79-6.83(1H, m), 7.15-7.30(2H, m), 7.56-7.65(1H, m), 8.13-8.19(1H, m), 8.80(1H, s) .
EI-MSm/z: 412(M⁺).

[Example 130] 4-[1-(6-Methoxy-3-pyridazinyl)-5-(thiazol-4-yl)-1H-pyrazole-3-carbonyl]morpholine

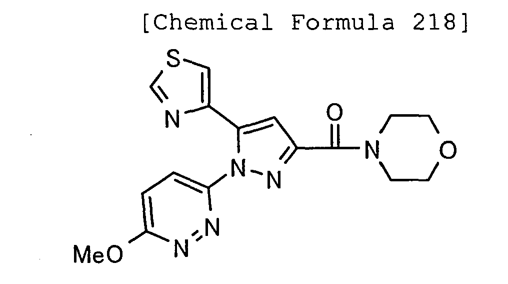

The title compound (0.231 g, 74%) was obtained as a solid by the same method as that in Example 5, using 1-(6-methoxy-3-pyridazinyl)-5-(thiazol-4-yl)-1H-pyrazole-3-carboxylic acid (0.250 g) of Reference Example 73 and morpholine (86 µL).
¹H-NMR(400MHz, CDCl₃)δ: 3.73-3.83(6H, m), 4.09-4.12(2H, m), 4.15(3H, s), 7.14-7.16(2H, m), 7.66(1H, m), 7.76(1H, d, J=9.0Hz), 8.73(1H, d, J=2.0Hz).
EI-MSm/z: 372(M⁺).

[Example 131] 1-[1-(6-Methoxy-3-pyridazinyl)-5-(thiazol-4-yl)-1H-pyrazole-3-carbonyl]-4-methylpiperazine

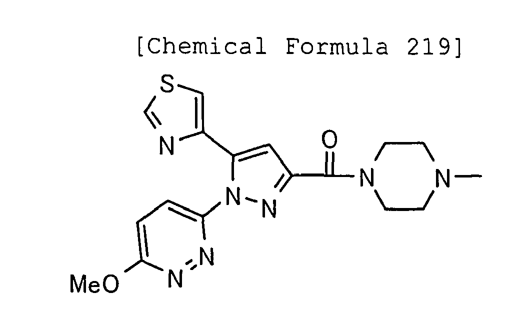

The title compound (0.225 g, 71%) was obtained as a solid by the same method as that in Example 5, using 1-(6-methoxy-3-pyridazinyl)-5-(thiazol-4-yl)-1H-pyrazole-3-carboxylic acid (0.250 g) of Reference Example 73 and N-methylpiperazine (0.110 mL).
¹H-NMR(400MHz, CDCl₃)δ: 2.35(3H, s), 2.46-2.54(4H, m), 3.87(2H, m), 4.06(2H, m), 4.14(3H, s), 7.12(1H, s), 7.15(1H, d, J=9.3Hz), 7.66(1H, d, J=2.0Hz), 7.79(1H, d, J=9.3Hz), 8.73(1H, m).
EI-MSm/z: 385(M⁺).

[Example 132] 1-[1-(6-Methoxy-3-pyridazinyl)-5-(thiazol-4-yl)-1H-pyrazole-3-carbonyl]-4-methoxypiperidine

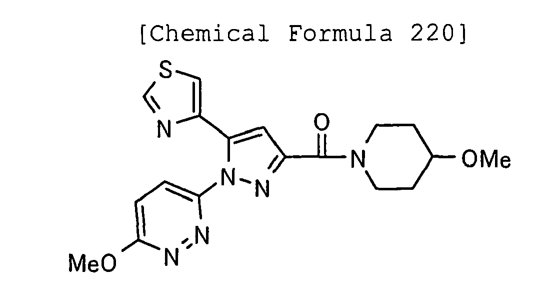

The title compound (0.276 g, 83%) was obtained as a solid by the same method as that in Example 5, using 1-(6-methoxy-3-pyridazinyl)-5-(thiazol-4-yl)-1H-pyrazole-3-carboxylic acid (0.250 g) of Reference Example 73 and 4-methoxypiperidine hydrochloride (0.188 g) of Reference Example 23.
¹H-NMR(400MHz, CDCl₃)δ: 1.67-1.95(4H, m), 3.38(3H, s), 3.48-3.58(2H, m), 3.68-3.72(1H, m), 4.07-4.11(1H, m), 4.14(3H, s), 4.22(1H, m), 7.09(1H, s), 7.14(1H, d, J=9.3Hz), 7.66(1H, d, J=2.0Hz), 7.81(1H, d, J=9.3Hz), 8.73(1H, d, J=2.0Hz).
EI-MSm/z: 400(M⁺).

[Example 133] 1-[1-(6-Methoxy-3-pyridazinyl)-5-(thiazol-4-yl)-1H-pyrazole-3-carbonyl]-4, 4-difluoropiperidine

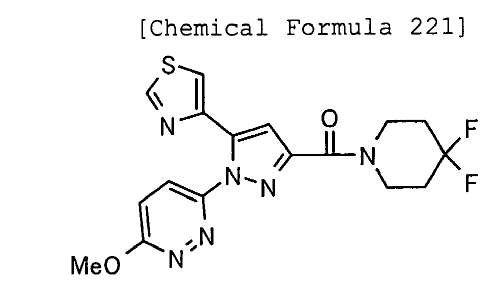

The title compound (0.266 g, 79%) was obtained as a solid by the same method as that in Example 5, using 1-(6-methoxy-3-pyridazinyl)-5-(thiazol-4-yl)-1H-pyrazole-3-carboxylic acid (0.250 g) of Reference Example 73 and 4, 4-difluoropiperidine hydrochloride (0.156 g) of Reference Example 18.
¹H-NMR(400MHz, CDCl₃)δ: 2.08(4H, m), 3.93(2H, m), 4.15(3H, s), 4.15(2H, m), 7.15-7.17(2H, m), 7.66-7.67(1H, m), 7.76(1H, d, J=9.3Hz), 8.73(1H, d, J=2.0Hz).
EI-MSm/z: 406(M⁺).

[Example 134] 1-[1-(6-Methoxy-3-pyridyl)-5-(1-methyl-1H-pyrazol-4-yl)-1H-pyrazole-3-carbonyl]hexahydropyridazine

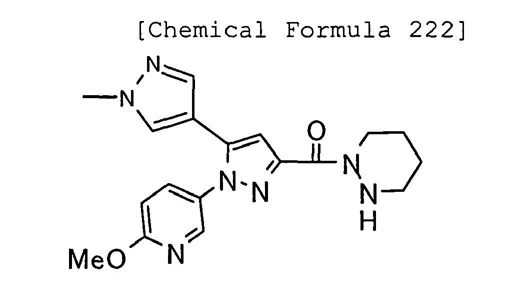

The title compound (0.202 g, 66%) was obtained as a solid by the same method as that in Example 5, using 1-(6-methoxy-3-pyridyl)-5-(1-methyl-1H-pyrazol-4-yl)-1H-pyrazole-3-carboxylic acid (0.250 g) of Reference Example 61 and hexahydropyridazine hydrochloride (0.159 g) of Reference Example 20.
¹H-NMR(400MHz, CDCl₃)δ: 1.68-1.83(4H, m), 3.03-3.05(2H, m), 3.87(3H, s), 3.87-4.25(2H, m), 3.99(3H, s), 6.80-6.82(1H, m), 6.88-6.95(1H, m), 7.21-7.28(2H, m), 7.58-7.60(1H, m), 8.22-8.23(1H, m).
EI-MSm/z: 367(M⁺).

[Example 135] 1-[1-(6-Methoxy-3-pyridazinyl)-5-(thiazol-4-yl)-1H-pyrazole-3-carbonyl]hexahydropyridazine

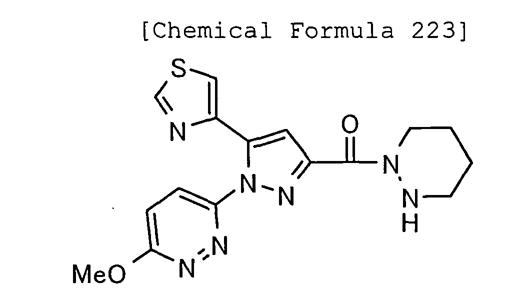

The title compound (0.208 g, 68%) was obtained as a solid by the same method as that in Example 5, using 1-(6-methoxy-3-pyridazinyl)-5-(thiazol-4-yl)-1H-pyrazole-3-carboxylic acid (0.250 g) of Reference Example 73 and hexahydropyridazine hydrochloride (0.157 g) of Reference Example 20.
¹H-NMR(400MHz, CDCl₃)δ: 1.70-1.86(4H, m), 3.03-3.08(2H, m), 3.88-4.22(2H, m), 4.15(3H, s), 7.14-7.28(2H, m), 7.67(1H, m), 7.78-7.93(1H, m), 8.73(1H, m).
EI-MSm/z: 371(M⁺).

[Example 136] 4-[1-(6-Methoxy-3-pyridyl)-5-(1-methyl-1H-pyrazol-4-yl)-1H-pyrazole-3-carbonyl]-1, 4-oxazepane

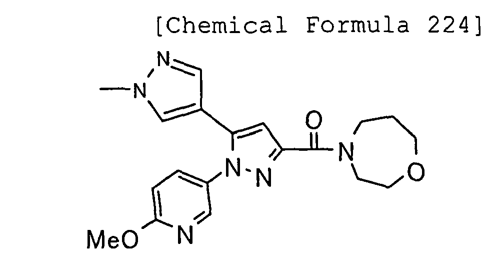

The title compound (0.267 g, 84%) was obtained as a solid by the same method as that in Example 5, using 1-(6-methoxy-3-pyridyl)-5-(1-methyl-1H-pyrazol-4-yl)-1H-pyrazole-3-carboxylic acid (0.250 g) of Reference Example 61 and 1, 4-oxazepane hydrochloride (0.138 g) of Reference Example 74.
¹H-NMR(400MHz, CDCl₃)δ: 2.00-2.09(2H, m), 3.78-3.92(6H, m), 3.88(3H, s), 3.99(3H, s), 4.06-4.13(2H, m), 6.81(1H, d, J=8.8Hz), 6.86-6.87(1H, m), 7.21-7.22(1H, m), 7.28-7.29(1H, m), 7.55-7.59(1H, m), 8.20-8.23(1H, m).
EI-MSm/z: 382(M⁺).

[Example 137] 4-[1-(6-Methoxy-3-pyridazinyl)-5-(thiazol-4-yl)-1H-pyrazole-3-carbonyl]-1, 4-oxazepane

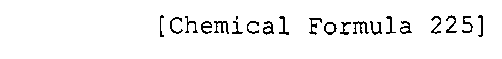

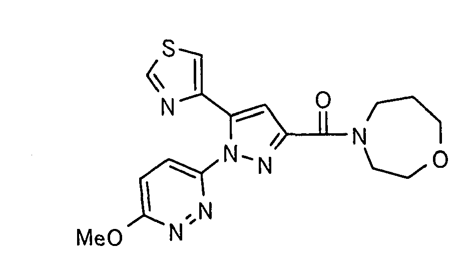

The title compound (0.264 g, 82%) was obtained as a solid, using 1-(6-methoxy-3-pyridazinyl)-5-(thiazol-4-yl)-1H-pyrazole-3-carboxylic acid (0.250 g) of Reference Example 73 and 1, 4-oxazepane hydrochloride (0.136 g) of Reference Example 74.
¹H-NMR(400MHz, CDCl₃)δ: 2.01-2.11(2H, m), 3.80-3.90(6H, m), 4.04-4.09(2H, m), 4.14(3H, s), 7.13-7.16(2H, m), 7.66-7.67(1H, m), 7.74-7.79(1H, m), 8.72-8.74(1H, m).
EI-MSm/z: 386(M⁺).

[Example 138] 1-[1-(6-Methoxy-3-pyridyl)-5-(thiazol-4-yl)-1H-pyrazole-3-carbonyl]hexahydropyridazine

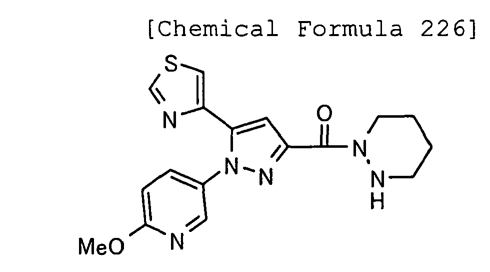

The title compound (0.202 g, 66%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and hexahydropyridazine hydrochloride (0.158 g) of Reference Example 20.
¹H-NMR(400MHz, CDCl₃)δ: 1.70-1.83(4H, m), 3.04-3.05(2H, m), 3.93-4.24(2H, m), 3.97(3H, s), 6.80(1H, d, J=8.8Hz), 7.16-7.29(2H, m), 7.61-7.64(1H, m), 8.18-8.19(1H, m), 8.79(1H, m).
ESI-MSm/z: 371(M+H)⁺.

[Example 139] 4-[1-(6-Methoxy-3-pyridyl)-5-(thiazol-4-yl)-1H-pyrazole-3-carbonyl]-1, 4-oxazepane

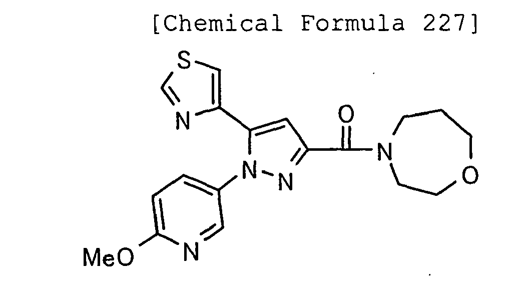

The title compound (0.272 g, 85%) was obtained as a solid by the same method as that in Example 5, using 1-(6-methoxy-3-pyridyl)-5-(thiazol-4-yl)-1H-pyrazole-3-carboxylic acid (0.250 g) of Reference Example 64 and 1, 4-oxazepane hydrochloride (0.137 g) of Reference Example 74.
¹H-NMR(400MHz, CDCl₃)δ: 2.02-2.10(2H, m), 3.79-3.89(6H, m), 3.97(3H, s), 4.06-4.12(2H, m), 6.79(1H, d, J=8.8Hz), 7.16-7.17(1H, m), 7.27-7.28(1H, m), 7.58-7.62(1H, m), 8.15-8.18(1H, m), 8.78-8.79(1H, m).
ESI-MSm/z: 386(M+H)⁺.

[Example 140] 1-[5-(5-Carbamoylmethoxy-2-pyridyl)-1-(6-methoxy-3-pyridyl)-1H-pyrazole-3-carbonyl)-4-methylpiperazine

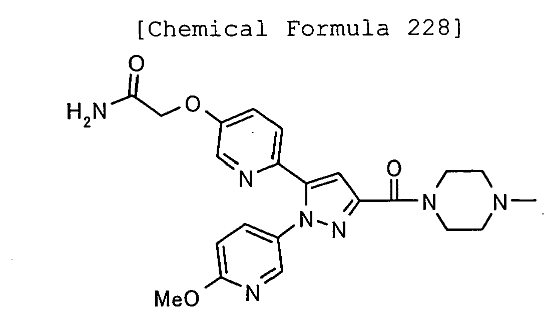

### 1) 1-[5-(5-Benzyloxy-2-pyridyl)-1-(6-methoxy-3-pyridyl)-1H-pyrazole-3-carbonyl]-4-methylpiperazine

1-[5-(5-Benzyloxy-2-pyridyl)-1-(6-methoxy-3-pyridyl)-1H-pyrazole-3-carbonyl]-4-methylpiperazine (0.995 g, 82%) was obtained as a solid by the same method as that in Example 5, using 5-(5-benzyloxy-2-pyridyl)-1-(6-methoxy-3-pyridyl)-1H-pyrazole-3-carboxylic acid (1.0 g) of Reference Example 76 and N-methylpiperazine (0.280 g).
¹H-NMR(400MHz, CDCl₃)δ: 2.34(3H, s), 2.42-2.52(4H, m), 3.80-3.90(2H, m), 3.95(3H, s), 4.07-4.13(2H, m), 5.10(2H, s), 6.75(1H, d, J=8.8Hz), 7.03(1H, s), 7.23-7.41(7H, m), 7.58(1H, dd, J=8.8, 2.7Hz), 8.11(1H, d, J=2.7Hz), 8.27(1H, d, J=2.7Hz).
EI-MSm/z: 484(M⁺).

### 2) 1-[5-(5-Hydroxy-2-pyridyl)-1-(6-methoxy-3-pyridyl)-1H-pyrazole-3-carbonyl]-4-methylpiperazine

To a solution of 1-[5-(5-benzyloxy-2-pyridyl)-1-(6-methoxy-3-pyridyl)-1H-pyrazole-3-carbonyl]-4-methylpiperazine (0.990 g) of the above in methanol (50 mL), 10% palladium-carbon (50% wet, 1.0 g) was added, and the resultant mixture was stirred for 2 hours at room temperature in the presence of hydrogen. After separating the catalyst by filtration, the solvent was evaporated under reduced pressure, and 1-[5-(5-hydroxy-2-pyridyl)-1-(6-methoxy-3-pyridyl)-1H-pyrazole-3-carbonyl]-4-methylpiperazine (895 mg, quantitative) was obtained as a solid.
¹H-NMR(400MHz, CDCl₃)δ: 2.55(3H, s), 2.75-2.90(4H, m), 3.93(3H, s), 3.93-4.05(2H, m), 4.25-4.40(2H, m), 6.72(1H, d, J=8.8Hz), 6.98(1H, s), 7.13-7.18(2H, m), 7.51(1H, dd, J=6.1, 2.7Hz), 8.07(1H, d, J=2.7Hz), 8.11(1H, d, J=2.7Hz).
EI-MSm/z: 394(M⁺).

### 3) Title compound

To a solution of the above hydroxyl product in N, N-dimethylformamide (10 mL), potassium carbonate (410 mg) and bromoacetamide (250 mg) were added at room temperature, and the resultant mixture was stirred for 20 hours. Water and chloroform were added to the reaction solution, and the mixture was partitioned. The organic layer was dried over anhydrous magnesium sulfate. After separation by filtration, a residue obtained by evaporating the solvent under reduced pressure was purified by silica gel thin layer chromatography (dichloromethane-methanol), to obtain the title compound (150 mg, 23%) as a solid.
¹H-NMR(400MHz, CDCl₃)δ: 2.37(3H, s), 2.45-2. 60 (4H, m), 3.80-3.95(2H, m), 3.95(3H, s), 4.10-4.20(2H, m), 5.74(1H, br s), 6.48 (1H, br s), 6.76 (1H, dd, J=8.8, 0.7Hz), 7.07 (1H, s), 7.23(1H, dd, J=8.8, 3.2Hz), 7.39(1H, dd, J=9.3, 0.5Hz), 7.59(1H, dd, J=8.8, 2.7Hz), 8.09(1H, dd, J=2.7, 0.7Hz), 8.25(1H, d, J=2.4Hz).
EI-MSm/z: 451(M⁺).

[Example 141] 1-[1-(6-Methoxy-3-pyridazinyl)-5-(1-methyl-1H-pyrazol-4-yl)-1H-pyrazole-3-carbonyl]-4, 4-difluoropiperidine

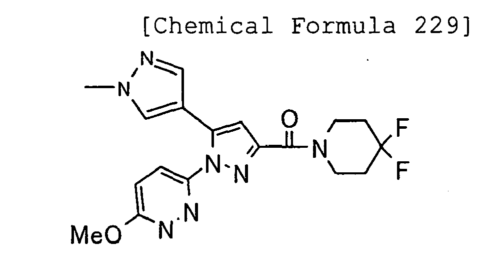

The title compound (0.227 g, 68%) was obtained as a solid by the same method as that in Example 5, using 1-(6-methoxy-3-pyridazinyl)-5-(1-methyl-1H-pyrazol-4-yl)-1H-pyrazole-3-carboxylic acid (0.250 g) of Reference Example 77 and 4, 4-difluoropiperidine hydrochloride (0.157 g) of Reference Example 18.
¹H-NMR(400MHz, CDCl₃)δ: 2.04-2.10 (4H, m), 3.92(3H, s), 3.92(2H, m), 4.11-4.14(2H, m), 4.20(3H, s), 6.88(1H, s), 7.15(1H, d, J=9.3Hz), 7.57(1H, s), 7.68-7.70(1H, m), 7.76(1H, s).
EI-MSm/z: 403(M⁺).

### [Test Example 1] Platelet aggregation suppressing effect

Human blood was collected using 3.13% sodium citrate in a volume 1/10 of the blood volume, as an anticoagulant. The collected blood was centrifuged at 180g for 10 minutes to separate platelet rich plasma (PRP) from the blood. After collecting the PRP fraction in the upper layer, the lower layer was further centrifuged at 1600g for 10 minutes to fractionate platelet poor plasma (PPP) in the upper layer. One µl of a solution of a compound obtained in the Examples was added to 200 µl of PRP, and the mixture was allowed to stand at 37°C for 2 minutes. Subsequently, 2 µl of collagen was added thereto, so as to induce platelet aggregation. The platelet aggregation rate was measured using PAM-12C (SSR Engineering, Inc.). By taking the light transmittance of PPP as the value indicating 100% aggregation, the aggregation rates at various concentrations of the compound of the Examples were determined, and the IC₅₀ value was calculated. The results are presented in Table 1.

### [Test Example 2] Verification of inhibitory action on cyclooxygenase-1 (COX-1) and cyclooxygenase-2 (COX-2)

For the measurement of the inhibitory activity of the compounds obtained in the Examples against COX-1 and COX-2, a COX Inhibitor Screening Assay Kit manufactured by Cayman Chemical Company (Catalog Nos. 560101 and 560121) was used.
Before starting the measurement, a reaction buffer, heme, arachidonic acid, SnCl₂, EIA buffer, a washing buffer, prostaglandin (PG)-screening EIA standard solution, PG-screening acetylcholinesterase (AchE), a tracer (chromogenic enzyme HRP conjugate), and PG-screening EIA antiserum were provided.

### (1) Production of PGF₂α by COX-1

A reaction solution containing a compound of the Examples (50 µM) and COX-1, or a reaction solution containing a compound of the Examples (300 µM) and COX-2 was allowed to stand at 37°C for 10 minutes, then 10 µl of arachidonic acid was added thereto, and the resultant mixture was allowed to stand at 37°C for 2 minutes. After the reaction, 50 µl of 1 N hydrochloric acid was added to the reaction mixture to stop the reaction, and 100 µl of a SnCl₂ solution was added thereto. The resultant mixture was allowed to stand at room temperature for 5 minutes.

### (2) Quantification of PGF₂α by ELISA

50 µl of antiserum (rabbit anti-PGF₂α antibody) was added to each well of a 96-well plate coated with mouse anti-rabbit IgG. Then, 50 µl of a solution prepared by diluting the reaction solution for PGF₂α production to 2000-folds, and 50 µl of an AchE tracer were added sequentially to the wells, and the plate was kept at room temperature for 18 hours. Each well was washed 5 times with the washing buffer to remove excessive AchE tracer, and then 200 µl of Ellman reagent was added to each well. The plate was kept in the dark room for 60 minutes, and then, absorbance at 405 nm was measured.

### (3) Calculation of inhibitory activity of the compound of Examples

A standard curve was produced using a PG-screening EIA standard solution, and the amount of PGF₂α production was determined from the absorbance obtained as described above. The COX-1 inhibition rate at 50 µM of the compound of the Examples, and the COX-2 inhibition rate at 300 µM of the compound of the Examples were calculated. The results are presented in Table 1.
Additionally, for the calculation of inhibition rate, the amount of PGF₂α production obtained using a reaction solution which does not contain the compound of the Examples was regarded as 100%.

**[Table 1]**

| Example | Inhibitory effect of collagen-induced platelet aggregation IC₅₀ (µM) | COX-1 inhibitory action at 50 µM (% inhibition) | COX-2 inhibitory action at 300 µM (% inhibition) |
|---|---|---|---|
| 1 | 0.21 | -13.3 | NT |
| 2 | 0.13 | -1.2 | NT |
| 3 | 0.22 | -21.2 | NT |
| 4 | 0.21 | -13.3 | NT |
| 5 | 0.21 | -35.9 | NT |
| 10 | 0.19 | 54.1 | NT |
| 12 | 0.32 | 25.9 | NT |
| 14 | 0.12 | 39.3 | NT |
| 16 | 0.12 | -5.2 | NT |
| 18 | 0.20 | -3.0 | NT |
| 20 | 0.12 | -17.0 | NT |
| 27 | 0.091 | -10.2 | NT |
| 31 | 0.18 | -10.5 | NT |
| 32 | 0.21 | -8.4 | NT |
| 33 | 0.097 | -9.5 | NT |
| 34 | 0.063 | -18.4 | NT |
| 38 | 0.37 | 1.2 | NT |
| 41 | 0.17 | -21.3 | NT |
| 73 | 0.15 | 5.9 | 15.5 |
| 105 | 0.94 | -9.6 | NT |
| 107 | 0.43 | -3.9 | NT |
| 115 | 0.11 | -21.2 | 23.4 |
| 118 | 0.079 | -12.7 | NT |
| 119 | 0.16 | 13.0 | NT |
| 121 | 0.71 | -0.7 | NT |
| 124 | 0.44 | 8.3 | NT |
| 126 | 0.36 | -6.4 | NT |
| 127 | 0.23 | -21.2 | NT |
| 128 | 0.10 | NT | NT |
| 129 | 0.26 | NT | NT |
| 131 | 0.32 | NT | NT |
| 132 | 0.19 | NT . | NT |
| 133 | 0.08 | NT | NT |
| 134 | 0.04 | NT | NT |
| 136 | 0.36 | NT | NT |
| 137 | 0.08 | NT | NT |
| 138 | 0.041 | NT | NT |
| 139 | 0.041 | NT | NT |
| 141 | 0.19 | 29.4 | NT |

| | | | |
|---|---|---|---|
| NT: Not Tested | | | |

As is clear from Table 1, the compound (I) of the invention, a salt thereof, or a solvate of the compound or the salt has a potent platelet aggregation suppressing effect, while not exhibiting COX-1 and COX-2 inhibitory effects.

## Claims

1. A compound represented by Formula (I): 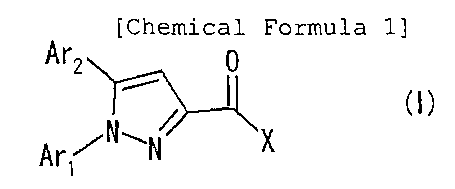 wherein Ar₁ represents a group represented by Formula (II) : 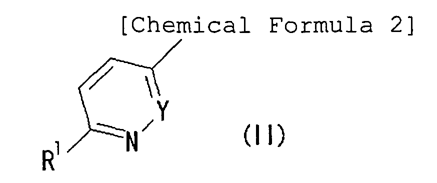 (wherein Y represents CH or a nitrogen atom; and R¹ represents a lower alkyl group or a lower alkoxy group);
Ar₂ represents a 5- or 6-membered aromatic heterocyclic group which may be substituted with 1 to 3 groups selected from a lower alkyl group which may be substituted, a lower alkynyl group, a carbamoyl group which may be substituted, a cyano group, an amino group which may be substituted, a lower alkoxy group which may be substituted, and a lower alkanoyl group; and
X represents a group represented by Formula (III): 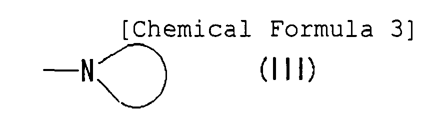 (wherein the cyclic structure represents a 4- to 7-membered alicyclic heterocyclic group which may contain a nitrogen atom or an oxygen atom as the constituent atom in addition to the nitrogen atom described in the formula, and which may be substituted with 1 to 4 groups or atoms selected from a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group, and a halogen atom), and
a salt thereof, or a solvate of the compound or the salt.

2. The compound according to claim 1, a salt thereof, or a solvate of the compound or the salt, wherein Ar₂ is a 5-membered nitrogen-containing aromatic heterocyclic group which may be substituted with 1 to 2 groups selected from a lower alkyl group which may be substituted, a lower alkynyl group, a carbamoyl group which may be substituted, a cyano group, an amino group which may be substituted, a lower alkoxy group which may be substituted, and a lower alkanoyl group.

3. The compound according to claim 2, a salt thereof, a solvate of the compound or the salt, wherein the 5-membered nitrogen-containing aromatic heterocyclic group is a pyrrolyl group, an imidazolyl group, a pyrazolyl group, a thiazolyl group, an oxazolyl group or a triazolyl group.

4. The compound according to claim 2, a salt thereof, a solvate of the compound or the salt, wherein the 5-membered nitrogen-containing aromatic heterocyclic group is a 1H-pyrrol-1-yl group, a 1H-pyrrol-2-yl group, a 1H-pyrrol-3-yl group, a 1H-imidazol-2-yl group, a 1H-imidazol-4-yl group, a 1H-pyrazol-3-yl group, a 1H-pyrazol-4-yl group, a thiazol-2-yl group, a thiazol-4-yl group, a thiazol-5-yl group, an oxazol-2-yl group, an oxazol-4-yl group, or a 1H-1, 2, 4-triazol-3-yl group.

5. The compound according to claim 1, a salt thereof, or a solvate of the compound or the salt, wherein Ar₂ is a 6-membered nitrogen-containing aromatic heterocyclic group which may be substituted with 1 to 2 groups selected from a lower alkyl group which may be substituted, a lower alkynyl group, a carbamoyl group which may be substituted, a cyano group, an amino group which may be substituted, a lower alkoxy group which may be substituted, and a lower alkanoyl group.

6. The compound according to claim 5, a salt thereof, or a solvate of the compound or the salt, wherein the 6-membered nitrogen-containing aromatic heterocyclic group is a pyridyl group, a pyrimidinyl group, or a pyrazinyl group.

7. The compound according to claim 5, a salt thereof, or a solvate of the compound or the salt, wherein the 6-membered nitrogen-containing aromatic heterocyclic group is a 2-pyridyl group, a 4-pyrimidinyl group, or a 2-pyrazinyl group.

8. The compound according to any one of claims 1 to 7, a salt thereof, or a solvate of the compound or the salt, wherein the Formula (III) is a 5-membered alicyclic heterocyclic group which may contain a nitrogen as the constituent atom in addition to the nitrogen atom described in the Formula (III), and which may be substituted with 1 to 4 groups or atoms selected from a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom.

9. The compound according to claim 8, a salt thereof, or a solvate of the compound or the salt, wherein the 5-membered alicyclic heterocyclic group is a pyrrolidinyl group or a pyrazolidinyl group.

10. The compound according to any one of claims 1 to 7, a salt thereof, or a solvate of the compound or the salt, wherein the group represented by Formula (III) is a 6-membered alicyclic heterocyclic group which may contain a nitrogen atom or an oxygen atom as the constituent atom in addition to the nitrogen atom described in the Formula (III), and which may be substituted with 1 to 4 groups or atoms selected from a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom.

11. The compound according to claim 10, wherein the 6-membered alicyclic heterocyclic group is a piperidino group, a piperazino group, a hexahydropyridazin-1-yl group, or a morpholino group.

12. The compound according to any one of claims 1 to 7, a salt thereof, or a solvate of the compound or the salt, wherein X is a group selected from a 2-methylpyrrolidino group, a 2, 5-dimethylpyrrolidino group, a 2-fluoromethylpyrrolidino group, a 2-trifluoromethylpyrrolidino group, a 2-hydroxymethylpyrrolidino group, a 2-methoxymethyl-5-methylpyrrolidino group, a 2-carbamoylpyrrolidino group, a 3-fluoropyrrolidino group, a 3, 3-difluoropyrrolidino group, a 2-methylpyrazolidin-1-yl group, a 2-formylpyrazolidin-1-yl group, a 2-methylsulfonylpyrazolidin-1-yl group, a piperidino group, a 3-methoxypiperidino group, a 3-fluoropiperidino group, a 4-methylpiperidino group, a 4-methoxypiperidino group, a 4-fluoropiperidino group, a 4, 4-difluoropiperidino group, a 2-aminocyclopropylpiperidino group, a 3-oxo-4-methylpiperazino group, a 3-oxo-4-ethylpiperazino group, a 4-methylpiperazino group, a hexahydropyridazin-1-yl group, a morpholino group, a 3-methylmorpholin-4-yl group, and a 1, 4-oxazepan-4-yl group.

13. The compound according to claim 1, wherein X is a morpholino group, a 4-methoxypiperidino group, a 4-fluoropiperidino group, or a 4, 4-difluoropiperidino group; and R¹ is a lower alkyl group.

14. 4-[1-(6-Methoxy-3-pyridyl)-5-(1-methyl-1H-pyrazol-4-yl)-1H-pyrazole-3-carbonyl]morpholine.

15. A pharmaceutical composition comprising the compound according to any one of claims 1 to 14, a salt thereof, or a solvate of the compound or the salt, and a pharmaceutically acceptable carrier.

16. A platelet aggregation suppressant comprising the compound according to any one of claims 1 to 14, a salt thereof, or a solvate of the compound or the salt, and a pharmaceutically acceptable carrier.

17. A prophylactic and/or therapeutic agent for ischemic diseases, comprising the compound according to any one of claims 1 to 14, a salt thereof, or a solvate of the compound or the salt, and a pharmaceutically acceptable carrier.

18. A medicine comprising the compound according to any one of claims 1 to 14, a salt thereof, or a solvate of the compound or the salt, as an active ingredient.

19. A platelet aggregation suppressant comprising the compound according to any one of claims 1 to 14, a salt thereof, or a solvate of the compound or the salt, as an active ingredient.

20. A prophylactic and/or therapeutic agent for ischemic diseases, comprising the compound according to any one of claims 1 to 14, a salt thereof, or a solvate of the compound or the salt, as an active ingredient.

21. A method of preventing and/or treating ischemic diseases, comprising administering an effective amount of the compound according to any one of claims 1 to 14, a salt thereof, or a solvate of the compound or the salt.

22. Use of the compound according to any one of claims 1 to 14, a salt thereof, or a solvate of the compound or the salt, for the production of medicines.
